# EUROPEAN PATENT APPLICATION

(11) **EP 2 457 896 A1**
(43) Date of publication of application: **30.05.2012**
(21) Application number: 11184402.3
(22) Date of filing: 12.07.2005
(51) Int. Cl.: C07D 207/16, C07D 207/22, C07D 207/24, C07D 209/42, C07D 209/44, C07D 211/76, C07D 213/64, C07D 233/10, C07D 263/06, C07D 277/06, C07D 487/04, C07D 487/06, C07D 513/04, C07K 5/06, C07K 5/08, C07K 5/10, C07D 207/273, A61K 38/06, A61P 35/00

(54) **Tripeptides as caspase modulators**

(30) Priority: 12.07.2004 US 587471 P; 23.05.2005 US 683875 P
(62) Divisional of application: 05771672.2
(71) Applicant: Idun Pharmaceuticals, Inc., San Diego, CA 92121 (US)
(72) Inventor: Chao, Bin, San Diego, CA 92130 (US); Deckwerth, Thomas, L., San Diego, CA 92130 (US); Furth, Paul, S., Bonita, CA 91902 (US); Linton, Steven, D., San Diego, CA 92131 (US); Spada, Alfred, P., Carlsbad, CA 92009 (US); Ullman, Brett, R., San Diego, CA 92105 (US); Weinhouse, Michael, I., Escondido, CA 92029 (US)
(74) Representative: Ritter, Thomas Kurt

(57) **Abstract**

Compounds, compositions and methods for treatment of hyperproliferative diseases, such as cancer are provided. 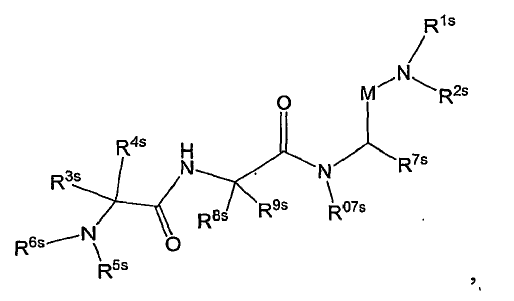 or a pharmaceutically acceptable derivative thereof,
where:
M is CO, SO or SO₂;
R^{1s}and R^{2s} are selected as follows:
(i) R^{1s} is hydrogen, alkyl, alkenyl, alkynyl, aryl, heteroaryl, cycloalkyl or heterocyclyI; and R^{2s} is selected from hydrogen, alkenyl, alkynyl, aryl, heteroaryl, cycloalkyl, heterocyclyl, OR¹¹ and NR¹⁵R¹⁶, or
(ii) R ^{1s}and R^{2s} together with the nitrogen atom on which they are substituted form a heterocyclic or heteroaryl ring;
R^{3s}, R^{4s} and R^{5s} are each independently selected from (i) or (ii) as follows:
(i) R^{3s} is hydrogen or alkyl; R^{4s} is alkyl; and R^{5s} is selected from hydrogen, alkyl, alkenyl, alkynyl, aryl, heteroaryl, heteroarylium, cycloalkyl, heterocyclyl and NR¹⁵R¹⁶;
(ii )R^{5s} and R^{3s} or R^{5s} and R^{4s} together with the atoms on which they are substituted form heterocyclic or heteroaryl ring and the other of R^{3s} or R^{4s} is selected as (ii);
R^{6s} is hydrogen, lower alkyl, lower alkenyl or lower alkynyl;
R^{7s} is hydrogen, alkyl, alkenyl, alkynyl, aryl, heteroaryl, cycloalkyl or heterocyclyl.
The other variables are as defined in the claims.

## Description

### FIELD

Compounds, compositions and methods for treatment of cell proliferative diseases are provided. The compounds provided are peptidomimetics of the N-terminal tetrapeptide of the mitochondrial protein Smac. It is believed that Smac promotes apotosis in cells through a pathway involving the Inhibitor of Apoptosis Proteins (IAPs). These peptidomimetics bind IAPs.

### BACKGROUND

Apoptosis or programmed cell death plays a central role in the development and homeostasis of all multi-cellular organisms. Alterations in apoptotic pathways have been implicated in many types of human pathologies, including developmental disorders, cancer, autoimmune diseases, as well as neurodegenerative disorders.

Thus, the programmed cell death pathways have become attractive targets for development of therapeutic agents. In particular, attention has been focused on anticancer therapies using pro-apoptotic agents such as conventional radiation and chemotherapy. These treatments are generally believed to trigger activation of the mitochondria-mediated apoptotic pathways. However, these therapies lack molecular specificity, and more specific molecular targets are needed.

Apoptosis is executed primarily by activated caspases, a family of cysteine proteases with aspartate specificity in their substrates. Caspases are produced in cells as catalytically inactive zymogens and must be proteolytically processed to become active proteases following a stimulus for apoptosis. In normal surviving cells that have not received an apoptotic stimulus, most caspases remain inactive. Even if some caspases are aberrantly activated, their proteolytic activity can be fully inhibited by a family of evolutionarily conserved proteins called inhibitors of apoptosis proteins or IAPs, see Deveraux & Reed, Genes Dev. 13: 239-252, 1999. Each of the IAPs contains 1-3 copies of the baculoviral IAP repeat, (BIR) domain and directly interacts with and inhibits the enzymatic activity of mature caspases. Several distinct mammalian IAPS including XIAP, survivin, and Livin/ML- LAP (Kasof &Gomes, J. Biol. Chem. 276: 3238-3246, 2001; Vucic et al. Curr. Biol. 10: 1359-1366, 2000; Ashhab et al. FEBS Lett. 495: 56-60,2001), have been identified, and they all exhibit anti-apoptotic activity in cell. As IAPs are expressed in most cancer cells, they may directly contribute to tumor progression and subsequent resistance to drug treatment.

In normal cells signaled to undergo apoptosis, however, the IAP-mediated inhibitory effect must be overcome, a process at least in part performed by a mitochondrial protein named Smac (second mitochondria-derived activator of caspases; Du et al. Cell 102:33-42, 2000) or DIABLO (direct IAP binding protein with low PI; Verhagen et al. Cell 102: 43-53, 2000). Smac, synthesized in the cytoplasm, is targeted to the inter-membrane space of mitochondria. Upon apoptotic stimuli, Smac is released from mitochondria back into the cytosol, together with cytochrome c. Whereas cytochrome c induces multimerization of Apaf-1 to activate procaspase-9 and ultimately caspase -3, Smac eliminates the inhibitory effect of multiple IAPS. Smac interacts with all IAPS that have been examined to date, including XIAP, c-IAP1, c-IAP2, and survivin (Du *et al*., 2000, supra; Verhagen *et al*., 2000, supra). Thus, Smac appears to be a cenral mediator of apoptosis in mammals.

It has been reported that Smac is capable of neutralizing XIAP by binding to a peptide binding pocket on the surface of BIR3. This binding prevents XIAP from exerting its apoptosis-suppressing function in the cell.

The use of peptides for in vivo administration as diagnostic or therapeutic agents is associated with certain disadvantages. These include short half-life due to proteolytic degradation in the body, low absorption through intestinal walls and potential immunogenic reactions, as well as expense involved in peptide synthesis. For these reasons, many current efforts in drug development focus on compounds that mimic the structure and biological activity of bioactive peptides, but possess improved pharmacologic properties and are easier or less expensive to synthesize.

Thus, there is a need for compounds that mimic Smac tetrapeptides. Such mimetics should possess the IAP-binding and apoptosis-promoting bioactivity of the Smac peptides, while also having the improved properties associated with non-peptide mimetics, for use as diagnostic and therapeutic agents in the treatment of cancer.

### Summary

Compounds for use in compositions and methods for promoting apoptosis are provided. In one embodiment, apoptosis is promoted in rapidly dividing cells. In particular, compounds for binding to a peptide binding pocket on the surface of BIR3 are provided. The compounds in the compositions and methods provided herein prevent XIAP from exerting its apoptosis-suppressing function in the cell.

Provided herein are compounds of formula: 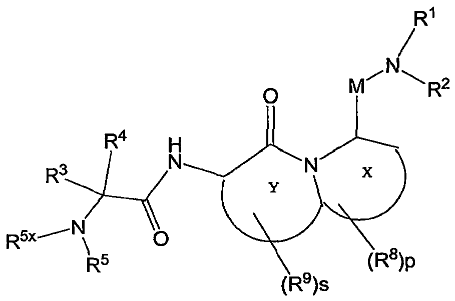 or pharmaceutically acceptable derivatives thereof,
where:
M is CO, SO or SO₂;
X and Y are each independently selected from 4-7 membered heterocyclic or heteroaryl rings containing one or two heteroatoms, in one embodiment one heteroatom, selected from O, S and N;
s and p are each independently 0-3;
R¹ and R² are each independently selected as follows:
   (i) R¹ is hydrogen, alkyl, alkenyl, alkynyl, aryl, heteroaryl, cycloalkyl or heterocyclyl; and R² is selected from hydrogen, alkenyl, alkynyl, aryl, heteroaryl, cycloalkyl, heterocyclyl, OR¹¹, NR¹⁵R¹⁶ and 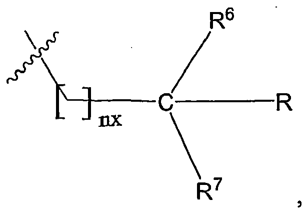 where nx is 0-6; R is hydrogen, alkyl, alkenyl, alkynyl, aryl, heteroaryl, heteroarylium, cycloalkyl, heterocyclyl, C(A)R¹⁰, OR¹¹ or NR¹⁵R¹⁶; and R⁶ and R⁷ are each independently selected from hydrogen, alkyl, alkenyl, alkynyl, aryl, heteroaryl, cycloalkyl and heterocyclyl; or
   (ii) R¹ and R² together with the nitrogen atom on which they are substituted form a heterocyclic or heteroaryl ring;

R³, R⁴ and R⁵ are each independently selected from (i) or (ii) as follows:
(i) R³ and R⁴ are each independently hydrogen, lower alkyl, lower alkenyl, alkynyl, lower cycloalkyl, lower heterocyclyl; and R⁵ is selected from hydrogen, alkyl, alkenyl, alkynyl, aryl, heteroaryl, cycloalkyl, heterocyclyl and NR¹⁵R¹⁶; or
(ii) R⁵ and R³ or R⁵ and R⁴ together with the atoms on which they are substituted form heterocyclic or heteroaryl ring and the other of R³ or R⁴ is selected as (i);

R^{5x} is hydrogen, lower alkyl, lower alkenyl or lower alkynyl;

R⁸ and R⁹ are each independently selected from (i) or (ii) as follows:
(i) R⁸ and R⁹ are each independently selected from lower alkyl, lower alkenyl, lower alkynyl, aryl, heteroaryl, heteroarylium, cycloalkyl, heterocyclyl, C(A)R¹⁰, halo, pseudohalo, OR¹¹, oxo, thio, S(D)nR¹², NR¹⁵R¹⁶, N⁺R¹⁵R¹⁶R¹⁷, =NR²⁵ and =CR¹³R¹⁴;
(ii) R⁸ and R⁹ together with the atoms on which they are substituted form a cycloalkyl, aryl, heterocyclic or heteroaryl ring; or
(iii) two R⁸ substituents together with the carbon atoms on which they are substituted form a cycloalkyl, aryl, heteroaryl or heterocyclyl ring,
with the proviso that when a) Y is a 7 membered heterocyclic ring with 1 heteroatom in the ring, b) X is a 5 membered heterocyclic ring with 1 heteroatom in the ring, c) R⁸ and R⁹ together with the atoms on which they are substituted form a phenyl ring, and d) nx is 0, then R, R⁶, and R⁷ are not heterocyclyl or heteroaryl.

In certain embodiments, the rings X, Y and the groups M, R, R¹- R⁸ and R^{5x} are selected such that the resulting compound binds to a peptide binding pocket on the surface of the BIR3 domain of XIAP. It is believed that such binding modulates activation of caspases, including caspase-9 by preventing XIAP from exerting its apoptosis-suppressing function.

Also provided are compounds of formula: 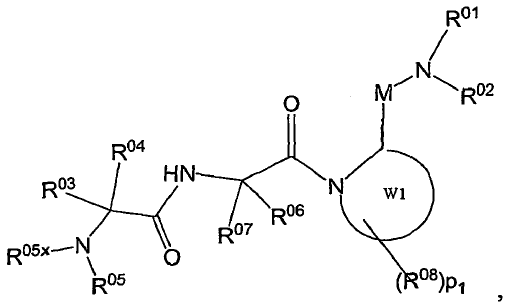 or a pharmaceutically acceptable derivative thereof, where:
W1 is a heterocyclic or heteroaryl ring containing 1 or 2 heteroatoms, in one embodiment one heteroatom, each independently selected from O, N and S;
M is CO, SO or SO₂;
R⁰¹ and R⁰² are each independently selected from (i) or (ii) as follows:
   (i) R⁰¹ is hydrogen, alkyl, alkenyl, alkynyl, aryl, heteroaryl, heteroarylium, cycloalkyl, heterocyclyl, C(A)R¹⁰, OR¹¹ or NR¹⁵R¹⁶; and R⁰² is selected from hydrogen, alkyl, alkenyl, alkynyl, aryl, heteroaryl, heteroarylium, cycloalkyl and heterocyclyl, or
   (ii) R⁰¹ and R⁰² together with the nitrogen atom on which they are substituted form a heterocyclic or heteroaryl ring;
R⁰³, R⁰⁴ and R⁰⁵ are each independently selected from (i) or (ii) as follows:
   (i) R⁰³ and R⁰⁴ are each independently hydrogen, lower alkyl, lower alkenyl, alkynyl, lower cycloalkyl, lower heterocyclyl; and R⁰⁵ is selected from hydrogen, alkyl, alkenyl, alkynyl, aryl, heteroaryl, cycloalkyl, heterocyclyl and NR¹⁵R¹⁶;
   (ii) R⁰⁵ and R⁰³ or R⁰⁵ and R⁰⁴ together with the atoms on which they are substituted form a heterocyclic or heteroaryl ring and the other of R⁰³ or R⁰⁴ is selected as (i);
R^{05x} is hydrogen, lower alkyl, lower alkenyl or lower alkynyl;
R⁰⁶ and R⁰⁷ are each independently hydrogen, alkyl, alkenyl, alkynyl, aryl, heteroaryl, cycloalkyl and heterocyclyl;
p₁ is 0-4;
R⁰⁸ is selected from
   i) alkyl, alkenyl, alkynyl, aryl, heteroaryl, heteroarylium, cycloalkyl, heterocyclyl, C(A)R¹⁰, halo, pseudohalo, OR¹¹, oxo, thio, S(D)nR¹² NR¹⁵R¹⁶, N⁺R¹⁵R¹⁶R¹⁷, =NR²⁵ and =CR¹³R¹⁴ ; or
   ii) two R⁰⁸ substituents together with the atoms on which they are substituted form an aryl, cycloalkyl, heteroaryl or heterocyclyl rings;
   with the proviso that when W1 is a heterocyclic ring with one heteroatom in the ring, p₁ is 0, R⁰¹ is hydrogen, R^{05x} is hydrogen or methyl, and R⁰⁵ is alkyl, optionally substituted with halo, hydroxy, alkoxy, mercapto, alkylthio, thiocyano or pseudohalo; then R⁰² is not hydrogen, alkyl, cycloalkyl, naphthyl or COR¹⁰, where R¹⁰ is alkyl or phenyl.

In certain embodiments, the groups W1, M, R⁰¹- R⁸, R^{05x} and Q¹ are selected such that the resulting compound is binds to a peptide binding pocket on the surface of the BIR3 domain of XIAP. It is believed that such binding prevents XIAP from exerting its apoptosis-suppressing function.

Also provided herein are compounds of formula: 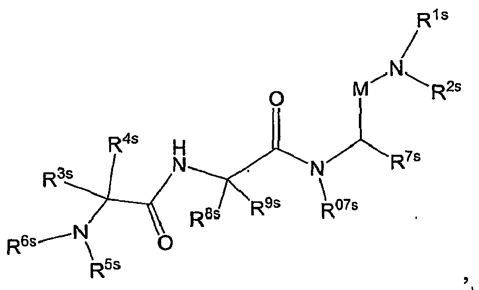 or pharmaceutically acceptable derivatives thereof, where:
M is CO, SO or SO₂;
R^{1s} and R^{2s} are selected as follows:
   (i) R^{1s} is hydrogen, alkyl, alkenyl, alkynyl, aryl, heteroaryl, cycloalkyl or heterocyclyl; and R^{2s} is selected from hydrogen, alkyl, alkenyl, alkynyl, aryl, heteroaryl, cycloalkyl, heterocyclyl, OR¹¹, NR¹⁵R¹⁶ or
   (ii) R^{1s} and R^{2s} together with the nitrogen atom on which they are substituted form a heterocyclic or heteroaryl ring; provided that both R^{1s} and R^{2s} are not hydrogen;
R^{3s}, R^{4s} and R^{5s} are each independently selected from (i) or (ii) as follows:
   (i) R^{3s} and R^{4s} are each independently hydrogen, lower alkyl, lower alkenyl, alkynyl, lower cycloalkyl, lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶;
   (ii) R^{5s} and R^{3s} or R^{5s} and R^{4s} together with the atoms on which they are substituted form heterocyclic or heteroaryl ring and the other of R^{3s} or R^{4s} is selected as (ii);
R^{6s} is hydrogen, lower alkyl, lower alkenyl or lower alkynyl;
R^{7s} is hydrogen, alkyl, alkenyl, alkynyl, aryl, heteroaryl, cycloalkyl or heterocyclyl;
R^{07s}, R^{8s} and R^{9s} are each selected as follows:
   (i) R^{8s} and R^{9s} are each independently hydrogen, alkyl, alkenyl, alkynyl, aryl, heteroaryl, cycloalkyl, heterocyclyl, and R^{07s} is alkyl, alkenyl, alkynyl, aryl or cycloalkyl; or
   (ii)) R^{8s} and R^{07s} or R^{9s} and R^{07s} together with the atoms on which they are substituted form a 5-7 membered heterocyclic or heteroaryl ring containing 1 or 2 heteroatoms in the ring;

In certain embodiments, the groups M, R^{1s}- R^{9s} and R^{07s} are selected such that the resulting compound binds to a peptide binding pocket on the surface of the BIR3 domain of XIAP. It is believed that such binding prevents XIAP from exerting its apoptosis-suppressing function.

Also provided herein are pharmaceutically-acceptable derivatives, including salts, esters, enol ethers, enol esters, solvates, hydrates and prodrugs of the compounds described herein. Pharmaceutically-acceptable salts, include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc, aluminum, and other metal salts, such as but not limited to sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates and fumarates.

Pharmaceutical compositions formulated for administration by an appropriate route and means containing effective concentrations of one or more of the compounds provided herein, or pharmaceutically acceptable derivatives thereof, that deliver amounts effective for the treatment, prevention, or amelioration of one or more symptoms of diseases or disorders that are modulated or otherwise affected by caspase activity, including caspase-9 activity, or in which caspase activity, including caspase-9 activity, is implicated, are also provided. The effective amounts and concentrations are effective for ameliorating any of the symptoms of any of the diseases or disorders.

Methods for treatment, prevention, or amelioration of one or more symptoms of diseases or disorders mediated by or in which casapase activity, including caspase-9 activity, is implicated, are provided. Such methods include methods of treatment, prevention and amelioration of one or more symptoms of hyperproliferative diseases, autoimmune diseases, psoriasis, hyperplasia and restenosis, using one or more of the compounds provided herein, or pharmaceutically acceptable derivatives thereof.

Methods of activating the caspase cascade by releasing caspase-9, using the compounds and compositions provided herein are provided. The compounds and compositions provided herein are active in assays that measure caspase-9 rescue using the assays provided herein. These methods include promotion of apoptosis by abrogating the inhibitory effects of IAPs, including XIAP, on caspase-9.

Methods of modulating IAP-mediated, including XIAP-mediated, inhibitory effect on caspases, including caspase-9, using one or more compounds or compositions provided herein are also provided. The methods are effected by contacting a composition containing the BIR3 domain of XIAP with one or more of the compounds or compositions.

Methods of treating, preventing, or ameliorating one or more symptoms of diseases or disorders which are affected by caspase activity, including caspase-9 activity or by inhibitory effect of IAP are also provided.

In practicing the methods, effective amounts of the compounds or compositions containing therapeutically effective concentrations of the compounds, which are formulated for systemic delivery, including parenteral, oral, or intravenous delivery, or for local or topical application, for the treatment of caspase, including caspase-9, mediated diseases or disorders, are administered to an individual exhibiting the symptoms of these diseases or disorders. The amounts are effective to ameliorate or eliminate one or more symptoms of the diseases or disorders.

Articles of manufacture containing packaging material, a compound or composition, or pharmaceutically acceptable derivative thereof, provided herein, which is effective for modulating the activity of caspases, including caspase-9, or for treatment, prevention or amelioration of one or more symptoms of caspases, including caspase-9, mediated diseases or disorders, or diseases or disorders in which caspase activity, including caspase-9 activity, is implicated, within the packaging material, and a label that indicates that the compound or composition, or pharmaceutically acceptable derivative thereof, is used for modulating the activity caspases, including caspase-9, or for treatment, prevention or amelioration of one or more symptoms of caspases, including caspase-9, mediated diseases or disorders, or diseases or disorders in which caspase activity, including caspase-9 activity, is implicated, are provided.

### DETAILED DESCRTPTION OF EMBODIMENTS

### A. Definitions

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

As used herein the term "caspase" is intended to mean a member of the family of cysteine aspartyl-specific proteases that cleave C-terminal to an aspartic acid residue in a polypeptide and are involved in cell death pathways leading to apoptosis. The term is intended to be consistent with its use in the art as described, for example, in Martin and Green, Cell 82:349-352 (1995) and includes caspase-1, caspase-2 caspase-3 caspase-4 caspase-5 caspase-6, caspase-7, caspase-8, caspase-9, caspase-10 and others.

As used herein the term "IAP" or "inhibitor of apoptosis" is intended to mean a protein that inhibits the proteolytic activity of a caspase. The term can include a protein that when bound to a caspase inhibits the proteolytic activity of the caspase. The term can also include a protein that inhibits the proteolytic activity of a downstream caspase by inhibiting the ability of an upstream caspase to process a precursor of the caspase to a mature form. Also included in the term is a protein that induces ubiquitination and degradation of a caspase.

As used herein, XIAP refers to Human X-linked IAP. XIAP is the best-characterized among the IAPs and is believed to directly inhibit particular caspases via its BIR domains.

As used herein, the term BIR refers to ~70-amino acid baculovirus IAP repeat, which is present in one to three copies as described, for example, in Deveraux et al., Genes and Development 13:239-252 (1999). BIR domains have been shown to exhibit distinct functions. For example, the second BIR domain of XIAP (BIR2) is a potent inhibitor for caspase-3, whereas the third BIR domain of XIAP (BIR3) targets caspase-9 (see Wu et al., Nature 408:1008-1012 (2000)).

As used herein, Smac refers to second mitochondria-derived activator of caspases. As used herein, DIABLO refers to direct IAP binding protein with low PI.

As used herein, a tag refers to a chemical entity that binds to a compound being tested to form a molecule that can be detected by florescence assays, radiometric assay, calorimetric assay or any other assay known in the art, including the fluorescence polarization assay as described herein.

As used herein, pharmaceutically acceptable derivatives of a compound include salts, esters, enol ethers, enol esters, acetals, ketals, orthoesters, hemiacetals, hemiketals, acids, bases, solvates, hydrates or prodrugs thereof. Such derivatives may be readily prepared by those of skill in this art using known methods for such derivatization. The compounds produced may be administered to animals or humans without substantial toxic effects and either are pharmaceutically active or are prodrugs. Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamineand other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and other metal salts, such as but not limited to sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates and fumarates. Pharmaceutically acceptable esters include, but are not limited to, alkyl, alkenyl, alkynyl, aryl, heteroaryl, aralkyl, heteroaralkyl, cycloalkyl and heterocyclyl esters of acidic groups, including, but not limited to, carboxylic acids, phosphoric acids, phosphinic acids, sulfonic acids, sulfmic acids and boronic acids. Pharmaceutically acceptable enol ethers include, but are not limited to, derivatives of formula C=C(OR) where R is hydrogen, alkyl, alkenyl, alkynyl, aryl, heteroaryl, aralkyl, heteroaralkyl, cycloalkyl ar heterocyclyl. Pharmaceutically acceptable enol esters include, but are not limited to, derivatives of formula C=C(OC(O)R) where R is hydrogen, alkyl, alkenyl, alkynyl, aryl, heteroaryl, aralkyl, heteroaralkyl, cycloalkyl ar heterocyclyl. Pharmaceutically acceptable solvates and hydrates are complexes of a compound with one or more solvent or water molecules, or 1 to about 100, or 1 to about 10, or one to about 2, 3 or 4, solvent or water molecules.

As used herein, treatment means any manner in which one or more of the symptoms of a disease or disorder are ameliorated or otherwise beneficially altered. Treatment also encompasses any pharmaceutical use of the compositions herein, such as use for treating a caspase, including caspase-9, mediated diseases or disorders, or diseases or disorders in which caspase activity, including caspase-9 activity, is implicated.

As used herein, amelioration of the symptoms of a particular disorder by administration of a particular compound or pharmaceutical composition refers to any lessening, whether permanent or temporary, lasting or transient that can be attributed to or associated with administration of the composition.

As used herein, the IC₅₀ refers to an amount, concentration or dosage of a particular test compound that achieves a 50% inhibition of a maximal response, such as modulation of caspase activity, in an assay that measures such response.

As used herein, EC₅₀ refers to a dosage, concentration or amount of a particular test compound that elicits a dose-dependent response at 50% of maximal expression of a particular response that is induced, provoked or potentiated by the particular test compound.

As used herein, a prodrug is a compound that, upon *in vivo* administration, is metabolized by one or more steps or processes or otherwise converted to the biologically, pharmaceutically or therapeutically active form of the compound. To produce a prodrug, the pharmaceutically active compound is modified such that the active compound will be regenerated by metabolic processes. The prodrug may be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo,* those of skill in this art, once a pharmaceutically active compound is known, can design prodrugs of the compound (see, e.g., Nogrady (1985) Medicinal Chemistry A Biochemical Approach, Oxford University Press, New York, pages 388-392).

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures. In the case of amino acid residues, such residues may be of either the L- or D-form. The configuration for naturally occurring amino acid residues is generally L. When not specified the residue is the L form. As used herein, the term "amino acid" refers to α-amino acids which are racemic, or of either the D- or L-configuration. The designation "d" preceding an amino acid designation (*e.g*., dAla, dSer, dVal, etc.) refers to the D-isomer of the amino acid. The designation "dl" preceding an amino acid designation (*e.g*., dlPip) refers to a mixture of the L- and D-isomers of the amino acid. It is to be understood that the chiral centers of the compounds provided herein may undergo epimerization *in vivo.* As such, one of skill in the art will recognize that administration of a compound in its (R) form is equivalent, for compounds that undergo epimerization *in vivo,* to administration of the compound in its (S) form.

As used herein, substantially pure means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound. The instant disclosure is meant to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

As used herein, the nomenclature alkyl, alkoxy, carbonyl, *etc*. is used as is generally understood by those of skill in this art.

As used herein, alkyl, alkenyl and alkynyl carbon chains, if not specified, contain from 1 to 20 carbons, or 1 to 16 carbons, and are straight or branched. Alkenyl carbon chains of from 2 to 20 carbons, in certain embodiments, contain 1 to 8 double bonds, and the alkenyl carbon chains of 2 to 16 carbons, in certain embodiments, contain 1 to 5 double bonds. Alkynyl carbon chains of from 2 to 20 carbons, in certain embodiments, contain 1 to 8 triple bonds, and the alkynyl carbon chains of 2 to 16 carbons, in certain embodiments, contain 1 to 5 triple bonds. Exemplary alkyl, alkenyl and alkynyl groups herein include, but are not limited to, methyl, ethyl, propyl, isopropyl, isobutyl, n-butyl, sec-butyl, tert-butyl, isopentyl, neopentyl, tert-penytyl and isohexyl. As used herein, lower alkyl, lower alkenyl, and lower alkynyl refer to carbon chains having from about 1 or about 2 carbons up to about 6 carbons. As used herein, "alk(en)(yn)yl" refers to an alkyl group containing at least one double bond and at least one triple bond.

As used herein, "cycloalkyl" refers to a saturated mono- or multicyclic ring system, in certain embodiments of 3 to 10 carbon atoms, in other embodiments of 3 to 6 carbon atoms; cycloalkenyl and cycloalkynyl refer to mono- or multicyclic ring systems that respectively include at least one double bond and at least one triple bond. Cycloalkenyl and cycloalkynyl groups may, in certain embodiments, contain 3 to 10 carbon atoms, with cycloalkenyl groups, in further embodiments, containing 4 to 7 carbon atoms and cycloalkynyl groups, in further embodiments, containing 8 to 10 carbon atoms. The ring systems of the cycloalkyl, cycloalkenyl and cycloalkynyl groups may be composed of one ring or two or more rings which may be joined together in a fused, bridged or spiro-connected fashion. "Cycloalk(en)(yn)yl" refers to a cycloalkyl group containing at least one double bond and at least one triple bond.

As used herein, "substituted alkyl," "substituted alkenyl," "substituted alkynyl," "substituted cycloalkyl," "substituted cycloalkenyl," and "substituted cycloalkynyl" refer to alkyl, alkenyl, alkynyl, cycloalkyl, cycloalkenyl and cycloalkynyl groups, respectively, that are substituted with one or more substituents, in certain embodiments one to three or four substituents, where the substituents are as defined herein, generally selected from Q¹.

As used herein, "aryl" refers to aromatic monocyclic or multicyclic groups containing from 6 to 19 carbon atoms. Aryl groups include, but are not limited to groups such as fluorenyl, substituted fluorenyl, phenyl, substituted phenyl, naphthyl and substituted naphthyl.

As used herein, "heteroaryl" refers to a monocyclic or multicyclic aromatic ring system, in certain embodiments, of about 5 to about 15 members where one or more, in one embodiment 1 to 3, of the atoms in the ring system is a heteroatom, that is, an element other than carbon, including but not limited to, nitrogen, oxygen or sulfur. The heteroaryl group may be optionally fused to a benzene ring. Heteroaryl groups include, but are not limited to, furyl, imidazolyl, pyrrolidinyl, pyrimidinyl, tetrazolyl, thienyl, pyridyl, pyrrolyl, N-methylpyrrolyl, quinolinyl and isoquinolinyl.

As used herein, a "heteroarylium" group is a heteroaryl group that is positively charged on one or more of the heteroatoms.

As used herein, "heterocyclyl" refers to a monocyclic or multicyclic non-aromatic ring system, in one embodiment of 3 to 10 members, in another embodiment of 4 to 7 members, in a further embodiment of 5 to 6 members, where one or more, in certain embodiments, 1 to 3, of the atoms in the ring system is a heteroatom, that is, an element other than carbon, including but not limited to, nitrogen, oxygen or sulfur. In embodiments where the heteroatom(s) is(are) nitrogen, the nitrogen is optionally substituted with alkyl, alkenyl, alkynyl, aryl, heteroaryl, aralkyl, heteroaralkyl, cycloalkyl, heterocyclyl, cycloalkylalkyl, heterocyclylalkyl, acyl, guanidino, or the nitrogen may be quaternized to form an ammonium group where the substituents are selected as above.

As used herein, "substituted aryl," "substituted heteroaryl" and "substituted heterocyclyl" refer to aryl, heteroaryl and heterocyclyl groups, respectively, that are substituted with one or more substituents, in certain embodiments one to three or four substituents, where the substituents are as defined herein, generally selected from Q¹.

As used herein, "aralkyl" refers to an alkyl group in which one of the hydrogen atoms of the alkyl is replaced by an aryl group.

As used herein, "heteroaralkyl" refers to an alkyl group in which one of the hydrogen atoms of the alkyl is replaced by a heteroaryl group.

As used herein, "halo", "halogen" or "halide" refers to F, Cl, Br or I.

As used herein, pseudohalides or pseudohalo groups are groups that behave substantially similar to halides. Such compounds can be used in the same manner and treated in the same manner as halides. Pseudohalides include, but are not limited to, cyano, thiocyanate, selenocyanate, trifluoromethoxy, and azide.

As used herein, "haloalkyl" refers to an alkyl group in which one or more of the hydrogen atoms are replaced by halogen. Such groups include, but are not limited to, chloromethyl, trifluoromethyl and 1-chloro-2-fluoroethyl.

As used herein, "haloalkoxy" refers to RO- in which R is a haloalkyl group.

As used herein, "sulfinyl" or "thionyl" refers to -S(O)-. As used herein, "sulfonyl" or "sulfuryl" refers to -S(O)₂-. As used herein, "sulfo" refers to -S(O)₂O-.

As used herein, "carboxy" refers to a divalent radical, -C(O)O-.

As used herein, "aminocarbonyl" refers to -C(O)NH₂.

As used herein, "alkylaminocarbonyl" refers to -C(O)NHR in which R is alkyl, including lower alkyl. As used herein, "dialkylaminocarbonyl" refers to -C(O)NR'R in which R and R are independently alkyl, including lower alkyl; "carboxamide" refers to groups of formula -NR'COR in which R' and R are independently alkyl, including lower alkyl.

As used herein, "diarylaminocarbonyl" refers to -C(O)NRR' in which R and R' are independently selected from aryl, including lower aryl, such as phenyl.

As used herein, "arylalkylaminocarbonyl" refers to -C(O)NRR' in which one of R and R' is aryl, including lower aryl, such as phenyl, and the other of R and R' is alkyl, including lower alkyl.

As used herein, "arylaminocarbonyl" refers to -C(O)NHR in which R is aryl, including lower aryl, such as phenyl.

As used herein, "hydroxycarbonyl" refers to -COOH.

As used herein, "alkoxycarbonyl" refers to -C(O)OR in which R is alkyl, including lower alkyl.

As used herein, "aryloxycarbonyl" refers to -C(O)OR in which R is aryl, including lower aryl, such as phenyl.

As used herein, "alkoxy" and "alkylthio" refer to RO- and RS-, in which R is alkyl, including lower alkyl.

As used herein, "aryloxy" and "arylthio" refer to RO- and RS-, in which R is aryl, including lower aryl, such as phenyl.

As used herein, "alkylene" refers to a straight, branched or cyclic, in certain embodiments straight or branched, divalent aliphatic hydrocarbon group, in one embodiment having from 1 to about 20 carbon atoms, in another embodiment having from 1 to 12 carbons. In a further embodiment alkylene includes lower alkylene. There may be optionally inserted along the alkylene group one or more oxygen, sulfur, including S(=O) and S(=O)₂ groups, or substituted or unsubstituted nitrogen atoms, including -NR- and -N⁺RR- groups, where the nitrogen substituent(s) is(are) alkyl, aryl, aralkyl, heteroaryl, heteroaralkyl or COR', where R' is alkyl, aryl, aralkyl, heteroaryl, heteroaralkyl, -OY or -NYY', where Y and Y' are each independently hydrogen, alkyl, aryl, heteroaryl, cycloalkyl or heterocyclyl. Alkylene groups include, but are not limited to, methylene (-CH₂-), ethylene (-CH₂CH₂-), propylene (-(CH₂)₃-), methylenedioxy (-O-CH₂-O-) and ethylenedioxy (-O-(CH₂)₂-O-). The term "lower alkylene" refers to alkylene groups having 1 to 6 carbons. In certain embodiments, alkylene groups are lower alkylene, including alkylene of 1 to 3 carbon atoms.

As used herein, "azaalkylene" refers to -(CRR)ₙ-NR-(CRR)ₘ-, where n and m are each independently an integer from 0 to 4. As used herein, "oxaalkylene" refers to -(CRR)ₙ-O-(CRR)ₘ-, where n and m are each independently an integer from 0 to 4. As used herein, "thiaalkylene" refers to -(CRR)ₙ-S-(CRR)ₘ-, -(CRR)ₙ-S(=O)-(CRR)ₘ-, and -(CRR)ₙ-S(=O)₂-(CRR)ₘ-, where n and m are each independently an integer from 0 to 4. In certain embodiments herein, the "R" groups in the definitions of azaalkylene, oxaalkylene and thiaalkylene are each independently selected from hydrogen and Q¹, as defined herein.

As used herein, "alkenylene" refers to a straight, branched or cyclic, in one embodiment straight or branched, divalent aliphatic hydrocarbon group, in certain embodiments having from 2 to about 20 carbon atoms and at least one double bond, in other embodiments 1 to 12 carbons. In further embodiments, alkenylene groups include lower alkenylene. There may be optionally inserted along the alkenylene group one or more oxygen, sulfur or substituted or unsubstituted nitrogen atoms, where the nitrogen substituent is alkyl. Alkenylene groups include, but are not limited to, -CH=CH-CH=CH- and -CH=CH-CH₂-. The term "lower alkenylene" refers to alkenylene groups having 2 to 6 carbons. In certain embodiments, alkenylene groups are lower alkenylene, including alkenylene of 3 to 4 carbon atoms.

As used herein, "alkynylene" refers to a straight, branched or cyclic, in certain embodiments straight or branched, divalent aliphatic hydrocarbon group, in one embodiment having from 2 to about 20 carbon atoms and at least one triple bond, in another embodiment 1 to 12 carbons. In a further embodiment, alkynylene includes lower alkynylene. There may be optionally inserted along the alkynylene group one or more oxygen, sulfur or substituted or unsubstituted nitrogen atoms, where the nitrogen substituent is alkyl. Alkynylene groups include, but are not limited to, -C≡C-C≡C-, -C≡C- and -C≡C-CH₂-. The term "lower alkynylene" refers to alkynylene groups having 2 to 6 carbons. In certain embodiments, alkynylene groups are lower alkynylene, including alkynylene of 3 to 4 carbon atoms.

As used herein, "alk(en)(yn)ylene" refers to a straight, branched or cyclic, in certain embodiments straight or branched, divalent aliphatic hydrocarbon group, in one embodiment having from 2 to about 20 carbon atoms and at least one triple bond, and at least one double bond; in another embodiment 1 to 12 carbons. In further embodiments, alk(en)(yn)ylene includes lower alk(en)(yn)ylene. There may be optionally inserted along the alkynylene group one or more oxygen, sulfur or substituted or unsubstituted nitrogen atoms, where the nitrogen substituent is alkyl. Alk(en)(yn)ylene groups include, but are not limited to, -C=C-(CH₂)ₙ-C≡C-, where n is 1 or 2. The term "lower alk(en)(yn)ylene" refers to alk(en)(yn)ylene groups having up to 6 carbons. In certain embodiments, alk(en)(yn)ylene groups have about 4 carbon atoms.

As used herein, "cycloalkylene" refers to a divalent saturated mono- or multicyclic ring system, in certain embodiments of 3 to 10 carbon atoms, in other embodiments 3 to 6 carbon atoms; cycloalkenylene and cycloalkynylene refer to divalent mono- or multicyclic ring systems that respectively include at least one double bond and at least one triple bond. Cycloalkenylene and cycloalkynylene groups may, in certain embodiments, contain 3 to 10 carbon atoms, with cycloalkenylene groups in certain embodiments containing 4 to 7 carbon atoms and cycloalkynylene groups in certain embodiments containing 8 to 10 carbon atoms. The ring systems of the cycloalkylene, cycloalkenylene and cycloalkynylene groups may be composed of one ring or two or more rings which may be joined together in a fused, bridged or spiro-connected fashion. "Cycloalk(en)(yn)ylene" refers to a cycloalkylene group containing at least one double bond and at least one triple bond.

As used herein, "substituted alkylene," "substituted alkenylene," "substituted alkynylene," "substituted cycloalkylene," "substituted cycloalkenylene," and "substituted cycloalkynylene" refer to alkylene, alkenylene, alkynylene, cycloalkylene, cycloalkenylene and cycloalkynylene groups, respectively, that are substituted with one or more substituents, in certain embodiments one to three or four substituents, where the substituents are as defined herein, generally selected from Q¹.

As used herein, "arylene" refers to a monocyclic or polycyclic, in certain embodiments monocyclic, divalent aromatic group, in one embodiment having from 5 to about 20 carbon atoms and at least one aromatic ring, in another embodiment 5 to 12 carbons. In further embodiments, arylene includes lower arylene. Arylene groups include, but are not limited to, 1,2-, 1,3- and 1,4-phenylene. The term "lower arylene" refers to arylene groups having 5 or 6 carbons.

As used herein, "heteroarylene" refers to a divalent monocyclic or multicyclic aromatic ring system, in one embodiment of about 5 to about 15 members where one or more, in certain embodiments 1 to 3, of the atoms in the ring system is a heteroatom, that is, an element other than carbon, including but not limited to, nitrogen, oxygen or sulfur.

As used herein, "heterocyclylene" refers to a divalent monocyclic or multicyclic non-aromatic ring system, in certain embodiments of 3 to 10 members, in one embodiment 4 to 7 members, in another embodiment 5 to 6 members, where one or more, including 1 to 3, of the atoms in the ring system is a heteroatom, that is, an element other than carbon, including but not limited to, nitrogen, oxygen or sulfur.

As used herein, "substituted arylene," "substituted heteroarylene" and "substituted heterocyclylene" refer to arylene, heteroarylene and heterocyclylene groups, respectively, that are substituted with one or more substituents, in certain embodiments one to three of four substituents, where the substituents are as defined herein, generally selected from Q¹.

As used herein, "alkylidene" refers to a divalent group, such as =CR'R", which is attached to one atom of another group, forming a double bond. Alkylidene groups include, but are not limited to, methylidene (=CH₂) and ethylidene (=CHCH₃). As used herein, "arylalkylidene" refers to an alkylidene group in which either R' or R" is an aryl group. "Cycloalkylidene" groups are those where R' and R" are linked to form a carbocyclic ring. "Heterocyclylidene" groups are those where at least one of R' and R" contain a heteroatom in the chain, and R' and R" are linked to form a heterocyclic ring.

As used herein, "amido" refers to the divalent group -C(O)NH-. "Thioamido" refers to the divalent group -C(S)NH-. "Oxyamido" refers to the divalent group - OC(O)NH-. "Thiaamido" refers to the divalent group -SC(O)NH-. "Dithiaamido" refers to the divalent group -SC(S)NH-. "Ureido" refers to the divalent group -HNC(O)NH-. "Thioureido" refers to the divalent group -HNC(S)NH-.

As used herein, "semicarbazide" refers to -NHC(O)NHNH-. "Carbazate" refers to the divalent group -OC(O)NHNH-. "Isothiocarbazate" refers to the divalent group - SC(O)NHNH-. "Thiocarbazate" refers to the divalent group -OC(S)NHNH-. "Sulfonylhydrazide" refers to the group -SO₂NHNH-. "Hydrazide" refers to the divalent group -C(O)NHNH-. "Azo" refers to the divalent group -N=N-. "Hydrazinyl" refers to the divalent group -NH-NH-.

Where the number of any given substituent is not specified (*e.g*., "haloalkyl"), there may be one or more substituents present. For example, "haloalkyl" may include one or more of the same or different halogens. As another example, "C₁₋₃alkoxyphenyl" may include one or more of the same or different alkoxy groups containing one, two or three carbons.

As used herein, the following terms have their accepted meaning in the chemical literature:
- AcOH: acetic acid
- CHCl₃: chloroform
- conc: concentrated
- DBU: 1,8-diazabicyclo[5.4.0]undec-7-ene
- DCM: dichloromethane
- DME: 1,2-dimethoxyethane
- DMF: *N*,*N*-dimethylformamide
- DMSO: dimethylsulfoxide
- EtOAc: ethyl acetate
- EtOH: ethanol (100%)
- Et₂O: diethyl ether
- Hex: hexanes
- H₂SO₄: sulfuric acid
- MeCN: acetonitrile
- MeOH: methanol
- Pd/C: palladium on activated carbon
- TEA: triethylamine
- THF: tetrahydrofuran
- TFA: trifluoroacetic acid

As used herein, the abbreviations for any protective groups, amino acids and other compounds, are, unless indicated otherwise, in accord with their common usage, recognized abbreviations, or the IUPAC-IUB Commission on Biochemical Nomenclature (see, (1972) Biochem. 11:942-944).

### B. Compounds

Compounds for use in compositions and methods for promoting apoptosis in rapidly dividing cells are provided. In particular, compounds for binding to a peptide binding pocket on the surface of BIR3 are provided. The compounds in the compositions and methods provided herein prevent XIAP from exerting its apoptosis-suppressing function in the cell.

In certain embodiments, the compounds are of formula: 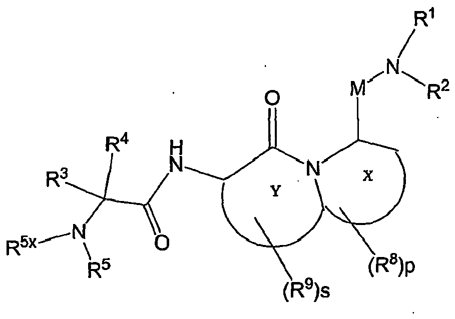 or pharmaceutically acceptable derivatives thereof, where:
M is CO, SO or SO₂;
X and Y are each independently selected from 4-7 membered heterocyclic and heteroaryl rings containing one or two heteroatoms, in one embodiment one heteroatom, each indepedently selected from O, N and S;
s and p are each independently 0-3;
R¹ and R² are each independently selected as follows:
   (i) R¹ is hydrogen, alkyl, alkenyl, alkynyl, aryl, heteroaryl, cycloalkyl or heterocyclyl; and R² is selected from hydrogen, alkenyl, alkynyl, aryl, heteroaryl, cycloalkyl, heterocyclyl, OR¹¹, NR¹⁵R¹⁶ and 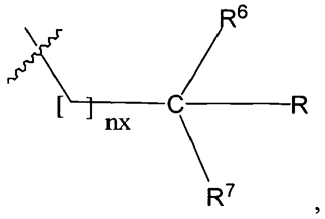 where nx is 0-6; R is hydrogen, alkyl, alkenyl, alkynyl, aryl, heteroaryl, heteroarylium, cycloalkyl, heterocyclyl, C(A)R¹⁰, OR¹¹ or NR¹⁵R¹⁶; and R⁶ and R⁷ are each independently selected from hydrogen, alkyl, alkenyl, alkynyl, aryl, heteroaryl, heteroarylium, cycloalkyl and heterocyclyl; or
   (ii) R¹ and R² together with the nitrogen atom on which they are substituted form a heterocyclic or heteroaryl ring;
R³, R⁴ and R⁵ are each independently selected from (i) or (ii) as follows:
   (i) R³ and R⁴ are each independently hydrogen, lower alkyl, lower alkenyl, alkynyl, lower cycloalkyl, lower heterocyclyl; and R⁵ is selected from hydrogen, alkyl, alkenyl, alkynyl, aryl, heteroaryl, cycloalkyl, heterocyclyl and NR¹⁵R¹⁶; or
   (ii) R⁵ and R³ or R⁵ and R⁴ together with the atoms on which they are substituted form heterocyclic or heteroaryl ring and the other of R³ or R⁴ is selected as (i);
R^{5x} is hydrogen, lower alkyl, lower alkenyl or lower alkynyl;
R⁸ and R⁹ are each independently selected from (i), (ii) or (iii) as follows:
   (i) R⁸ and R⁹ are each independently selected from lower alkyl, lower alkenyl, lower alkynyl, aryl, heteroaryl, cycloalkyl, heterocyclyl, C(A)R¹⁰, halo, pseudohalo, OR¹¹, oxo, thio, S(D)nR¹², NR¹⁵R¹⁶, N⁺R¹⁵R¹⁶R¹⁷, =NR²⁵ and =CR¹³R¹⁴;
   (ii) R⁸ and R⁹ together with the atoms on which they are substituted form a cycloalkyl, aryl, heterocyclic or heteroaryl ring; or
   (iii) two R⁸ substituents together with the carbon atoms on which they are substituted form a cycloalkyl, aryl, heteroaryl or heterocyclyl ring;
A is O, S or NR²⁵;
R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
D is O or NR²⁵;
n is 0, 1 or 2;
when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
R¹³ and R¹⁴ are each independently selected from hydrogen, alkyl, alkenyl, alkynyl, aryl, heteroaryl, heteroarylium, cycloalkyl, heterocyclyl, C(A)R²⁹, OR²⁵ or NR³²R³³;
R¹⁵, R¹⁶ and R¹⁷ are each independently selected from (a) and (b) as follows:
   (a) hydrogen, alkyl, alkenyl, alkynyl, aryl, heteroaryl, heteroarylium, cycloalkyl, heterocyclyl, C(A)R²⁹, OR²⁵ or NR³²R³³; or
   (b) any two of R¹⁵, R¹⁶ and R¹⁷ together form alkylene, alkenylene, alkynylene, heteroalkylene, and the other is selected as in (a);
R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R₂₁ together form alkylene, alkenylene, alkynylene, heteroalkylene;
R²², R²³ and R²⁴ are each independently selected as in (i) or (ii) as follows:
   (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
   (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
   (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
   (ii) R²⁷ and R²⁸ together form alkylene, alkenylene, alkynylene, heteroalkylene;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
R, R^{5x} and R¹-R³³ are each independently unsubstituted or substituted with one or more substituents, in one embodiment one to five substituents, in another embodiment one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylakyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁴-R⁵¹R⁵²R⁵¹, P(R⁵⁰)₂, p(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-)or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one or more substituents, in one embodiment one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, diallcylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹,
with the proviso that when a) Y is a 7 membered heterocyclic ring with 1 heteroatom in the ring, b) X is a 5 membered heterocyclic ring with 1 heteroatom in the ring, c) R⁸ and R⁹ together with the atoms on which they are substituted form a phenyl ring, and d) nx is 0, then R, R⁶, and R⁷ are not heterocyclyl or heteroaryl.

In other embodiments, the compounds have formula: 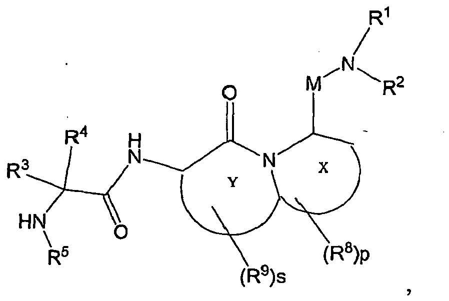 wherein the variables are as defined elsewhere herein. In other embodiments, the compounds have formula: 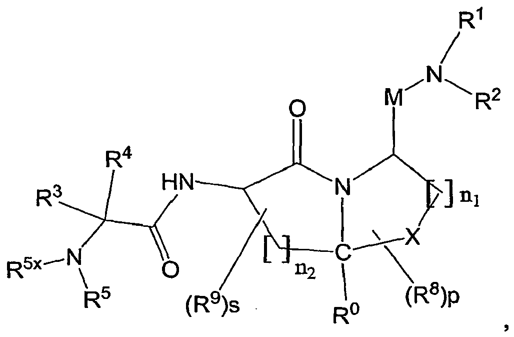 where
X is NH, NR⁸, S, O, CH₂, CHR⁸ or C(R⁸)₂;
R⁰ is H or R⁸_{;}
n₁ and n₂ are each independently 0-3 and s and p are each independently selected as follows:
when n₁ is 0, p is 0 or 1;
when n₁ is 1, p is 0, 1 or 2;
when n₁ is 2, p is 0, 1, 2 or 3;
when n₁ is 3, p is 0, 1, 2, 3 or 4;
when n₂ is 0, s is 0 or 1;
when n₂ is 1, s is 0, 1 or 2;
when n₂ is 2, s is 0, 1, 2 or 3; and
when n₂ is 3, s is 0, 1, 2, 3 or 4 and other variables are as defined elsewhere herein.

In other embodiments, the compounds are selected such that
M is CO;
n₁ is 3 and n₂ is 1;
R¹ is hydrogen or lower alkyl;
R² is substituted or unsubstituted aryl, NR¹⁵R¹⁶ or 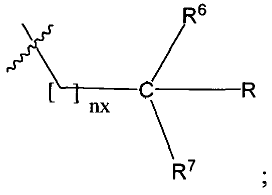 nx is 0 or 1; R⁶ is selected from hydrogen and substituted or unsubstituted alkyl; R⁷ is selected from hydrogen, substituted or unsubstituted alkyl, and substituted or unsubstituted cycloalkyl;
R is hydrogen or substituted or unsubstituted alkyl;
R³, R⁴ and R⁵ are each independently selected from (i) or (ii) as follows:
(i) R³ and R⁴ are each independently hydrogen or substituted or unsubstituted alkyl; and R⁵ is selected from hydrogen, substituted or unsubstituted alkyl, substituted or unsubstituted cycloalkyl and NR¹⁵R¹⁶;
(ii) R⁵ and R³ or R⁵ and R⁴ together with the atoms on which they are substituted form substituted or unsubstituted heterocyclic or substituted or unsubstituted heteroaryl ring and the other of R³ or R⁴ is selected as (i);
R^{5x} is hydrogen or lower alkyl;
R⁸ and R⁹ are each independently selected from (i), (ii) or (iii) as follows:
i) R⁸ and R⁹ are each independently selected from substituted or unsubstituted lower alkyl and oxo;
ii) R⁸ and R⁹ together with the atoms on which they are substituted form substituted or unsubstituted aryl ring, wherein substituents when present, are selected from Q¹; or
(iii) two R⁸ substituents together with the carbon atoms on which they are substituted form an aryl ring and R⁹ is selected as above.

In certain embodiments, R¹ is hydrogen or substituted or unsubstituted lower alkyl.

In certain embodiments, R¹ is hydrogen.

In certain embodiments, R² is selected from hydrogen, substituted or unsubstituted aryl, substituted or unsubstituted cycloalkyl and NR¹⁵R¹⁶, wherein substituents when present are selected from Q¹.

In certain embodiments, R² is selected from carboxyaralkyl, aryl, cycloalkyl and arylalkylamine.

In certain embodiments, R² is selected from benzylmethyl, carboxybenzyl, naphthyl, tetrahydronaphthyl and methylphenylamine.

In certain embodiments, R² is tetrahydronaphthyl.

In certain embodiments, R² is selected from

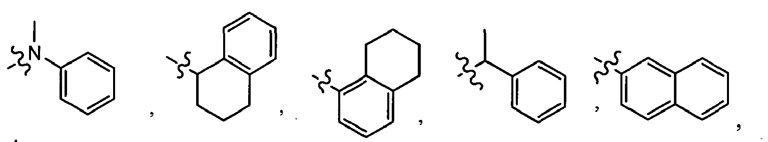

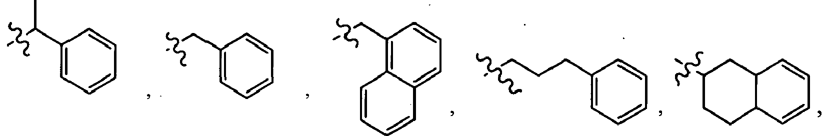

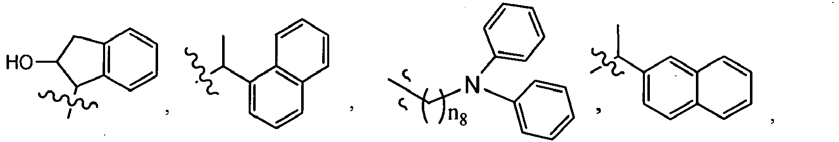

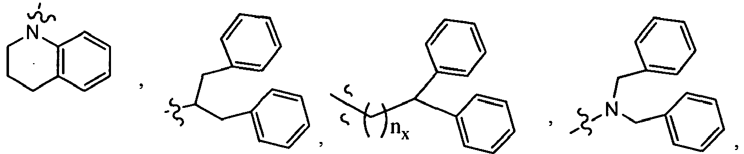

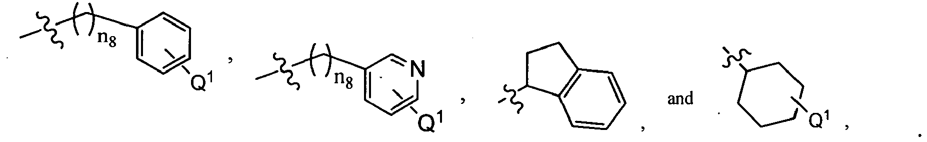

In certain embodiments, n₈ is 0 to 3. In other embodiments, n₈ is 0, 1 or 2.

In certain embodiments, R² is selected from

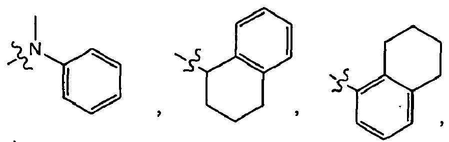

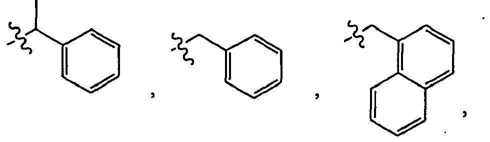

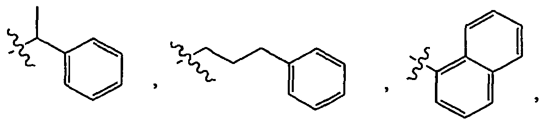

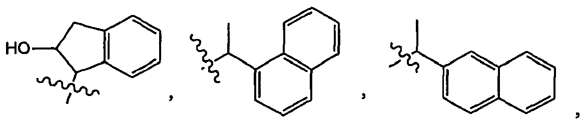

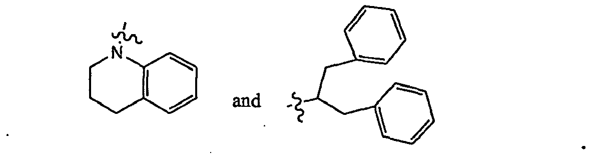

In certain embodiments, the X ring is a 5 membered heterocyclic ring with two heteroatoms in the ring.

In certain embodiments, the X ring is a thiazole ring.

In certain embodiments, the X ring is pyrrolidine ring.

In certain embodiments, the Y ring is pyrrolidine ring.

In certain embodiments, the X and Y rings are selected from: 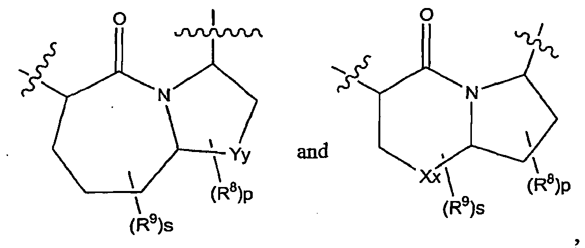 where Y_{y} is S, O or NR⁸ and Xₓ is S, O or NR⁹_{.}

In certain embodiments, the X and Y rings are selected from: 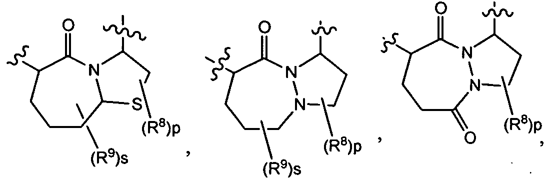 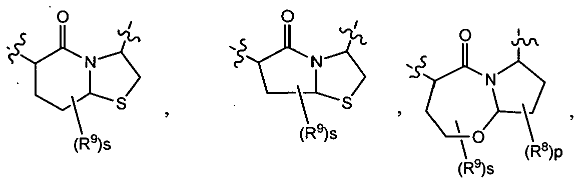 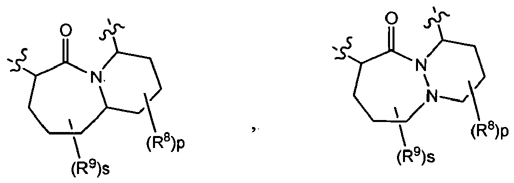 and

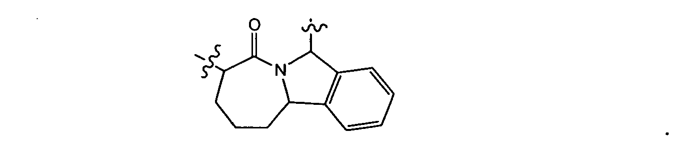

In certain embodiments, the X and Y rings form a fused bicyclic ring:

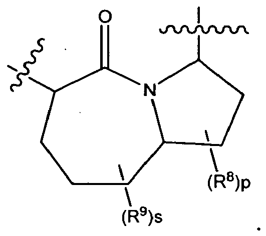

In other embodiment, R⁸ is lower alkyl, oxo or OR¹¹.

In other embodiment, R⁸ is lower alkyl or oxo.

In other embodiment, R⁹ is lower alkyl, oxo or OR¹¹.

In other embodiment, R⁹ is lower alkyl or oxo.

In certain embodiments, R⁸ and R⁹ are selected as follows: (i) R⁸ and R⁹ together with the atoms on which they are substituted form an aryl ring or (ii) two R⁸ substituents together with the carbon atoms on which they are substituted form an aryl ring.

In certain embodiments, R⁸ and R⁹ together with the atoms on which they are substituted form an aryl ring.

In other embodiments, R⁸ and R⁹ together with the atoms on which they are substituted form a phenyl ring.

In certain embodiments, two R⁸ substituents together with the carbon atoms on which they are substituted form an aryl ring.

In certain embodiments, two R⁸ substituents together with the carbon atoms on which they are substituted form a phenyl ring.

In other embodiments, R³ and R⁴ are each independently hydrogen or lower alkyl.

In other embodiments, R³ is hydrogen.

In other embodiments, R³ is lower alkyl.

In other embodiments, R³ is methyl or ethyl.

In other embodiments, R³ is methyl.

In other embodiments, R⁴ is hydrogen.

In other embodiments, R⁴ is lower alkyl.

In other embodiments, R⁴ is methyl or ethyl.

In other embodiments, R⁴ is methyl.

In other embodiments, R⁵ is alkyl, cycloalkylalkyl or aralkyl.

In other embodiments, R⁵ is methyl, cyclohexylmethyl, cyclopropylmethyl, isopropyl, benzyl or phenylpropyl.

In other embodiments, R⁵ and R³ or R⁵ and R⁴ together with the atoms on which they are substituted form a heterocyclic or heteroaryl ring and the other of R³ or R⁴ is selected from hydrogen and lower alkyl.

In other embodiments, R⁵ and R³ form an optinally substituted heterocyclic ring selected from:

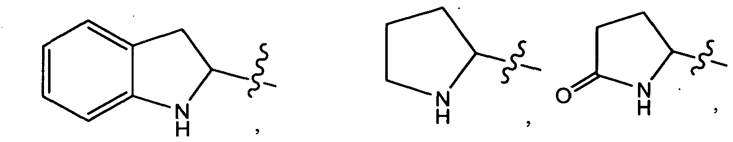

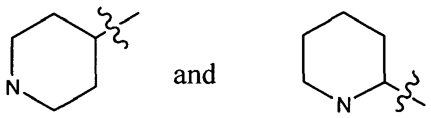

In other embodiments, R⁵ and R³ form a heterocyclic ring selected from:

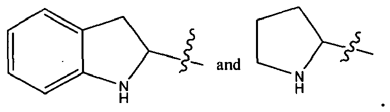

In certain embodiments, R^{5x} is hydrogen or lower alkyl.

In certain embodiments, R^{5x} is hydrogen or methyl.

In certain embodiments, the compounds have formula: 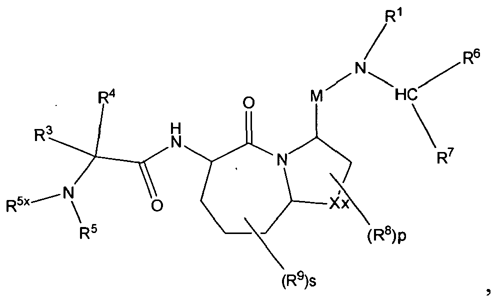 where the variables are as defined elsewhere herein. In certain embodiments, the compounds have formula: 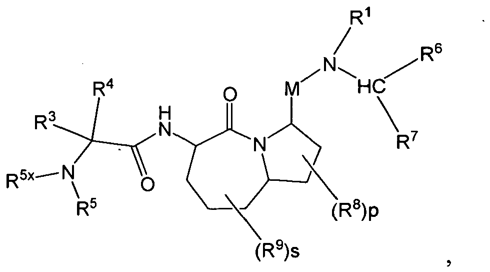 where the variables are as defined elsewhere herein.

In other embodiments, the compounds have formula: 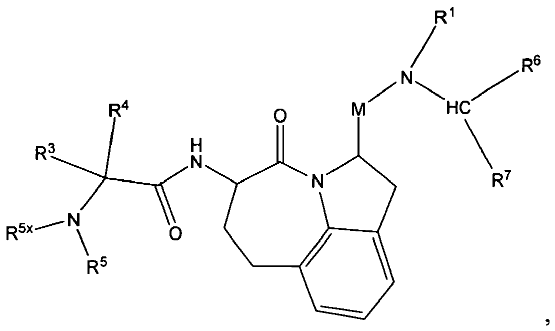 where the variables are as defined elsewhere herein.

In other embodiments, the compounds have formula 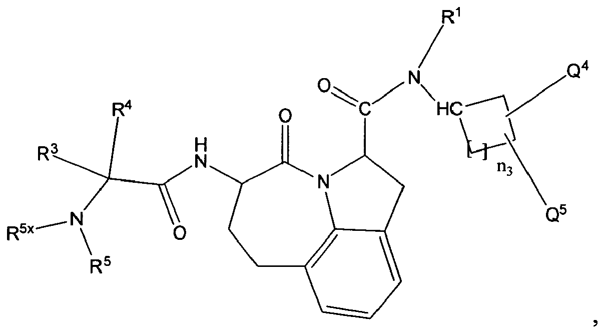 where n₃ is 1-3, Q⁴ and Q⁵ are each independently alkyl, cycloalkyl, aryl or Q⁴ and Q⁵ together with the carbon atoms on which they are substituted form a cycloalkyl or aryl ring and other variables are as defined elsewhere herein.

In other embodiments, the compounds have formula: 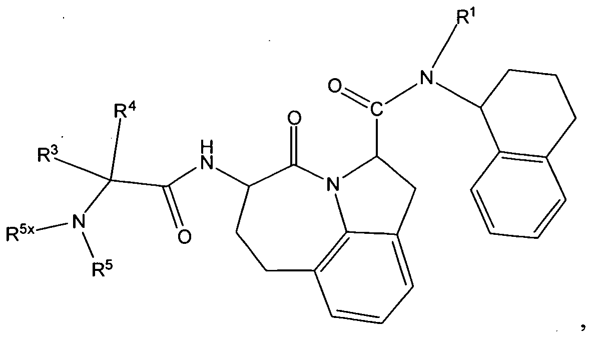 where the variables are as defined elsewhere herein.

In other embodiments, the compounds have formula: 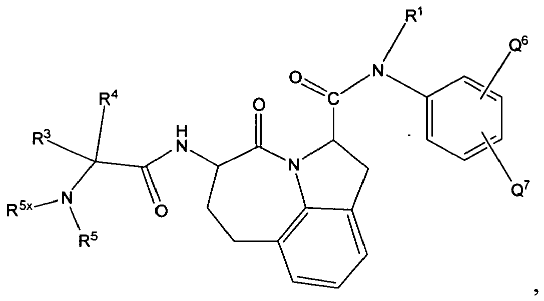 where Q⁶ and Q⁷ are each independently alkyl, cycloalkyl, aryl or Q⁶ and Q⁷ together with the carbon atoms on which they are substituted form a cycloalkyl or aryl ring and other variables are as defined elsewhere herein.

In other embodiments, the compounds have formula: 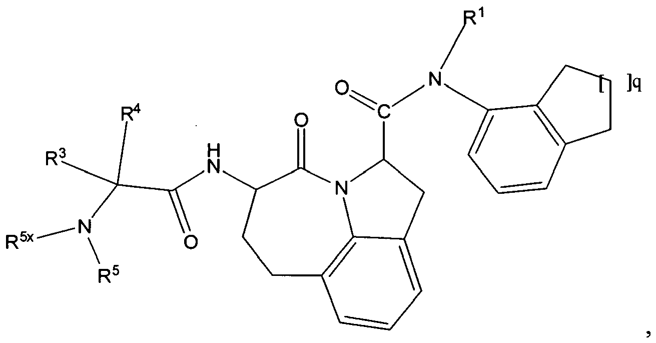 where q is 1-3 and other variables are as defined elsewhere herein.

In certain embodiments, the compounds have formula

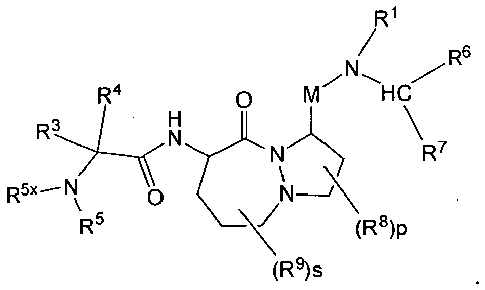

In certain embodiments, the compounds provided are of formula: 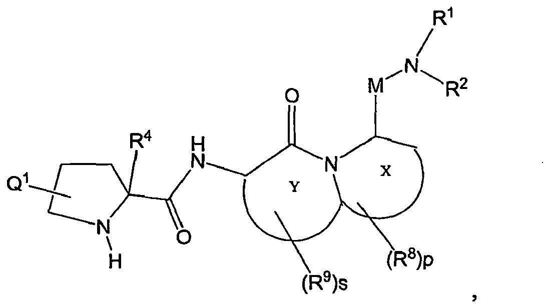 where the variables are as defined elsewhere herein.

In certain embodiments, the compounds provided are of formula: 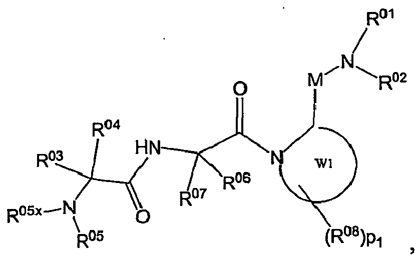 or pharmaceutically acceptable derivatives thereof, where,
M is CO, SO or SO₂ ; W1 is a 5-7 membered heterocyclic or heteroaryl ring containing 1 or 2 heteroatoms, one embodiment one heteroatom, each independently selected from O, S and N;
R⁰¹ and R⁰² are each independently selected from (i) or (ii) as follows:
(i) R⁰¹ is hydrogen, alkyl, alkenyl, alkynyl, aryl, heteroaryl, cycloalkyl or heterocyclyl; and R⁰² is selected from hydrogen, alkyl, alkenyl, alkynyl, aryl, heteroaryl, cycloalkyl, heterocyclyl, OR¹¹, NR¹⁵R¹⁶, or
(ii) R⁰¹ and R⁰² together with the nitrogen atom on which they are substituted form a heterocyclic or heteroaryl ring;
R⁰³, R⁰⁴ and R⁰⁵ are each independently selected from (i) or (ii) as follows:
(i) R⁰³ and R⁰⁴ are each independently hydrogen, lower alkyl, lower alkenyl, alkynyl, lower cycloalkyl, lower heterocyclyl; and R⁰⁵ is selected from hydrogen, alkyl, alkenyl, alkynyl, aryl, heteroaryl, cycloakyl, heterocyclyl and NR¹⁵R¹⁶;
(ii) R⁰⁵ and R⁰³ or R⁰⁵ and R⁰⁴ together with the atoms on which they are substituted form a heterocyclic or heteroaryl ring and the other of R⁰³ or R⁰⁴ is selected as (i);
R^{05x} is hydrogen, lower alkyl, lower alkenyl or lower alkynyl;
R⁰⁶ and R⁰⁷ are each independently hydrogen, alkyl, alkenyl, alkynyl, aryl, heteroaryl, cycloalkyl and heterocyclyl;
p₁ is 0-4;
R⁰⁸ is selected from
i) alkyl, alkenyl, alkynyl, aryl, heteroaryl, heteroarylium, cycloalkyl, heterocyclyl, C(A)R¹⁰, halo, pseudohalo, OR¹¹, oxo, thio, S(D)ₙR¹², NR¹⁵R¹⁶, N⁺R¹⁵R¹⁶R¹⁷, =NR²⁵ and =CR¹³R¹⁴; or
ii) two R⁰⁸ substituents together with the atoms on which they are substituted form an aryl, cycloalkyl, heteroaryl or heterocyclyl ring;
A is O, S or NR²⁵;
R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
D is O or NR²⁵;
n is 0, 1 or 2;
when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
R¹³ and R¹⁴ are each independently selected from hydrogen, alkyl, alkenyl, alkynyl, aryl, heteroaryl, heteroarylium, cycloalkyl, heterocyclyl, C(A)R²⁹, OR²⁵ or NR³²R³³;
R¹⁵, R¹⁶ and R¹⁷ are each independently selected from (a) and (b) as follows:
(a) hydrogen, alkyl, alkenyl, alkynyl, aryl, heteroaryl, heteroarylium, cycloalkyl, heterocyclyl, C(A)R²⁹, OR²⁵ or NR³²R³³; or
(b) any two of R¹⁵, R¹⁶ and R¹⁷ together form alkylene, alkenylene, alkynylene, heteroalkylene, and the other is selected as in (a);
R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R21 together form alkylene, alkenylene, alkynylene, heteroalkylene;
R²², R²³ and R²⁴ are each independently selected as in (i) or (ii) as follows:
(i) R²², R²³ and R²⁴ are each independently hydrogen, alkyl, alkenyl, alkynyl, aryl, heteroaryl, heteroarylium, cycloalkyl, heterocyclyl, R²⁵ or NR³²R³³; or
(ii) any two of R²², R²³ and R²⁴ together form alkylene, alkenylene, alkynylene, heteroalkylene; and the other is selected as in (i);
R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
(i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
(ii) R²⁷ and R²⁸ together form alkylene, alkenylene, alkynylene, heteroalkylene;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
R⁰¹-R⁰⁸ and R¹⁰-R³³ are each independently unsubstituted or substituted with one or more substituents, in one embodiment one to five substituents, in another embodiment one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one or more substituents, in one embodiment one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹;
with the proviso that when W1 is a heterocyclic ring with one heteroatom in the ring, p is 0, R⁰¹ is hydrogen, R^{05x} is hydrogen or methyl, and R⁰⁵ is alkyl, optionally substituted with halo, hydroxy, alkoxy, mercapto, alkylthio, thiocyano, pesudohalo, then R⁰² is not hydrogen, alkyl, cycloalkyl, naphthyl or COR¹⁰, where R¹⁰ is alkyl or phenyl.

In certain embodiments, the compounds provided are of formula: 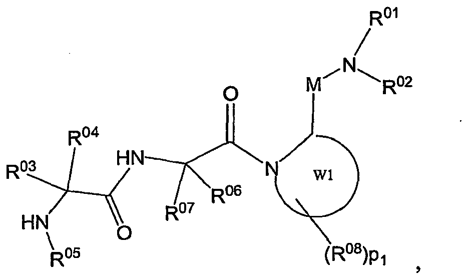 where W1 is a 5-7 membered heterocyclic ring containing 1 or 2 heteroatoms;
M is CO;
R⁰¹ is hydrogen, alkyl, alkenyl or alkynyl; and R⁰² is selected from hydrogen, alkyl, alkenyl, alkynyl, aryl, heteroaryl, cycloalkyl, heterocyclyl, NR¹⁵R¹⁶;
R⁰³, R⁰⁴ and R⁰⁵ are each independently selected from (i) or (ii) as follows:
(i) R⁰³ and R⁰⁴ are each independently hydrogen and alkyl and R⁰⁵ is selected form hydrogen, alkyl and aryl and NR¹⁵R¹⁶;
(ii) R⁰⁵ and R⁰³ or R⁰⁵ and R⁰⁴ together with the atoms on which they are substituted form a heterocyclic ring and the other of R⁰³ or R⁰⁴ is selected as (i);
   R⁰⁶ and R⁰⁷ are each independently hydrogen, alkyl and cycloalkyl;
   p₁ is 0-2;
   R⁰⁸ is selected from
i) R⁰⁸ is lower alkyl or oxo, or
ii) two R⁰⁸ substituents together with the atoms on which they are substituted form an aryl ring;
   R⁰¹-R⁰⁸ are each independently unsubstituted or substituted with one or more substituents, in one embodiment one to five substituents, in another embodiment one, two or three substituents, each independently selected from Q¹.

In certain embodiments, R⁰¹ is hydrogen or substituted or unsubstituted lower alkyl.

In certain embodiments, R⁰¹ is hydrogen.

In certain embodiments, R⁰² is selected from hydrogen, substituted or unsubstituted alkyl, substituted or unsubstituted aryl, substituted or unsubstituted cycloalkyl and NR¹⁵R¹⁶, wherein substituents when present are selected from Q¹.

In certain embodiments, R⁰² is selected from carboxyaralkyl, aralkyl, aryl, cycloalkyl and arylalkylamine.

In certain embodiments, R⁰² is selected from carboxyaralkyl, aralkyl, aryl, bicyclic aryl, bicyclic cycloalkyl and arylalkylamine.

In certain embodiments, R⁰² is selected from 2-benzyl-3-phenylpropyl, benzylmethyl, carboxybenzyl, naphthyl, tetrahydronaphthyl, methylphenylamine, benzyloxy, 3-(benzyloxy)phenyl, benzylphenyl, benzyl, 3-(trifluoromethyl)benzyl, 3-fluorobenzyl, 3-fluorophenyl, 3-trifluoromethoxyphenyl, 2,2,4,4-tetramethylbutyl, pyridine-3-ylmethyl, 2-(pyridine-3-yl)ethyl, 3-trifluoromethylphenyl, 2-(3-fluorophenyl)ethyl and 3-chlorophenyl.

In certain embodiments, R⁰² is selected from 2-benzyl-3-phenylpropyl, benzylmethyl, carboxybenzyl, naphthyl, tetrahydronaphthyl and methylphenylamine.

In certain embodiments, R⁰² is tetrahydronaphthyl.

In certain embodiments, R⁰² is selected from

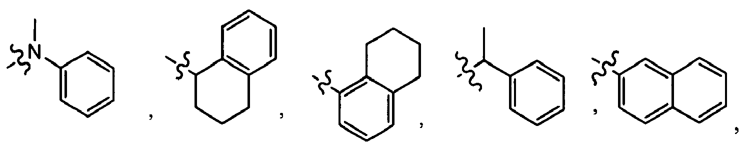

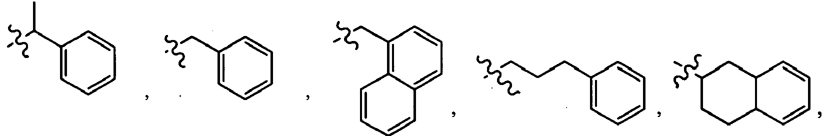

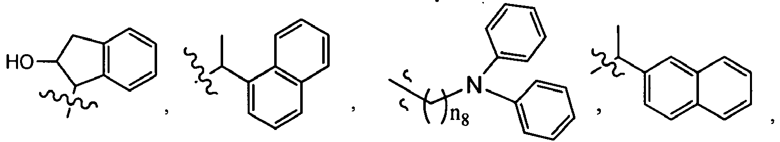

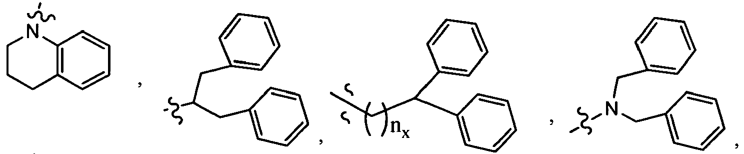

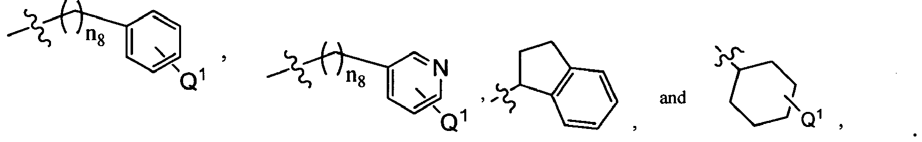

In certain embodiments, R⁰² is selected from

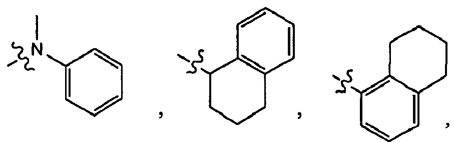

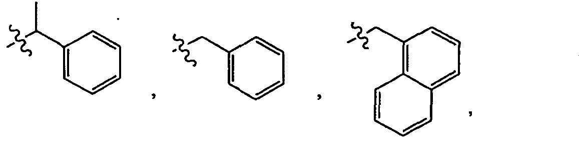

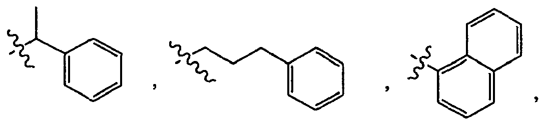

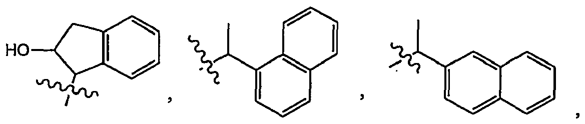

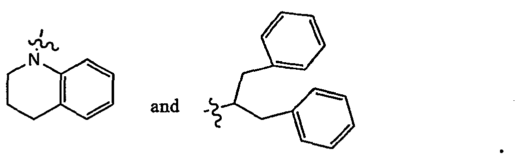

In certain embodiments, the W1 ring is a 5 membered heterocyclic ring with two heteroatoms in the ring. In certain embodiments, the heteroatoms are selected from O, S and N. In certain embodiments, the heteroatom is N. In certain embodiments, the heteroatom is S. In certain embodiments, the heteroatom is O.

In certain embodiments, the W1 ring is a thiazole ring.

In certain embodiments, the W1 ring is pyrrolidine ring.

In certain embodiments, the W1 ring is selected from: 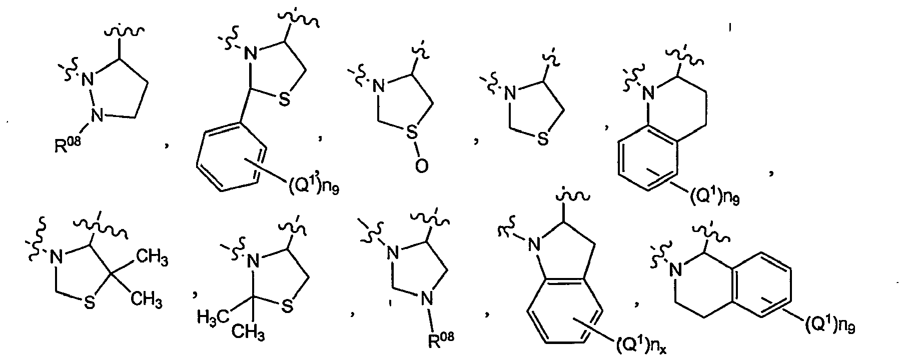 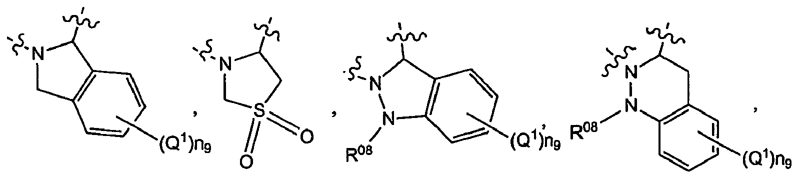 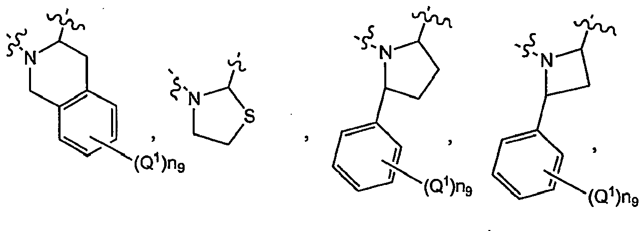 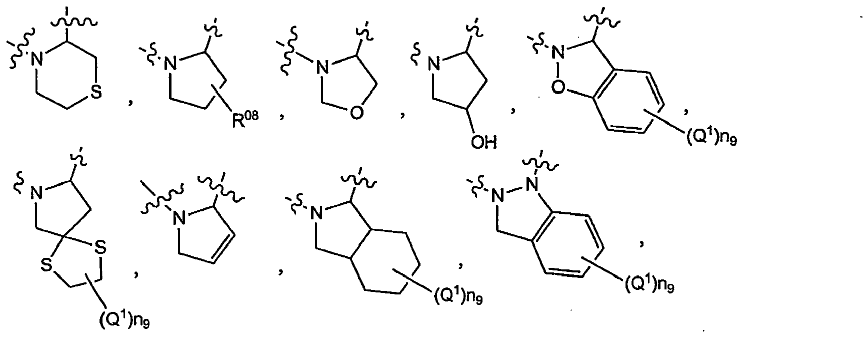 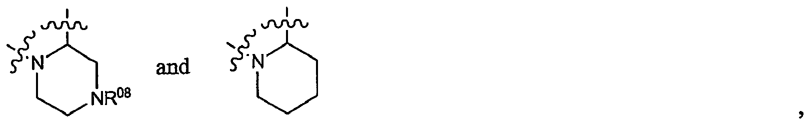 where n₉ is 0-5.

In certain embodiments, the W1 ring is selected from:

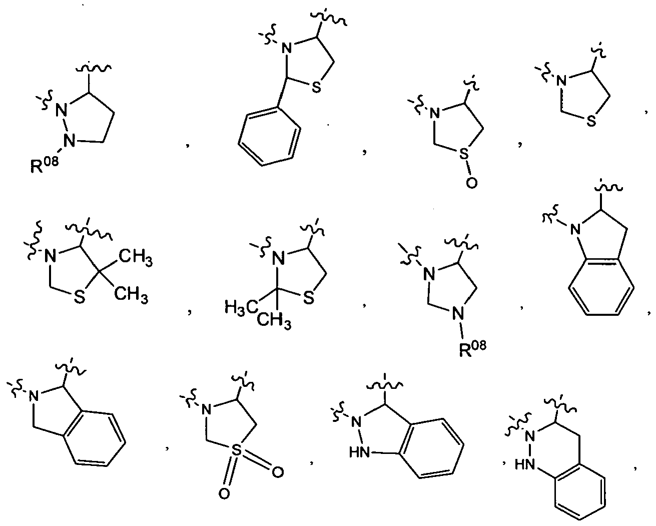

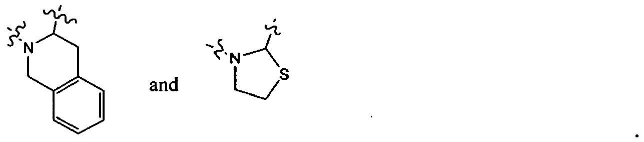

In other embodiments, R⁰³ and R⁰⁴ are each independently hydrogen or lower alkyl.

In other embodiments, R⁰³ is hydrogen.

In other embodiments, R⁰³ is lower alkyl.

In other embodiments, R⁰³ is methyl.

In other embodiments, R⁰⁴ is hydrogen.

In other embodiments, R⁰⁴ is lower alkyl.

In other embodiments, R⁰⁴ is methyl.

In other embodiments, R⁰⁵ is hydrogen, alkyl, cycloalkylalkyl, aralkyl or NR¹⁵R¹⁶, where R¹⁵ and R¹⁶ are each independently hydrogen or lower alkyl.

In other embodiments, R⁰⁵ is hygrogen, methyl, propyl, cyclohexylmethyl, cyclopropylmethyl, isopropyl, benzyl, 3-phenylpropyl or NH₂.

In other embodiments, R⁰⁵ is hygrogen, methyl, cyclohexylmethyl, cyclopropylmethyl, isopropyl, benzyl, phenylpropyl or NH₂.

In other embodiments, R⁰⁵ and R⁰³ or R⁰⁵ and R⁰⁴ together with the atoms on which they are substituted form a heterocyclic or heteroaryl ring and other of R⁰³ or R⁰⁴ is selected from hydrogen and lower alkyl.

In other embodiments, R⁰⁵ and R⁰³ form an optinally substituted heterocyclic ring selected from:

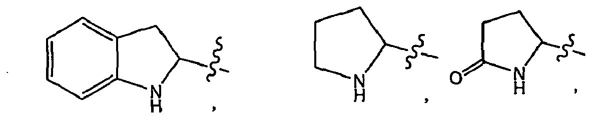

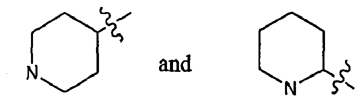

In other embodiments, R⁰⁵ and R⁰³ form a heterocyclic ring selected from:

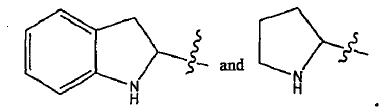

In certain embodiments, R^{5x} is hydrogen or lower alkyl.

In certain embodiments, R^{5x} is hydrogen or methyl.

In other embodiment, R⁰⁶ and R⁰⁷ are each independently hydrogen or alkyl. In other embodiment, R⁰⁶ is hydrogen or alkyl.

In other embodiment, R⁰⁶ is hydrogen, tertiary butyl or isopropyl.

In other embodiment, R⁰⁷ is hydrogen.

In other embodiment, R⁰⁸ is lower alkyl, oxo or OR¹¹.

In other embodiment, R⁰⁸ is lower alkyl or oxo.

In other embodiment, two R⁰⁸ substituents together with the atoms on which they are substituted form a phenyl ring.

In certain embodiments, the compounds provided herein are of formula: 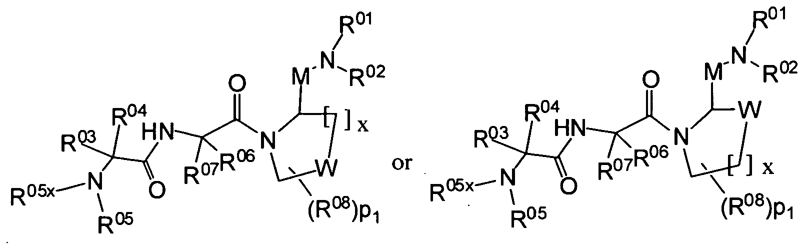 where W is S, N, NH, NR⁰⁸, CH or CHR⁰⁸; x is 1-3 and other variables are as defined elsewhere herein.

In certain embodiments, the compounds provided herein are of formula: 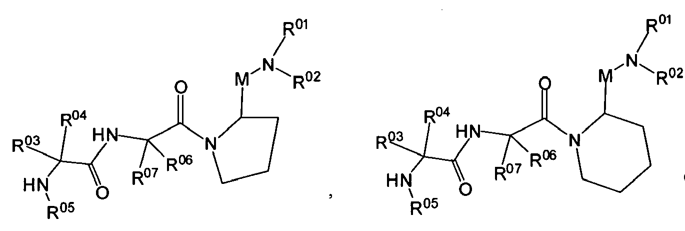 or 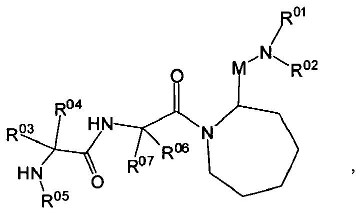 where the variables are as defined elsewhere herein. In certain embodiments, the compounds provided herein are of formula: 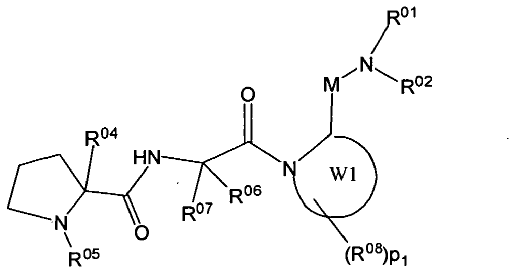 where the variables are as defined elsewhere herein.

In certain embodiments, the compounds provided herein are of formula: 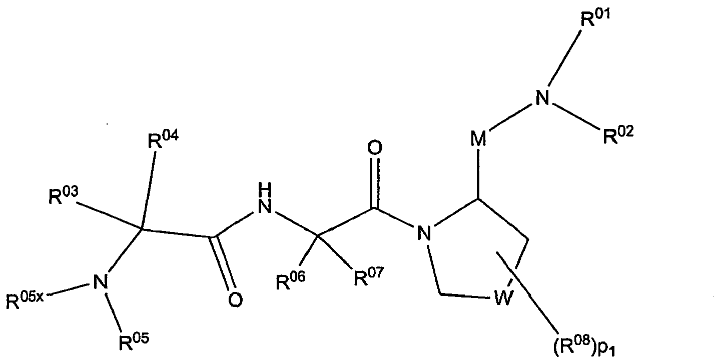 where the variables are as defined elsewhere herein.

In certain embodiments, the compounds provided herein are of formula: 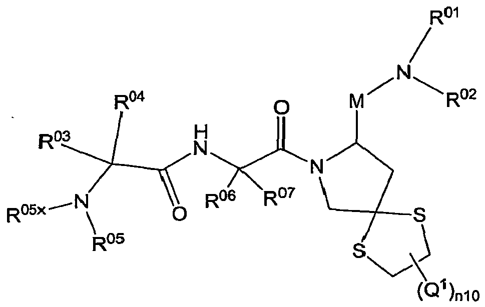 or 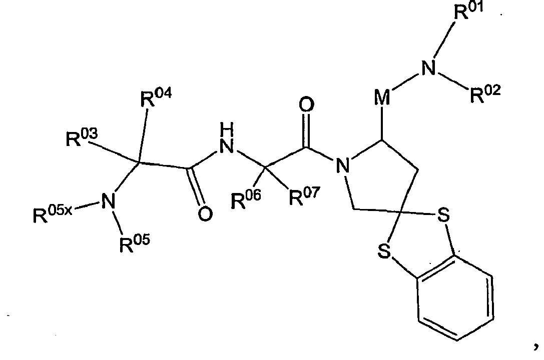 where n10 is 0 to 2 and the variables are as defined elsewhere herein.

In certain embodiments, the compounds provided herein are of formula: 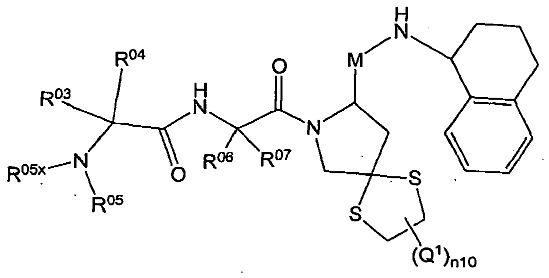 or 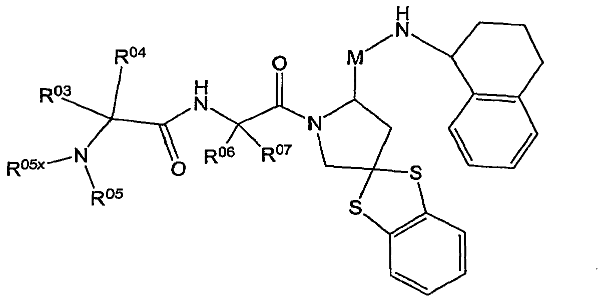 where the variables are as defined elsewhere herein.

In certain embodiments, the compounds provided herein are of formula: 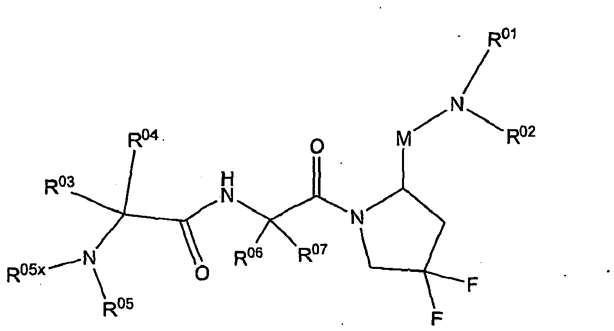 or 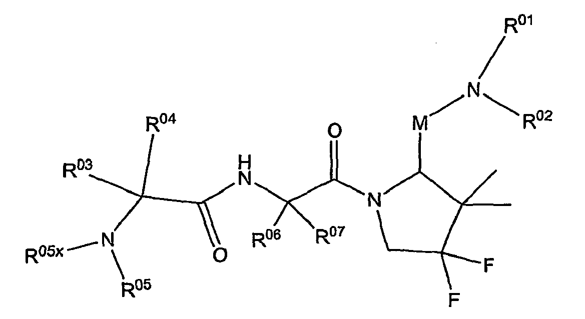 where the variables are as defined elsewhere herein.

In certain embodiments, the compounds provided herein are of formula: 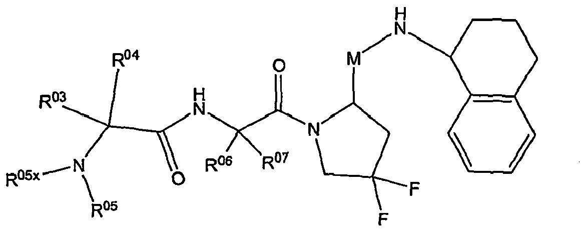 or 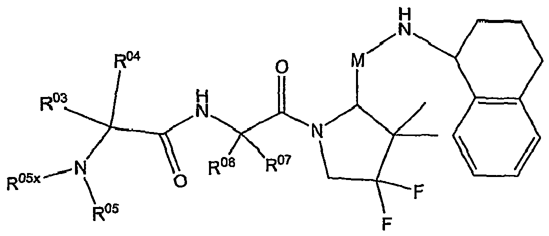 where the variables are as defined elsewhere herein.

In certain embodiments, the compounds provided herein are of formula: 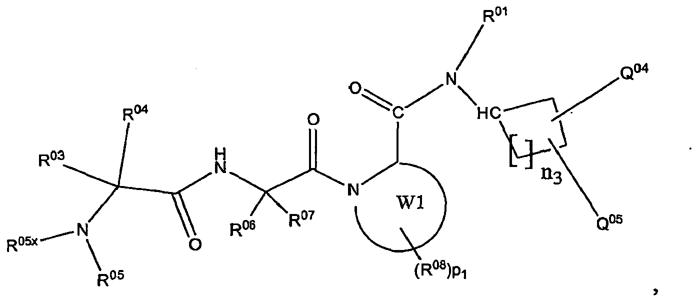 where n₃ is 1-3, Q⁰⁴ and Q⁰⁵ are each independently alkyl, cycloalkyl, aryl or Q⁰⁴ and Q⁰⁵ together with the carbon atoms on which they are substituted form a cycloalkyl or aryl ring and other variables are as defined elsewhere herein.

In certain embodiments, the compounds provided herein are of formula: 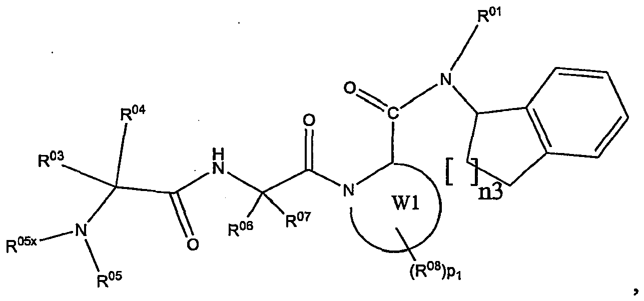 wherein the variables are as defined elsewhere herein.

In certain embodiments, the compounds provided herein are of formula:

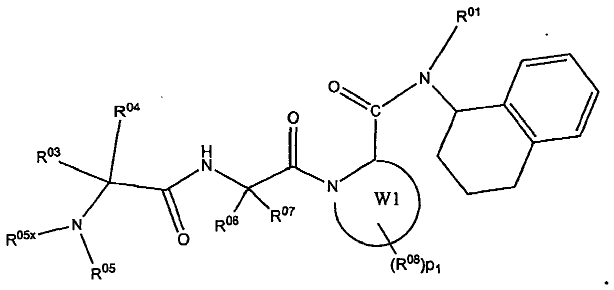

In certain embodiments, the compounds provided herein are of formula: 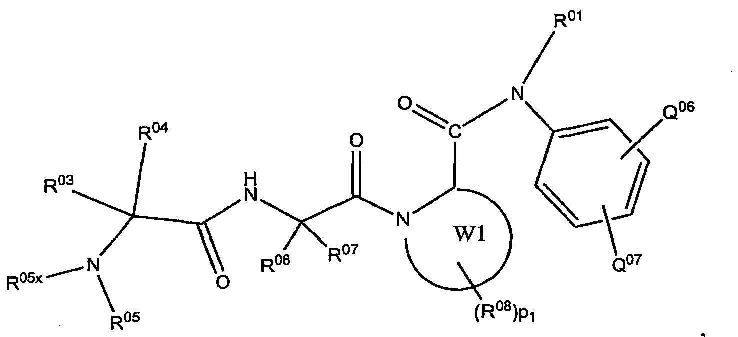 where Q⁰⁶ and Q⁰⁷ are each independently alkyl, cycloalkyl, aryl or Q⁰⁶ and Q⁰⁷ together with the carbon atoms on which they are substituted form a cycloalkyl or aryl ring and other variables are as defined elsewhere herein.

In certain embodiments, the compounds provided herein are of formula: 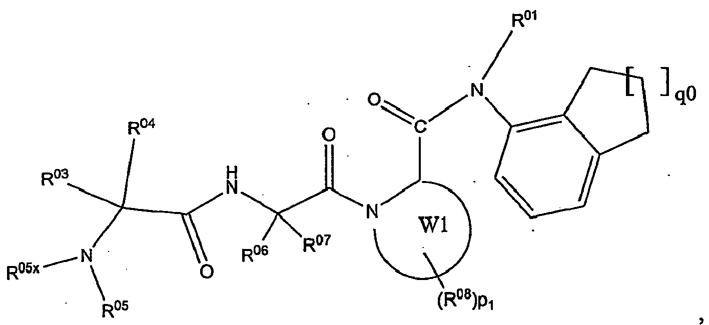 where q⁰ is 1-3 and other variables are as defined elsewhere herein.

In certain embodiments, the compounds provided are of formula: 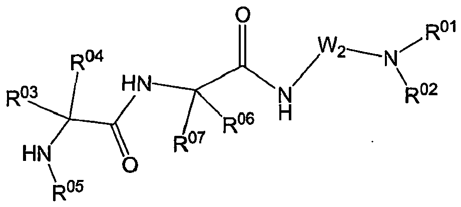 or pharmaceutically acceptable derivatives thereof, where W2 is an optionally substituted 5-7 membered heteroaryl ring containing 1-3 heteroatoms selected from O, S and N, wherein the substituents, when present, are selected from R⁰⁸. In certain embodiments, the W2 is a 5 membered ring. In certain embodiments, the W2 ring contains 1 heteroatom. In other embodiments, the ring W2 contains 2 heteroatoms. In other embodiments, the ring W2 contains 3 heteroatoms.

In certain embodiments, the compounds provided are of formula: 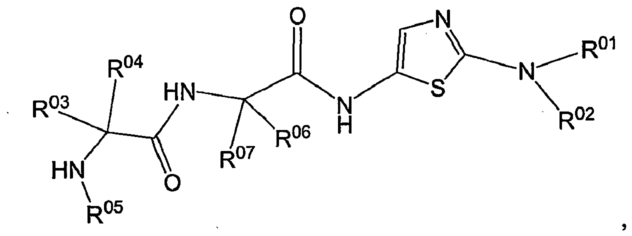 wherein the variables are as described elsewhere herein.

In certain embodiments, the compounds provided are of formula: 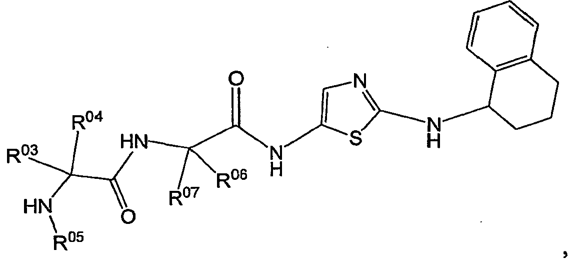 wherein the variables are as described elsewhere herein.

In certain embodiments, the compounds provided are of formula:

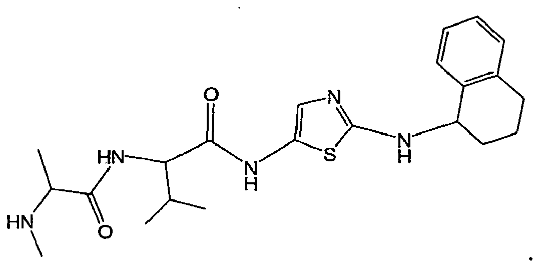

In certain embodiments, the compounds provided are of formula: 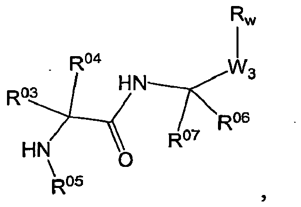 or pharmaceutically acceptable derivatives thereof, where
W3 is an optionally substituted 5-7 membered heteroaryl ring containing 1-3 heteroatoms selected from O, S and N, wherein the substituents, when present, are selected from R⁰⁸;
R_{w} is hydrogen, halo, pseudohalo, alkyl, alkenyl, alkynyl, aryl, heteroaryl, cycloalkyl, heterocyclyl, C(A)R¹⁰, OR¹¹, oxo, thio, S(D)nR¹² or NR¹⁵R¹⁶; and the other variables are as described elsewhere herein.

In certain embodiments, R_{w} is hydrogen, C(A)R¹⁰ or NR¹⁵R¹⁶; and the other variables are as described elsewhere herein.

In certain embodiments, W3 is a 5 membered ring. In certain embodiments, W3 ring contains 1 heteroatom. In other embodiments, the ring W3 contains 2 heteroatoms. In other embodiments, the ring W3 contains 3 heteroatoms.

In certain embodiments, the compounds provided are of formula: 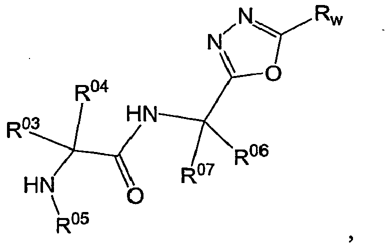 wherein the variables are as described elsewhere herein. In certain embodiments, the compounds provided are of formula: 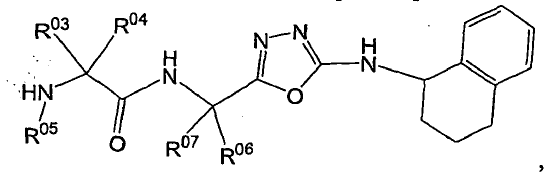 wherein the variables are as described elsewhere herein. In certain embodiments, the compounds provided are of formula:

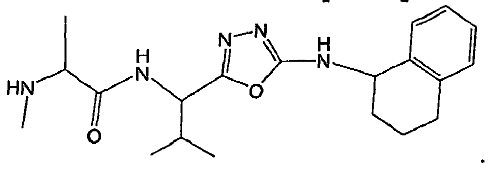

Also provided herein are compounds of formula: 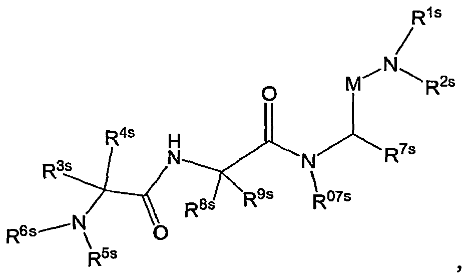 or pharmaceutically acceptable derivatives thereof, where:
M is CO, SO or SO₂;
R^{1s} and R^{2s} are selected as follows:
   (i) R^{1s} is hydrogen, alkyl, alkenyl, alkynyl, aryl, heteroaryl, cycloalkyl or heterocyclyl; and R^{2s} is selected from hydrogen, alkyl, alkenyl, alkynyl, aryl, heteroaryl, cycloalkyl, heterocyclyl, OR¹¹, NR¹⁵R¹⁶ or
   (ii) R^{1s} and R^{2s} together with the nitrogen atom on which they are substituted form a heterocyclic or heteroaryl ring; provided that both R^{1s} and R^{2s} are not hydrogen;
   R^{3s}, R^{4s} and R^{5s} are each independently selected from (i) or (ii) as follows:
      (i) R^{3s} and R^{4s} are each independently hydrogen, lower alkyl, lower alkenyl, alkynyl, lower cycloalkyl, lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶;
      (ii) R^{5s} and R^{3s} or R^{5s} and R^{4s} together with the atoms on which they are substituted form heterocyclic or heteroaryl ring and the other of R^{3s} or R^{4s} is selected as (ii);
R^{6s} is hydrogen, lower alkyl, lower alkenyl or lower alkynyl;
R^{7s} is hydrogen, alkyl, alkenyl, alkynyl, aryl, heteroaryl, cycloalkyl or heterocyclyl;
R^{07s}, R^{8s} and R^{9s} are each selected as follows:
   (i) R^{8s} and R^{9s} are each independently hydrogen, alkyl, alkenyl, alkynyl, aryl, heteroaryl, cycloalkyl, heterocyclyl, and R^{07s} is alkyl, alkenyl, alkynyl, aryl or cycloalkyl; or
   (ii)) R^{8s} and R^{07s} or R^{9s} and R^{07s} together with the atoms on which they are substituted form a 5-7 membered heterocyclic or heteroaryl ring containing 1 or 2 heteroatoms in the ring;
A is O, S or NR²⁵;
R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
D is O or NR²⁵.
n is 0, 1 or 2;
when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
R¹³ and R¹⁴ are each independently selected from hydrogen, alkyl, alkenyl, alkynyl, aryl, heteroaryl, heteroarylium, cycloalkyl, heterocyclyl, C(A)R²⁹, OR²⁵ or NR³²R³³;
R¹⁵, R¹⁶ and R¹⁷ are each independently selected from (a) and (b) as follows:
   (a) hydrogen, alkyl, alkenyl, alkynyl, aryl, heteroaryl, heteroarylium, cycloalkyl, heterocyclyl, C(A)R²⁹, OR²⁵ or NR³²R³³; or
   (b) any two of R¹⁵, R¹⁶ and R¹⁷ together form alkylene, alkenylene, alkynylene, heteroalkylene, and the other is selected as in (a);
R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R21 together form alkylene, alkenylene, alkynylene, heteroalkylene;
R²², R²³ and R²⁴ are each independently selected as in (i) or (ii) as follows:
   (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
   (ii) any two of R²², R²³ and R²⁴ together form alkylene, alkenylene, alkynylene, heteroalkylene; and the other is selected as in (i);
R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
   (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
   (ii) R²⁷ and R²⁸ together form alkylene, alkenylene, alkynylene, heteroalkylene;
R²⁹ is hydrogen, alkyl, alkenyl, alkynyl, aryl, heteroaryl, heteroarylium, cycloalkyl, heterocyclyl, OR⁴⁰ or _{NR}³²_{R}³³_{;}
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
R^{1s}-R^{9s}, R^{07s} and R¹⁰-R³³ are each independently unsubstituted or substituted with one or more substituents, in one embodiment one to five substituents, in another embodiment one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfmyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one or more substituents, in one embodiment one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, alyl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, p(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfmyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹.

In other embodiment, R^{1s}-R^{9s}, R^{07s} and R¹⁰-R³³ are each independently unsubstituted or substituted with one, two or three Q¹ substituents.

In other embodiment, each Q¹ substituent is independently substituted with one, two or three Q² substituents.

In other embodiment, the compounds have formula: 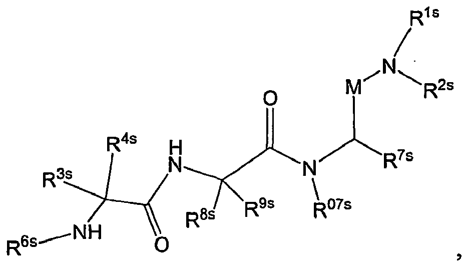 where M is CO;
R^{1s} is hydrogen;
R^{2s} is selected from aryl, heteroaryl, cycloalkyl, and heterocyclyl;

R^{3s}, R^{4s} and R^{5s} are each independently selected from (i) or (ii) as follows:
(i) R^{3s} is hydrogen or alkyl; R^{4s} is alkyl and R^{5s} is selected form hydrogen, alkyl and aryl;
(ii) R^{5s} and R^{3s} or R^{5s} and R^{4s} together with the atoms on which they are substituted form a heterocyclic ring and the other of R^{3s} or R^{4s} is selected as (i);
R^{6s} is hydrogen, or lower alkyl;
R^{7s} is hydrogen or alkyl;
R^{07s}, R^{8s} and R^{9s} are each selected as follows:
(i) R^{8s} and R^{9s} are each independently hydrogen or alkyl; and R^{07s} is lower alkyl; or
(ii) ) R^{8s} and R^{07s} or R^{9s} and R^{07s} together with the atoms on which they are substituted form a 5-7 membered heterocyclic or heteroaryl ring containing 1 heteroatom in the ring;

R^{1s}-R^{8s} and R^{07s} are each independently unsubstituted or substituted with one to five substituents, each independently selected from Q¹ and other variables are as defined elsewhere herein.

In other embodiment, R^{1s} is hydrogen; and R^{2s} is selected from aryl, heteroaryl, cycloalkyl, cycloalkylalkyl and heterocyclyl.

In other embodiment, R^{1s} is hydrogen.

In other embodiment, R^{2s} is selected from substituted or unsubstituted aryl, substituted or unsubstituted aralkyl and substituted or unsubstituted cycloalkyl.

In other embodiment, R^{2s} is selected from substituted or unsubstituted aryl and substituted or unsubstituted cycloalkyl.

In other embodiment, R^{2s} is selected from bicyclic aryl and bicyclic cycloalkyl. In other embodiment, R^{2s} is tetrahydronaphthyl.

In other embodiment, R^{2s} is selected from

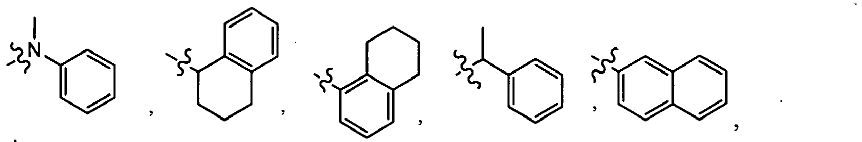

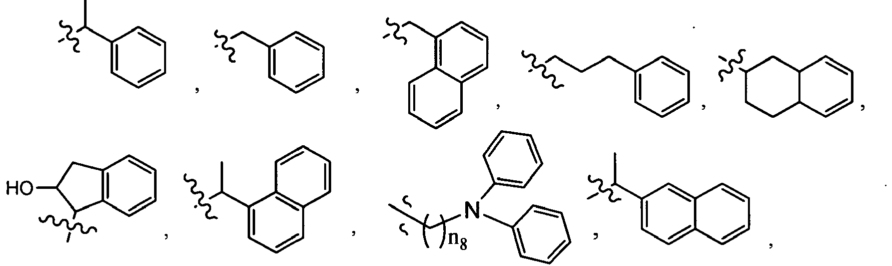

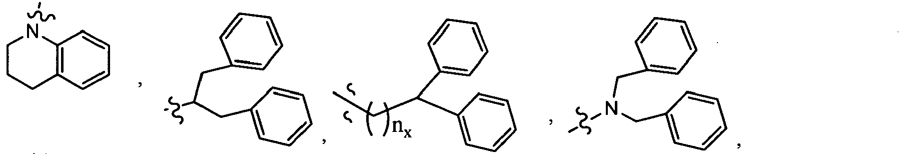

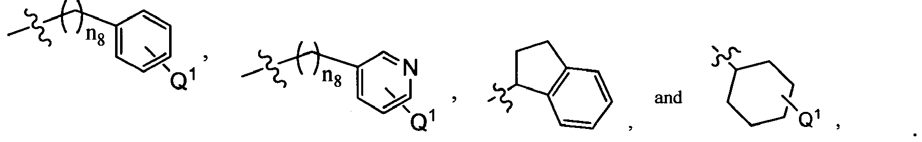

In other embodiment, R^{2s} is selected from

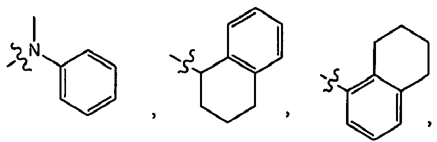

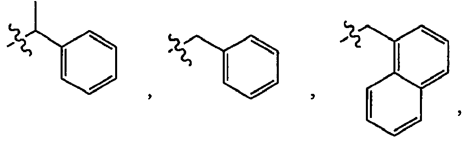

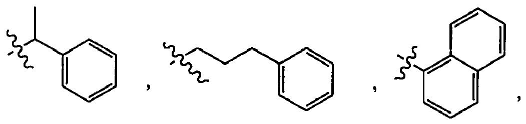

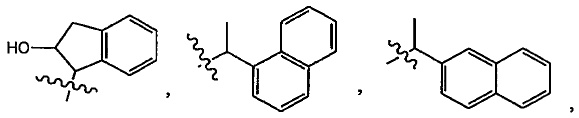

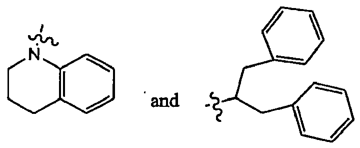

In other embodiment, R^{3s} is hydrogen.

In other embodiment, R^{3s} is lower alkyl.

In other embodiment, R^{3s} is methyl.

In other embodiment, R^{4s} is lower alkyl.

In other embodiment, R^{4s} is methyl.

In other embodiment, R^{5s} is hydrogen, alkyl, cycloalkylalkyl or aralkyl.

In other embodiment, R^{5s} is methyl.

In other embodiment, R^{5s} and R^{3s} or R^{5s} and R^{4s} together with the atoms on which they are substituted form a heterocyclic or heteroaryl ring and other of R^{3s} or R^{4s} is selected from hydrogen and lower alkyl.

In other embodiments, R^{5s} and R^{3s} form an optinally substituted heterocyclic ring selected from:

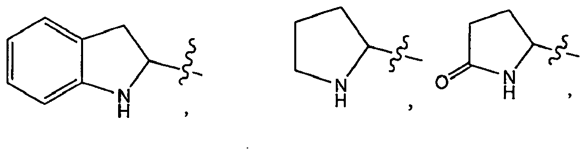

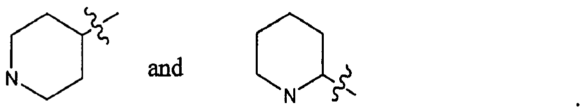

In other embodiments, R^{5s} and R^{3s} form a heterocyclic ring selected from:

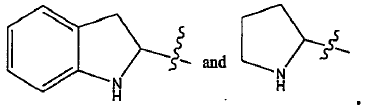

In other embodiment, R^{6s} is hydrogen or lower alkyl.

In other embodiment, R^{6s} is hydrogen or methyl.

In other embodiment, R^{7s} is hydrogen, alkyl, aralkoxycarbonylaminoalkyl or aralkoxyalkyl.

In other embodiment, R^{7s} 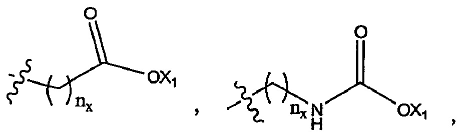 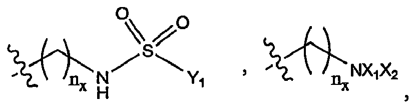 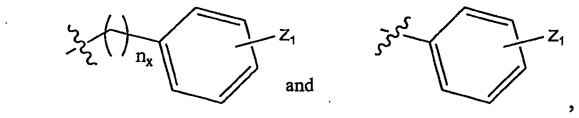 where nₓ is 1-6;
X₁ and X₂ are selected as follows:
(i) X₁ is selected from hydrogen, alkyl, aryl, aralkyl and X₂ is selected from hydrogen, alkyl, aryl, aralkyl, NX₄X₅, and CAX₃, or
(ii) X₁ and X₂ together with the nitrogen atom on which they are substituted form a heteroaryl or heterocyclic ring;
X₃ is hydrogen, alkyl, aryl or NH₂;
A is O, S or NX₄;
X₄ is hydrogen, alkyl, or aryl;
Y₁ is alkyl, aryl, aralkyl;
Z₁ hydrogen, halogen, alkyl, alkenyl, alkynyl, haloalkyl, aryl, aralkyl, nitro and COX₃; and

X₁, X₂ and Y₁ can be substituted with one or more, in some embodiments, 1, 2 or 3 substituents selected from Z₁.

In other embodiment, R^{7s} is hydrogen, benzyloxymethyl, benzyloxyethyl, benzyloxybenzyl, benzyl, 4- benzyloxycarbonylaminobutyl, 5-benzyloxycarbonylaminopentyl or methyl.

In other embodiment, R^{7s} is hydrogen, benzyloxymethyl, benzyloxyethyl, benzyloxycarbonylaminobutyl or methyl.

In other embodiment, R^{07s} is alkyl or aralkyl.

In other embodiment, R^{07s} is lower alkyl.

In other embodiment, R^{07s} is methyl or 2-phenylethyl.

In other embodiment, R^{8s} is hydrogen or alkyl.

In other embodiment, R^{8s} is hydrogen or isopropyl.

In other embodiment, R^{9s} is hydrogen or alkyl.

In other embodiment, R^{9s} is hydrogen.

In other embodiment, the compounds provided herein have formula: 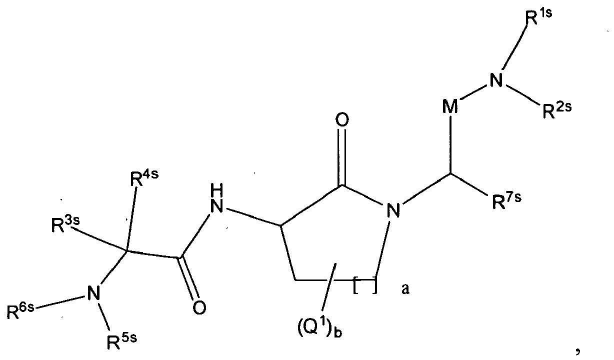 where a is 1-3 and b is 0-4 and is selected as follows:
when a is 1, b is 0, 1 or 2,
when a is 2, b is 0, 1, 2 or 3,
when a is 3, b is 0, 1, 2, 3 or 4, and other variables are as defined elsewhere
herein.

In other embodiment, the compounds have formula: 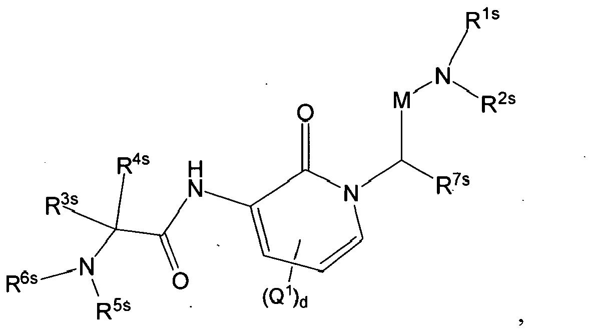 where d is 0-3 and other variables are as defined elsewhere herein.

In other embodiment, the compounds have formula: 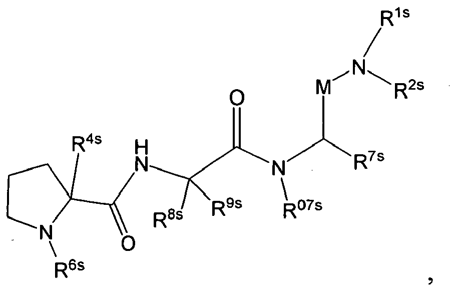 where the variables are as defined elsewhere herein.

In other embodiment, the compounds have formula: 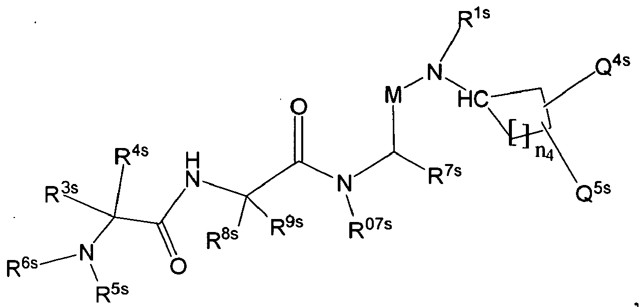 where n₄ is 1-3, Q^{4s} and Q^{5s} are each independently alkyl, cycloalkyl, aryl or Q^{4s} and Q^{5s} together with the carbon atoms on which they are substituted form a cycloalkyl or aryl ring and other variables are as defined elsewhere herein.

In other embodiment, the compounds have formula: 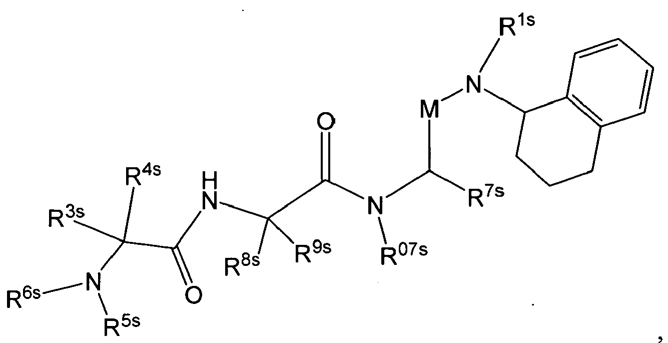 where the variables are as defined elsewhere herein.

In other embodiment, the compounds have formula: 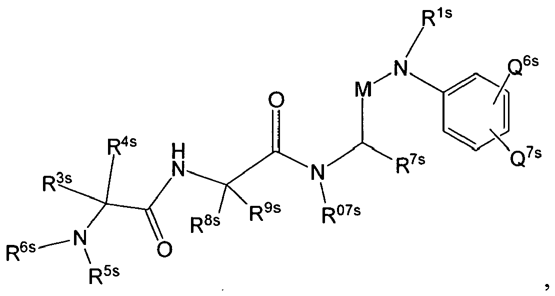 where Q^{6s} and Q^{7s} are each independently alkyl, cycloalkyl, aryl or Q^{6s} and Q^{7s} together with the carbon atoms on which they are substituted form a cycloalkyl or aryl ring and other variables are as defined elsewhere herein.

In other embodiment, the compounds have formula: 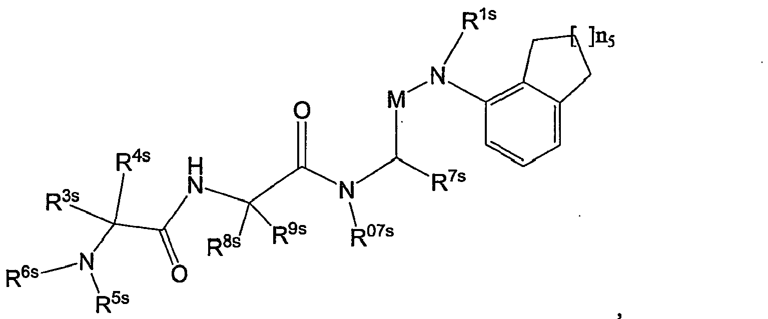 where n₅ is 1-3 and other variables are as defined elsewhere herein.

Also provided herein are compounds of formula: 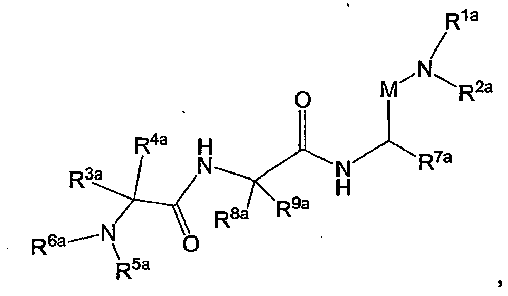 or pharmaceutically acceptable derivatives thereof, where:
M is CO, SO or SO₂;
R^{1a} and R^{2a} are selected as follows:
   (i) R^{1a} is hydrogen, alkyl, alkenyl, alkynyl, aryl, heteroaryl, cycloalkyl or heterocyclyl; and R^{2a} is selected from hydrogen, alkyl, alkenyl, alkynyl, aryl, heteroaryl, cycloalkyl, heterocyclyl, OR¹¹, NR¹⁵R¹⁶ or
   (ii) R^{1a} and R^{2a} together with the nitrogen atom on which they are substituted form a heterocyclic or heteroaryl ring; provided that both R^{1a} and R^{2a} are not hydrogen,
R^{3a}, R^{4a} and R^{5a} are each independently selected from (i) or (ii) as follows:
   (i) R^{3a} and R^{4a} are each independently hydrogen, lower alkyl, lower alkenyl, alkynyl, lower cycloalkyl, lower heterocyclyl; and R^{5a} is selected from hydrogen, alkyl, alkenyl, alkynyl, aryl, heteroaryl, heteroarylium, cycloalkyl, heterocyclyl and NR¹⁵R¹⁶;
   (ii) R^{5a} and R^{3a} or R^{5a} and R^{4a} together with the atoms on which they are substituted form heterocyclic or heteroaryl ring and the other of R^{3a} or R^{4a} is selected as (ii);
R^{6a} is hydrogen, lower alkyl, lower alkenyl or lower alkynyl;
R^{7a} is hydrogen, alkyl, alkenyl, alkynyl, aryl, C(A)R²⁹, heteroaryl, cycloalkyl or heterocyclyl;
R^{8a} and R^{9a} are each independently hydrogen, alkyl, alkenyl, alkynyl, aryl, heteroaryl, cycloalkyl, heterocyclyl;
A is O, S or NR²⁵;
R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
R¹⁵ and R¹⁶ are each independently selected hydrogen, alkyl, alkenyl, alkynyl, aryl, heteroaryl, heteroarylium, cycloalkyl, heterocyclyl, C(A)R²⁹, OR²⁵ or NR³²R³³; or
R²², R²³ and R²⁴ are each independently selected as in (i) or (ii) as follows:
   (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
   (ii) any two of R²², R²³ and R²⁴ together form alkylene, alkenylene, alkynylene, heteroalkylene; and the other is selected as in (i);
R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
   (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
   (ii) R²⁷ and R²⁸ together form alkylene, alkenylene, alkynylene, heteroalkylene;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
R^{1a}-R^{9a} and R¹⁰-R³³ are each independently unsubstituted or substituted with one or more substituents, in one embodiment one to five substituents, in another embodiment one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfmyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one or more substituents, in one embodiment one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, p(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹.

In other embodiment, R^{1a}-R^{9a}, and R¹⁰-R³³ are each independently unsubstituted or substituted with one, two or three Q¹ substituents.

In other embodiment, each Q¹ substituent is independently substituted with one, two or three Q² substituents.

In certain embodiments, the compounds have formula: 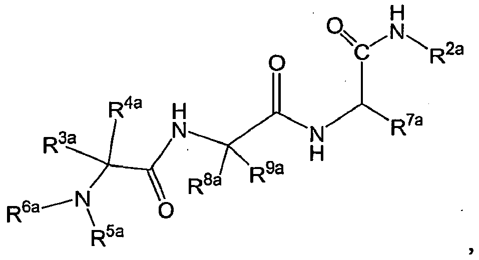 or pharmaceutically acceptable derivatives thereof, where:
R^{2a} is selected from substituted or unsubstituted arylalkyl and substituted or unsubstituted cycloalkyl;
R^{3a}, R^{4a} and R^{5a} are each independently selected from (i) or (ii) as follows:
   (i) R^{3a} and R^{4a} are each independently hydrogen, lower alkyl, lower alkenyl, alkynyl, lower cycloalkyl, lower heterocyclyl; and R^{5a} is selected from hydrogen, alkyl, alkenyl, alkynyl, aryl, heteroaryl, heteroarylium, cycloalkyl, heterocyclyl and NR¹⁵R¹⁶;
   (ii) R^{5a} and R^{3a} or R^{5a} and R^{4a} together with the atoms on which they are substituted form heterocyclic or heteroaryl ring and the other of R^{3a} or R^{4a} is selected as (ii);
R^{6a} is hydrogen, lower alkyl, lower alkenyl or lower alkynyl;
R^{7a} is hydrogen, alkyl, alkenyl, alkynyl, C(A)R29, aryl, heteroaryl, cycloalkyl or heterocyclyl;
R^{8a} and R^{9a} are each independently hydrogen, alkyl, alkenyl, alkynyl, aryl, heteroaryl, cycloalkyl, heterocyclyl; and
R²⁹ is hydrogen, alkyl, alkenyl, alkynyl, aryl, heteroaryl, heteroarylium, cycloalkyl or heterocyclyl,
R^{1a}-R^{9a} are each independently unsubstituted or substituted with one to five substituents, each independently selected from Q¹ and other variables are as defined elsewhere herein.

In other embodiment, R^{1a} is hydrogen; and R^{2a} is selected from aryl, heteroaryl, cycloalkyl, cycloalkylalkyl and heterocyclyl.

In other embodiment, R^{1a} is hydrogen.

In other embodiment, R^{2a} is selected from substituted or unsubstituted aryl, substituted or unsubstituted aralkyl and substituted or unsubstituted cycloalkyl.

In other embodiment, R^{2a} is selected from substituted or unsubstituted aryl and substituted or unsubstituted cycloalkyl.

In other embodiment, R^{2a} is selected from bicyclic aryl and bicyclic cycloalkyl. In other embodiment, R^{2a} is tetrahydronaphthyl.

In certain embodiments, R^{2a} is selected from:

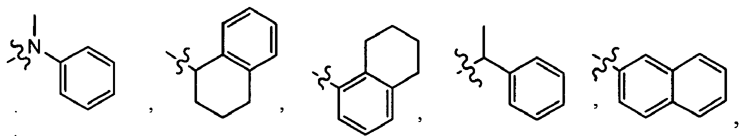

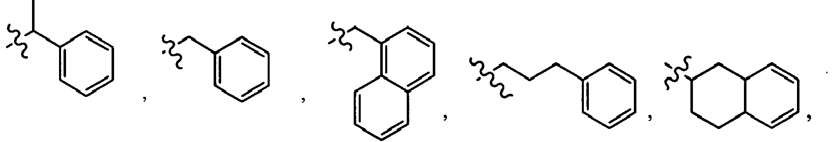

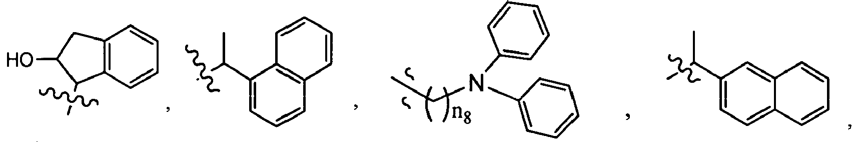

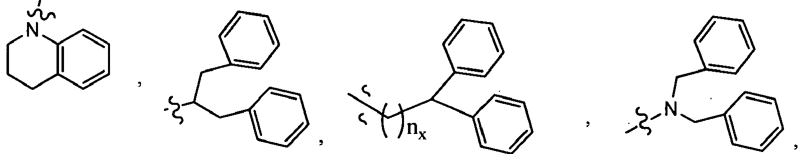

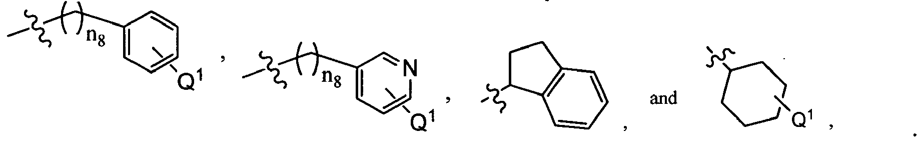

In certain embodiments, R^{2a} is selected from substituted or unsubstituted arylalkyl and substituted or unsubstituted cycloalkyl. In other embodiment, R^{2a} is tetrahydronaphthyl.

In certain embodiments, R^{2a} is selected from:

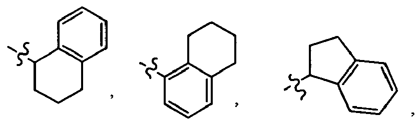

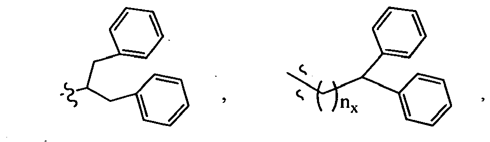

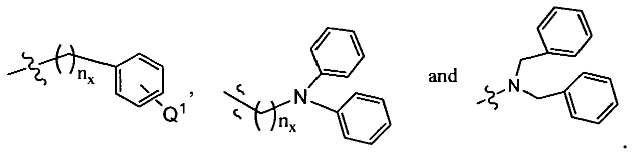

In other embodiment, R^{3a} is hydrogen.

In other embodiment, R^{3a} is lower alkyl.

In other embodiment, R^{3a} is methyl.

In other embodiment, R^{4a} is lower alkyl.

In other embodiment, R^{4a} is methyl.

In other embodiment, R^{5a} is hydrogen, alkyl, cycloalkylalkyl or aralkyl.

In other embodiment, R^{5a} is methyl.

In other embodiment, R^{5a} and R^{3a} or R^{5a} and R^{4a} together with the atoms on which they are substituted form a heterocyclic or heteroaryl ring and other of R^{3a} or R^{4a} is selected from hydrogen and lower alkyl.

In other embodiments, R^{5a} and R^{3a} form an optinally substituted heterocyclic ring selected from:

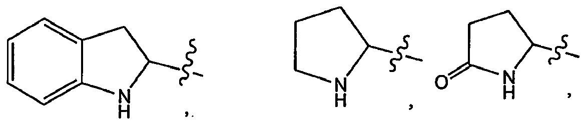

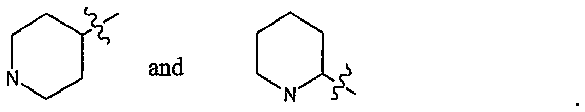

In other embodiments, R^{5a} and R^{3a} form a heterocyclic ring selected from:

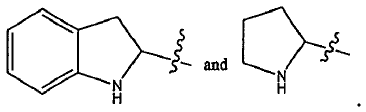

In other embodiment, R^{6a} is hydrogen or lower alkyl.

In other embodiment, R^{6a} is hydrogen or methyl.

In other embodiments, R^{7a} 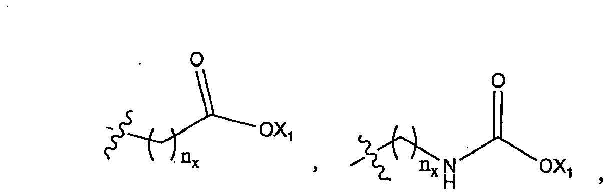 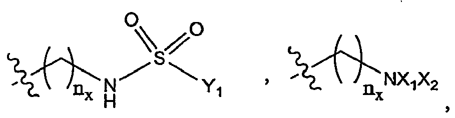 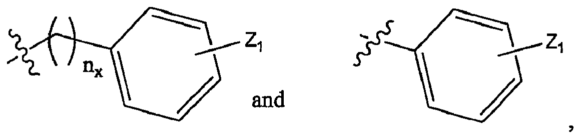 where nx is 1-6, X₁ is selected from hydrogen, alkyl, aryl, aralkyl;
X₂ is selected from hydrogen, alkyl, aryl, aralkyl, COX₃, where X₃ is alkyl or aryl;
Y₁ is alkyl, aryl, aralkyl;
Z₁ hydrogen, halogen, alkyl, alkenyl, alkynyl, haloalkyl, aryl, aralkyl, nitro and COX₃; and
X₁, X₂ and Y₁ can be substituted with one or more, in some embodiments, 1, 2 or 3 substituents selected from Z₁.

In other embodiment, R^{7a} is hydrogen, benzyloxymethyl, benzylthiomethyl, 2-benzyloxyethyl, benzyloxybenzyl, benzyl, 4-aminobutyryl, 3-aminopropyl, 4-benzyloxycarbonylaminobutyryl, 3- benzyloxycarbonylaminopropyl, 4-(benzyloxycarbonylamino)benzyl, 4-(2-chlorobenzyloxycarbonylamino)butyl, 4-(p-tolylsulfonylamino)butyryl, 3-(p-tolylsulfonylamino)propyl, butyryl, 5-benzyloxycarbonylaminopentyl, cyclohexyl, methyl, 4-methylsulfonylaminobutyryl, 4-(4-butyl)-sulfonylaminobutyryl, 4-benzylsulfonylaminobutyryl, 4-trifluoromefhylsulfbnylaminobutyryl, 4-(4-methoxyphenylsulfonylamino)butyryl, 4-(4-nitrophenylsulfonylamino)butyryl, benzyloxycarbonylmethyl, 2-(benzyloxycarbonyl)ethyl, 2-aminoethyl, propyl, 4-(N, N-dimethylamino)butyryl, 4-(N, N-dibutyrylamino)butyryl, 4-acetylaminobutyryl, 2-hydroxyethyl and phenyl.

In other embodiment, R^{7a} is hydrogen, alkyl, aralkylthioalkyl, aralkoxycarbonylaminoalkyl or aralkoxyalkyl.

In certain embodiments, nx is 1, 2, 3, 4 or 5.

In certain embodiments, Y₁ is methyl, butyl, benzyl, tolyl, trifluoromethyl, methoxyphenyl, and nitrophenyl.

In certain embodiments, X₁ is benzyl or chlorobenzyl.

In certain embodiments, X₂ is selected from hydrogen, methyl, butyl, and acetyl.

In certain embodiments, X₃ is alkyl, aryl or aralkyl. In other embodiment, X₃ is methyl.

In other embodiment, R^{8a} is hydrogen or alkyl.

In other embodiment, R^{8a} is hydrogen, isopropyl or tert-butyl.

In other embodiment, R^{9a} is hydrogen or alkyl.

In other embodiment, R^{9a} is hydrogen.

In other embodiment, the compounds provided herein have formula: 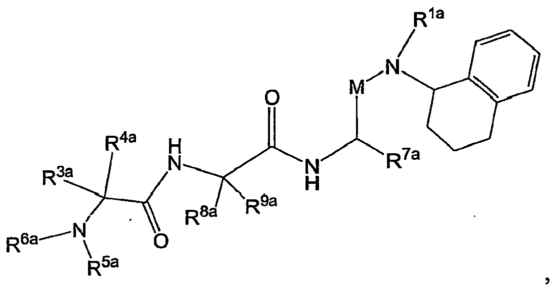 where the variables are as defined elsewhere herein.

In other embodiment, the compounds provided herein have formula: 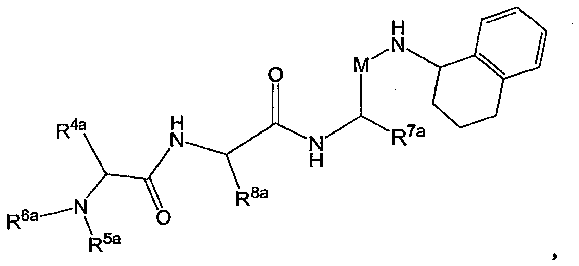 where the variables are as defined elsewhere herein.

In other embodiment, the compounds provided herein have formula: 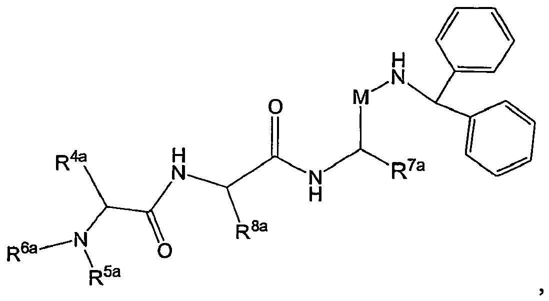 where the variables are as defined elsewhere herein.

In other embodiment, the compounds provided herein have formula: 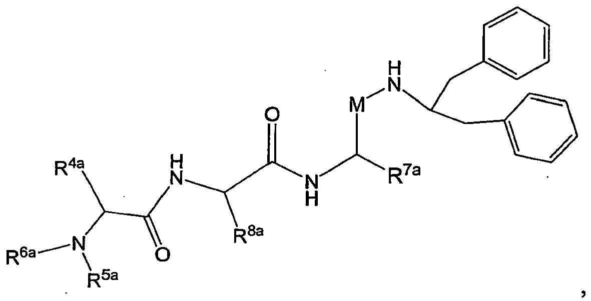 where the variables are as defined elsewhere herein.

Also provided herein are compounds of formula: 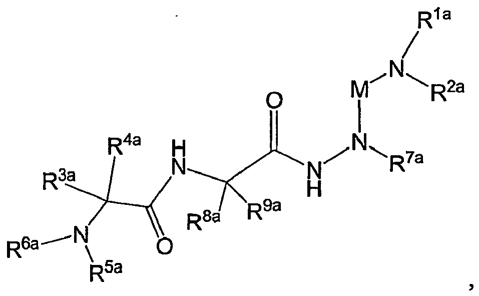 or pharmaceutically acceptable derivatives thereof, where the variables are as described elsewhere herein.

In other embodiment, the compounds provided herein have formula: 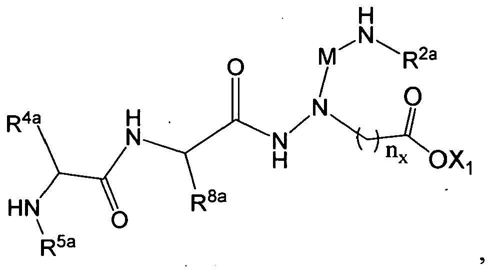 where the variables are as defined elsewhere herein.

In other embodiment, the compounds provided herein have formula:zz 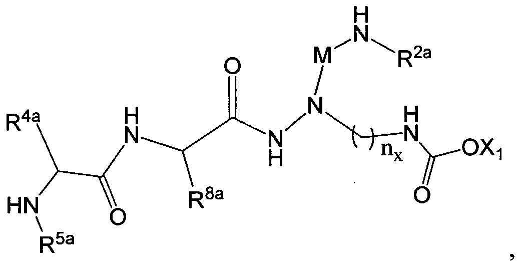 where the variables are as defined elsewhere herein.

In other embodiment, the compounds provided herein have formula: 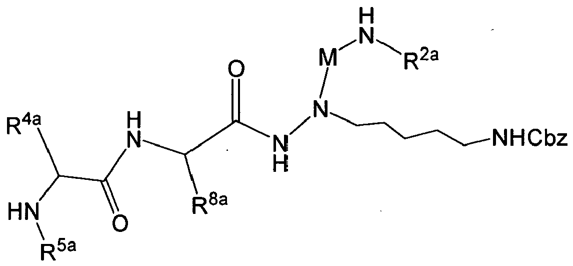 where the variables are as defined elsewhere herein.

In other embodiment, the compounds are selected from Table 1.

**Table 1**

| COMPOUND | EC₅₀ for binding to BIR3 | EC₅₀ for Caspase-9 rescue |
|---|---|---|
| 2-Methylamino-N-{2-oxo-1-[(1,2,3,4-tetrahydro-naphthalen-1-ylcarbamoyl)-methyl]-azepan-3-yl}-propionamide; compound with trifluoro-acetic acid | B | C |
| 5-(2-Methylamino-propionylamino)-4-oxo-1,2,4,5,6,7-hexahydro-azepino[3,2,1-hi]indole-2-carboxylic 'acid (1,2,3,4-tetrahydro-naphthalen-1-yl)-amide | A | B |
| 1-[2-(2-Isobutylamino-propionylamino)-3,3-dimethyl-butyryl]-pyrrolidine-2-carboxylic acid (1,2,3,4-tetrahydro-naphthalen-1-yl)-amide | A | B |
| 3,3-Dimethyl-2-(2-methylamino-propionylamino)-N-phenethyl-N-[(1,2,3,4-tetrahydro-naphthalen-1-ylcarbamoyl)-methyl]-butyramide TFA salt | B | C |
| 1-{2-[2-(Cyclopropylmethyl-amino)-propionylamino]-3,3-dimethyl-butyryl}-pyrrolidine-2-carboxylic acid (1,2,3,4-tetrahydro-naphthalen-1-yl)-amide | A | B |
| 3-[3-Methyl-2-(2-methylamino-propionylamino)-butyryl]-thiazolidine-4-carboxylic acid (1,2,3,4-tetrahydro-naphthalen-1-yl)-amide | A | A |
| 1-[3,3-Dimethyl-2-(2-propylamino-propionylamino)-butyryl]-pyrrolidine-2-carboxylic acid (1,2,3,4-tetrahydro-naphthalen-1-yl)-amide | A | B |
| N-{2,2-Dimethyl-1-[2-(N'-methyl-N'-phenyl-hydrazinocarbonyl)-pyrrolidine-1-carbonyl]-propyl}-2-methylamino-propionamide | A | B |
| 6-(2-Methylamino-propionylamino)-5-oxo-octahydro-pyrrolo[1,2-a]azepine-3-carboxylic acid (1,2,3,4-tetrahydro-naphthalen-1-yl)-amide | A | A |
| 3-[3-Methyl-2-(2-methylamino-propionylamino)-butyryl]-oxazolidine-4-carboxylic acid (1,2,3,4-tetrahydro-naphthalen-1-yl)-amide | A | A |
| 1-[3-Methyl-2-(2-methylamino-propionylamino)-butyryl]-octahydro-indole-2-carboxylic acid (1,2,3,4-tetrahydro-naphthalen-1-yl)-amide | A | A |
| 3-[3-Methyl-2-(2-methylamino-propionylamino)-butyryl]-thiazolidine-2-carboxylic acid (1,2,3,4-tetrahydro-naphthalen-1-yl)-amide | A | A |
| {5-(2-Methylamino-propionylamino)-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester | A | A |
| 3-[3-Methyl-2-(2-methylamino-propionylamino)-butyryl]-1,1-dioxo-116-thiazolidine-4-carboxylic acid (1,2,3,4-tetrahydro-naphthalen-1-yl)-amide | A | A |
| 2-[3-Methyl-2-(2-methylamino-propionylamino)-butyryl]-2,3-dihydro-1H-isoindole-1-carboxylic acid (1,2,3,4-tetrahydro-naphthalen-1-yl)-amide (1,2,3,4-tetrahydro-naphthalen-1-yl)-amide | A | A |
| [5-{Methyl-[3-methyl-2-(2-methylamino-propionylamino)-butyryl]-amino}-5-(1,2,3,4-tetrahydro-naphthalen-1-ylcarbamoyl)-pentyl]-carbamic acid benzyl ester | A | B |
| 8-(2-Methylamino-propionylamino)-9-oxo-hexahydro-pyrazolo[1,2-a][1,2]diazepine-1-carboxylic acid (1,2,3,4-tetrahydro-naphthalen-1-yl)-amide | A | B |
| 2-[3-Methyl-2-(2-methylamino-propionylamino)-butyryl]-1,2,3,4-tetrahydro-isoquinoline-3-carboxylic acid (1,2,3,4-tetrahydro-naphthalen-1-yl)-amide | A | A |
| N-[2-Benzyloxy-1-(1,2,3,4-tetrahydro-naphthalen-1-ylcarbamoyl)-ethyl]-3-methyl-2-(2-methylamino-propionylamino)-butyramide | A | B |
| 1-[3,3-Dimethyl-2-(2-methylamino-propionylamino)-butyryl]-pyrrolidine-2-carboxylic acid benzhydryl-amide | A | A |
| 3-[3-Methyl-2-(2-methylamino-propionylamino)-butyryl]-thiazolidine-4-carboxylic acid benzhydryl-amide | A | A |
| 3-[3-Methyl-2-(2-methylamino-propionylamino)-butyryl]-thiazolidine-4-carboxylic acid (1-benzyl-2-phenyl-ethyl)-amide | A | A |
| N-{1-[4-(N',N'-Diphenyl-hydrazinocarbonyl)-thiazolidine-3-carbonyl]-2-methyl-propyl}-2-methylamino-propionamide | A | B |
| N-{1-[4-(N',N'-Dibenzyl-hydrazinocarbonyl)-thiazolidine-3-carbonyl]-2-methyl-propyl}-2-methylamino-propionamide | A | A |
| 5,5-Dimethyl-3-[3-methyl-2-(2-methylamino-propionylamino)-butyryl]-thiazolidine-4-carboxylic acid (1,2,3,4-tetrahydro-naphthalen-1-yl)-amide | A | A |
| 2-[3-Methyl-2-(2-methylamino-propionylamino)-butyryl]-1,2,3,4-tetrahydro-isoquinoline-3-carboxylic acid benzhydryl-amide | A | B |
| 2-[3-Methyl-2-(2-methylamino-propionylamino)-butyryl]-1,2,3,4-tetrahydro-isoquinoline-3-carboxylic acid (1-benzyl-2-phenyl-ethyl)-amide | A | B |
| 2-[3-Methyl-2-(2-methylamino-propionylamino)-butyryl]-1,2,3,4-tetrahydro-isoquinoline-3-carboxylic acid (1-benzyl-2-phenyl-ethyl)-amide | A | B |
| [5-[3-Methyl-2-(2-methylamino-propionylamino)-butyrylamino]-5-(1,2,3,4-tetrahydro-naphthalen-1-ylcarbamoyl)-pentyl]-carbamic acid benzyl ester | A | A |
| 1-[3,3-Dimethyl-2-(2-methylamino-propionylamino)-butyryl]-pyrrolidine-2-carboxylic acid (3,3-diphenyl-propyl)-amide | B | C |
| N-{1-[2-(N',N'-Dibenzyl-hydrazinocarbonyl)-pyrrolidine-1-carbonyl]-2,2-dimethyl-propyl}-2-methylamino-propionamide | A | B |
| N-{1-[3-(N',N'-Diphenyl-hydrazinocarbonyl)-3,4-dihydro-1H-isoquinoline-2-carbonyl]-2-methyl-propyl}-2-methylamino-propionamide | C | C |
| 7-[3-Methyl-2-(2-methylamino-propionylamino)-butyryl]-1,4-dithia-7-aza-spiro[4.4]nonane-8-carboxylic acid (1,2,3,4-tetrahydro-naphthalen-1-yl)-amide | A | B |
| [4-[3-Methyl-2-(2-methylamino-propionylamino)-butyrylamino]-4-(1,2,3,4-tetrahydro-naphthalen-1-ylcarbamoyl)-butyl]-carbamic acid benzyl ester | A | A |
| 6-Amino-2-[3-methyl-2-(2-methylamino-propionylamino)-butyrylamino]-hexanoic acid (1,2,3,4-tetrahydro-naphthalen-1-yl)-amide | A | A |
| [3-[3-Methyl-2-(2-methylamino-propionylamino)-butyrylamino]-3-(1,2,3,4-tetrahydro-naphthalen-1-ylcarbamoyl)-propyl]-carbamic acid benzyl ester | A | B |
| 8-(2-Methylamino-propionylamino)-5,9-dioxo-hexahydro-pyrazolo[1,2-a][1,2]diazepine-1-carboxylic acid (1,2,3,4-tetrahydro-naphthalen-1-yl)-amide | A | B |
| [5-[3-Methyl-2-(2-methylamino-propionylamino)-butyrylamino]-5-(1,2,3,4-tetrahydro-naphthalen-1-ylcarbamoyl)-pentyl]-carbamic acid 2-chloro-benzyl ester | A | A |
| 2-[3-Methyl-2-(2-methylamino-propionylamino)-butyrylamino]-6-(toluene-4-sulfonylamino)-hexanoic acid (1,2,3,4-tetrahydro-naphthalen-1-yl)-amide | A | A |
| 2-[3-Methyl-2-(2-methylamino-propionylamino)-butyrylamino]-hexanoic acid (1,2,3,4-tetrahydro-naphthalen-1-yl)-amide | A | B |
| 5-Amino-2-[3-methyl-2-(2-methylamino-propionylamino)-butyrylamino]-pentanoic acid (1,2,3,4-tetrahydro-naphthalen-1-yl)-amide | A | B |
| (5-{[3-Methyl-2-(2-methylamino-propionylamino)-butyryl]-[(1,2, 3,4-tetrahydro-naphthalen-1-ylcarbamoyl)-methyl]-amino}-pentyl)-carbamic acid benzyl ester | A | B |
| N-[3-Benzyloxy-1-(1,2,3,4-tetrahydro-naphthalen-1-ylcarbamoyl)-propyl]-3-methyl-2-(2-methylamino-propionylamino)-butyramide | A | B |
| 3-Methyl-2-(2-methylamino-propionylamino)-N-[1-(1,2,3,4-tetrahydro-naphthalen-1-ylcarbamoyl)-ethyl]-butyramide | A | A |
| N-[Cyclohexyl-(1,2,3,4-tetrahydro-naphthalen-1-ylcarbamoyl)-methyl]-3-methyl-2-(2-methylamino-propionylamino)-butyramide | A | A |
| N-{3,7-Dioxo-1-pentyl-2-[(1,2,3,4-tetrahydro-naphthafen-1-ylcarbamoyf)-methyl]-[1,2]diazepan-4-yl}-2-methylamino-propionamide | B | C |
| 6-Methanesulfonylamino-2-[3-methyl-2-(2-methylamino-propionylamino)-butyrylamino]-hexanoic acid (1,2,3,4-tetrahydro-naphthalen-1-yl)-amide | A | A |
| 2-[3-Methyl-2-(2-methylamino-propionylamino)-butyrylamino]-6-phenylmethanesulfonylamino-hexanoic acid (1,2,3,4-tetrahydro-naphthalen-1-yl)-amide | A | A |
| 2-[3-Methyl-2-(2-methylamino-propionylamino)-butyrylamino]-6-trifluoromethanesulfonylamino-hexanoic acid (1,2,3,4-tetrahydro-naphthalen-1-yl)-amide | A | C |
| 6-(4-Methoxy-benzenesulfonylamino)-2-[3-methyl-2-(2-methylamino-propionylamino)-butyrylamino]-hexanoic acid (1,2,3,4-tetrahydro-naphthalen-1-yl)-amide | A | A |
| 2-[3-Methyl-2-(2-methylamino-propionylamina)-butyrylamino]-6-(4-nitro-benzenesulfonylamino)-hexanoic acid (1,2,3,4-tetrahydro-naphthalen-1-yl)-amide | A | A |
| 6-(Butane-1-sulfonylamino)-2-[3-methyl-2-(2-methylamino-propionylamino)-butyrylamino]-hexanoic acid (1,2,3,4-tetrahydro-naphthalen-1-yl)-amide | A | A |
| 4-[3-Methyl-2-(2-methylamino-propionylamino)-butyryl]-thiomorpholine-3-carboxylic acid (1,2,3,4-tetrahydro-naphthalen-1-yl)-amide | A | B |
| (S)-2-((S)-N,3-dimethyl-2-((S)-2-(methylamino)propanamido)butanamido)-N-((R)-1,2,3,4-tetrahydronaphthalen-1-yl)-5-(3-Htosylguanidino)pentanamide; 2,2,2-trifluoroacetic acid salt | A | A |
| 3-[3-Methyl-2-(2-methylamino-propionylamino)-butyrylamino]-N-(1,2,3,4-tetrahydro-naphthalen-1-yf)-succinamic acid benzyl ester | A | B |
| 4-[3-Methyl-2-(2-methylamino-propionylamino)-butyrylamino]-4-(1,2,3,4-tetrahydro-naphthalen-1-ylcarbamoyl)-butyric acid benzyl ester | A | B |
| N-[3-Amino-1-(1,2,3,4-tetrahydro-naphthalen-1-ylcarbamoyl)-propyl]-3-methyl-2-(2-methylamino-propionylamino)-butyramide | A | B |
| N-[3-Hydroxy-1-(1,2,3,4-tetrahydro-naphthalen-1-ylcarbamoyl)-propyl]-3-methyl-2-(2-methylamino-propionylamino)-butyramide | A | B |
| 6-Dimethylamino-2-[3-methyl-2-(2-methylamino-propionylamino)-butyrylamino]-hexanoic acid (1,2,3,4-tetrahydro-naphthalen-1-yl)-amide | A | A |
| 3-[3-Methyl-2-(2-methylamino-propionylamino)-butyryl]-thiazolidine-4-carboxylic acid indan-1-yfamide | A | A |
| 6-Acetylamino-2-[3-methyl-2-(2-methylamino-propionylamino)-butyrylamino]-hexanoic acid (1,2,3,4-tetrahydro-naphthalen-1-yl)-amide | A | B |
| 3-Methyl-2-(2-methylamino-propionylamino)-N-[phenyl-(1,2,3,4-tetrahydro-naphthalen-1-ylcarbamoyl)-methyl]-butyramide | A | A |
| 3-Methyl-2-(2-methylamino-propionylamino)-N-[(1,2,3,4-tetrahydro-naphthalen-1-ylcarbamoyl)-methyl]-butyramide | B | B |
| 6-Dibutylamino-2-[3-methyl-2-(2-methylamino-propionylamino)-butyrylamino]-hexanoic acid (1,2,3,4-tetrahydro-naphthalen-1-yl)-amide | A | A |
| 3-Methyl-2-(2-methylamino-propionylamino)-N-[2-phenyl-1-(1,2,3,4-tetrahydro-naphthalen-1-ylcarbamoyl)-ethyl]-butyramide | B | C |
| 3-[3-Methyl-2-(2-methylamino-propionylamino)-butyrylamino]-N-(1,2,3,4-tetrahydro-naphthalen-1-yl)-succinamic acid | NC | NC |
| 4-[3-Methyl-2-(2-methylamino-propionylamino)-butyrylamino]-4-(1,2,3,4-tetrahydro-naphthalen-1-ylcarbamoyl)-butyric acid | NC | NC |

Average EC₅₀ of the compounds provided herein for relief of BIR3-mediated Caspase-9 inhibition provided in Table 1 are as follows: A < 1 µM, B = 1-10 µM, C >10 µM, and NC = not calculated or inactive. Average EC₅₀ of the compounds provided herein for binding to BIR3 domain of rat XIAP provided in Table 1 are as follows: A < 1 µM, B = 1-10 µM, C >10 µM, and NC = not calculated or inactive.

### C. Preparation of the compounds

Starting materials in the synthesis examples provided herein are either available from commercial sources or via literature procedures. All commercially available compounds were used without further purification unless otherwise indicated. HPLC was performer on a Zorbax C8 4.6x150mm column, eluting with a gradient of 10% to 50% acetonitrile in water containing 0.1 % TFA over 20 minutes.

The following illustrations depict general preparations of compounds claimed herein and consist of reactions typically known to one skilled in the art of chemical synthesis. The substituents refered in the schemes are described elsewhere herein. Also it will be apparent to one skilled in the art that many of the products could exist as one or more isomers, that is E/Z isomers, enantiomers and/or diastereomers.

The coupling reactions carried out in the following schemes are performed in the presence of a standard peptide coupling reagent such as the combination of dicyclohexylcarbodiimide (DCC) and 1-hydroxy-benzotriazole (HOBt), as well as the BOP (benzotrazolyloxy-tris-(dimethylamino)phosphonium hexaflurophosphate) reagent, pyBOP (benzotrazolyl-tris(N-pyrolidinyl(phosphoniumhexafluorophosphate), TU (O-benzotrazolyly-tetramethylisouronium-hexafluorophosphate), HATU (O-(7-azabenzotriazol-1-yl)-N,N,N',N'-tetramethyluronium hexafluorophosphate), HBTU (O-benzotriazol-1-yl-N, N,N',N'-tetramethyluronium hexafluorophosphate) EEDQ (1-ethyloxycarbonyl-2-ethyloxy-1,2-dihydroquinoline), and T3P (propane phosphonic acid anhydride) reagents, the combination of 1-ethyl(3,3'-dimethyl-1'-aminopropyl)carbodiimide (EDCI) and HOBt, and the like, as discussed in J. Jones, Amino Acid and Peptide Synthesis, Steven G. Davis ed., Oxford University Press, Oxford, pp. 25-41 (1992); M. Bodanzsky, Principles of Peptide Synthesis, Hafnee et al. ed., Springer-Verlag, Berlin Heidelberg, pp. 9-58 and pp. 202-251 (1984); M. Bodanzsky, Peptide Chemistry, A Practical Textbook, Springer-Verlag, Berlin Heidelberg, pp. 55-73 and pp. 129-180; and H Wissman, H.J. Kleiner, Angew Chem Int. Ed., 1980, 19, 133ff., V. Brandmeier, M. Feigel, M. Bremer, Angew. Chem. Int. Ed., 1997, 36, 486-488, and H Wissman, Phosphorus and Sulfur, 1987, 30, 645-648, all of which are herein incorporated by reference.

Compounds of formula **6-9** are synthesized from readily available starting materials using conventional techniques as discussed below in Scheme 1. A suitably N-protected proline **1** or proline analog is coupled using a standard peptide coupling reaction to an amine or hydrazine to obtain compound **2**. The proline nitrogen protecting group is removed, and the compound is then coupled to nitrogen protected compound **2a**. This nitrogen protecting group is removed, and the resulting compound **4** is coupled to the compound **4a**. Final nitrogen deprotection of compound **5** yields compound of Formula **6**. Alternatively, the proline analog could be coupled to an N-protected-N-methyl dipeptide of choice.

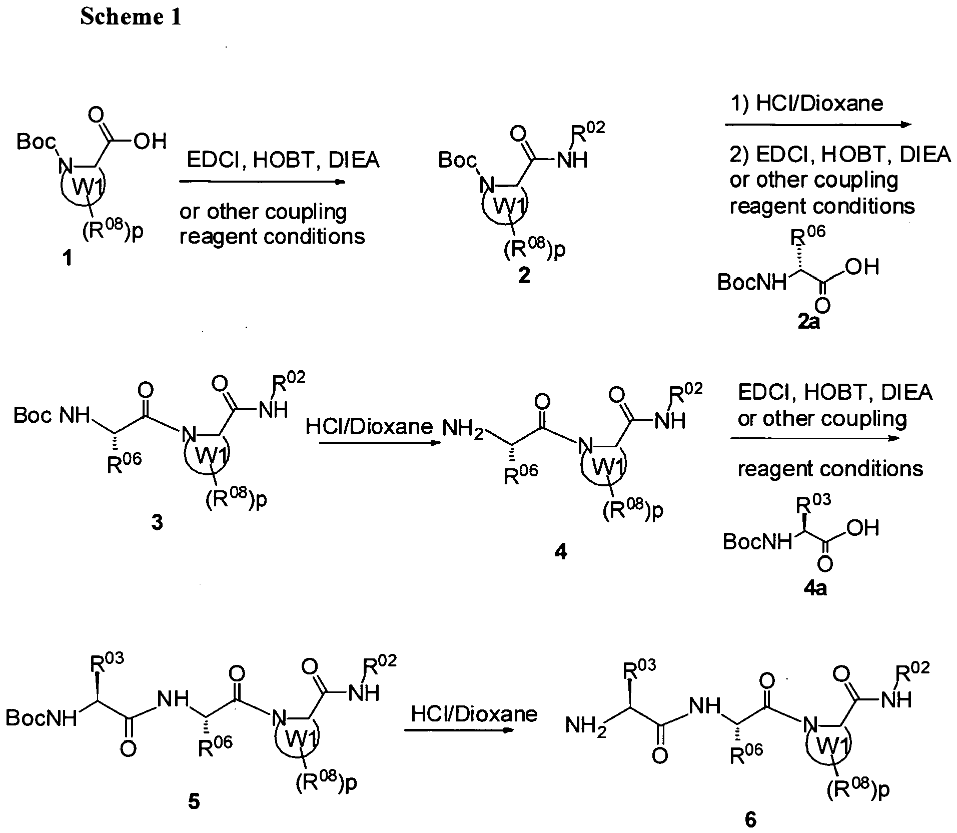

N-terminus analogues of Formula **7-9** could be prepared by reductive amination, acylation or sulfonamide formation of compound 6 leading to compounds of formula **7-9**.

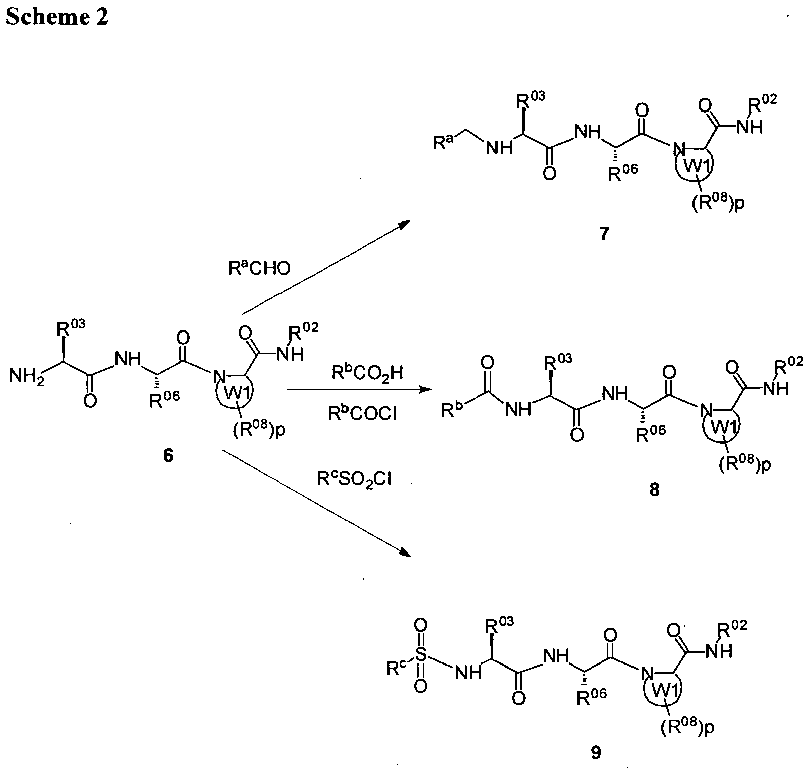

Hydrazide analog (compound **10**) of compound **6** can be prepared as follows:

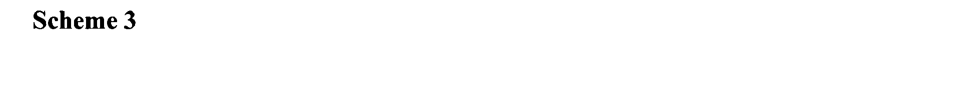

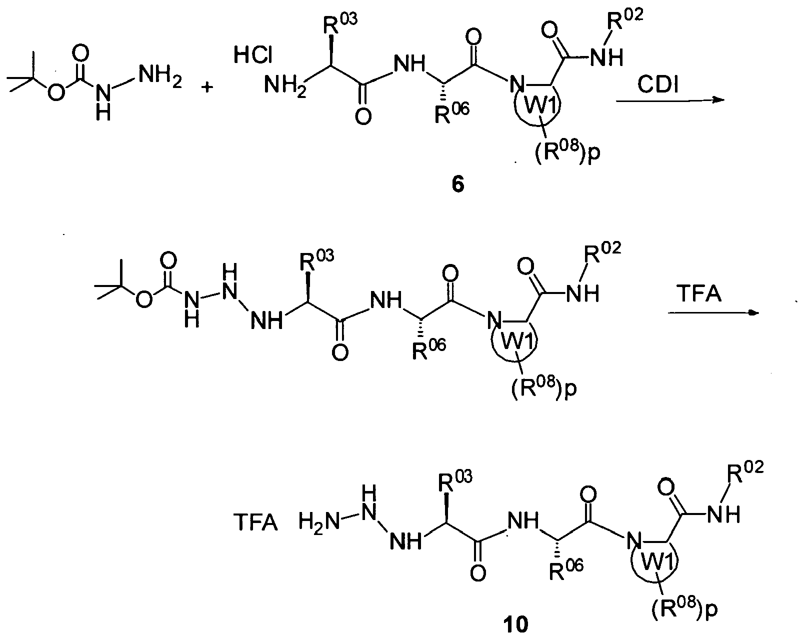

Compounds of formula **14** are synthesized from readily available starting materials using reaction sequence depicted below in Scheme 4. A suitably protected proline analog **11**, such as benzyl ester, is coupled using a standard peptide coupling reaction to the N-protected amino acid **2a** of choice to obtain compound **12**. The nitrogen protecting group is removed, and the resulting compound is coupled to the N-protected-N-methyl amino acid **5a** of choice. The benzyl ester is removed by hydrogenolysis, and the resulting carboxylic acid is coupled to the amine or hydrazine of choice. Final nitrogen deprotection yields compounds of Formula **14**. Alternatively, the proline analog could be coupled to an N-protected-N-methyl dipeptide of choice.

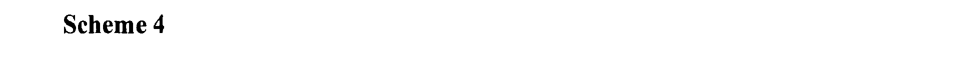

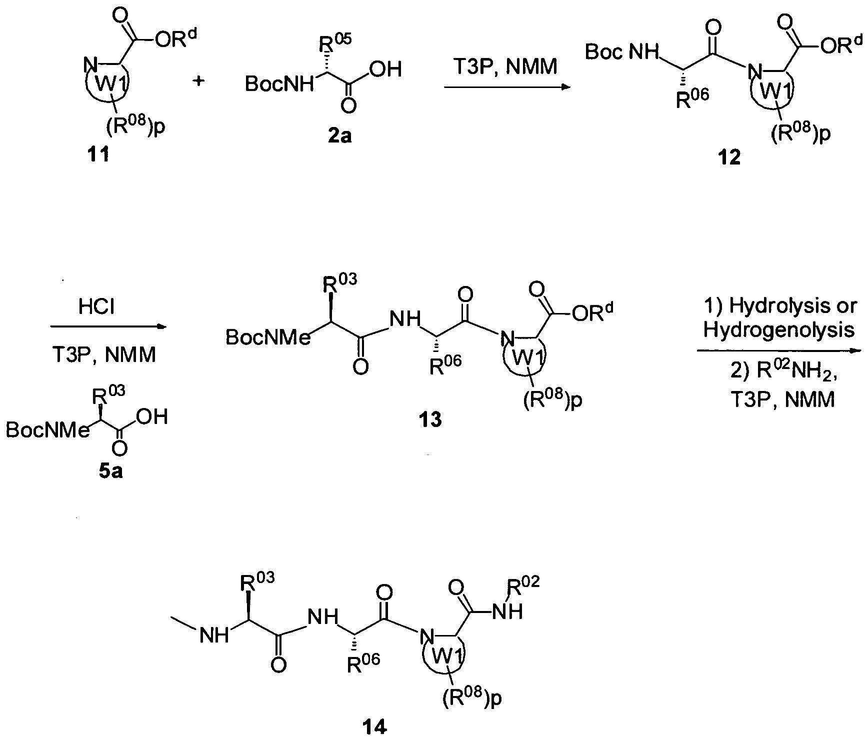

Tricyclic compounds of formula **16** is prepared by coupling the suitably protected N 5-Amino-4-oxo-1,2,4,5,6,7-hexahydro-azepino[3,2,1-hi]indole-2-carboxylic acid with the amine, hydrazine, or amino acid of choice using standard peptide coupling conditions. The nitrogen protecting group is removed, and the resulting compound is coupled to the N-protected-N-methyl amino acid of choice. Final nitrogen deprotection yields compounds of Formula **16**.

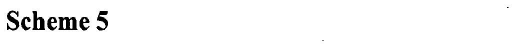

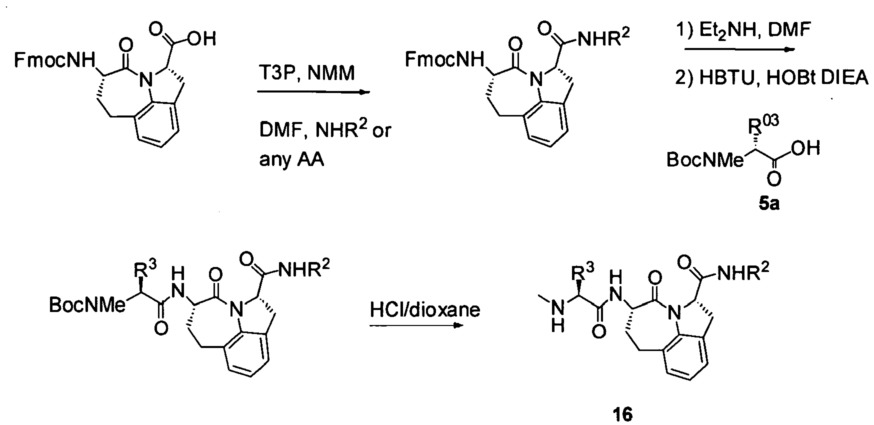

Lactam analogues of Formula **21** are synthesized from readily available starting materials using the methods shown in Scheme 6. A suitably N-protected lactam **17** is coupled using a standard peptide coupling conditions to the amine or hydrazine of choice. The nitrogen protecting group is removed, and the resulting compound **19** is coupled to the N-protected-N-methyl amino acid of choice. Final nitrogen deprotection yields compounds of Formula **21**.

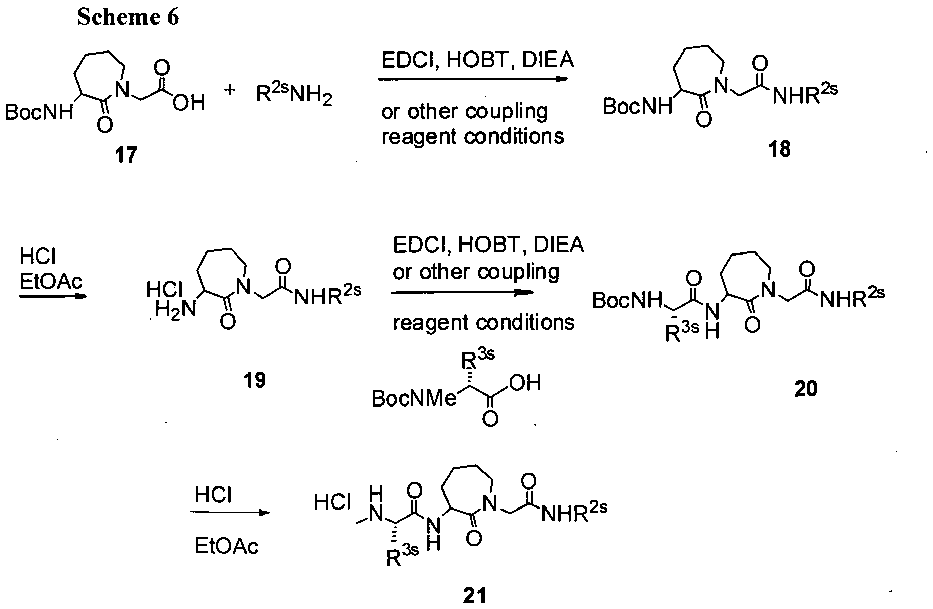

Compounds of Formula **26** are synthesized from readily available starting materials using the methods shown in Scheme 7. A suitably N-protected lactam is coupled using a standard peptide coupling conditions to the amine or hydrazine of choice. The nitrogen protecting group is removed, and the resulting compound **24** is coupled to the N-protected-N-methyl amino acid **5a** of choice. Final nitrogen deprotection yields compounds of Formula **26**.

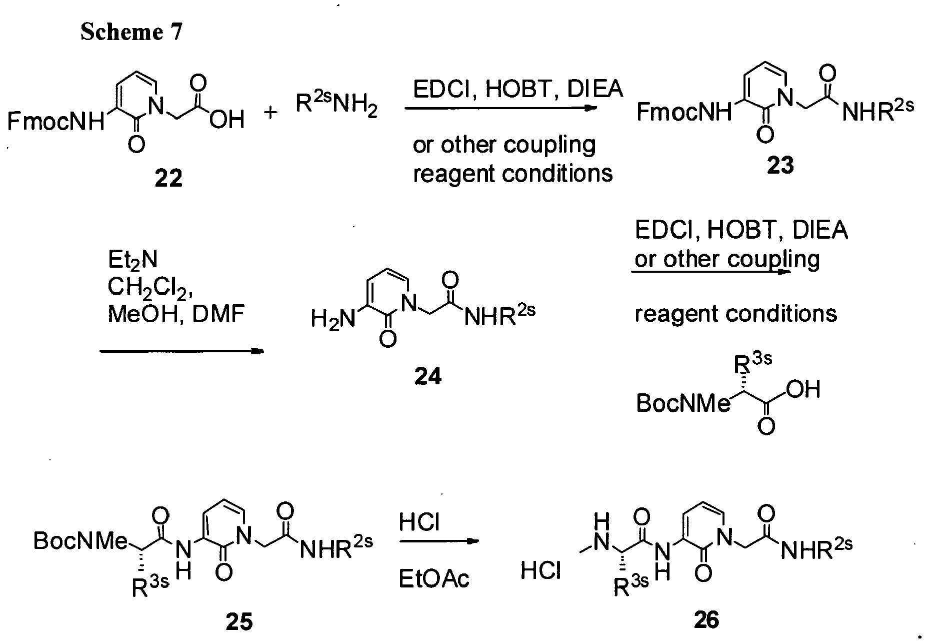

Compounds of the Formula **27** are prepared according to the methods outlined in Scheme 8. A suitably protected glutamic acid derivative can be reduced to an alcohol and subsequently oxidized under mild conditions to the corresponding aldehyde. Condensation with cysteine under basic conditions yields the 6-tert-Butoxycarbonylamino-5-oxo-hexahydro-thiazolo[3,2-a]pyridine-3-carboxylic acid. The terminal carboxylic acid is then coupled to the amine or hydrazine of choice using standard coupling conditions, followed by N-terminal nitrogen deprotection. The resulting compound is coupled to the N-protected-N-methyl amino acid of choice. Final nitrogen deprotection yields compounds of Formula **27**.

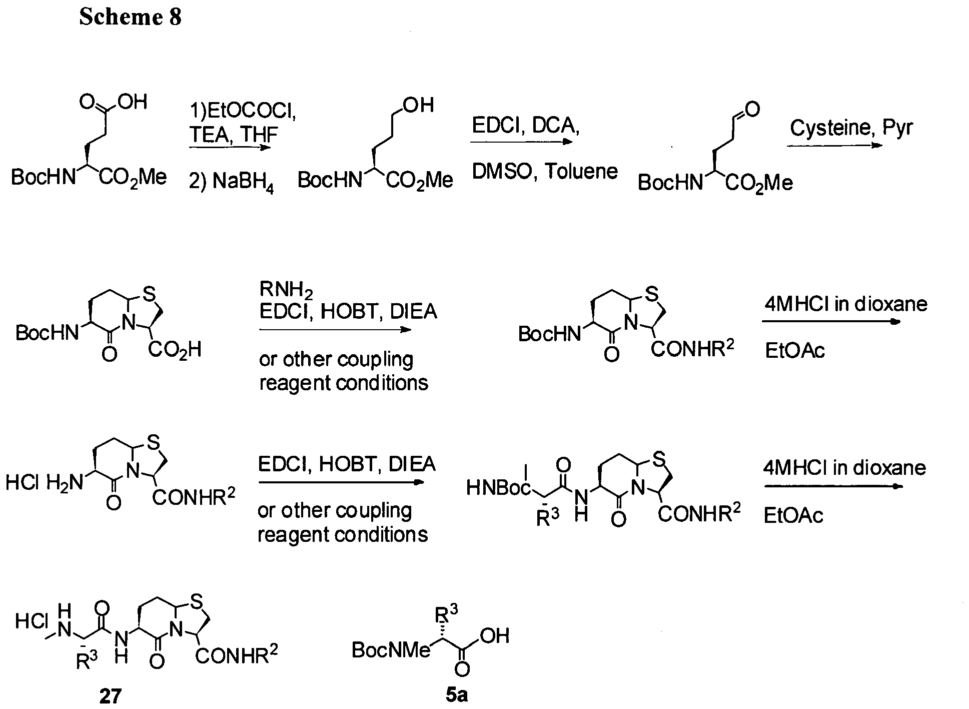

Compounds of the Formula **28** may be prepared according to the methods outlined in Scheme 9. A suitably protected pyroglutamic acid derivative is ring-opened using a vinyl grignard reaction yielding a gamma vinyl ketone glutamic acid derivative. The ketone is reduced, mesylated and cyclized to yield the protected 5-vinyl-pyrrolidine. The nitrogen protecting group is removed and the pyrrolidine coupled using standard coupling conditions to a suitably protected allyl glycine derivative. Olefin metathesis allows for ring formation. The ester is then deprotected and coupled to the amine or hydrazine of choice employing standard coupling conditions. The nitrogen protecting group is then removed, and the resulting compound is coupled to the N-protected-N-methyl amino acid of choice. Final nitrogen deprotection yields compounds of Formula **28**.

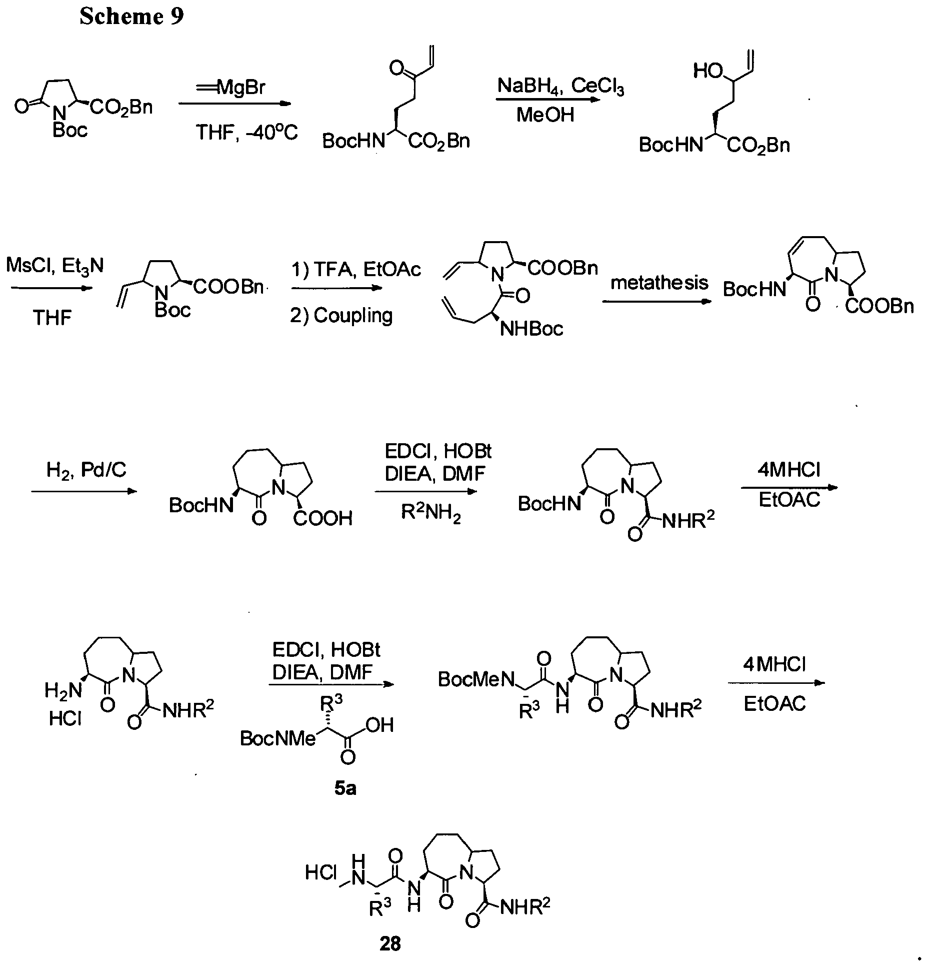

Compounds of the Formula **29** may be prepared according to the methods outlined in Scheme 10. A suitably protected ornithine derivative can be deprotected and concomitantly cyclized with a glyoxal derivative. The resulting lactam is coupled using a standard peptide coupling conditions to an amine or hydrazine of choice. The nitrogen protecting group is removed, and the resulting compound is coupled to the N-protected-N-methyl amino acid of choice. Final nitrogen deprotection yields compounds of Formula **29**.

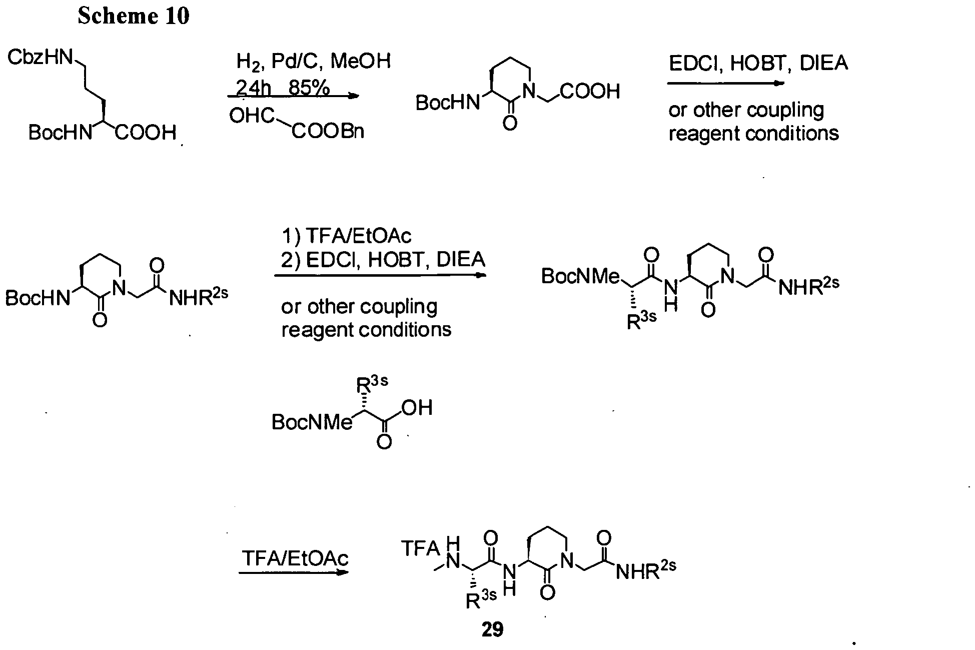

Compounds of the Formula **30** may be prepared according to the methods outlined in Scheme 11. A suitably protected methionine derivative can be coupled to glycine methyl ester employing standard coupling conditions. The sulfur is then methylated, followed by deprotonation of the glycine nitrogen causes cyclization to the lactam and concomitant hydrolysis of the methyl ester. This lactam acid is coupled using a standard peptide coupling conditions to an amine or hydrazine of choice. The nitrogen protecting group is removed, and the resulting compound is coupled to the N-protected-N-methyl amino acid of choice. Final nitrogen deprotection yields compounds of Formula **30**.

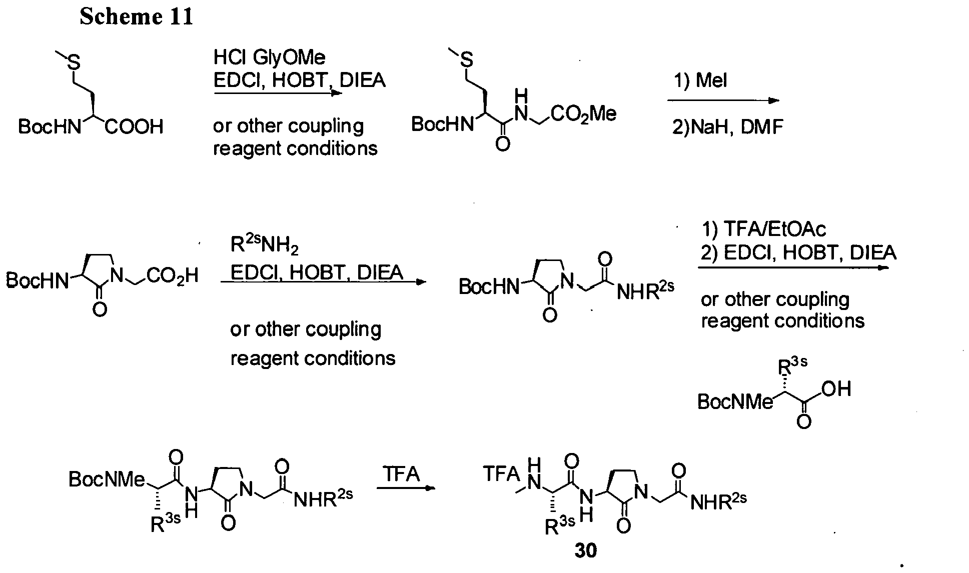

Compounds of the Formula **31** are prepared according to the methods outlined in Schemes 12 and 13 (see, Liu, et al., "Constrained peptidomimetics: building bicyclic analogs of pyrazoline derivatives", Tetrahedron, 2003, 59:8515-8523; Carreira et al., "Asymmetric dipolar cycloadditions of Me3SiCHN2 Synthesis of a novel class of amino acids: Azaprolines", J. Am. Chem. Soc., 1997, 119: 8379-8380). A commercially available chiral auxiliary is acylated, then treated with trimethylsilyldiazomethane in a dipolar cycloaddition reaction, which after further treatment with acid yields the Δ²-pyrazoline. Reduction, carbamate protection, and subsequent chiral auxillary cleavage yields the Δ²-pyrazoline-5-carboxylic acid ester.

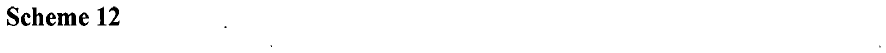

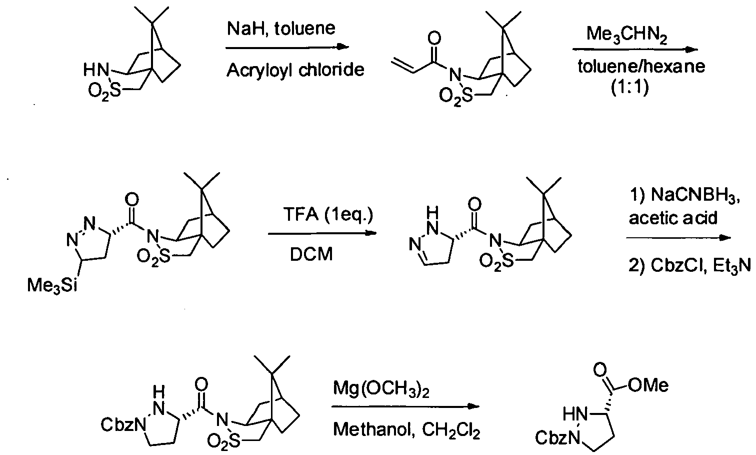

The Δ²-pyrazoline-5-carboxylic acid ester is coupled to a phthalyl-protected allyl glycine acid chloride under Schotten-Baumann conditions. Hydroboration and mild oxidation yields an aldehyde intermediate that cyclizes to the 7-5 bicyclic system upon deprotection of the pyrazoline. The nitrogen protecting group is then removed, and the resulting compound is coupled to the N-protected-N-methyl amino acid of choice. The carboxylic acid ester is hydrolyzed and coupled to the amine or hydrazine of choice employing standard coupling conditions. Final nitrogen deprotection yields compounds of Formula **31**.

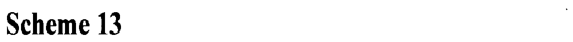

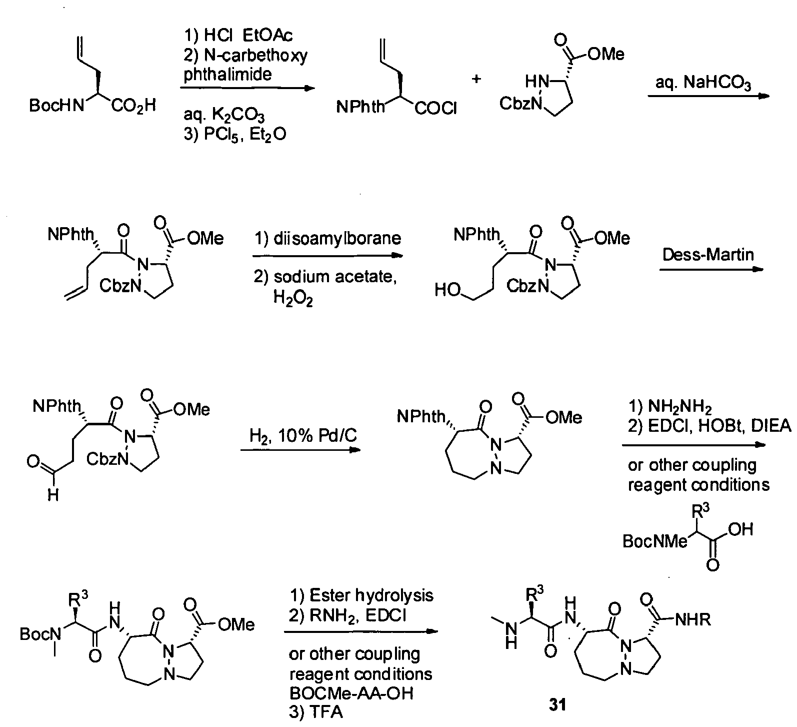

The following reactions schemes illustrate preparation of further compounds provided herein: 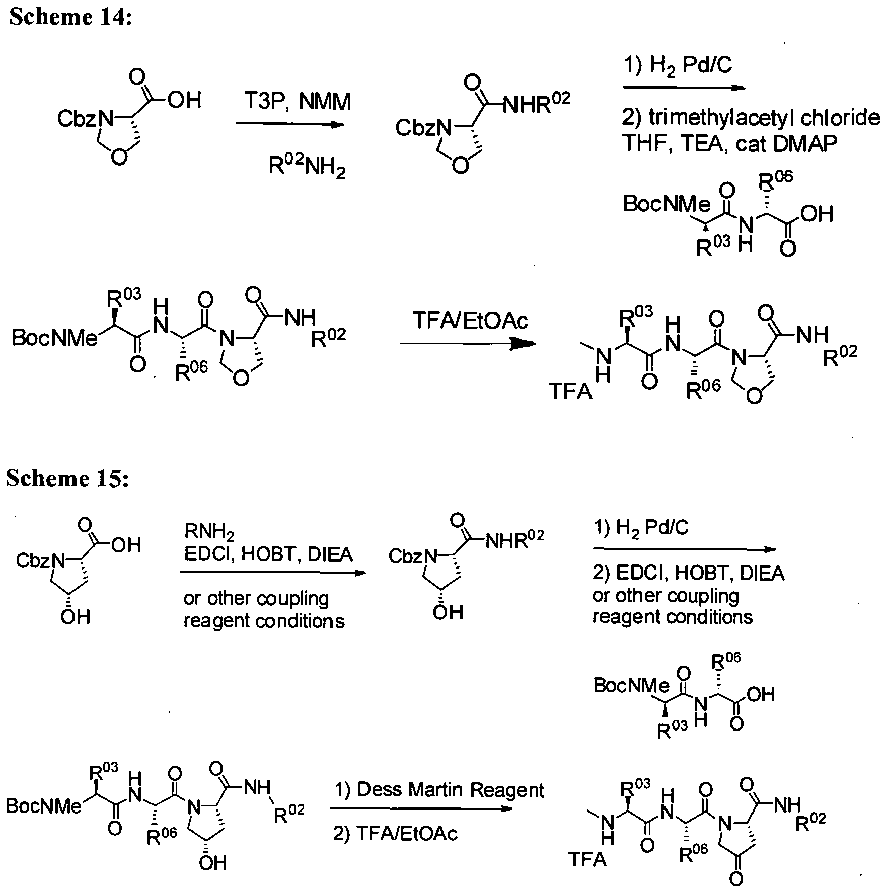 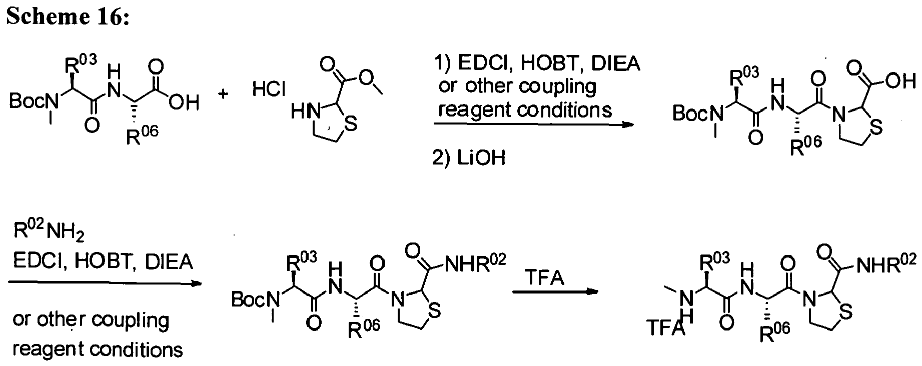 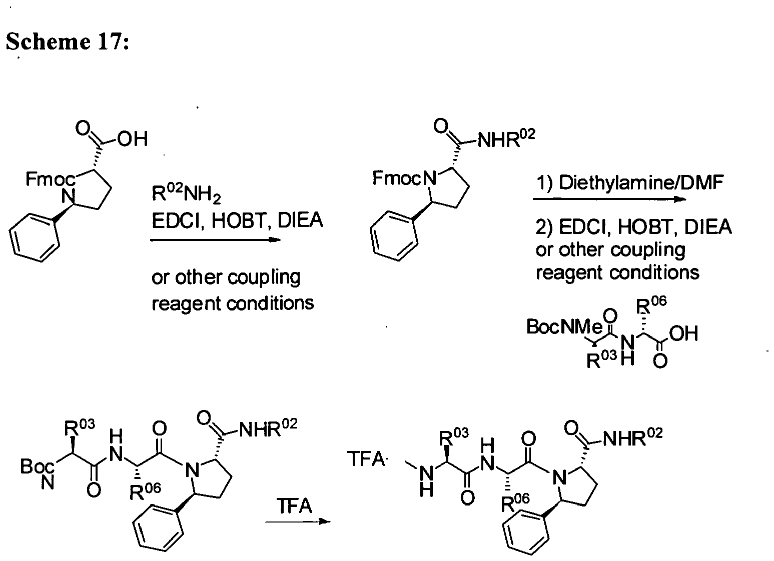 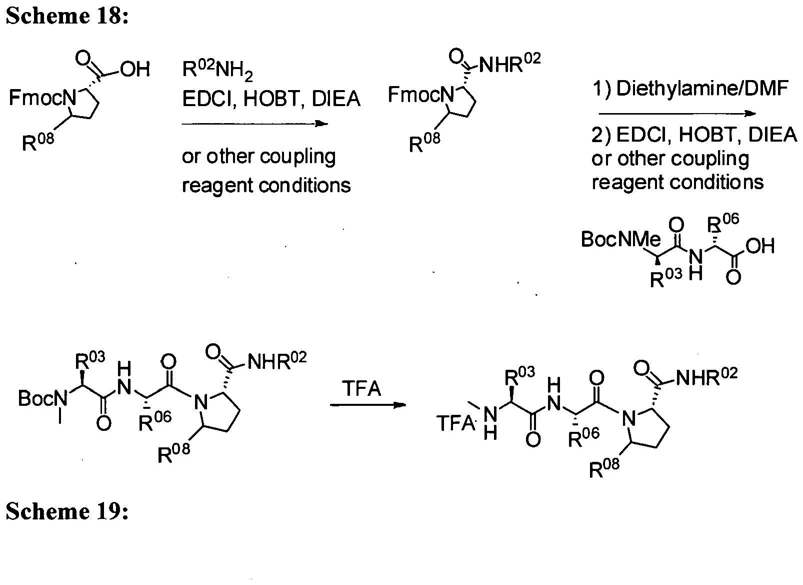 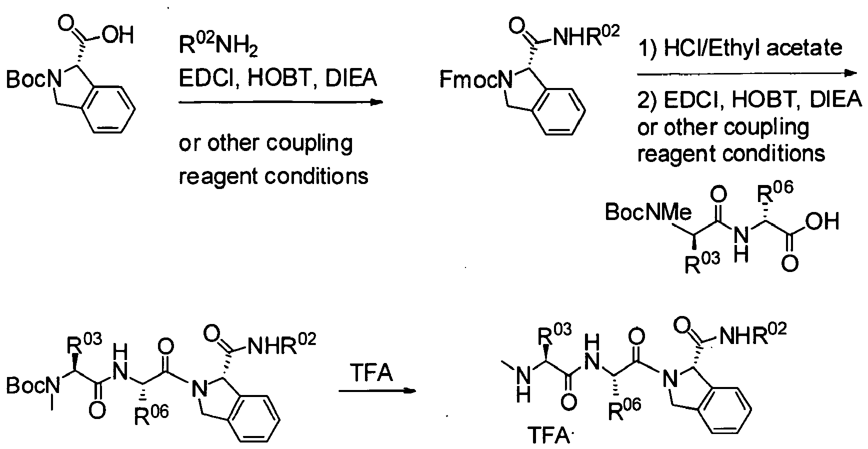 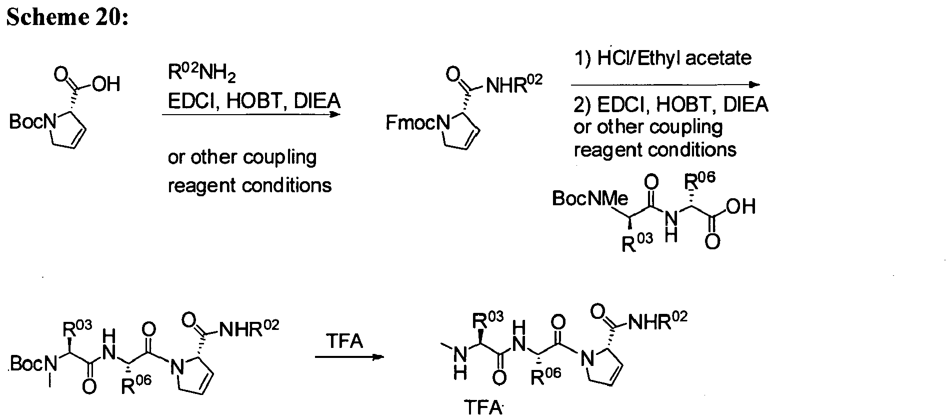 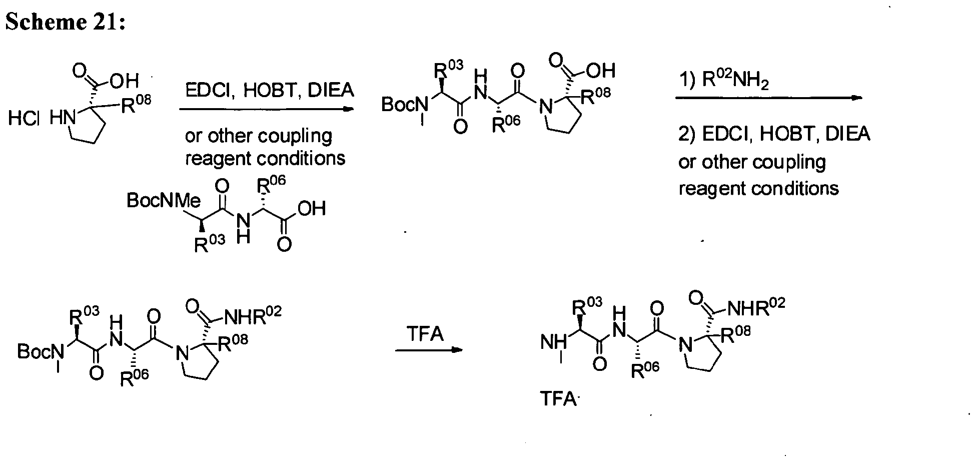 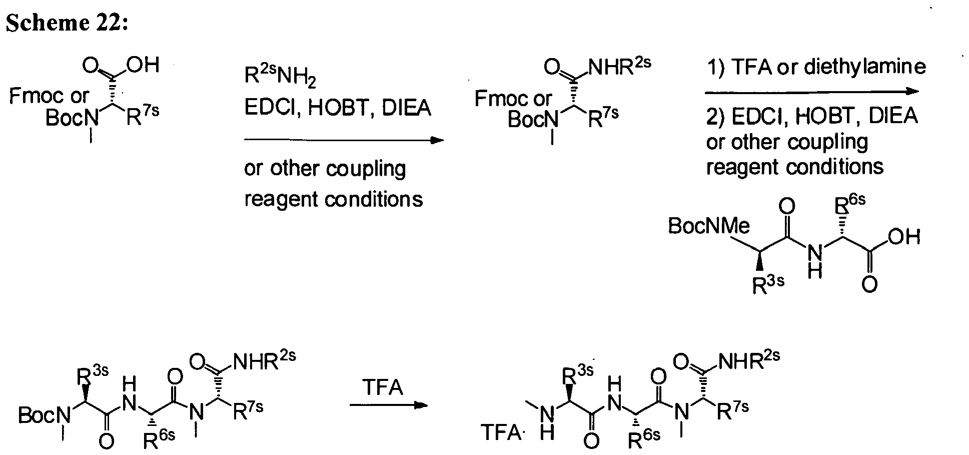 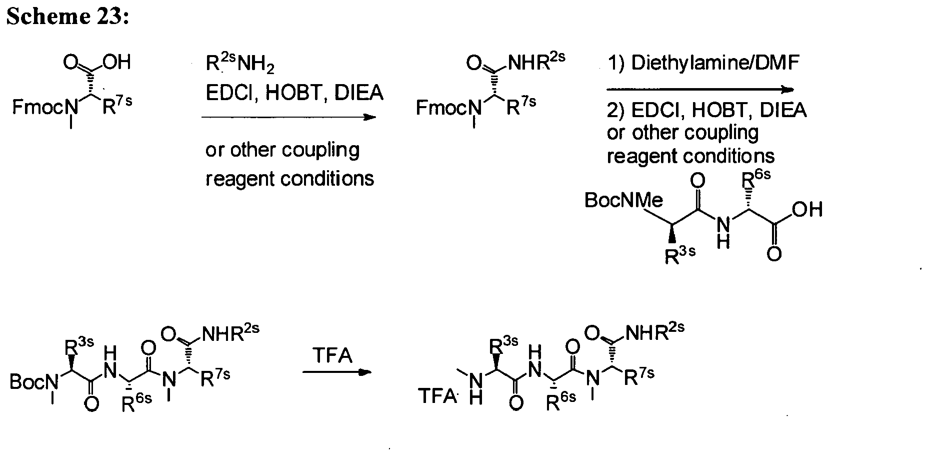 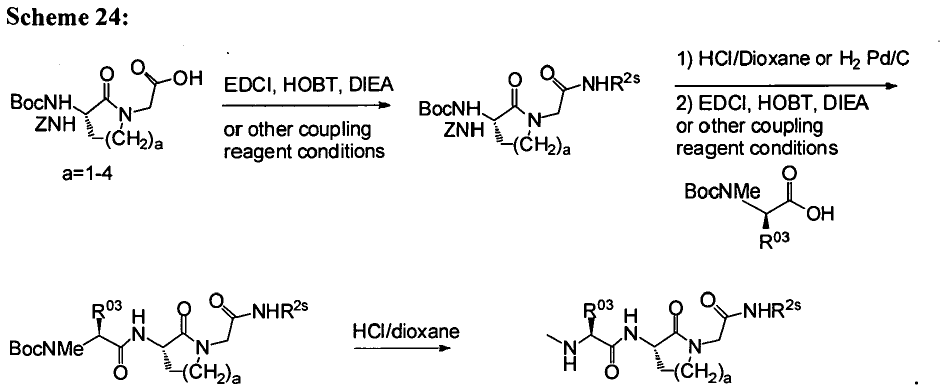 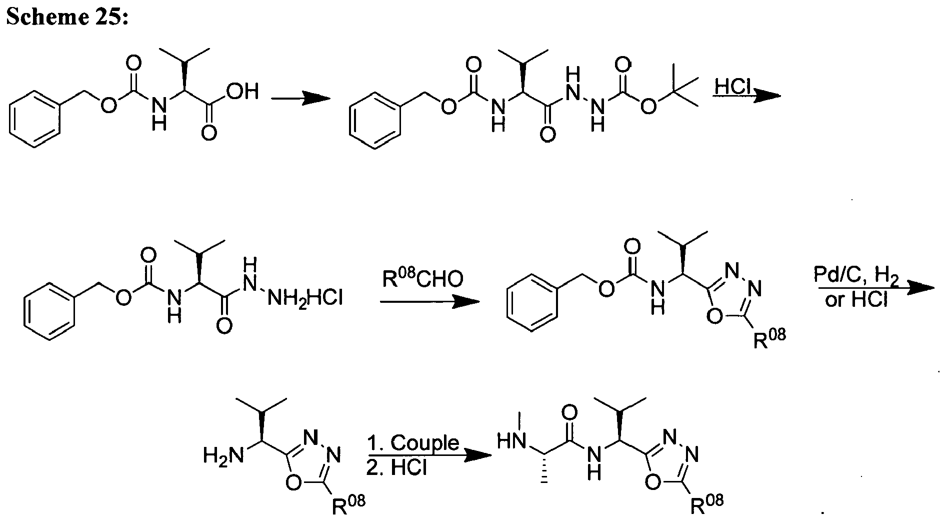 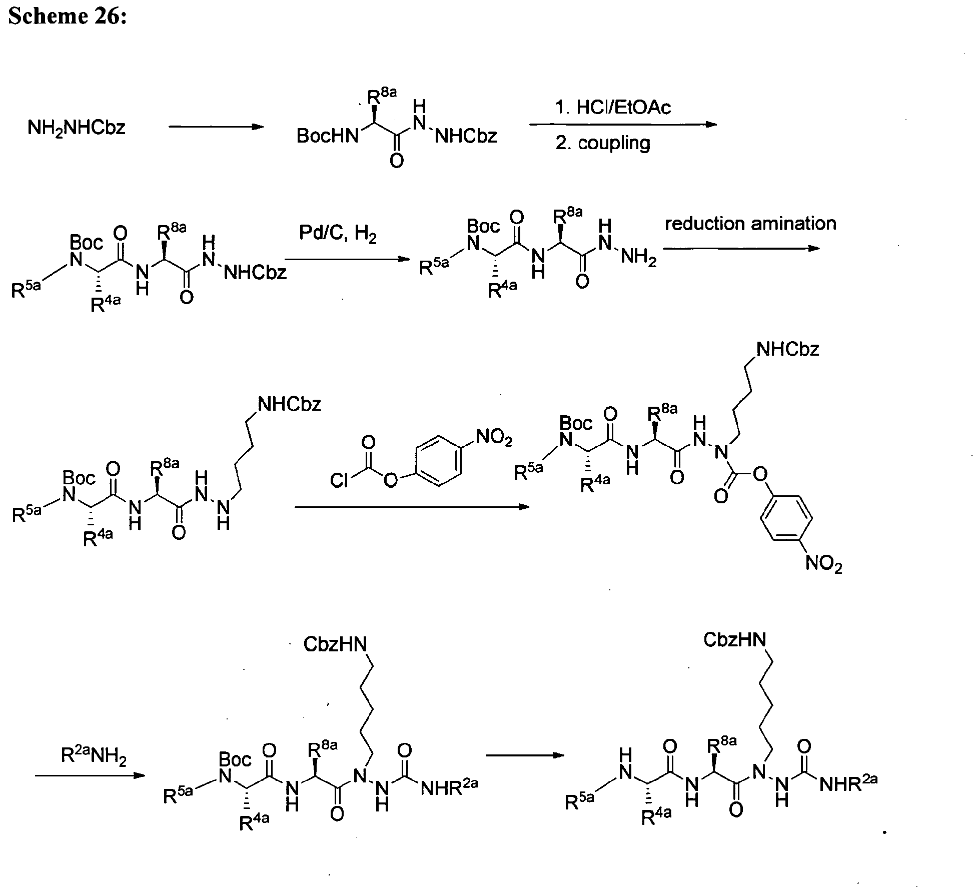 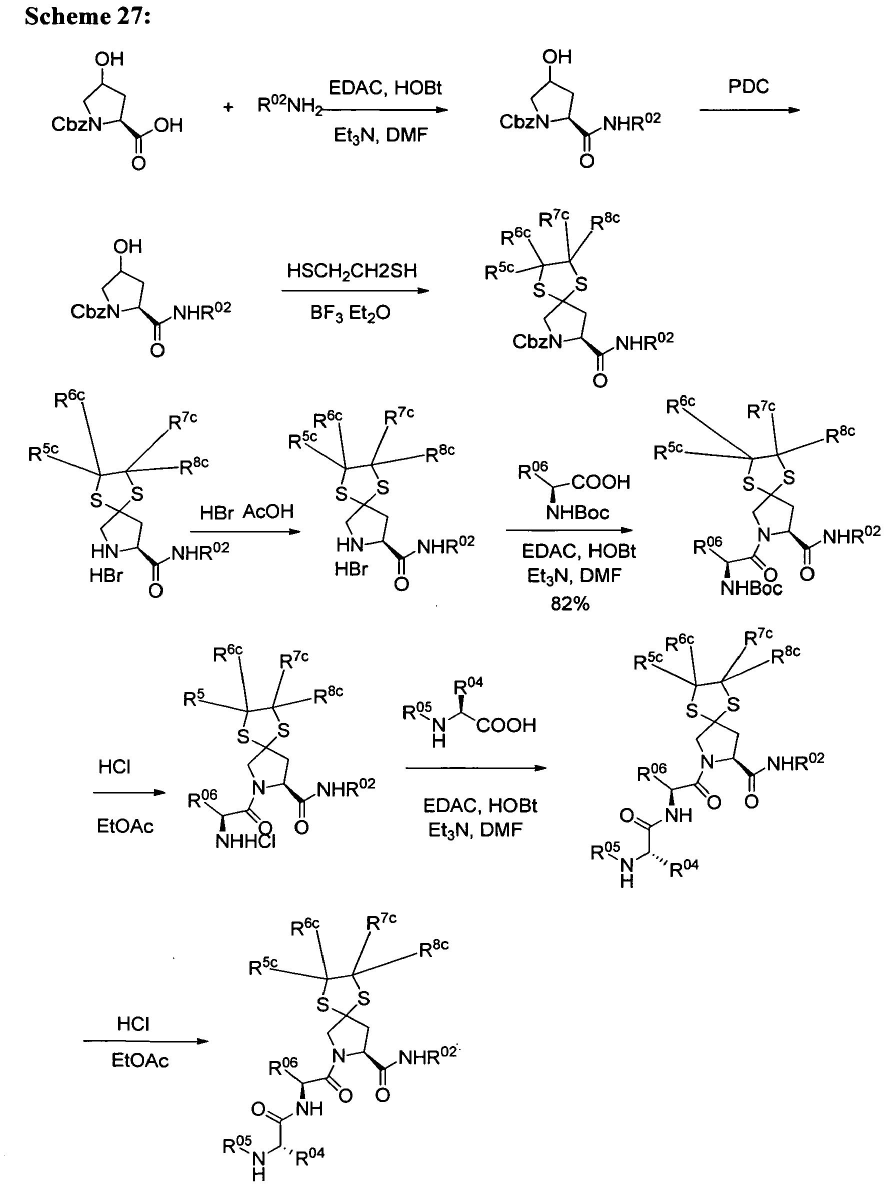 where R^{5c}, R^{6c}, R^{7c} and R^{8c} are each independently selected from hydrogen, halogen and alkyl. In certain embodiments, R^{5c}, R^{6c}, R^{7c} and R^{8c} are each independently selected from hydrogen and fluoro.

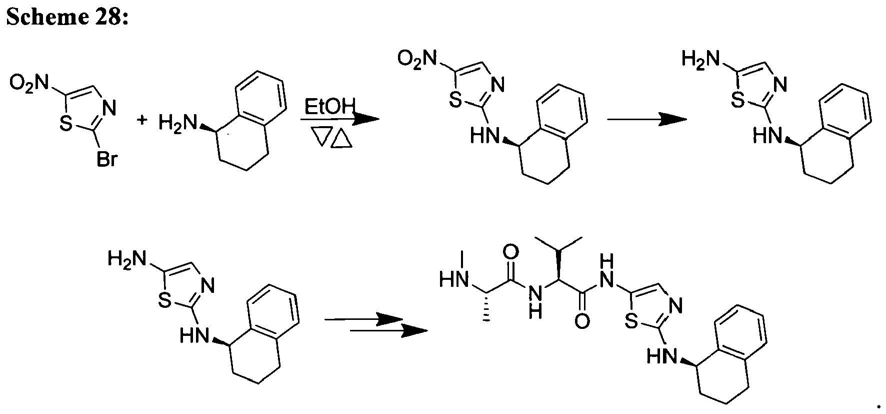

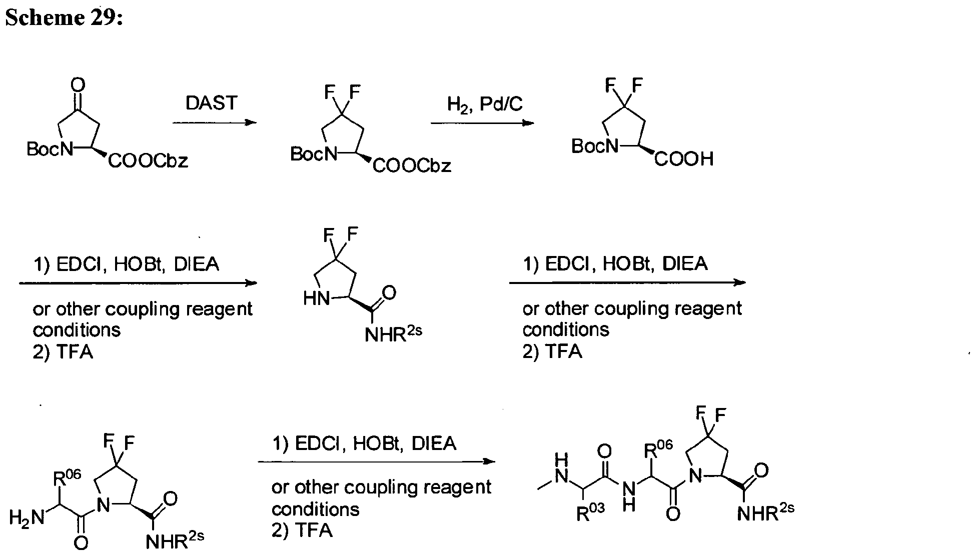

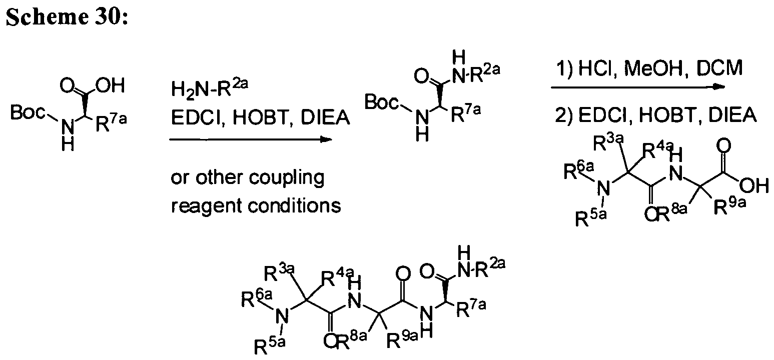

### D. Formulation of pharmaceutical compositions

The pharmaceutical compositions provided herein contain therapeutically effective amounts of one or more of the N-terminus smac tetrapeptide analogs provided herein that are useful in the prevention, treatment, or amelioration of one or more of the symptoms of hyperproliferative diseases or disorders associated with caspase activity, including caspase-9 activity. Such diseases or disorders include, but are not limited to, hyperproliferative diseases, autoimmune diseases, psoriasis, hyperplasia and restenosis.

The compositions contain one or more compounds provided herein. The compounds are preferably formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. Typically the compounds described above are formulated into pharmaceutical compositions using techniques and procedures well known in the art (see, *e.g.*, Ansel Introduction to Pharmaceutical Dosage Forms, Fourth Edition 1985, 126).

In the compositions, effective concentrations of one or more compounds or pharmaceutically acceptable derivatives is (are) mixed with a suitable pharmaceutical carrier or vehicle. The compounds may be derivatized as the corresponding salts, esters, enol ethers or esters, acids, bases, solvates, hydrates or prodrugs prior to formulation, as described above. The concentrations of the compounds in the compositions are effective for delivery of an amount, upon administration, that treats, prevents, or ameliorates one or more of the symptoms of diseases or disorders associated with caspase activity or in which caspase activity is implicated. Such diseases or disorders include, but are not limited to, hyperproliferative diseases, autoimmune diseases, psoriasis, hyperplasia and restenosis.

Typically, the compositions are formulated for single dosage administration. To formulate a composition, the weight fraction of compound is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, the compounds may be formulated as the sole pharmaceutically active ingredient in the composition or may be combined with other active ingredients. Liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in *in vitro* and *in vivo* systems described herein and then extrapolated therefrom for dosages for humans.

The concentration of active compound in the pharmaceutical composition will depend on absorption, inactivation and excretion rates of the active compound, the physicochemical characteristics of the compound, the dosage schedule, and amount administered as well as other factors known to those of skill in the art. For example, the amount that is delivered is sufficient to ameliorate one or more of the symptoms of diseases or disorders associated with caspase activity or in which caspase activity is implicated, as described herein.

Typically a therapeutically effective dosage should produce a serum concentration of active ingredient of from about 0.1 ng/ml to about 50-100 µg/ml. The pharmaceutical compositions typically should provide a dosage of from about 0.001 mg to about 2000 mg of compound per kilogram of body weight per day. Pharmaceutical dosage unit forms are prepared to provide from about 1 mg to about 1000 mg and preferably from about 10 to about 500 mg of the essential active ingredient or a combination of essential ingredients per dosage unit form.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Pharmaceutically acceptable derivatives include acids, bases, enol ethers and esters, salts, esters, hydrates, solvates and prodrug forms. The derivative is selected such that its pharmacokinetic properties are superior to the corresponding neutral compound.

Thus, effective concentrations or amounts of one or more of the compounds described herein or pharmaceutically acceptable derivatives thereof are mixed with a suitable pharmaceutical carrier or vehicle for systemic, topical or local administration to form pharmaceutical compositions. Compounds are included in an amount effective for ameliorating one or more symptoms of, or for treating or preventing diseases or disorders associated with caspase activity or in which caspase activity is implicated, as described herein. The concentration of active compound in the composition will depend on absorption, inactivation, excretion rates of the active compound, the dosage schedule, amount administered, particular formulation as well as other factors known to those of skill in the art.

The compositions are intended to be administered by a suitable route, including orally, parenterally, rectally, topically and locally. For oral administration, capsules and tablets are presently preferred. The compositions are in liquid, semi-liquid or solid form and are formulated in a manner suitable for each route of administration. Preferred modes of administration include parenteral and oral modes of administration. Oral administration is presently most preferred.

Solutions or suspensions used for parenteral, intradermal, subcutaneous, or topical application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or single or multiple dose vials made of glass, plastic or other suitable material.

In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate. Derivatives of the compounds, such as prodrugs of the compounds may also be used in formulating effective pharmaceutical compositions.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active compounds and derivatives thereof are typically formulated and administered in unit-dosage forms or multiple-dosage forms. Unit-dose forms as used herein refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

The composition can contain along with the active ingredient: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polvinylpyrrolidine, celluloses and derivatives thereof, povidone, crospovidones and other such binders known to those of skill in the art. Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, for example, acetate, sodium citrate, cyclodextrine derivatives, sorbitan monolaurate, triethanolamine sodium acetate, triethanolamine oleate, and other such agents. Actual methods of preparing such dosage forms are known, or will be apparent, to those skilled in this art; for example, see Remington's Pharmaceutical Sciences, Mack Publishing Company, Easton, Pa., 15th Edition, 1975. The composition or formulation to be administered will, in any event, contain a quantity of the active compound in an amount sufficient to alleviate the symptoms of the treated subject.

Dosage forms or compositions containing active ingredient in the range of 0.005% to 100% with the balance made up from non-toxic carrier may be prepared. For oral administration, a pharmaceutically acceptable non-toxic composition is formed by the incorporation of any of the normally employed excipients, such as, for example pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain 0.001%-100% active ingredient, preferably 0.1-85%, typically 75-95%.

The active compounds or pharmaceutically acceptable derivatives may be prepared with carriers that protect the compound against rapid elimination from the body, such as time release formulations or coatings.

The compositions may include other active compounds to obtain desired combinations of properties. The compounds provided herein, or pharmaceutically acceptable derivatives thereof as described herein, may also be advantageously administered for therapeutic or prophylactic purposes together with another pharmacological agent known in the general art to be of value in treating one or more of the diseases or medical conditions referred to hereinabove, such as diseases or disorders associated with caspase activity or in which caspase activity is implicated. It is to be understood that such combination therapy constitutes a further aspect of the compositions and methods of treatment provided herein.

### 1. Compositions for oral administration

Oral pharmaceutical dosage forms are either solid, gel or liquid. The solid dosage forms are tablets, capsules, granules, and bulk powders. Types of oral tablets include compressed, chewable lozenges and tablets which may be enteric-coated, sugar-coated or film-coated. Capsules may be hard or soft gelatin capsules, while granules and powders may be provided in non-effervescent or effervescent form with the combination of other ingredients known to those skilled in the art.

In certain embodiments, the formulations are solid dosage forms, preferably capsules or tablets. The tablets, pills, capsules, troches and the like can contain any of the following ingredients, or compounds of a similar nature: a binder; a diluent; a disintegrating agent; a lubricant; a glidant; a sweetening agent; and a flavoring agent.

Examples of binders include microcrystalline cellulose, gum tragacanth, glucose solution, acacia mucilage, gelatin solution, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emetic-coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

If oral administration is desired, the compound could be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, sprinkle, chewing gum or the like. A syrup may contain, in addition to the active compounds, sucrose as a sweetening agent and certain preservatives, dyes and colorings and flavors.

The active materials can also be mixed with other active materials which do not impair the desired action, or with materials that supplement the desired action, such as antacids, H2 blockers, and diuretics. The active ingredient is a compound or pharmaceutically acceptable derivative thereof as described herein. Higher concentrations, up to about 98% by weight of the active ingredient may be included.

Pharmaceutically acceptable carriers included in tablets are binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are especially useful in the formation of chewable tablets and lozenges.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms.

Solvents include glycerin, sorbitol, ethyl alcohol and syrup. Examples of preservatives include glycerin, methyl and propylparaben, benzoic add, sodium benzoate and alcohol. Examples of non-aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia. Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic adds include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such fruits, and synthetic blends of compounds which produce a pleasant taste sensation.

For a solid dosage form, the solution or suspension, in for example propylene carbonate, vegetable oils or triglycerides, is preferably encapsulated in a gelatin capsule. Such solutions, and the preparation and encapsulation thereof, are disclosed in U.S. Patent Nos 4,328,245; 4,409,239; and 4,410,545. For a liquid dosage form, the solution, e.g., for example, in a polyethylene glycol, may be diluted with a sufficient quantity of a pharmaceutically acceptable liquid carrier, e.g., water, to be easily measured for administration.

Alternatively, liquid or semi-solid oral formulations may be prepared by dissolving or dispersing the active compound or salt in vegetable oils, glycols, triglycerides, propylene glycol esters (e.g., propylene carbonate) and other such carriers, and encapsulating these solutions or suspensions in hard or soft gelatin capsule shells. Other useful formulations include those set forth in U.S. Patent Nos. Re 28,819 and 4,358,603. Briefly, such formulations include, but are not limited to, those containing a compound provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

In all embodiments, tablets and capsules formulations may be coated as known by those of skill in the art in order to modify or sustain dissolution of the active ingredient. Thus, for example, they may be coated with a conventional enterically digestible coating, such as phenylsalicylate, waxes and cellulose acetate phthalate.

### 2. Injectables, solutions and emulsions

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow-release or sustained-release system, such that a constant level of dosage is maintained (see, *e.g.*, U.S. Patent No. 3,710,795) is also contemplated herein. Briefly, a compound provided herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylenevinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the subject.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions include EDTA. Pharmaceutical carriers also include ethyl alcohol, polyethylene glycol and propylene glycol for water miscible vehicles and sodium hydroxide, hydrochloric acid, citric acid or lactic acid for pH adjustment.

The concentration of the pharmaceutically active compound is adjusted so that an injection provides an effective amount to produce the desired pharmacological effect. The exact dose depends on the age, weight and condition of the patient or animal as is known in the art.

The unit-dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. Typically a therapeutically effective dosage is formulated to contain a concentration of at least about 0.1 % w/w up to about 90% w/w or more, preferably more than 1% w/w of the active compound to the treated tissue(s). The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The compound may be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and may be empirically determined.

### 3. Lyophilized powders

Of interest herein are also lyophilized powders, which can be reconstituted for administration as solutions, emulsions and other mixtures. They may also be reconstituted and formulated as solids or gels.

The sterile, lyophilized powder is prepared by dissolving a compound provided herein, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, typically, about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (10-1000 mg, preferably 100-500 mg) or multiple dosages of the compound. The lyophilized powder can be stored under appropriate conditions, such as at about 4 °C to room temperature.

Reconstitution of this lyophilized powder with water for injection provides a formulation for use in parenteral administration. For reconstitution, about 1-50 mg, preferably 5-35 mg, more preferably about 9-30 mg of lyophilized powder, is added per mL of sterile water or other suitable carrier. The precise amount depends upon the selected compound. Such amount can be empirically determined.

### 4. Topical administration

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds or pharmaceutically acceptable derivatives thereof may be formulated as aerosols for topical application, such as by inhalation (see, e.g., U.S. Patent Nos. 4,044,126, 4,414,209, and 4,364,923, which describe aerosols for delivery of a steroid useful for treatment of inflammatory diseases, particularly asthma). These formulations for administration to the respiratory tract can be in the form of an aerosol or solution for a nebulizer, or as a microfme powder for insufflation, alone or in combination with an inert carrier such as lactose. In such a case, the particles of the formulation will typically have diameters of less than 50 microns, preferably less than 10 microns.

The compounds may be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered.

These solutions, particularly those intended for ophthalmic use, may be formulated as 0.01 % - 10% isotonic solutions, pH about 5-7, with appropriate salts.

### 5. Compositions for other routes of administration

Other routes of administration, such as topical application, transdermal patches, and rectal administration are also contemplated herein.

For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. The typical weight of a rectal suppository is about 2 to 3 gm.

Tablets and capsules for rectal administration are manufactured using the same pharmaceutically acceptable substance and by the same methods as for formulations for oral administration.

### 6. Articles of manufacture

The compounds or pharmaceutically acceptable derivatives may be packaged as articles of manufacture containing packaging material, a compound or pharmaceutically acceptable derivative thereof provided herein, which is effective for modulating the activity of caspases, including caspase-9 receptors, or for treatment, prevention or amelioration of one or more symptoms of caspase, including caspase-9, mediated diseases or disorders, or diseases or disorders in which caspase activity, including caspase-9 activity, is implicated, within the packaging material, and a label that indicates that the compound or composition, or pharmaceutically acceptable derivative thereof, is used for modulating the activity of caspases, including caspase-9, or for treatment, prevention or amelioration of one or more symptoms of caspase, including caspase-9, mediated diseases or disorders, or diseases or disorders in which caspase activity, including caspase-9 activity, is implicated.

The articles of manufacture provided herein contain packaging materials. Packaging materials for use in packaging pharmaceutical products are well known to those of skill in the art. See, *e.g.*, U.S. Patent Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated as are a variety of treatments for any disease or disorder in which caspase activity, including caspase -9 activity, is implicated as a mediator or contributor to the symptoms or cause.

### E. Evaluation of the Activity of the Compounds

Standard physiological, pharmacological and biochemical procedures are available for testing the compounds to identify those that possess biological activities that modulate the activity of caspases, including caspase-9. Such assays include, for example, biochemical assays such as binding assays, measuring fluorescence polarization and Caspase-9 rescue assay.

In fluorescence polarization assays a fluorescent probe is used. The binding affinity of compounds to BIR3 is measured by competitive displacement of a fluorescent probe using fluorescence polarization anisotropy. Fluorescence polarization assays therefore provide a way of detecting binding and quantifying the binding affinity of compounds to the BIR3 domain by measuring changes in fluorescence polarization that occur as a result of the displacement of a trace amount of the probe by the compound. The methods provided herein, in certain embodiments use hexapeptide H-Ala-Val-Pro-Phe-Ala-Lys-OH covalently modified on the epsilon-amino group of the C-terminal lysine with 5-carboxyfluorescein as a probe. In other embodiments, the probe used is 6-(6-Hydroxy-3-oxo-3H-xanthen-9-yl)-N-{5-(2-methylamino-propionylamino)-6-oxo-6-[4-(1,2,3,4-tetrahydro-naphthalen-1-ylcarbamoyl)-thiazolidin-3-yl]-hexyl}-isophthalamic acid.

Additionally the compounds and compositions can be evaluated for their ability to achieve relief of BIR3-mediated Caspase-9 inhibition by measuring Caspase-9 reactivation. The fluorogenic substrate Ac-LEHD-7-amido-4-methylcoumarin is used in these methods. EC₅₀ values of tested BIR3 inhibitors or Caspase-9 rescuers is calculated from the percentage of Caspase-9 reactivation in the mixtures containing serially diluted concentrations of tested compound. Typically, 10-point curves are generated for such EC₅₀ determination.

### F. Methods of use of the compounds and compositions

Methods of use of the compounds and compositions provided herein are also provided. The methods involve both *in vitro* and *in vivo* uses of the compounds and compositions for promoting caspase activity, including caspase-9 activity, and for treatment, prevention, or amelioration of one or more symptoms of diseases or disorder that are modulated by caspase activity, including caspase-9 activity, or in which caspase activity, including caspase-9 activity, is implicated. The methods are effected by contacting a composition containing the BIR3 domain of XIAP with one or more of the compounds or compositions.

Methods of inducing apoptosis and of promoting apoptosis are provided. As described above, caspases, including caspase-9 are implicated in modulating apoptosis.

Method of reducing the inhibitory effect IAPs, including XIAP, by contacting a cell with one or more compounds or compositions provided herein, are provided. In certain embodiments, methods provided herein are for modulating BIR domain of an IAP. In certain embodiments, methods provided herein are for modulating BIR domain of XIAP. In certain embodiments, methods provided herein are for modulating BIR3 domain of IAP. In certain embodiments, methods provided herein are for antagonizing BIR domain of an IAP. In certain embodiments, methods provided herein are for antagonizing BIR domain of XIAP. In certain embodiments, methods provided herein are for antagonizing BIR3 domain of XIAP.

Methods of treatment, prevention, or amelioration of one or more symptoms of a disease or disorder which is affected by activity of caspases, including caspase-9 and by inhibitory effect of IAP are provided.

Methods of treatment, prevention, or amelioration of one or more symptoms of hyperproliferative diseases, autoimmune diseases, psoriasis, hyperplasia and restenosis are provided. The hyperproliferative diseases treated by the methods provided herein include the cancers which are resistant to apoptosis due to the expression of IAPs. Examples of such cancer types include, but are not limited to neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, toung carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometriuni carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, nonsmall cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

Examples of autoimmune diseases treated by methods provided herein include, but are not limited to collagen diseases such as rheumatoid arthritis, Lupus erythematodes disseeminatus, Sharp syndrome, CREST syndrome (calcinosis, Raynaud syndrome, esophageal dysmotility, teleangiectasia), dermatomyositis, vasculitis (Morbus Wegener) and Sjögren and syndrome, renal diseases such as Goodpasture syndrome, rapidly-progressing glomerulonephritis and membrane-proliferative glomerulonephritis type II, endocrine diseases such as type-I diabetes, autoimmune polyendocrinopathy-candidiasis-ectodermal dystrophy (APECED), autoimmune parathyreoidism, pernicious anemia, gonad insufficiency, idiopathic Morbus Addison, hyperthyreosis, Hashimoto thyreoiditis and primary myxedemia, skin diseases such as Pemphigus vulgaris, bullous pemphigoid, Herpes gestationis, Epidermolysis bullosa Erythema multiforme major, liver diseases such as primary biliary cirrhosis, autoimmune cholangitis, autoimmune hepatitis type-1, autoimmune hepatitis type-2, primary sclerosing cholangitis, neuronal diseases such as multiple sclerosis, myastenia gravis, myasthenic Lambert-Eaton syndrom e, acquired neuromyotony, Guillain-Barré syndrome (Müller-Fischer syndrome), Stiff-man syndrome, cerebellar degeneration, ataxia, opsoklonus, sensoric neuropathy and achalasia, blood diseases such as autoimmune hemolytic anemia idiopathic thrombocytopenic purpura (Morbus Werlhof), infectious diseases with associated autoimmune reactions such as AIDS , Malaria and Chagas disease.

### G. Combination Therapy

The compounds provided herein may be administered as the sole active ingredient or in combination with other active ingredients. Other active ingredients that may be used in combination with the compounds provided herein include but are not limited to compounds known to modulate caspase activity, other compounds for use in treating, preventing, or ameliorating one or more symptoms of caspase mediated diseases and disorders, and-angiogenesis agents, anti-tumor agents, other cancer treatments and autoimmune agents.f Such compounds include, in general, but are not limited to, alkylating agents, toxins, antiproliferative agents and tubulin binding agents. Classes of cytotoxic agents for use herein include, for example, the anthracycline family of drugs, the vinca drugs, the mitomycins, the bleomycins, the cytotoxic nucleosides, the pteridine family of drugs, diynenes, the maytansinoids, the epothilones, the taxanes and the podophyllotoxins.

The following examples are included for illustrative purposes only and are not intended to limit the scope of the subject matter claimed herein.

### Example 1

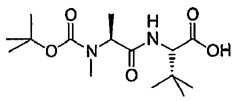

### 2-[2-(tert-Butoxycarbollyl-methyl-amino)-propionylamino]-3,3-dimethyl-butyric acid

### Part A: 2-[2-(tert-Butoxycarbonyl-methyl-amino)-propionylamino]-3,3-dimethyl-butyric acid methyl ester

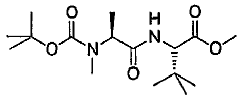

A solution of 2-(*tert*-butoxycarbonyl-methyl-amino)-propionic acid (1.76 g, 8.68 mmol) and 2-amino-3,3-dimethyl-butyric acid methyl ester; hydrochloride salt (1.58 g, 8.70 mmol) in tetrahydrofuran (THF) (100 mL) was added 1-ethyl-3-(3-dimethylaminopropyl)carbodiimide hydrochloride (EDCI) (3.2 g, 16.7 mmol) and 1-hydroxybenzotriazole monohydrate (HOBt) (1.5 g, 11.1 mmol), followed by N-methyl-2-pyrrolidinone (2 mL) and 4-methyl morpholine (4 mL). This mixture was stirred at room temperature for 18 hours. The reaction mixture was treated with water (30 mL), then partitioned between ethyl acetate/water. The organic phase was washed with 1N HCl (2 x 50 mL), saturated sodium bicarbonate solution (100 mL) and brine solution (50 mL), dried over Na₂SO₄ and evaporated to dryness to give the title compound (2.79 g, >95%) as a glassine solid. TLC (50% ethyl acetate/hexane) R_{f}= 0.51; MS (ES) for C₁₆H₃₀N₂O₅ (MW=330.42): positive 331 (M+H), negative 329 (M-H).

### Part B: 2-[2-(tert-Butoxycarbonyl-methyl-amino)-propionylamino]-3,3-dimethyl-butyric acid

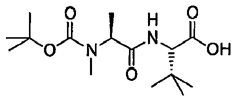

A solution of 2-[2-(*tert*-butoxycarbonyl-methyl-amino)-propionylamino]-3,3-dimethyl-butyric acid methyl ester (2.70 g, 8.16 mmol) in tetrahydrofuran (THF) (20 mL), methanol (MeOH) (10 mL) and water (20 mL) was added a 2 N LiOH solution (16 mL, 32 mmol). This mixture was stirred at room temperature for 18 hours. The reaction mixture was treated with 1 N HCl (100 mL), then partitioned with ethyl acetate. The organic phase was washed with brine solution (50 mL), dried over Na₂SO₄ and evaporated to dryness to give the title compound (2.58 g, >95%) as a crystalline solid. TLC (ethyl acetate) R_{f}= 0.2 streaks; MS(ES) for C₁₅H₂₈N₂O₅ (MW=316.39): positive 317 (M+H), negative 315 (M-H).

### Example 2

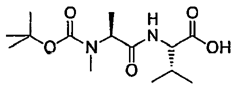

### 2-[2-(tert-Butoxycarbonyl-methyl-amino)-propionylamino]-3-methyl-butyric acid

### Part A: 2-[2-(tert-Butoxycarbonyl-methyl-amino)-propionylamino]-3-methyl-butyric acid methyl ester

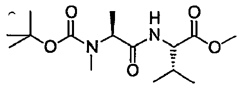

A solution of 2-(*tert*-butoxycarbonyl-methyl-amino)-propionic acid (5.30 g, 26.1 mmol) and 2-amino-3-methyl-butyric acid methyl ester; hydrochloride salt (6.03 g, 36.0 mmol) in tetrahydrofuran (THF) (100 mL) was added 1-ethyl-3-(3-dimethylaminopropyl)carbodiimide hydrochloride (EDCI) (10.2 g, 53.2 mmol) and 1-hydroxybenzotriazole monohydrate (HOBt) (5.17 g, 38.2 mmol), followed by *N*-methyl-2-pyrrolidinone (6 mL) and 4-methyl morpholine (12 mL). This mixture was stirred at room temperature for 18 hours. The reaction mixture was treated with water (30 mL), then partitioned between ethyl acetate/water. The organic phase was washed with 1N HCl (2 x 75 mL), saturated sodium bicarbonate solution (150 mL) and brine solution (50 mL), dried over Na₂SO₄ and evaporated to dryness to give the title compound (8.25 g, >95%) as a colorless oil. TLC (50% ethyl acetate/hexane) R*_{f}*= 0.52; MS(ES) for C₁₅H₂₈N₂O₅ (MW=316.39): positive 317 (M+H), negative 315 (M-H).

### Part B: 2-[2-(tert-Butozycarbonyl-methyl-amino)-propionylamino]-3-methyl-butyric acid

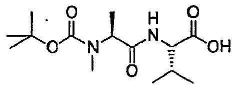

A solution of 2-[2-(*tert*-butoxycarbonyl-methyl-amino)-propionylamino]-3-methyl-butyric acid methyl ester (8.25 g, 26.0 mmol) in tetrahydrofuran (THF) (40 mL), methanol (MeOH) (20 mL) and water (40 mL) was added a 2 N LiOH solution (40 mL, 80 mmol). This mixture was stirred at room temperature for 18 hours. The reaction mixture was treated with 1 N HCl (150 mL), then partitioned with ethyl acetate. The organic phase was washed with brine solution (50 mL), dried over Na₂SO₄ and evaporated to dryness to give the title compound (7.89 g, >95%) as a crystalline solid. TLC (ethyl acetate) R*_{f}*= 0.2 streaks; MS(ES) for C₁₄H₂₆N₂O₅ (MW=302.37): positive 303 (M+H), negative 301 (M-H).

### Example 3

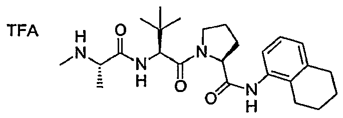

### 1-[3,3-Dimethyl-2-(2-methylamino-propionylamino)-butyryl]-pyrrolidine-2-carboxylic acid (5,6,7,8-tetrahydro-naphthalen-1-yl)-amide trifluoro-acetic acid salt

### Part A: 1-(2-tert-Butoxycarbonylamino-3,3-dimethyl-butyryl)-pyrrolidine-2-carboxylic acid benzyl ester

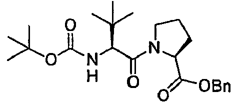

To a cooled (0 °C) solution of 2-tert-Butoxycarbonylamino-3,3-dimethyl-butyric acid (2.313g, 10mmol) in 40 mL of methylene chloride under nitrogen was added 1-hydroxybenzotriazole (HOBt, 1.684g, 11 mmol) followed by addition of 1-(3-dimethylaminopropyl)-3-ethylcarbodiimide hydrochloride (EDCI, 2.876g, 15mmol). The solution was stirred for 20 minutes. Pyrrolidine-2-carboxylic acid benzyl ester hydrochloride salt (2.418g, 10mmol) was added followed by N-methylmorpholine (NMM, 3.30mL, 30mmol). The solution was stirred for18 hours while allowing to warm to room temperature. Solvent was removed under reduced pressure. Reaction mixture was taken up in ethyl acetate (80 mL). This was washed with 5% KHSO₄ (3x30 mL), saturated sodium bicarbonate solution (3x30 mL), and brine (30 mL). The solution was dried over Na₂SO₄ and solvent was removed under reduced pressure to yield the title compound (4.185g >99 %).

### Part B: 1-(2-Amino-3,3-dimethyl-butyryl)-pyrrolidine-2-carbozylic acid benzyl ester hydrochloride salt

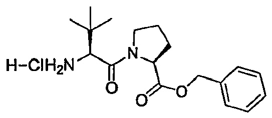

To a solution of 1-(2-tert-Butoxycarbonylamino-3,3-dimethyl-butyryl)-pyrrolidine-2-carboxylic acid benzyl ester (4.185g, 10mmol) in 20 mL of ethyl acetate was added 6M HCl in ethyl acetate (10 mL). The solution was stirred for 3 hours. Solvent was removed under reduced pressure. The resulting oil was dried on a vacuum pump for 18 hours to yield the title compound (3.548g >99 %).

### Part C: 1-{2-[2-(tert-Butoxycarbonyl-methyl-amino)-propionylamino]-3,3-dimethyl-butyryl}-pyrrolidine-2-carbozylic acid benzyl ester

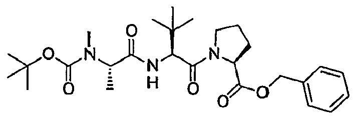

To a cooled (0 °C) solution of 2-(tert-Butoxycarbonyl-methyl-amino)-propionic acid (2.032g, 10mmol) in 40 mL of methylene chloride under nitrogen was added HOBt (1.684g, 11 mmol) followed by addition of EDCI (2.876g, 15mmol). The solution was stirred for 20 minutes. 1-(2-Amino-3,3-dimethyl-butyryl)-pyrrolidine-2-carboxylic acid benzyl ester hydrochloride salt (3.545g, 10mmol) was added followed by NMM (3.30mL, 30mmol). The solution was stirred for 16 hours while allowing to warm to room temperature. Solvent was removed under reduced pressure. Reaction mixture was taken up in ethyl acetate (80 mL). This was washed with 5% KHSO₄ (3x30 mL), saturated sodium bicarbonate solution (3x30 mL), and brine (30 mL). The solution was dried over Na₂SO₄ and concentrated under reduced pressure to yield the title compound (4.395g, 87.3 %).

### Part D: 1-{2-[2-(tert-Butoxycarbonyl-methyl-amino)-propionylamino]-3,3-dimethyl-butyryl}-pyrrolidine-2-carboxylic acid

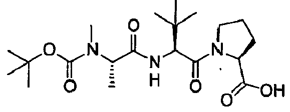

To a nitrogen flushed solution of 1-{2-[2-(tert-Butoxycarbonyl-methyl-amino)-propionylamino]-3,3-dimethyl-butyryl}-pyrrolidine-2-carboxylic acid benzyl ester (4.390g, 8.72 mmol) in 25 mL of ethyl acetate was added 10%w/w Pd/C (0.439g), and the mixture was stirred for 3.5 hours under hydrogen (1 atm). The mixture was filtered through celite to remove Pd/C and the solvent was removed under reduced pressure to yield the title compound (3.601g, >99 %).

### Part E: 1-{2,2-Dimethyl-1-[2-(5,6,7,8-tetrahydro-naphthalen-1-ylcarbamoyl)-pyrrolidine-1-carbonyl]-propylcarbamoyl}-ethyl)-methyl-carbamic acid tert-butyl ester

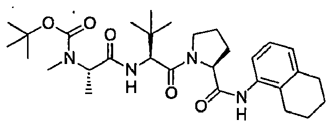

To a solution of 1-{2-[2-(tert-Butoxycarbonyl-methyl-amino)-propionylamino]-3,3-dimethyl-butyryl}-pyrrolidine-2-carboxylic acid (0.207 g, 0.5 mmol) in ethyl acetate (1 mL) was added propane phosponic acid anhydride (T3P, 50% w/w soln in ethyl acetate) (0.91 mL, 1.5 mmol). The resulting solution was stirred for 10 minutes, then 5,6,7,8-Tetrahydro-naphthalen-1-ylamine (69.7 µL, 0.5 mmol) was added followed by addition of NMM (0.55 mL, 5.0 mmol). The solution was stirred for 2 hours, then diluted with ethyl acetate (10 mL), washed with 5% KHSO₄ (3x10 mL), saturated sodium bicarbonate soluton (3x10 mL), and brine (10 mL). The solution was dried over Na₂SO₄ and concentrated under reduced pressure to yield the title compound (0.126 g, 46.5 %) TLC (1:1 Ethyl acetate(Hexanes) R_{f} = 0.37.

### Part F: 1-[3,3-Dimethyl-2-(2-methylamino-propionylamino)-butyryl]-pyrrolidine-2-carboxylic acid (5,6,7,8-tetrahydro-naphthalen-1-yl)-amide trifluoro-acetic acid salt

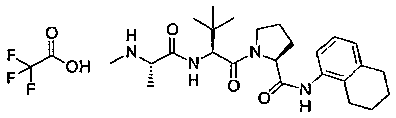

To a solution of (1-{2,2-Dimethyl-1-[2-(5,6,7,8-tetrahydro-naphthalen-1-ylcarbamoyl)-pyrrolidine-1-carbonyl]-propylcarbamoyl}-ethyl)-methyl-carbamic acid tert-butyl ester (0.125 g, 0.23 mmol) in ethyl acetate (2 mL) was added 6M HCl in ethyl acetate (3 mL). After stirring for 1 hour the solvent was removed under reduced pressure. The residue was triturated in ethyl ether then purified using reverse phase HPLC, using a C18 Vydec column and eluting with a gradient of 5% to 50% acetonitrile in water containing 0.1% TFA over 30 minutes, yielding the title compound as a white solid (90.1 mg, 70.4 %). Analytical HPLC Rₜ-10.518 min. MS(ES) for C₂₇H₃₉F₃N₄O₅ (MW=556.62): positive 443 (M+H) negative 441/555 (M-H).

### Example 4

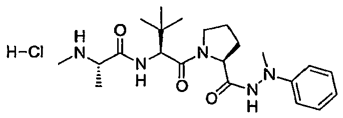

### N-{2,2-Dimethyl-1-[2-(N'-methyl-N'-phenyl-hydrazinocarbonyl)-pyrrolidine-1-carbonyl]-propyl}-2-methylamino-propionamide hydrochloride salt

Example 4 was prepared using appropriate starting materials and using the same procedures used to prepare example 3 Purification of example 3 via reverse phase HPLC was not necessary. The title compound could be isolated after ether tritration (0.163 g, 79.8. Analytical HPLC Rₜ-7.775 min. MS (ES) for C₂₂H₃₆ClN₅O₃ (MW=454.01): positive 418 (M+H) negative 416/452 (M-H).

### Example 5

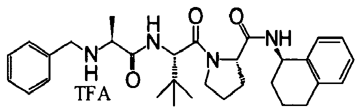

### 1-[2-(2-Benzylamino-propionylamino)-3,3-dimethyl-butyryl]pyrrolidine-2-carboxylic acid (1,2,3,4-tetrahydro-naphthalen-1-yl)-amide Trifluoroacetic acid salt

### Part A: 2-(1,2,3,4-Tetrahydro-naphthalen-1-ylcarbamoyl)-pyrrolidine-1-carboxylic acid tert-butyl ester

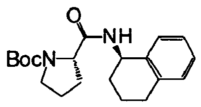

The Boc-Proline (18.40 g, 85.48), 1,2,3,4-Tetrahydro-naphthalen-1-ylamine (15.102g, 102.58mmol, 1.2eq.), EDCI (24.555g,128.22mmol, 1.5eq.), and HOBt (15.694g, 102.58mmol, 1.2eq.) were combined and dissolved in acetonitrile (341.93mL, 0.25M). After stirring for ten minutes DIEA (73.03mL, 427.41mmol, 5eq.) was added and the mixture was stirred at room temperature for 18 hours. The reaction mixture was concentrated under reduced pressure then re-suspended in 3L ethyl acetate and washed with 300mL of 1M HCl (2X), brine, saturated sodium bicarbonate (2X), brine. The ethyl acetate was dried (Na₂SO₄ and solvent was removed to yield white foam with R_{f}= 0.43 (1:1, hexane/ethyl acetate).

### Part B: Pyrrolidine-2-carboxylic acid (1,2,3,4-tetrahydro-naphthalen-1-yl)-amide HCl Salt

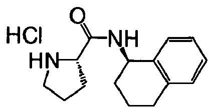

2-(1,2,3,4-Tetrahydro-naphthalen-1-ylcarbamoyl)-pyrrolidine-1-carboxylic acid tert-butyl ester (85.48mmol) was dissolved in ethyl acetate (285mL). To this was added 4M HCl in dioxane (95mL, 380mmol, 4.444eq.) and the mixture was stirred at room temperature for 18 hours. The resulting suspension was filtered and the solid was rinsed with diethyl ether and dried under vacuum.

### Part C: {2,2-Dimethyl-1-[2-(1,2,3,4-tetrahydro-naphthalen-1-ylcarbamoyl)-pyrrolidine-1-carbonyl]-propyl}-carbamic acid tert-butyl ester

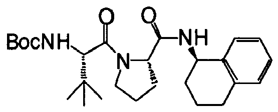

The Boc-tButylGlycine (3.969g, 17.16mmol), pyrrolidine-2-carboxylic acid (1,2,3,4-tetrahydro-naphthalen-1-yl)-amide HCl salt (5.78g, 20.59mmol, 1.2eq.), EDCI (4.929g,25.74mmol, 1.5eq.), and HOBt (3.151g, 20.59mmol, 1.2eq.) were combined and dissolved in acetonitrile (68.64mL, 0.25M). After stirring for ten minutes DIEA (14.66mL, 85.80mmol, 5eq.) was added and the mixture was stirred at room temperature for 18 hours. The reaction mixture was concentrated under reduced pressure and was re-suspended in 3L ethyl acetate and washed with 300mL of 1M HCl (2X), brine, saturated sodium bicarbonate (2X), brine. The ethyl acetate was dried (Na₂SO₄) and solvent was removed to yield white foam.

### Part D: 1-(2-Amino-3,3-dimethyl-butyryl)-pyrrolidine-2-carboxylic acid (1,2,3,4-tetrahydro-naphthalen-1- yl)-Amide HCl Salt

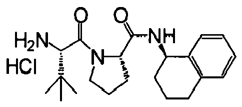

{2,2-Dimethyl-1-[2-(1,2,3,4-tetrahydro-naphthalen-1-ylcarbamoyl)-pyrrolidine-1-carbonyl]-propyl}-carbamic acid tert-butyl ester (17.16mmol) was dissolved in ethyl acetate (57.2mL). To this was added 4M HCl in dioxane (19.1mL, 76.27mmol, 4.444eq.). The mixture was stirred at room temperature for 18 hours. The resulting suspension was filtered and the solid was rinsed with diethyl ether and dried under vacuum.

### Part E: (1-{2,2-Dimethyl-1-[2-(1,2,3,4-tetrahydro-naphthalen-1-ylcarbamoyl)-pyrrolidine-1-carbonyl]-propylcarbamoyl}-ethyl)-carbamic acid tert-butyl ester

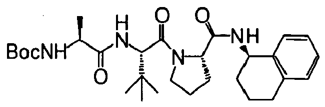

The Boc-Alanine (0.700g, 3.7mmol), 1-(2-Amino-3,3-dimethyl-butyryl)-pyrrolidine-2-carboxylic acid (1,2,3,4-tetrahydro-naphthalen-1-yl)-amide HCl salt (1.603g, 4.07mmol, 1.1 eq.), EDCI (1.063g, 5.55mmol, 1.5eq.), and HOBt (0.679g, 4.44mmol, 1.2eq.) were combined and dissolved in acetonitrile (14.8mL, 0.25M). After stirring for ten minutes, DIEA (3.16mL, 18.50mmol, 5eq.) was added and the mixture was stirred at room temperature for 18 hours. The reaction mixture was concentrated under reduced pressure and was re-suspended in 250mL ethyl acetate and washed with 25mL of 1M HCl(2X), brine, saturated sodium bicarbonate(2X), brine. The ethyl acetate was dried (Na₂SO₄) and solvent was removed to yield the crude title compound.

### Part F: 1-[2-(2-Amino-propionylamino)-3,3-dimethyl-butyryl]-pyrrolidine-2-carboxylic acid (1,2,3,4-tetrahydro-naphthalen-1-yl)-amideHCl salt

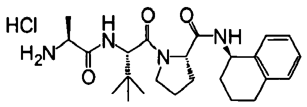

(1-{2,2-Dimethyl-1-[2-(1,2,3,4-tetrahydro-naphthalen-1-ylcarbamoyl)-pyrrolidine-1-carbonyl]-propylcarbamoyl}-ethyl)-carbamic acid tert-butyl ester (3.7mmol) was dissolved in ethyl acetate (15mL). To this was added 4M HCl in dioxane (5mL, 20mmol, 5.4eq.). The mixture was stirred at room temperature for 18 hours. The resulting suspension was filtered and the solid was rinsed with diethyl ether and dried under vacuum.

### Part G: 1-[2-(2-Benzylamino-propionylamino)-3,3-dimethyl-butyryl]-pyrrolidine-2-carboxylic acid (1,2,3,4-tetrahydro-naphthalen-1-yl)-amide

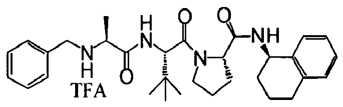

1-[2-(2-Amino-propionylamino)-3,3-dimethyl-butyryl]-pyrrolidine-2-carboxylic acid (1,2,3,4-tetrahydro-naphthalen-1-yl)-amideHCl salt (54mg, 0.12mmol) was dissolved in THF (1.16mL, 0.1M). To this was added triethylamine (16µL, 0.12mmol, leq.) and benzaldehyde (12 µL, 0.12mmol, leq.). The mixture was stirred for 2 hours, at which time sodium borohydride (13mg, 0.35mmol, 3eq.) was added. This mixture was stirred at room temperature for 18 hours. The reaction mixture was then filtered, and the precipitate rinsed with THF. The THF portions were combined and solvent was removed under pressure. The crude material was purified using reverse phase HPLC, using a C18 Vydac column and eluting with a gradient of 5% to 50% acetonitrile in water containing 0.1% TFA over 30 minutes, yielding (0.027g). MS(ES) for C₃₁H₄₂N₄O₃ (MW=518.7): positive 519.34 (MH+), negative 517.22 (MH-). Analytical HPLC Rₜ=11.23 min. The compounds in the following Table were prepared using appropriate starting materials and using the same procedures used to prepare example 5

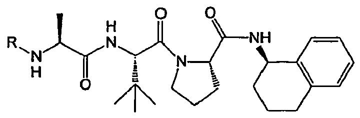

| Example | R = | Mass | (M+H) | (M-H) | Rₜ*(Min) |
|---|---|---|---|---|---|
| 6 | Cyclohexylmethyl | 524.74 | 525.39 | 523.28 | 13.23 |
| 7 | Isobutyl | 484.67 | 485.4 | 483.28 | 10.14 |
| 8 | Phenylpropyl | 546.74 | 547.4 | 545.26 | 11.9 |
| 9 | n-Propyl | 470.65 | 471.45 | 469.32 | 9.73 |
| 10 | Cyclopropylmethyl | 482.66 | 483.38 | 481.26 | 11.21 |

| | | | | | |
|---|---|---|---|---|---|
| *using analytical HPLC method defined at the beginning of the experimental section. | | | | | |

### Example 11

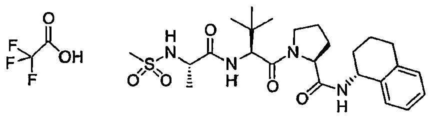

### 1-[2-(2-Methanesulfonylamino-propionylamino)-3,3-dimethyl-butyryl]pyrrolidine-2-carboxylic acid (1,2,3,4-tetrahydro-naphthalen-1-yl)-amide triofluoro-acetic acid salt

To a cooled (0 °C) solution of 1-[2-(2-Amino-propionylamino)-3,3-dimethyl-butyryl]-pyrrolidine-2-carboxylic acid (1,2,3,4-tetrahydro-naphthalen-1-yl)-amide hydrochloride salt (0.465g, 1 mmol) and DIEA (0.38 mL, 2.2 mmol) in THF (2 mL) was added methanesulfonyl chloride (77.4 µL, 1 mmol). The solution was stirred for 90 minutes while warming to room temperature. The reaction mixture was then diluted with ethyl acetate (20 mL), washed with water (3x10 mL) and brine (10 mL), dried over Na₂SO₄ and concentrated under reduced pressure to yield the title compound (0.490g, 96.7 %). 0.245g of the title compound was further purified using reverse phase HPLC, using a C18 Vydac column and eluting with a gradient of 5% to 50% acetonitrile in water containing 0.1 % TFA over 30 minutes, yielding the title compound (52mg, 16.8 %). Analytical HPLC Rₜ- 11.100 min. MS(ES) for C₂₇H₃₉F₃N₄O₇S (MW=620.68): positive 507 (MH+), negative 505/619 (MH-).

### Example 12

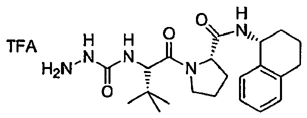

### Azaglycine-tert-butylglycine-Proline-(1,2,3,4-tetrahydro-naphthalen-1-yl)-amine; trifluoroacetic acid salt

### Part A: tBOC-Azaglycine-tert-butylglycine-Proline-(1,2,3,4-tetrahydro-naphthalen-1-yl)-amine

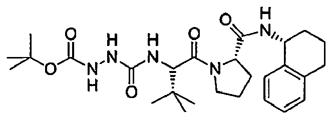

The 1-(2-amino-3,3-dimethyl-butyryl)-pyrrolidine-2-carboxylic acid (1,2,3,4-tetrahydro-naphthalen-1-yl)-amide; hydrochloride salt (330 mg; 0.838 mmol) was added to a solution of 1,1'-carbonyldiimidazole (CDI) (670 mg, 4.13 mmol) in dichloromethane (DCM) (15 mL). Slowly, triethylamine (TEA) was added to this mixture, and stirred at r.t. for 2 hours. The *tert*-butyl carbazate (780 mg, 5.90 mmol) was added, and the stirring continued for 18 hours. The reaction mixture was treated with water (20 mL), then partitioned between ethyl acetate/water. The organic phase was washed with 1 N HCl (25 mL), saturated sodium bicarbonate solution (50 mL) and brine solution (25 mL), dried over Na₂SO₄ and evaporated to dryness. The residue was purified by medium pressure column chromatography on silica gel eluting with ethyl acetate/hexane (0-100%) to give the title compound (350 mg, 81%) as a glassine solid. TLC (ethyl acetate) R*_{f}*= 0.18; MS (ES) for C₂₇H₄₁N₅O₅ (MW=515.65): positive 516 (M+H), negative 514 (M-H).

### Part B: Azaglycine-tert-butylglycine-Proline-(1,2,3,4-tetrahydro-naphthalen-1-yl)-amine; trifluoroacetic acid salt

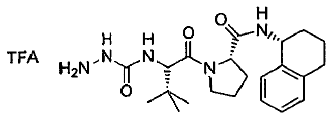

The *t*BOC-Azaglycine-*tert*-butylglycine-Proline-(1,2,3,4-tetrahydro-naphthalen-1-yl)-amine (301 mg, 0.383 mmol) was dissolved in dichloromethane (DCM) (20 mL). Trifluoroacetic acid (TFA) (5 mL) was added. After stirring at room temperature for 3 hours, the solvent was evaporated. The crude material was purified using reverse phase HPLC, using a C18 Vydec column and eluting with a gradient of 5% to 60% acetonitrile in water containing 0.1% TFA over 30 minutes, yielding the title compound as a white solid (145 mg, 71%). Analytical HPLC Rₜ-9.06 min); MS (ES) for C₂₂H₃₃N₅O₃ (MW=415.53): positive 416 (M+H), negative 414 (M-H).

### Example 13

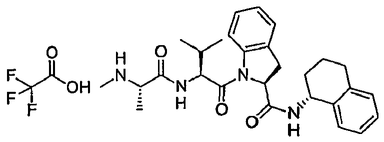

### 1-[3-Methyl-2-(2-methylamino-propionylamino)-butyryl]-1,3-dihydro-1H-indole-1-carboxylic acid (1,2,3,4-tetrahydro-naphthalen-1-yl)-amide trifluoro-acetic acid salt

### Part A: 2,3-Dihydro-1H-indole-2-carboxylic acid ethyl ester; hydrochloride

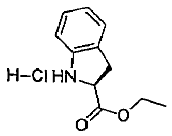

To a solution of 2,3-Dihydro-1*H*-indole-2-carboxylic acid (2.173 g, 10 mmol) in ethanol (20 mL) was added 6M HCl in ethyl acetate (3 mL). The resulting solution was stirred for 18 hours. Solvent was removed under reduced pressure. The resulting solid was triturated in ether to yield the title compound (2.555 g , 95.4 %).

### Part B: 1-(2-Benzyloxycarbonylamino-3-methyl-butyryl)-2,3-dihydro-1H-indole-2-carboxylic acid ethyl ester

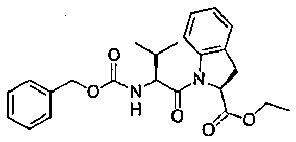

To a solution of 2-Benzyloxycarbonylamino-3-methyl-butyric acid (0.754 g, 3 mmol) and pyridine (0.29 mL, 3.6 mmol) in methylene chloride (9 mL) was added cyanuric fluoride (0.30 mL, 3.6 mmol). After stirring for 2.5 hours the resulting precipitate was filtered off and washed with methylene chloride. The mother liquor was diluted with ethyl acetate (30 mL); washed with saturated sodium bicarbonate solution (20 mL), 5% KHSO₄ (20 mL), and brine (20 mL); reduced under vacuum and taken up in methylene chloride (6 mL). The solution was cooled to -30 °C.

A solution of 2,3-Dihydro-1*H*-indole-2-carboxylic acid ethyl ester; hydrochloride (0.723 g, 2.7 mmol) was partitioned between saturated sodium bicarbonate solution_{(aq)} (20 mL)and ethyl acetate (20 mL), layers were separated, and the aqueous layers were extracted with ethyl acetate (2x20 mL). Combined extracts were washed with brine, dried over Na₂SO₄, and solvent was removed under reduced pressure.

This was dissolved in methylene chloride (6 mL) with 2,6-di-tert-butyl pyridine (0.74 mL, 3.3 mmol) and added to the acid fluoride solution. The resulting mixture was stirred for 18 hours; diluted with ethyl acetate (30 mL); washed with 5% KHSO₄ (3x20 mL), saturated sodium bicarbonate solution (3x20 mL), and brine (20 mL). The solution was dried over Na₂SO₄ and solvent was removed under reduced pressure. Purified via flash column chromatography (1:3 ethyl acetate/hexane) to yield the title compound (1.09 g, 86.9 %). TLC (1:3 Ethyl acetate/Hexane) R_{f} = 0.48.

### Part C: 1-(2-Benzyloxycarbonylamino-3-methyl-butyryl)-2,3-dihydro-1H-indole-2-carboxylic acid

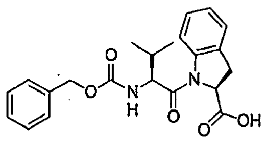

To a solution of 1-(2-Benzyloxycarbonylamino-3-methyl-butyryl)-2,3-dihydro-1H-indole-2-carboxylic acid ethyl ester (0.959 g, 2.06 mmol) in 1:1 dioxane/water (6 mL) was added 1 N LiOH (2.06 mL). The solution was stirred for 3 hours, then diluted with water (20 mL) and washed with ethyl ether (3x10 mL), acidified (pH~2), extracted with ethyl acetate (3x20 mL). Combined ethyl acetate layers were washed with brine (20 mL), dried over Na₂SO₄, and reduced under vacuum to yield the title compound (0.899 g, >99 %)

### Part D: {2-Methyl-1-[2-(1,2,3,4-tetrahydro-naphthalen-1-ylcarbamoyl)-2,3-dihydro-indole-1-carbonyl]-propyl}-carbamic acid benzyl ester

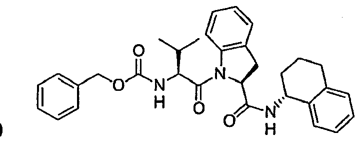

To a solution of 1-(2-Benzyloxycarbonylamino-3-methyl-butyryl)-2,3-dihydro-1H-indole-2-carboxylic acid (0.899 g, 2.06 mmol) in ethyl acetate (20 mL) was added propane phosponic acid anhydride (T3P, 50% w/w soln in ethyl acetate (3.75 mL, 6.18 mmol). The resulting solution was stirred for 10 min. 1,2,3,4-Tetrahydro-naphthalen-1-ylamine (0.28 mL, 2.06 mmol) was added followed by the addition of NMM (2.26 mL, 20.6 mmol). The resulting solution was stirred for 1 hour, diluted with ethyl acetate (50 mL), washed with 5% KHSO₄ (3x20 mL), saturated sodium bicarbonate solution (3x20 mL), and brine (20 mL). The solution was dried over Na₂SO₄ and solvent was removed under reduced pressure to give the title compound (1.075 g, 92.3 %). TLC (1:1 ethyl acetate/hexane) R_{f} = 0.70.

### Part E: 1-(2-Amino-3-methyl-butyryl)-2,3-dihydro-1H-indole-2-carboxylic acid (1,2,3,4-tetrahydro-naphthalen-1-yl)-amide hydrobromide salt

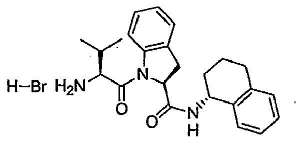

To a solution of 30 % acetic acid in HBr (1 mL) was added {2-Methyl-1-[2-(1,2,3,4-tetrahydro-naphthalen-1-ylcarbamoyl)-2,3-dihydro-indole-1-carbonyl]-propyl}-carbamic acid benzyl ester (0.500 g, 0.885 mmol). The resulting solution was stirred for 45 minutes and diluted with ethyl ether (10 mL). The resulting precipitate was filtered off and washed with ethyl ether and hexanes. Dried on vacuum pump and carried on to the next step without further purification, assuming quantitative yield.

### Part F: Methyl-(1-{2-methyl-1-[2-(1,2,3,4-tetrahydro-naphthalen-1-ylcarbamoyl)-2,3-dihydro-indole-1-carbonyl]-propylcarbamoyl}-ethyl)-carbamic acid tert-butyl ester

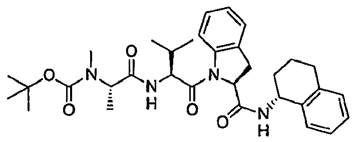

To a solution of 2-(tert-Butoxycarbonyl-methyl-amino)-propionic acid (0.180 g, 0.885 mmol) in ethyl acetate (8 mL) was added propane phosponic acid anhydride (T3P, 50% w/w soln in ethyl acetate ( 1.61 mL, 2.66 mmol). The resulting solution was stirred for 10 minutes, then 1-(2-Amino-3-methyl-butyryl)-2,3-dihydro-1H-indole-2-carboxylic acid (1,2,3,4-tetrahydro-naphthalen-1-yl)-amide hydrobromide salt (0.418 g, 0.885 mmol) was added followed by addition of NMM (0.97 mL, 8.85 mmol). The solution was stirred for 2 hours, then diluted with ethyl acetate (80 mL), washed with 5% KHSO₄ (3x40 mL), saturated sodium bicarbonate solution (3x40 mL), and brine (40 mL). The solution was dried over Na₂SO₄ and solvent was removed under reduced pressure. The crude material was purified via flash column chromatography (1:1 ethyl acetate/hexanes) to yield the title compound (97.0 mg, 19.0 %). TLC (1:1 ethyl acetate/Hexane R_{f} = 0.52.

### Part G: 1-[3-Methyl-2-(2-methylamino-propionylamino)-butyryl]-2,3-dihydro-1H-indole-2-carboxylic acid (1,2,3,4-tetrahydro-naphthalen-1-yl)-amide trifluoro-acetic acid salt

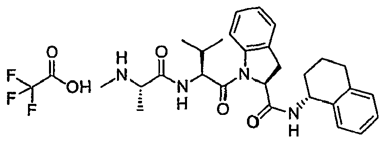

To a solution of **Methyl-(1-{2-methyl-1-{2-(1,2,3,4-tetrahydro-naphthalen-1-ylcarbamoyl)-2,3-dihydro-indole-1-carbonyl]-propylcarbamoyl}-ethyl)-carbamic acid tert-butyl ester** (97 mg, 0.168 mmol) in ethyl acetate (1 mL) was added 6M HCl in ethyl acetate (2 mL). The resulting solution was stirred for 18 hours, after which solvent was removed under reduced pressure. The crude product was purified using reverse phase HPLC, using a C18 Vydec column and eluting with a gradient of 5% to 50% acetonitrile in water containing 0.1% TFA over 30 minutes, yielding the title compound (37 m, 37.3 %). Analytical HPLC Rₜ-11.5 min. MS(ES) for C₃₀H₃₇F₃N₄O₅ (MW=590.64): positive 477 (M+H) negative 475/589 (M-H).

### Example 14

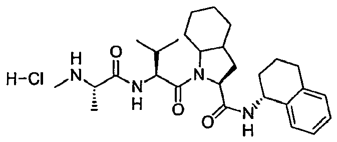

### 1-[3-Methyl-2-(2-methylamino-propionylamino)-butyryl]-octahydro-indole-1-carboxylic acid (1,2,3,4-tetrahydro-naphthalen-1-yl)-amide hydrochloride salt

### Part A: 2-(1,2,3,4-Tetrahydro-naphthalen-1-ylcarbamoyl)-octahydro-indole-1-carboxylic acid tert-butyl ester

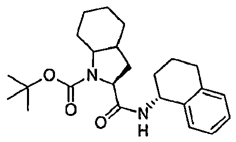

To a solution of Octahydro-indole-1,2-dicarboxylic acid 1-tert-butyl ester (0.539 g, 2.0 mmol) in ethyl acetate (4 mL) was added propane phosponic acid anhydride (T3P, 50% w/w soln in ethyl acetate) (3.64 mL, 6.0 mmol). The resulting solution was stirred for 10 minutes, then 1,2,3,4-Tetrahydro-naphthalen-1-ylamine (0.30 mL, 3.0 mmol) was added followed by the addition of NMM (2.20 mL, 20.0 mmol). The resulting solution was stirred for 1 hour, diluted with ethyl acetate (50 mL), washed with 5% KHSO₄ (3x20 mL), saturated sodium bicarbonate solution (3x20 mL), and brine (20 mL). The solution was dried over Na₂SO₄ and solvent was removed under reduced pressure. Purified via flash column chromatography (1:3 ethyl acetate/hexane) to give the title compound (0.654 g, 82.0 %). TLC (1:3 Ethyl acetate/Hexane) R_{f} = 0.40.

### Part B: Octahydro-indole-2-carboxylic acid (1,2,3,4-tetrahydro-naphthalen-1-yl)-amide hydrochloride salt

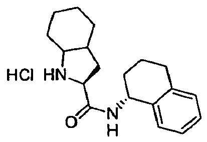

To a solution of 2-(1,2,3,4-Tetrahydro-naphthalen-1-ylcarbam-oyl)-octahydroindole-1-carboxylic acid tert-butyl ester (0.654 g, (1.64 mmol) in ethyl acetate (2 mL) was added 6M HCl in ethyl acetate (3 mL). The solution was stirred for 3 hours and the solvent was removed under reduced pressure. The resulting solid was triturated in ethyl ether to yield the title compound (0.500 g, 91.1 %).

### Part C: {2-Methyl-1-[2-(1,2,3,4-tetrahydro-naphthalen-1-ylcarbamoyl)-octahydro-indole-1-carbonyl]-propyl}-carbamic acid tert-butyl ester

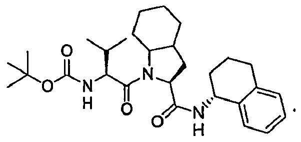

To a solution of 2-tert-Butoxy-carbonylamino-3-methyl-butyric acid (0.324 g, 1.49 mmol) in ethyl acetate (3 mL) was added phosponic acid anhydride (T3P, 50% w/w soln in ethyl acetate) ( 2.72 mL, 4.48 mmol). The resulting solution was stirred for 10 minutes, then Octahydro-indole-2-carboxylic acid (1,2,3,4-tetrahydro-naphthalen-1-yl)-amide hydrochloride salt (0.500 g, 1.49 mmol) was added followed by addition of NMM (1.64 mL, 14.9 mmol). The solution was stirred for 40minutes then diluted with ethyl acetate (80 mL), washed with 5% KHSO₄ (3x40 mL), saturated sodium bicarbonate solution (3x40 mL), and brine (40 mL). he solution was dried over Na₂SO₄ and solvent was removed under reduced pressure. The crude material was purified via flash column chromatography, yielding the title compound (0.581 g, 78.3 %). TLC (1:1 Ethyl acetate/Hexane) R_{f}: 0.81.

### Part D: 1-(2-Amino-3-methyl-butyryl)-octahydro-indole-2-carboxylic acid (1,2,3,4-tetrahydro-naphthalen-1-yl)-amide hydrochloride salt

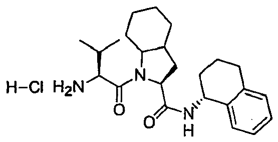

To a solution of {2-Methyl-1-[2-(1,2,3,4-tetrahydro-naphthalen-1-ylcarbamoyl)-octahydro-indole-1-carbonyl]-propyl}-carbamic acid tert-butyl ester (0.574 g, 1.15 mmol) in ethyl acetate (2 mL) was added 6M HCl in ethyl acetate (2 mL). The resulting solution was stirred for 18 hours, then the solvent was removed via reduced pressure, and the resulting crude solid was triturated in ethyl ether to yield the title compound (0.476 g, 95.4 %).

### Part E: Methyl-(1-{2-methyl-1-[2-(1,2,3,4-tetrahydro-naphthalen-1-ylcarbamoyl)-octahydro-indole-1-carbonyl]-propylcarbamoyl}-ethyl)-carbamic acid tert-butyl ester

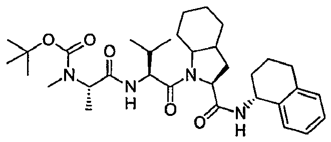

To a solution of 2-(tert-Butoxycarbonyl-methyl-amino)-propionic acid (0.223 g, 1.10 mmol) in ethyl acetate (2 mL) was added propane phosponic acid anhydride (T3P, 50% w/w soln in ethyl acetate) ( 1.99 mL, 3.24 mmol). The resulting solution was stirred for 10 minutes, followed by addition of 1-(2-Amino-3-methyl-butyryl)-octahydro-indole-2-carboxylic acid (1,2,3,4-tetrahydro-naphthalen-1-yl)-amide hydrochloride salt (0.476 g, 1.10 mmol), further followed by addition of NMM (1.21 mL, 11.0 mmol). The solution was stirred for 2 hours, then diluted with ethyl acetate (80 mL), washed with 5% KHSO₄ (3x40 mL), saturated sodium bicarbonate solution (3x40 mL), and brine (40 mL). The solution was dried over Na₂SO₄ and solvent was removed under reduced pressure. The crude material was purified via flash column chromatography (1:1 Ethyl acetate/hexanes) to yield the title compound (0.401 g, 62.8 %). TLC (1:1 ethyl acetate/hexane) R*_{f}*. 0.60.

### Part F: 1-[3-Methyl-2-(2-methylamino-propionylamino)-butyryl]-octahydro-indole-2-carboxylic acid (1,2,3,4-tetrahydro-naphthalen-1-yl)-amide hydrochloride salt

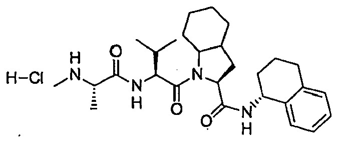

To a solution of Methyl-(1-{2-methyl-1-[2-(1,2,3,4-tetrahydro-naphthalen-1-ylcarbamoyl)-octahydro-indole-1-carbonyl]-propylcarbamoyl} -ethyl)-carbamic acid tert-butyl ester (0.400 g, 0.686 mmol) in ethyl acetate (1 mL) was added 6M HCl in ethyl acetate (0.5 mL). The resulting solution was stirred for 18 hours, followed by removal of solvent under reduced pressure. The crude product was triturated in ethyl ether to yield the title compound (0.300 g, 84.2 %). Analytical HPLC Rₜ-10.589 min. MS(ES) for C₂₈H₄₃ClN₄O₃ (MW=519.12): positive 483 (M+H) negative 481/517 (M-H).

### Example 15

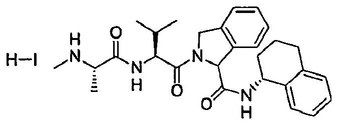

### 2-[3-Methyl-2-(2-methylamino-propionylamino)-butyryl]-2,3-dihydro-1H-isoindole-1-carboxylic acid (1,2,3,4-tetrahydro-naphthalen-1-yl)-amide hydriodide salt

### Part A: 1-(1,2,3,4-Tetrahydro-naphthalen-1-ylcarbamoyl)-1,3-dihydro-isoindole-2-carboxylic acid tert-butyl ester

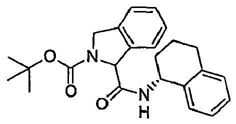

To a solution of 1,3-Dihydro-isoindole-1,2-dicarboxylic acid 2-tert-butyl ester (0.527 g, 2.0 mmol) in ethyl acetate (4 mL) was added propane phosponic acid anhydride (T3P, 50% w/w soln in ethyl acetate) (3.64 mL, 6.0 mmol). The resulting solution was stirred for 10 minutes, followed by addition of 1,2,3,4-Tetrahydro-naphthalen-1-ylamine (0.30 mL, 3.0 mmol), and further followed by the addition of NMM (2.20 mL, 20.0 mmol). The resulting solution was stirred for 1 hour, diluted with ethyl acetate (50 mL), washed with 5% KHSO₄ (3x20 mL), saturated sodium bicarbonate solution (3x20 mL), and brine (20 mL). The solution was dried over Na₂SO₄ and solvent was removed under reduced pressure. The crude material was purified via flashcolumn chromatography (1:3 Ethyl acetate/hexanes) to yield the title compound (0.740 g, 94.3 %). TLC (1:3 ethyl acetate/hexane) R_{f}= 0.31.

### Part B: 2,3-Dihydro-1H-isoindole-1-carboxylic acid (1,2,3,4-tetrahydro-naphthalen-1-yl)-amide; hydrochloride salt

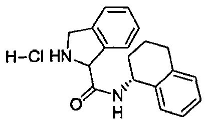

To a solution of 1**-(1,2,3,4-Tetrahydro-naphthalen-1-ylcarbamoyl)-1,3-dihydro-isoindole-2-carboxylic acid tert-butyl ester** (0.740 g, 1.885 mmol) in ehyl acetate (2 mL), was added 6M HCl in ethyl acetate (3 mL). The solution was stirred for 90 minutes followed by concentration of the reaction mixture under reduced pressure. The resulting solid was triturated in ethyl ether to give 0.572 g (92.3 %)

### Part C: {2-Methyl-1-[1-(1,2,3,4-tetrahydro-naphthalen-1-ylcarbamoyl)-1,3-dihydro isoindole-2-carbonyl]-propyl}-carbamic acid tert-butyl ester

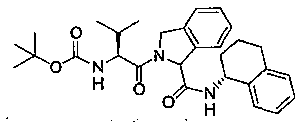

To a solution of 2-tert-Butoxy-carbonylamino-3-methyl-butyric acid (0.378 g, 1.74 mmol), 2,3-Dihydro-1H-isoindole-1-carboxylic acid (1,2,3,4-tetrahydro-naphthalen-1-yl)-amide; hydrochloride salt (1,2,3,4-tetrahydro-naphthalen-1-yl)-amide; hydrochloride salt (0.572 g, 1.74mmol), and NMM (1.91 mL, 17.4 mmol) in ethyl acetate (3 mL) was added propane phosponic acid anhydride (T3P, 50% w/w soln in ethyl acetate) ( 3.16 mL, 5.22 mmol). The resulting solution was stirred for 30 minutes. The reaction mixture was then diluted with ethyl acetate (100 mL), and washed with 5% KHSO₄ (3x50 mL), saturated sodium bicarbonate solution (3x50 mL), and brine (50 mL). The solution was dried over Na₂SO₄ and solvent was removed under reduced pressure. The crude material was purified via flash column chromatography (1:3 ethyl acetate/hexanes) yielding the title compound (0.754 g, 88.3%) TLC (1:1 ethyl acetate/hexane) R_{f} = 0.63.

### Part D: 2-(2-Amino-3-methyl-butyryl)-2,3-dihydro-1H-isoindole-1-carboxylic acid (1,2,3,4-tetrahydro-naphthalen-1-yl)-amide; hydrochloride salt

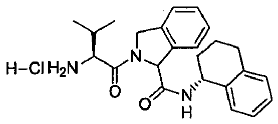

To a solution of {2-Methyl-1-[1-(1,2,3,4-tetrahydro-naphthalen-1-ylcarbamoyl)-1,3-dihydro-isoindole-2-carbonyl]-propyl}-carbamic acid tert-butyl ester (0.520 g, 1.06 mmol) in ethyl acetate (10 mL) was added 6M HCl in ethyl acetate (5 mL). The resulting solution was stirred for 18 hours, followed by filtration of the resulting precipitate, which was washed with ethyl acetate and hexanes to give the title compound (0.342 g, 75.3 %).

### Part E: Methyl-(1-{2-methyl-1-[1-(1,2,3,4-tetrahydro-naphthalen-1-ylcarbamoyl)-1,3-dihydro-isoindole-2-carbonyl]-propylcarbamoyl}-ethyl)-carbamic acid tert-butyl ester

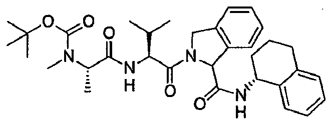

To a solution of 2-(tert-Butoxycarbonyl-methyl-amino)-propionic acid (0.162 g, 0.80 mmol), 2-(2-Amino-3-methyl-butyryl)-2,3-dihydro-1H-isoindole-1-carboxylic acid (1,2,3,4-tetrahydro-naphthalen-1-yl)-amide; hydrochloride salt (0.342 g, 0.80 mmol), and NMM (0.88 mL, 8.0 mmol) in ethyl acetate (1.6 mL) was added propane phosponic acid anhydride (T3P, 50% w/w soln in ethyl acetate) ( 1.45 mL, 2.4 mmol). The resulting solution was stirred for 30 minutes. The reaction mixture was then diluted with ethyl acetate (80 mL), washed with 5% KHSO₄ (3x40 mL), saturated sodium bicarbonate solution (3x40 mL), and brine (40 mL). The solution was dried over Na₂SO₄ and solvent was removed under reduced pressure. The crude material was purified via flash column chromatography (1:1 Ethyl acetate/hexanes) yielding the title compound and its diastereomer (0.155 g (less polar) and 0.131 g (more polar) for a combined yield of 0.246 g (53.4 %). TLC (1:1 Ethyl acetate/Hexane) R_{f} = 0.47 and 0.28 (respectively).

### Part F: 2-[3-Methyl-2-(2-methylamino-propionylamino)-butyryl]-2,3-dihydro-1H-isoindole-1-carboxylic acid (1,2,3,4-tetrahydro-naphthalen-1-yl)-amide hydriodide salt (less polar)

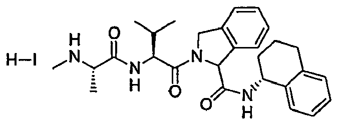

To a solution of **Methyl-(1-{2-methyl-l-[1-(1,2,3,4-tetrahydro-naphthalen-1-ylcarbamoyl)-1,3-dihydro-isoindole-2-carbonyl]-propylcarbamoyl}-ethyl)-carbamic acid tert-butyl ester** (less polar product from preceding reaction) (0.122 g, 0.212 mmol) in methylene chloride (2 mL) was added trimethylsilyl iodide (TMSI) 60.2 µL, 0.423 mmol). After 10 minutes the solution was quenched with methanol (0.5 mL); concentrated under reduced pressure and the resulting solid triturated in ethyl ether to yield the title compound (0.102 g. 79.7 %) Analytical HPLC Rₜ: 10.930 min. MS (ES) for C₂₈H₃₇IN₄O₃ (MW=604.53): positive 477 (M+H) negative 475/603 (M-H).

The more polar product from the preceding reaction was treated in a similar manner with TMSI and triturated with ethyl ether to yield the title compound, presumably as the opposite epimer at the dihydro-isoindole center (0.121 g (91.0 %). Analytical HPLC Rₜ-10.722 min: MS (ES) for C₂₈H₃₇IN₄O₃ (MW=604.53): positive 477 (M+H) negative 475/603 (M-H).

### Example 16

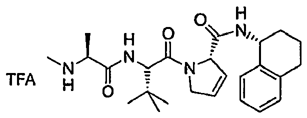

### 1-[3,3-Dimethyl-2-(2-methylamino-propionylamino)-butyryl]-2,5-dihydro-1H-pyrrole-2-carboxylic acid (1,2,3,4-tetrahydro-naphthalen-1-yl)-amide; trifluoroacetic acid salt

### Part A: 2-(1,2,3,4-Tetrahydro-naphthalen-1-ylcarbamoyl)-2,5-dihydro-pyrrole-1-carboxylic acid tert-butyl ester

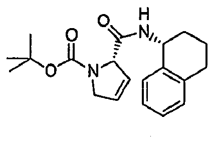

A solution of 2,5-dihydro-pyrrole-1,2-dicarboxylic acid 1-tert-butyl ester (392.4 mg, 1.84 mmol) and (R)-1,2,3,4-tetrahydro-naphthalen-1-ylamine (564.3 mg, 3.83 mmol) in tetrahydrofuran (THF) (40 mL) was added 1-ethyl-3-(3-dimethylaminopropyl)carbodiimide hydrochloride (EDCI) (860 mg, 4.48 mmol) and 1-hydroxybenzotriazole monohydrate (HOBt) (450 mg, 3.33 mmol), followed by N-methyl-2-pyrrolidinone (0.75 mL) and 4-methyl morpholine (0.75 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (630 mg, >95%) as a glassine solid. TLC (50% ethyl acetate/hexane) R_{f}= 0.31; MS (ES) for C₂₀H₂₆N₂O₃ (MW=342.43): positive 343 (M+H).

### Part B 2,5-Dihydro-1H-pyrrole-2-carboxylic acid (1,2,3,4-tetrahydro-naphthalen-1-yl)-amide; trifluoroacetic acid salt

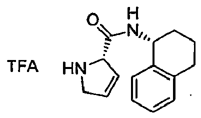

The 2-(1,2,3,4-tetrahydro-naphthalen-1-ylcarbamoyl)-2,5-dihydro-pyrrole-1-carboxylic acid *tert*-butyl ester (517.2 mg, 1.51 mmol) was dissolved in dichloromethane (DCM) (10 mL). Trifluoroacetic acid (TFA) (4 mL) was added. After stirring at room temperature for 1 hour, the solvent was evaporated to give the title compound as a crystalline solid (538 mg, >95%). MS(ES) for C₁₅H₁₈N₂O (MW=242.32): positive 243 (M+H).

### Part C: (1-{2,2-Dimethyl-1-[2-(1,2,3,4-tetrahydro-naphthalen-1-ylcarbamoyl)-2,5-dihydro-pyrrole-1-carbonyl]-propylcarbamoyl}-ethyl)-methyl-carbamic acid tert-butyl ester

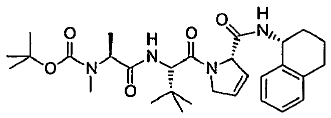

A solution of 2-[2-(tert-butoxycarbonyl-methyl-amino)-propionylamino]-3,3-dimethyl-butyric acid (507.6 mg, 1.60 mmol) and 2,5-dihydro-1*H*-pyrrole-2-carboxylic acid (1,2,3,4-tetrahydro-naphthalen-1-yl)-amide; trifluoroacetic acid salt (538 mg, 1.51 mmol) in tetrahydrofuran (THF) (50 mL) was added 1-ethyl-3-(3-dimethylaminopropyl)carbodiimide hydrochloride (EDCI) (600 mg, 3.13 mmol) and 1-hydroxybenzotriazole monohydrate (HOBt) (300 mg, 2.22 mmol), followed by *N*-methyl-2-pyrrolidinone (1.5 mL) and 4-methyl morpholine (1 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (565 mg, 69%) as a glassine solid. TLC (50% ethyl acetate/hexane) R*_{f}*= 0.15; MS (ES) for C₃₀H₄₄N₄O₅ (MW=540.69): positive 541 (M+H), negative 539 (M-H).

### Part D: 1-[3,3-Dimethyl-2-(2-methylamino-propionylamino)-butyryl]-2,5-dihydro-1H-pyrrole-2-carboxylic acid (1,2,3,4-tetrahydro-naphthalen-1-yl)-amide; trifluoroacetic acid salt

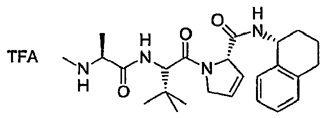

The (1-{2,2-dimethyl-1-[2-(1,2,3,4-tetrahydro-naphthalen-1-ylcarbamoyl)-2,5-dihydro-pyrrole-1-carbonyl]-propylcarbamoyl}-ethyl)-methyl-carbamic acid *tert*-butyl ester (519 mg, 0.960 mmol) was dissolved in dichloromethane (DCM) (10 mL). Trifluoroacetic acid (TFA) (4.5 mL) was added. After stirring at room temperature for 2 hours, the solvent was evaporated. The crude material was purified using reverse phase HPLC, using a C18 Vydec column and eluting with a gradient of 5% to 60% acetonitrile in water containing 0.1 % TFA over 30 minutes, yielding the title compound as a white solid (79 mg). Analytical HPLC Rₜ-9.39 min); MS (ES) for C₂₅H₃₆N₄O₃ (MW=440.58): positive 441 (M+H), negative 439 (M-H).

### Example 17

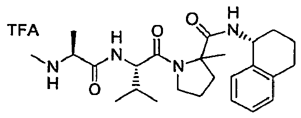

### 2-Methyl-1-[3-methyl-2-(2-methylamino-propionylamino)-butyryl]-pyrrolidine-2-carboxylic acid (1,2,3,4-tetrahydro-naphthalen-1-yl)-amide; trifluoroacetic acid salt

### Part A: 1-{2-[2-(tert-Butoxycarbonyl-methyl-amino)-propionylamino]-3-methyl-butyryl}-2-methyl-pyrrolidine-2-carboxylic acid

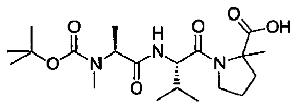

A solution of 2-methyl-pyrrolidine-2-carboxylic acid; hydrochloride salt (330 mg, 2.55 mmol) in tetrahydrofuran (THF) (40 mL) was added 1-ethyl-3-(3-dimethylaminopropyl)carbodiimide hydrochloride (EDCI) (750 mg, 3.01 mmol) and 1-hydroxybenzotriazole monohydrate (HOBt) (300 mg, 2.22 mmol), followed by *N*-methyl-2-pyrrolidinone (1 mL) and 4-methyl morpholine (1 mL). After this mixture was stirred at r.t. for 2 hrs, 2-[2-(*tert*-butoxycarbonyl-methyl-amino)-propionylamino]-3-methyl-butyric acid (436 mg, 1.44 mmol) was added. The resulting mixture was stirred at room temperature overnight. The reaction mixture was treated with water (20 mL), then partitioned between ethyl acetate/water. The organic phase was washed with 1N HCl (100 mL) and brine solution (50 mL), dried over Na₂SO₄ and evaporated to dryness to give the title compound (596 mg, 95%) as a sticky solid without further purification. TLC (50% ethyl acetate/hexane) R*_{f}*= 0.1 streaks; MS (ES) for C₂₀H₃₅N₃O₆ (MW= 413.51): positive 414 (M+H), negative 412 (M-H).

### Part B: Methyl-(1-{2-methyl-1-[2-methyl-2-(1,2,3,4-tetrahydro-naphthalen-1-ylcarbamoyl)-pyrrolidine-1-carbonyl]-propylcarbamoyl}-ethyl)-carbamic acid tert-butyl ester

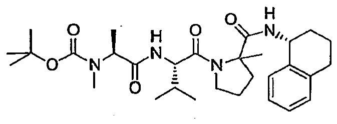

A solution of 1-{2-[2-(*tert*-butoxycarbonyl-methyl-amino)-propionylamino]-3-methyl-butyryl}-2-methyl-pyrrolidine-2-carboxylic acid (527.5 mg, 1.28 mmol) and (R)-1,2,3,4-tetrahydro-naphthalen-1-ylamine (570.2 mg, 3.87 mmol) in tetrahydrofuran (THF) (40 mL) was added 1-ethyl-3-(3-dimethylaminopropyl)carbodiimide hydrochloride (EDCI) (700 mg, 3.65 mmol) and 1-hydroxybenzotriazole monohydrate (HOBt) (375 mg, 2.77 mmol), followed by *N*-methyl-2-pyrrolidinone (1 mL) and 4-methyl morpholine (1 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (86.5 mg, 12%) as a glassine solid. TLC (50% ethyl acetate/hexane) R*_{f}*= 0.08; MS (ES) for C₃₀H₄₆N₄O₅ (MW=542.71): positive 543 (M+H), negative 541 (M-H). **Part C: 2-Methyl-1-[3-methyl-2-(2-methylamino-propionylamino)-butyryl]-pyrrolidine-2-carboxylic acid (1,2,3,4-tetrahydro-naphthalen-1-yl)-amide; trifluoroacetic acid salt**

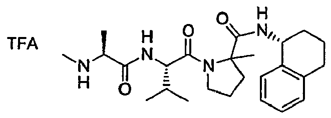

The methyl-(1-{2-methyl-1-[2-methyl-2-(1,2,3,4-tetrahydro-naphthalen-1-ylcarbamoyl)-pyrrolidine-1-carbonyl]-propylcarbamoyl}-ethyl)-carbamic acid *tert*-butyl ester (64 mg, 0.118 mmol) was dissolved in dichloromethane (DCM) (5 mL). Trifluoroacetic acid (TFA) (2 mL) was added. After stirring at room temperature for 2.5 hours, the solvent was evaporated. The crude material was purified using reverse phase HPLC, using a C18 Vydec column and eluting with a gradient of 5% to 60% acetonitrile in water containing 0.1% TFA over 30 minutes, yielding title compound as a white solid (46.5 mg). Analytical HPLC Rr9.32 min; MS (ES) for C₂₅H₃₈N₄O₃ (MW=442.59): positive 443 (M+H), negative 441 (M-H).

### Example 18

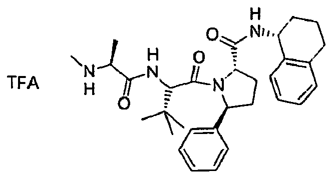

### 1-[3,3-Dimethyl-2-(2-methylamino-propionylamino)-butyryl]-5-phenyl-pyrrolidine-2-carboxylic acid (1,2,3,4-tetrahydro-naphthalen-1-yl)-amide; trifluoroacetic acid salt

### Part A: 2-Phenyl-5-(1,2,3,4-tetrahydro-naphthalen-1-ylcarbamoyl)-pyrrolidine-1-carboxylic acid 9H-fluoren-9-ylmethyl ester

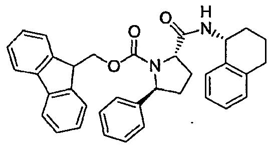

A solution of (2S,5R)-5-phenyl-pyrrolidine-1,2-dicarboxylic acid 1-(9*H*-fluoren-9-ylmethyl) ester (1001 mg, 2.42 mmol) and (R)-1,2,3,4-tetrahydro-naphthalen-1-ylamine (520 mg, 3.50 mmol) in tetrahydrofuran (THF) (40 mL) was added 1-ethyl-3-(3-dimethylaminopropyl)carbodiimide hydrochloride (EDCI) (950 mg, 4.95 mmol) and 1-hydroxybenzotriazole monohydrate (HOBt) (450 mg, 3.33 mmol), followed by *N*-methyl-2-pyrrolidinone (0.75 mL) and 4-methyl morpholine (0.75 mL). This mixture was stirred at room temperature for 5 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (1310 mg, >95%) as a glassine solid. TLC (25% ethyl acetate/hexane) R*_{f}*= 0.12; MS (ES) for C₃₆H₃₄N₂O₃ (MW=542.67): positive 543 (M+H).

### Part B: 5-Phenyl-pyrrolidine-2-carboxylic acid (1,2,3,4-tetrahydro-naphthalen-1-yl)-amide

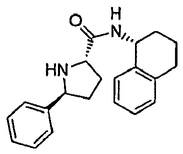

A solution of 2-phenyl-5-(1,2,3,4-tetrahydro-naphthalen-1-ylcarbamoyl)-pyrrolidine-1-carboxylic acid 9*H*-fluoren-9-ylmethyl ester (498 mg, 0.94 mmol) in dimethylformamide (5 mL) was treated with diethylamine (DEA) (5 mL) at room temperature for 2 hours. The DEA was evaported, and the resulting solution was used without further purification in the subsequent reaction. TLC (ethyl acetate) R*_{f}*= 0.47; MS (ES) for C₂₁H₂₄N₂O (MW=320.43): positive 321 (M+H), negative 319 (M-H).

### Part C: (1-{2,2-Dimethyl-1-[2-phenyl-5-(1,2,3,4-tetrahydro-naphthalen-1-ylcarbamoyl)-pyrrolidine-1-carbonyl]-propylcarbamoyl}-ethyl)-methyl-carbamic acid tert-butyl ester

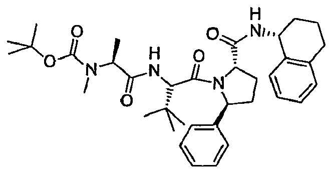

A solution of 2-[2-(tert-butoxycarbonyl-methyl-amino)-propionylamino]-3,3-dimethyl-butyric acid (379 mg, 1.20 mmol) and 5-phenyl-pyrrolidine-2-carboxylic acid (1,2,3,4-tetrahydro-naphthalen-1-yl)-amide (0.94 mmol in DMF from above reaction) in tetrahydrofuran (THF) (50 mL) was added 1-ethyl-3-(3-dimethylaminopropyl)carbodiimide hydrochloride (EDCI) (500 mg, 2.61 mmol) and 1-hydroxybenzotriazole monohydrate (HOBt) (250 mg, 1.85 mmol), followed by *N*-methyl-2-pyrrolidinone (0.75 mL) and 4-methyl morpholine (0.75 mL). This mixture was stirred over 6 days. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (199 mg, 34%) as a glassine solid. TLC (75% ethyl acetate/hexane) R*_{f}*= 0.47; MS (ES) for C₃₆H₅₀N₄O₅ (MW=618.81): positive 619 (M+H), negative 617 (M-H).

### Part D: 1-[3,3-Dimethyl-2-(2-methylamino-propionylamino)-butyryl]-5-phenyl-pyrrolidine-2-carboxylic acid (1,2,3,4-tetrahydro-naphthalen-1-yl)-amide; trifluoroacetic acid salt

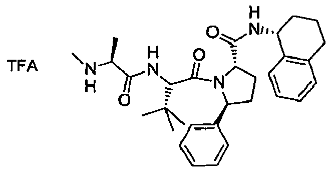

The (1-{2,2-dimethyl-1-[2-phenyl-5-(1,2,3,4-tetrahydro-naphthalen-1-ylcarbamoyl)-pyrrolidine-1-carbonyl]-propylcarbamoyl}-ethyl)-methyl-carbamic acid *tert*-butyl ester (185.5 mg, 0.300 mmol) was dissolved in dichloromethane (DCM) (5 mL). Trifluoroacetic acid (TFA) (2 mL) was added. After stirring at room temperature for 1.5 hours, the solvent was evaporated. The crude material was purified by reverse phase HPLC, using a C18 Vydec column and eluting with a gradient of 5% to 60% acetonitrile in water containing 0.1% TFA over 30 minutes, yielding the appropriate fractions were collected and solvents removed to give the title compound as a white solid (17.5 mg). Analytical HPLC Rₜ-11.17 min); MS(ES) for C₃₆H₅₀N₄O₅ (MW=518.69): positive 519 (M+H), negative 517 (M-H).

### Example 19

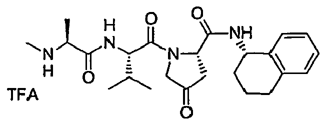

### 1-[3-Methyl-2-(2-methylamino-propionylamino)-butyryl]-4-oxo-pyrrolidine-2-carboxylic acid (1,2,3,4-t etrahydro-naphthalen-1-yl)-amide

### Part A: 4-Hydroxy-2-(1,2,3,4-tetrahydro-naphthalen-1-ylcarbamoyl)-pyrrolidine-1-carboxylic acid benzyl ester

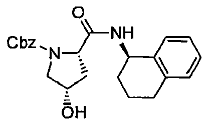

To a solution of 4-hydroxy-pyrrolidine-1,2-dicarboxylic 1-benzyl ester (2.07 g, 7.81 mmol) in dimethylformamide (DMF, 15 mL) was added 1-hydroxybenzotriazole (HOBt, 1.37 g, 10.15 mmol), 1-(3-dimethylaminopropyl)-3-ethylcarbodiimide hydrochloride (EDCI, 1.95 g, 10.15 mmol), *R*-1,2,3,4-tetrahydro-1-naphthylamine (1.28 g, 8.95 mmol), and diisopropylethylamine (DIEA, 2 mL, 11.72 mmol). The resulting mixture was stirred at room temperature for 18 hours. It was then diluted with ethyl acetate (100 mL), and washed successively with aq. 5% KHSO₄, saturated sodium bicarbonate solution, brine, dried (Na₂SO₄), and concentrated under reduced pressure. After removal of solvent, the residue was triturated with ether to give the pure title compound (0.7g, 88%).TLC (40% ethyl acetate/hexane) R_{f}= 0.18. MS (ES) for C₂₃H₃₈N₂O₄ (MW=394.46): positive 395(M+H).

### Part B: (1-{1-[4-Hydroxy-2-(1,2,3,4-tetrahydro-naphthalen-1-ylcarbamoyl)-pyrrolidine-1-carbonyl]-2-methyl-propylcarbamoyl}-ethyl)-methyl-carbamic acid tert-butyl ester

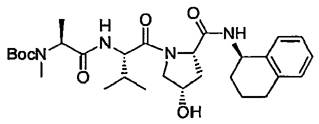

To a solution of 4-Hydroxy-2-(1,2,3,4-tetrahydro-naphthalen-1-ylcarbamoyl)-pyrrolidine-1-carboxylic acid benzyl ester ( 1.31g, 5.07 mmol) in methanol (20 mL) was added 10% Pd/C (200 mg). The reaction mixture was stirred under hydrogen (latm) for 18 hours, filtrated, then concentrated to yield the crude residue that was dissolved in DMF (20 mL) followed by addition of 2-[2-(tert-Butoxycarbonyl-methyl'-amino)-propionylamino]-3-methyl-butyric acid (1g, 3.31 mmol), HOBt (582 mg, 4.3 mmol), EDCI (824 mg, 4.3 mmol), and DIEA (0.86 mL, 4.95 mmol). The resulting mixture was stirred at room temperature for 18 hours. The crude reaction mixture was then diluted with ethyl acetate (100 mL), washed successively with aq. 5% KHSO₄, saturated sodium bicarbonate solution, brine, dried (Na₂SO₄), and concentrated under reduced pressure. The crude mixture was purified by flash column chromatography on silica gel eluting with using 5% methanol/ methylene chloride to afford the title compound (1.4 g, 80%). TLC (5% methanol/ methylene chloride) R_{f}= 0.31. MS (ES) for C₂₉H₄₄N₄O₆ (MW=544.68): positive 545 (M+H).

### Part C: 1-[3-Methyl-2-(2-methylamino-propionylamino)-butyryl]-4-oxo-pyrrolidine-2-carboxylic acid (1,2,3,4-tetrahydro-naphthalen-1-yl)-amide

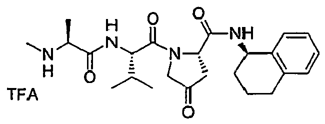

To a solution of (1-{1-[4-Hydroxy-2-(1,2,3,4-tetrahydro-naphthalen-1-ylcarbamoyl)-pyrrolidine-1-carbonyl]-2-methyl-propylcarbamoyl} -ethyl)-methyl-carbamic acid tert-butyl ester (260 mg, 0.48 mmol) in methylene chloride (5 mL) was added Dess-Martin reagent (448 mg, 1.06 mmol). The reaction mixture was stirred at room temperature for 2 hours, then diluted with ethyl acetate (10 mL), washed with saturated sodium bicarbonate solution, dried (Na₂SO₄), and concentrated to give the corresponding ketone. The crude ketone was then dissolved in ethyl acetate (3 mL) followed by addition of 4M HCl in 1,4-dioxane HCl (0.36 mL,) and stirred at room temperature for 18 hours. The crude product was concentrated under reduced pressure and purified by reverse phase HPLC, using a C18 Vydec column and eluting with a gradient of 5% to 40% acetonitrile in water containing 0.1% TFA over 30 minutes to yield the title compound (80 mg, 30%). Analytical HPLC Rₜ = 10.035 min. MS (ES) for C₂₄H₃₆N₄O₄ (MW=442.57): positive 443 (M+H).

### Example 20

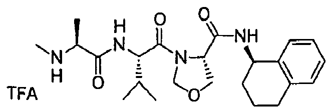

### 3-[3-Methyl-2-(2-methylamino-propionylamino)-butyryl]-oxazolidine-4-carboxylic acid (1,2,3,4-tetrahy dro-naphthalen-1-yl)-amide

### Part A: 4-(1,2,3,4-Tetrahydro-naphthalen-1-ylcarbamoyl)-oxazolidine-3-carboxylic acid benzyl ester

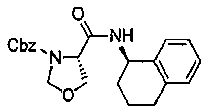

To a solution of oxazolidine-3,4-dicarboxylic acid 3-benzyl ester (3.12 g, 12.42 mmol) in dimethylformamide (DMF, 6 mL) was added 1-hydroxybenzotriazole (HOBt, 2.68 g, 19.9 mmol), 1-(3-dimethylaminopropyl)-3-ethylcarbodiimide hydrochloride (EDCI, 3.8 g, 19.82 mmol), R-1,2,3,4-tetrahydro-1-naphthylamine ( 2.97 g , 19.91 mmol), and diisopropylethylamine (DIEA, 4.33 mL, 19.9 mmol) and the resulting mixture was stirred at room temperature for 18 hours. It was then diluted with ethyl acetate (100 mL), washed successively with aq. 5% KHSO₄, saturated sodium bicarbonate solution, brine, dried (Na₂SO₄), and concentrated under reduced pressure. The crude residue was purified by flash column chromatography on silica gel eluting with 35% ethyl acetate/hexane to furnish the title compound (4.6g, 97%). TLC (40% ethyl acetate/hexane) R_{f}= 0.29. MS (ES) for C₂₂H₂₄N₂O₄ (MW=380.44): positive 381 (M+H).

### Part B: 3-[3-Methyl-2-(2-methylamino-propionylamino)-butyryl]-oxazolidine-4-carboxylic acid (1,2,3,4-tetrahydro-naphthalen-1-yl)-amide

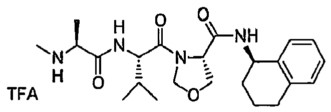

To a solution of 4-(1,2,3,4-Tetrahydro-naphthalen-1-ylcarbamoyl)-oxazolidine-3-carboxylic acid benzyl ester (784 mg, 2.05 mmol) in ethyl acetate (9 mL) was added 20% Pd(OH)₂ (150 mg) and triethylamine (0.75 mL). The resulting mixture was stirred under hydrogen (1 atm) for 1.5 hours. Filtration and concentration under reduced pressure gave the corresponding amine. In a separate flask was placed 2-[2-(tert-Butoxycarbonylmethyl'-amino)-propionylamino]-3-methyl-butyric acid (620 mg, 2.05 mmol) in tetrahydrofuran (THF, 7mL) triethylamine (0.32 ml, 2.26 mmol), trimethylacetyl chloride, and catalytic N,N-dimethylaminopyridine (DMAP, 15 mg, 0.12 mmol). The resulting mixture was stirred at 0°C for 1.5h followed by addition of the amine. The reaction mixture was stirred at room temperature for 18 hours, then quenched with aq. 5% KHSO₄, and diluted with ethyl acetate (20 mL). The organic phase was washed with saturated sodium bicarbonate solution, and brine to give a crude product that, without further purification, was dissolved in 2M HCl in ethyl acetate (1 mL). The reaction mixture was stirred at room temperature for 18 hours. The crude material was concentrated under reduced pressure and purified by reverse phase HPLC, using a C18 Vydec column and eluting with a gradient of 5% to 40% acetonitrile in water containing 0.1% TFA over 30 minutes to give the desired product (20 mg, 2.3%). Analytical HPLC Rₜ = 9.017 min. MS (ES) for C₂₃H₃₄N₄O₄ (MW=430.54): positive 431(M+H).

### Example 21

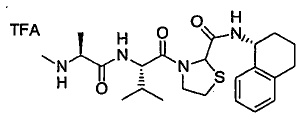

### 3-[3-Methyl-2-(2-methylamino-propionylamino)-butyryl]-thiazolidine-2-carboxylic acid (1,2,3,4-teirahydro-naphthalen-1-yl)-amide, trifluoroacetic acid salt

### Part A: 3-{2-[2-(tert-Butoxycarbonyl-methyl-amino)-propionylamino]-3-methyl-butyryl}-thiazolidine-2-carboxylic acid methyl ester

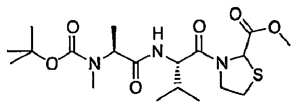

A solution of 2-[2-(*tert*-butoxycarbonyl-methyl-amino)-propionylamino]-3-methyl-butyric acid (747 mg, 2.47 mmol) and thiazolidine-2-carboxylic acid methyl ester; hydrochloride salt (504.4 mg, 2.75 mmol) in tetrahydrofuran (THF) (40 mL) was added 1-ethyl-3-(3-dimethylaminopropyl)carbodiimide hydrochloride (EDCI, 995 mg, 5.19 mmol) and 1-hydroxybenzotriazole monohydrate (HOBt, 520 mg, 3.84 mmol), followed by *N*-methyl-2-pyrrolidinone (1 mL) and 4-methyl morpholine (2 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (565 mg, 56%) as a very light yellow oil. TLC (50% ethyl acetate/hexane) R*_{f}*= 0.52; MS(ES) for C₁₉H₃₃NO₆S (MW=431.55): positive 432 (M+H), negative 430 (M-H).

### Part B: 3-{2-[2-(tert-Butoxycarbonyl-methyl-amino)-propionylamino]-3-methyl-butyryl}-thiazolidine-2-carboxylic acid

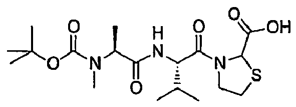

A solution of 3-{2-[2-(*tert*-butoxycarbonyl-methyl-amino)-propionylamino]-3-methyl-butyryl}-thiazolidine-2-carboxylic acid methyl ester (503.5 mg, 1.17 mmol) in tetrahydrofuran (THF) (10 mL), methanol (MeOH) (1 mL) and water (3 mL) was added a 2 N LiOH solution (3 mL, 6 mmol). This mixture was stirred at r.t. overnight. The reaction mixture was treated with 1 N HCl (50 mL), then partitioned with ethyl acetate. The organic phase was washed with brine solution (25 mL), dried over Na₂SO₄ and evaporated to dryness to give the title compound (487 mg, >95%) as a colorless oil. TLC (ethyl acetate) R_{f}= 0.1 streaks; MS(ES) for C₁₈H₃₁N₃O₆S (MW=417.52): positive 418 (M+H).

### Part C: Methyl-(1-{2-methyl-1-[2-(1,2,3,4-tetrahydro-naphthalen-1-ylcarbamoyl)-thiazolidine-3-carbonyl]-propylcarbamoyl}-ethyl)-carbamic acid tert-butyl ester

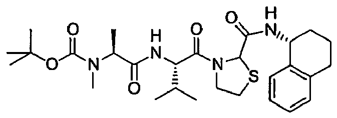

A solution of 3-{2-[2-(*tert*-butoxycarbonyl-methyl-amino)-propionylamino]-3-methyl-butyryl}-thiazolidine-2-carboxylic acid (424 mg, 1.01 mmol) and (R)-1,2,3,4-tetrahydro-naphthalen-1-ylamine (306.9 mg, 2.08 mmol) in tetrahydrofuran (THF) (40 mL) was added 1-ethyl-3-(3-dimethylaminopropyl)carbodiimide hydrochloride (EDCI) (510 mg, 2.66 mmol) and 1-hydroxybenzotriazole monohydrate (HOBt) (400 mg, 2.96 mmol), followed by *N*-methyl-2-pyrrolidinone (0.75 mL) and 4-methyl morpholine (0.75 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and saturated brine solution (50 mL), dried over Na₂SO₄ and evaporated to dryness. The residue was purified by medium pressure column chromatography on silica gel eluting with ethyl acetate/hexane (0-100%) to give the title compound (77 mg, 14%) as a tan solid. TLC (50% ethyl acetate/hexane) R_{f}= 0.11; MS (ES) for C₂₈H₄₂N₄O₅S (MW=546.72): positive 547 (M+H), negative 545 (M-H).

### Part D: 3-[3-Methyl-2-(2-methylamino-propionylamino)-butyryl]-thiazolidine-2-carboxylic acid (1,2,3,4-tetrahydro-naphthalen-1-yl)-amide; trifluoroacetic acid salt

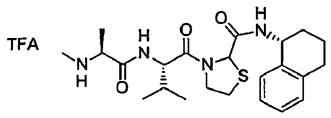

The methyl-(1-{2-methyl-1-[2-(1,2,3,4-tetrahydro-naphthalen-1-ylcarbamoyl)-thiazolidine-3-carbonyl]-propylcarbamoyl}-ethyl)-carbamic acid *tert*-butyl ester (58 mg, 0.106 mmol) was dissolved in dichloromethane (DCM) (5 mL). Trifluoroacetic acid (TFA) (1.5 mL) was added. After stirring at room temperature for 2 hours, the solvent was evaporated. The crude material was purified by reverse phase HPLC, using a C18 Vydec column and eluting with a gradient of 5% to 60% acetonitrile in water containing 0.1 % TFA over 30 minutes; the appropriate fractions were collected and solvents removed to give the title compound as a white solid (20.5 mg). Analytical HPLC: 63.8% at 9.44 min and 34.6% at 9.18 min; two diastereomers present); MS (ES) for C₂₃H₃₄N₄O₃S (MW=446.61): positive 447 (M+H), negative 445 (M-H).

### Example 22

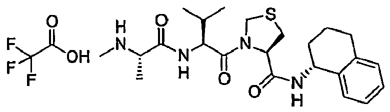

### 3-[3-Methyl-2-(2-methylamino-propionylamino)-butyryl]-thiazolidine-4-carboxylic acid (1,2,3,4-tetrahydro-naphthalen-1-yl)-amide trifluoro-acetic acid salt

### Part A: 4-(1,2,3,4-Tetrahydro-naphthalen-1-ylcarbamoyl)-thiazolidine-3-carboxylic acid tert-butyl ester

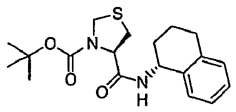

To a solution of Thiazolidine-3,4-dicarboxylic acid 3-tert-butyl ester (2.333 g, 10 mmol) in ethyl acetate (30 mL) was added propane phosponic acid anhydride (T3P, 50% w/w soln in ethyl acetate) (18.2 mL, 30 mmol). The resulting solution was stirred for 10 min. 1,2,3,4-Tetrahydro-naphthalen-1-ylamine (1.37 mL, 10 mmol) was added followed by the addition of N-methylmorpholine (NMM, 11.0 mL, 120 mmol). The resulting solution was stirred for 3 hours, diluted with ethyl acetate (100 mL), washed with 5% KHSO₄ (3x50 mL), saturated sodium bicarbonate solution (3x50 mL), and brine (50 mL). The solution was dried over Na₂SO₄ and solvent was removed under reduced pressure. Purified via flash column chromatography (1:1 ethyl acetate/hexanes) to yield the title compound (3.111 g, 85.8 %) TLC (1:1 ethyl acetate/hexanes) R_{f}. 0.73.

### Part B: Thiazolidine-4-carboxylic acid (1,2,3,4-tetrahydro-naphthalen-1-yl)-amide; hydrochloride

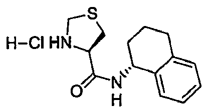

To a solution of 4-(1,2,3,4-Tetrahydro-naphthalen-1-ylcarbamoyl)-thiazolidine-3-carboxylic acid tert-butyl ester (3.10 g, 8.55 mmol) in ethyl acetate (17 mL) was added 6M HCl in ethyl acetate (20 mL). The solution was stirred for 1 hour and the resulting precipitate was filtered and washed with ethyl ether and heaxanes to give the title cpd as a **white solid (2.430 g, 95.1 %).**

### Part C: {2-Methyl-1-[4-(1,2,3,4-tetrahydro-naphthalen-1-ylcarbamoyl)-thiazolidine-3-carbonyl]-propyl}-carbamic acid benzyl ester

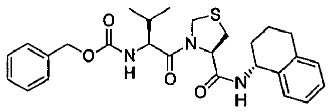

To a solution of 2-Benzyloxycarbonylamino-3-methyl-butyric acid (0.503 g, 2 mmol) and pyridine (0.19 mL, 2.4 mmol) in methylene chloride (6 mL) was added cyanuric fluoride (0.20 mL, 2.4 mmol). After stirring for 2.5 hours the resulting precipitate was filtered off and washed with methylene chloride. The mother liquor was diluted with ethyl acetate (20 mL); washed with saturated sodium bicarbonate solution (15 mL), 5% KHSO₄ (15 mL), and brine (15 mL); reduced under vacuum and taken up in methylene chloride (4 mL). The solution was cooled to -30 °C. A solution of Thiazolidine-4-carboxylic acid (1,2,3,4-tetrahydro-naphthalen-1-yl)-amide; hydrochloride (0.598 g, 2 mmol) was partitioned between saturated sodium bicarbonate solution (20 mL)and ethyl acetate (20 mL), layers were separated. Extracted with ethyl acetate (2x20 mL). Combined extracts were washed with brine, dried over Na₂SO₄, and solvent was removed under reduced pressure. This was taken up in methylene chloride (4 mL) and 2,6-di-t-Butyl-pyridine (0.49 mL, 2.2 mmol) was added. This was added to the acid fluoride solution. The resulting mixture was stirred for 18 hours; diluted with ethyl acetate (30 mL); washed with 5% KHSO₄ (3x20 mL), saturated sodium bicarbonate solution (3x20 mL), and brine (20 mL). The solution was dried over Na₂SO₄ and solvent was removed under reduced pressure. Purified via flash column chromatography (1:1 Ethyl acetate/hexanes) to give the title compound (0.882 g, 89.0 %).

### Part D: 3-(2-Amino-3-methyl-butyryl)-thiazolidine-4-carboxylic acid (1,2,3,4-tetrahydro-naphthalen-1-yl)-amide hydrobromide salt

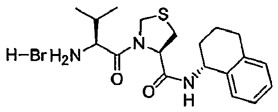

To a solution of 30 % acetic acid in HBr (3 mL) was added {2-Methyl-1-[4-(1,2,3,4-tetrahydro-naphthalen-1-ylcarbamoyl)-thiazolidine-3-carbonyl]-propyl}-carbamic acid benzyl ester (0.882 g, 1.78 mmol). The resulting solution was stirred for 1 hour and diluted with ethyl ether (20 mL). The resulting precipitate was filtered off and washed with ethyl ether and hexanes. Dried on vacuum pump and used as is assuming quantitative yield.

### Part E: Methyl-(1-{2-methyl-1-[4-(1,2,3,4-tetrahydro-naphthalen-1-ylcarbamoyl)-thiazolidine-3-carbonyl]-propylcarbamoyl}-ethyl)-carbamic acid tert-butyl ester

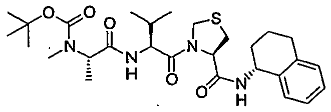

To a solution of 2-(tert-Butoxycarbonyl-methyl-amino)-propionic acid (0.362 g, 1.78 mmol) in ethyl acetate (4 mL) was added propane phosponic acid anhydride (T3P, 50% w/w soln in ethyl acetate) (3.24 mL, 5.34 mmol). The resulting solution was stirred for 10 minutes, then 3-(2-Amino-3-methyl-butyryl)-thiazolidine-4-carboxylic acid (1,2,3,4-tetrahydro-naphthalen-1-yl)-amide hydrobromide salt (0.788 g, 1.78 mmol) was added followed by addition of NMM (1.96 mL, 17.8 mmol). The solution was stirred for 3.5 hours, then diluted with ethyl acetate (100 mL), washed with 5% KHSO₄ (3x40 mL), saturated sodium bicarbonate solution (3x40 mL), and brine (40 mL). The solution was dried over Na₂SO₄ and solvent was removed under reduced pressure. Purified via flash column chromatography (1:1 ethyl acetate/hexane) to yield the title compound (0.587 g, 66.3 %). TLC (1:1 ethyl acetate/hexane) R_{f} = 0.44.

### Part F: 3-[3-Methyl-2-(2-methylamino-propionylamino)-butyryl]-thiazolidine-4-carboxylic acid (1,2,3,4-tetrahydro-naphthalen-1-yl)-amide trifluoro-acetic acid salt

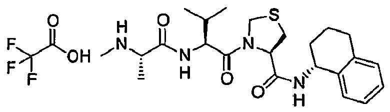

To a solution of Methyl-(1-{2-methyl-1-[4-(1,2,3,4-tetrahydro-naphthalen-1-ylcarbamoyl)-thiazolidine-3-carbonyl]-propylcarbamoyl}-ethyl)-carbamic acid tert-butyl ester (0.585 g, 1.07 mmol) in ethyl acetate (2mL) was added 6M HCl in ethyl acetate (3mL). The resulting solution was stirred for 1 hour and solvent was removed under reduced pressure. The crude product was triturated in ethyl ether to give yield the title compound as a white solid (0.440 g, 91.1 %, Analytical HPLC: 86 % pure). This solid was further purified using reverse phase HPLC, using a C18 Vydec column and eluting with a gradient of 5% to 50% acetonitrile in water containing 0.1% TFA over 30 minutes yielding the title compound (0.152 g, 25.3 %). Analytical HPLC Rₜ-8.948 min; MS (ES) for salt C₂₅H₂₉N₃O₄ (MW=560.63) positive 447 (M+H), negative 445/559 (M-H).

### Example 23

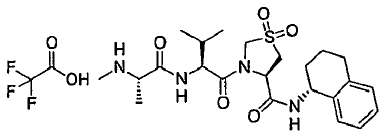

### 3-[3-Methyl-2-(2-methylamino-propionylamino)-butyryl]-1,1-dioxo-1λ⁶-thiazolidine-4-carboxylic acid (1,2,3,4-tetrahydro-naphthalen-1-yl)-amide trifluoro-acetic acid salt

### Part A: (1-{1-[1,1-Dioxo-4-(1,2,3,4-tetrahydro-naphthalen-1-ylcarbamoyl)-1λ⁶-thiazolidine-3-carbonyl]-2-ethyl-propylcarbamoyl}-ethyl)-methyl-carbamic acid tert-butyl ester

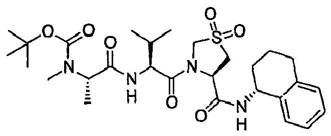

To a solution of Methyl-(1-{2-methyl-1-[4-(1,2,3,4-tetrahydro-naphthalen-1-ylcarbamoyl)-thiazolidine-3-carbonyl]-propylcarbamoyl}-ethyl)-carbamic acid tert-butyl ester (0.180 g, 0.33 mmol) in ethyl acetate (3 mL) was added 77 % mCPBA (0.224 g, 1 mmol). The resulting mixture was stirred for 18 hours. Diluted with ethyl acetate (20 mL); washed with saturated sodium bicarbonate solution (3x10 mL), and brine (10 mL); dried over Na₂SO₄ and the solvent was removed under reduced pressure. The crude material was purified via flash column chromatography (1:1 ethyl acetate/hexane) to yield the title compound (0.131 g, 68.9 %). TLC (ethyl acetate) R_{f} = 0.59.

### Part B: 3-[3-Methyl-2-(2-methylamino-propionylamino)-butyryl]-1,1-dioxo-1λ⁶-thiazolidine-4-carboxylic acid (1,2,3,4-tetrahydro-naphthalen-1-yl)-amide trifluoro-acetic acid salt

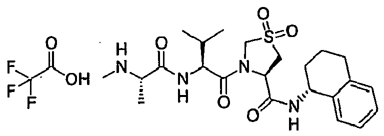

To a solution of (1-{1-[1,1-Dioxo-4-(1,2,3,4-tetrahydro-naphthalen-1-ylcarbamoyl)-1λ⁶-thiazolidine-3-carbonyl]-2-ethyl-propylcarbamoyl}-ethyl)-methyl-carbamic acid tert-butyl ester (0.175 g, 0.302 mmol) in ethyl acetate (1mL) was added 6M HCl in ethyl acetate (0.6 mL). The resulting solution was stirred for 1 hour and solvent was removed under reduced pressure. The crude product was triturated in ethyl ether and purified using reverse phase HPLC, using a C 18 Vydec column and eluting with a gradient of 5% to 50% acetonitrile in water containing 0.1% TFA over 30 minutes yielding the title compound (35 mg, 19.6 %). Analytical HPLC Rₜ-9.328 min. MS(ES) for C₂₅H₃₅F₃N₄O₇S (MW=592.64) positive 479 (M+H), negative 477/591 (M-H).

### Example 24

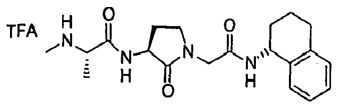

### 2-Methylamino-N-{2-oxo-1-[(1,2,3,4-tetrahydro-naphthalen-1-ylcarbamoyl)-methyl]-pyrrolidin-3-yl}-p ropionamide

### Part A: (2-tert-Butoxycarbonylamino-4-methylsulfanyl-butyrylamino)-acetic acid methyl ester

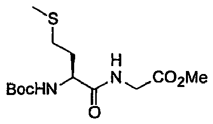

To a solution of 2-tert-butoxy carbonylamino 4-methyl sulfanyl-butyric acid (5 g, 20.24 mmol) in dimethylformamide (DMF, 50 mL) at room temperature under nitrogen was added 1-hydroxybenzotriazole (HOBt, 3.56 g, 26.32 mmol), 1-(3-dimethylaminopropyl)-3-ethylcarbodiimide hydrochloride (EDCI, 5.05 g, 26.32 mmol), glycine methyl ester (4.24 g, 30.36 mmol), and diisopropyethylamine (DIEA, 4.6 mL, 26.32 mmol) and the resulting mixture was stirred at room temperature for 18 hours. The reaction was diluted with ethyl acetate (100 mL) and washed successively with aq. 5% KHSO₄, saturated sodium bicarbonate solution, brine, dried (Na₂SO₄) and concentrated under reduced pressure. The crude residue was purified by flash column chromatography on silica gel eluting with 30% ethyl acetate/hexane to afford the title compound (5.32 g, 82%). TLC (30% ethyl acetate/hexane) R_{f}= 0.17.

### Part B: ({2-Oxo-1-[(1,2,3,4-tetrahydro-naphthalen-2-ylcarbamoyl)-methyl]-pyrrolidin-3-yl}-carbamic acid tert-butyl ester

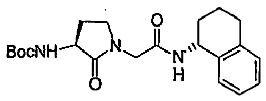

(2-tert-Butoxycarbonylamino-4-methylsulfanyl-butyrylamino)-acetic acid methyl ester (2.15 g, 6.74 mmol) was dissolved in methyliodide (13 mL, excess) and the mixture was stirred at room temperature for 7 hours. The excess methyliodide was decanted and the crude product was dried to give the the sulfonium iodide that was dissolved in a 1:1 of mixture of dimethylformamide/methylene chloride (140 mL). To the resulting solution at 0°C was added sodium hydride in one portion and then stirred for 2.5 hours. Methyl acetate (45 mL) and H₂O (10 mL) were added and the resulting mixture was stirred for 18 hours at room temperature. The mixture was then concentrated under reduced pressure and partitioned between H₂O (20 mL) and methylene chloride (20 mL). The aqueous layer was acidified to pH=3 with 0.5 M citric acid and extracted 3 times with methylene chloride. The combined organic layers were dried (Na₂SO₄) and concentrated to afford the acid product. This was directly coupled with R-1,2,3,4-tetrahydro-1-naphthylamine (992mg, 6.74 mmol) using HOBt (1.18 g, 8.76 mmol), EDCI (1.68 g, 8.76 mmol), and DIEA (1.5 mL, 8.76 mmol) and the resulting mixture was stirred at room temperature for 18 hours. The reaction was diluted with ethyl acetate (100 mL) and washed successively with aq. 5% KHSO₄, saturated sodium bicarbonate solution, brine, dried (Na₂SO₄) and concentrated under reduced pressure. The crude residue was purified by flash column chromatography on silica gel eluting with 5% methanol/ methylene chloride gave the desired product (1.3 g, 50%). MS (ES) for C₂₁H₂₉N₃O1₄ (MW=387.47): positive 388(M+H).

### Part C: 2-Methylamino-N-12-oxo-1-[(1,2,3,4-tetrahydro-naphthalen-1-ylcarbamoyl)-methyl]-pyrrolidin-3-yl}-propionamide

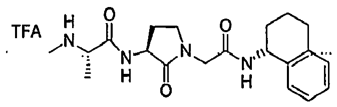

({2-Oxo-1-[(1,2,3,4-tetrahydro-naphthalen-2-ylcarbamoyl)-methyl]-pyrrolidin-3-yl}-carbamic acid tert-butyl ester (319 mg, 0.82 mmol) was dissolved in 20% TFA/methylene chloride (3 mL) and the mixture was stirred at room temperature for 1 hour then concentrated under reduced pressure. The crude amine was then taken up in DMF (5 mL) followed by addition of Boc-Me-Ala-OH (259 mg, 1.27 mmol), HOBt (212 mg, 1.57 mmol), EDCI ( 301 mg, 1.57 mmol), and DIEA ( 0.29 mL, 1.64 mmol). The resulting mixture was stirred at room temperature for 18 hours then diluted with ethyl acetate (50 mL), washed successively with aq.5% KHSO4, saturated sodium bicarbonate solution, brine, dried (Na₂SO₄) and concentrated under reduced pressure. The crude residue was dissolved in ethyl acetate (3 mL) followed by addition of 4M HCl in 1,4-dioxane (0.62 mL). The reaction mixture was stirred at room temperature for 18 hours then purified by reverse phase HPLC, using a C18 Vydec column and eluting with a gradient of 5% to 40% acetonitrile in water containing 0.1% TFA over 30 minutes to afford the desired product (50 mg, 16%). Analytical HPLC Rₜ: 7.224 min. MS (ES) for C₂₀H₂₈N₄O₃ (MW=372.46): positive 373(M+H).

### Example 25

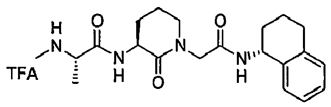

### 2-Methylamino-N-(2-oxo-1[(1,2,3,4-tetrahydro-naphthalen-1-ylcarbamoyl)-methyl]-piperidin-3-yl}-propionamide

### Part A: (3-tert-Butoxycarbonylamino-2-oxo-piperidin-1-yl)-acetic acid

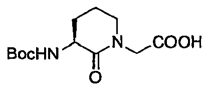

To a solution of Boc-Glu(OBzl)-OH (2g, 5.46 mmol) in methanol (150 mL) was added glyoxylic acid (503 mg, 5.46 mmol). The resulting mixture was stirred under hydrogen (1 atm) at room temperature for 18 hours. It was then concentrated under reduced pressure and purified by flash column chromatography on silica gel eluting with 10% methanol/ methylene chloride to yield the title compound (1.26 g, 85%). MS (ES) for C₁₂H₂₀N₂O₅ (MW=272.30): positive 273(M+H).

### Part B: ({2-Oxo-1-[(1,2,3,4-tetrahydro-naphthalen-1-ylcarbamoyl)-methyl]-piperidin-3-yl}-carbamic acid tert-butyl ester

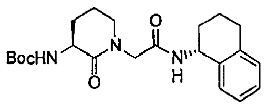

To a solution of (3-tert-Butoxycarbonylamino-2-oxo-piperidin-1-yl)-acetic acid (305 mg, 1.12 mmol) in dimethylformamide (DMF, 5 mL) was added 1-hydroxybenzotriazole (HOBt, 242 mg, 1.79 mmol), 1-(3-dimethylaminopropyl)-3-ethylcarbodiimide hydrochloride (EDAC, 344 mg, 1.79 mmol), R-1,2,3,4-tetrahydro-1-naphthylamine (231 mg, 1.57 mmol), and diisopropylethylamine (DIEA, 0.32 mL, 1.79 mmoL); the resulting mixture was stirred at room temperature for 18 hours. It was then diluted with ethyl acetate (50 mL) and washed successively with aq. 5% KHSO₄, sat. NaHCO₃, brine, and concentrated to give the crude product that was purified by flash column chromatography on silica gel eluting with 15% methanol/ methylene chloride to afford the title compound ( 387 mg, 86%). TLC (5% methanol/ methylene chloride) R_{f}= 0.39.

### Part C: Methyl-(1-{2-oxo-1-[(1,2,3,4-tetrahydro-naphthalen-1-ylcarbamoyl)-methyl]-piperidin-3-ylcarbamoyl}-ethyl)-carbamic acid tert-butyl ester

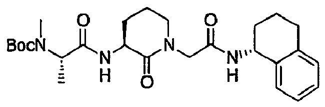

To a solution of ({2-Oxo-1-[(1,2,3,4-tetrahydro-naphthalen-1-ylcarbamoyl)-methyl]-piperidin-3-yl}-carbamic acid tert-butyl ester (401.5mg, 0.95 mmol) in ethyl acetate (5 mL) was added 3 eq. Of HCl ( 0.75 mL, 4M HCl in 1,4-dioxane). The reaction mixture was stirred at room temperature overnight, concentrated to give a residue that was dissolved in DMF (10 mL) followed by addition of Boc-Me-Ala-OH ( 243 mg, 1.57 mmol), HOBt ( 243mg, 1.79 mmol), (EDAC ( 343 mg, 1.79 mmoL), and DIEA ( 0.39 mL, 2.24 mmol). The resulting solution was stirred at room temperature overnight, diluted with Ethyl acetate ( 40 mL), washed with aq.5%KHSO₄, saturated sodium bicarbonate solution, brine, dried (Na₂SO₄) and concentrated. The crude product was purified by flash column chromatography on silica gel eluting with 6% ethanol/methylene chloride to afford the title compound (370 mg, 80%). TLC (6% methanol/methylene chloride) R_{f}= 0.24.

### Part D: 2-Methylamino-N-{2-oxo-1-[(1,2,3,4-tetrahydro-naphthalen-1-ylcarbamoyl)-methyl]-piperidin-3-yl}-propionamide

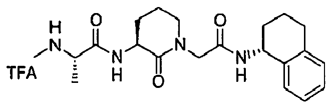

To a solution of Methyl-(1-{2-oxo-1-[(1,2,3,4-tetrahydro-naphthalen-1-ylcarbamoyl)-methyl]-piperidin-3-ylcarbamoyl}-ethyl)-carbamic acid tert-butyl ester (370 mg, 0.76 mmol) in ethyl acetate (2 mL) was added 4M HCl in 1,4-dioxane (0.57 mL, 3 eq,). The reaction mixture was stirred at room temperature for 18 hours, concentrated under reduced pressure, then purified by reverse phase HPLC, using a C18 Vydec column and eluting with a gradient of 5% to 40% acetonitrile in water containing 0.1% TFA over 30 minutes to give the title compound (114 mg, 30%).Analytical HPLC Rₜ = 7.542 min. MS (ES) for C₂₁H₃₀N₄O₃ (MW=386.49): positive 387(M+H).

### Example 26

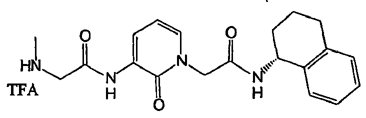

### 2-[3-(2-Methylamino-acetylamino)-2-oxo-2H-pyridin-1-yl]-N-(1,2,3,4-tetrahydronaphthalen-1-yl)-acetamide trifluoroacetic acid salt

### Part A: 2-Ozo-1-[(1,2,3,4-tetrahydro-naphthalen-1-ylcarbamoyl)-methyl]-1,2-dihydro-pyridin-3-yl}-carbamic acid 9H-fluoren-9-ylmethyl ester

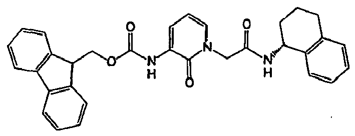

To a slurry of [3-(9H-Fluoren-9-ylmethoxycarbonylamino)-2-oxo-2H-pyridin-1-yl] acetic acid (.527mg, 1.35mmol) in ethyl acetate (5.4 mL) was added propane phosponic acid anhydride (T3P, 50% w/w soln in ethyl acetate) (1.29g, 2.02mmol, 1.5eq.) until a clear homogeneous mixture was achieved. To this was added 1,2,3,4-Tetrahydro-naphthalen-1-ylamine (238mg, 1.62mmol, 1.2eq.), followed by N-methylmorpholine (NMM, 0.74mL, 6.75mmol, 5eq.). The mixture was stirred for 25 minutes then diluted to 100 mL with ethyl acetate. The reaction was washed with 20mL 1M HCl followed by 10mL washes with 1M HCl and brine. The ethyl acetate was dried (Na₂SO₄) and solvent was removed to yield an oil that was purified by flash column chromatography on silica gel eluting with 50% hexanes in ethyl acetate to yield the title compound as a white solid. TLC (50% ethyl acetate/hexane) R*_{f}*= 0.4.

### Part B: 2-(3-Amino-2-oxo-2H-pyridin-1-yl)-N-(1,2,3,4-tetrahydro-naphthalen-1-yl)-acetamide

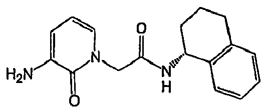

-Oxo-1-[(1,2,3,4-tetrahydro-naphthalen-1-ylcarbamoyl)-methyl]-1,2-dihydropyridin-3-yl}-carbamic acid 9H-fluoren-9-ylmethyl ester was dissolved in methylene chloride/ methanol/ dimethylformamide (1:1:1) (6 mL). Diethyl amine was added and the mixture was stirred 3 hours. The reaction was diluted with ethyl acetate (50mL) and extracted with 10mL 1 M HCl (2X). The combined HCl extracts were made basic with saturated sodium bicarbonate and extracted with 20 mL ethyl acetate (2X). The combined ethyl acetate layers were dried (Na₂SO₄) and solvent was removed to yield 97mg of an oil.

### Part C: Methyl-({2-oxo-1-[(1,2,3,4-tetrahydro-naphthalen-1-ylcarbamoyl)-methyl]-1,2-dihydro-pyridin-3-ylcarbamoyl}-methyl)-carbamic acid tert-butyl ester

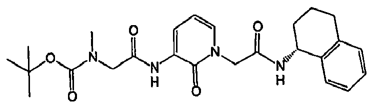

Boc-N-Me-Alanine (40mg, .20mmol), 2-(3-Amino-2-oxo-2H-pyridin-1-yl)-N-(1,2,3,4-tetrahydro-naphthalen-1-yl)-acetamide (72mg, .22mmol, 1.1eq.), 1-(3-dimethylaminopropyl)-3-ethylcarbodiimide hydrochloride (EDCI, 57mg, .300mmol, 1.5eq.), and 1-hydroxybenzotriazole (HOBt, 36mg, .24mmol, 1.2eq.) were combined and dissolved in acetonitrile (0.79mL, 0.25M). After stirring for ten minutes diisopropylethylamine (DIEA, 0.17mL, 0.98 mmol, 5eq.) was added and the mixture was stirred at room temperature for 18 hours. Acetonitrile was removed in vacuo, the mixture was re-suspended in 50mL ethyl acetate and washed with 5mL of 1M HCl (2X), brine, saturated sodium bicarbonate (2X), brine. The ethyl acetate was dried (Na₂SO₄) and solvent was remove to give the title compound (98mg).

### Part D: 2-[3-(2-Methylamino-acetylamino)-2-oxo-2H-pyridin-1-yl]-N-(1,2,3,4tetrahydro-naphthalen-1-yl)-acetamide trifluoroacetic acid salt

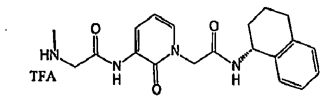

Methyl-({2-oxo-1-[(1,2,3,4-tetrahydro-naphthalen-1-ylcarbamoyl)-methyl]-1,2-dihydro-pyridin-3-ylcarbamoyl) -methyl)-carbamic acid tert-butyl ester was dissolved in ethyl acetate (3mL). To this was added 4M HCl in dioxane (1mL, 4mmol, 20eq.). The mixture was stirred at room temperature for 18 hours. The reaction mixture was concentrated under reduced pressure, then the crude material was purified using reverse phase HPLC, using a C18 Vydac column and eluting with a gradient of 5% to 40% acetonitrile in water containing 0.1 % TFA over 30 minutes, yielding the title compound (0.018g). MS(ES) for C₂₁H₂₆N₄O₃ (MW=382.4): positive 383.36 (M+H), negative 381.21 (M-H). Analytical HPLC Rₜ= 8.07min.

### Example 27

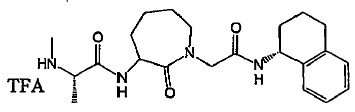

### 2-Methylamino-N-{2-oxo-1-[(1,2,3,4-tetrahydro-naphthalen-1-ylcarbamoyl)-methyl]-azepan-3-yl}-propionamide Trifluoroacetic acid salt

### Part A: {2-Oxo-1-[(1,2,3,4-tetrahydro-naphthalen-1-ylcarbamoyl)-methyl]-azepan-3-yl}-carbamic acid tert-butyl ester

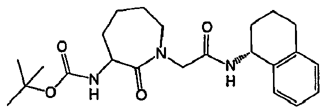

The (3-tert-Butoxycarbonylamino-2-oxo-azepan-1-yl)-acetic acid (586mg, 18.80mmol), 1,2,3,4-Tetrahydro-naphthalen-1-ylamine (362mg, 2.46mmol, 1.2eq.), 1-(3-dimethylaminopropyl)-3-ethylcarbodiimide hydrochloride (EDCI, 588mg,3.07mmol, 1.5eq.), and 1-hydroxybenzotriazole (HOBt, 376mg, 2.46mmol, 1.2eq.) were combined and dissolved in acetonitrile (8.19mL, 0.25M). After stirring for ten minutes, diisopropylethylamine (DIEA, 1.75mL, 10.23mmol, 5eq.) was added and the mixture was stirred 18hr. Acetonitrile was removed in vacuo, the mixture was re-suspended in 100mL ethyl acetate and washed with 10mL of 1M HCl (2X), brine, saturated sodium bicarbonate (2X), brine. The ethyl acetate was dried (Na2SO4) and solvent was removed to yield thick yellow oil.

### Part B: 2-(3-Amino-2-oxo-azepan-1-yl)-N-(1,2,3,4-tetrahydro-naphthalen-1-yl)-acetamide; HCl salt

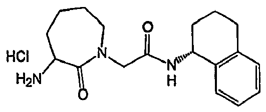

{2-Oxo-1-[(1,2,3,4-tetrahydro-naphthalen-1-ylcarbamoyl)-methyl]-azepan-3-yl}-carbamic acid tert-butyl ester (2.05mmol) was dissolve in ethyl acetate (6.83mL). To this was added 4M HCl in dioxane (2.28mL, 9.111mmol, 4.4eq.). The mixture was stirred at room temperature for 18 hours, followed by concentration of the reaction under reduced pressure to dryness yielding a hydroscopic tan foam.

### Part C: Methyl-(1-12-oxo-1-[(1,2,3,4-tetrahydro-naphthalen-1-ylcarbamoyl)-methyl]-azepan-3-ylcarbamoyl)-ethyl)-carbamic acid tert-butyl ester

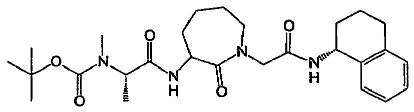

Boc-N-Me-Alanine (35mg, 172mmol), 2-(3-Amino-2-oxo-azepan-1-yl)-N-(1,2,3,4-tetrahydro-naphthalen-1-yl)-acetamide; HCl salt (2.05mmol, 1.2eq.), EDCI (49mg,.258mmol, 1.5eq.), and HOBt (32mg, 0.207mmol, 1.2eq.) were combined and dissolved in acetonitrile (0.7mL, 0.25M). After stirring for ten minutes DIEA (0.147mL, 0.861 mmol, 5eq.) was added and the mixture was stirred at room temperature for 18 hours. Acetonitrile was removed in vacuo, the mixture was re-suspended in 50mL ethyl acetate and washed with 5mL of 1M HCl (2X), brine, saturated sodium bicarbonate (2X), brine. The ethyl acetate was dried (Na₂SO₄) and solvent was removed to give the title compound (0.105 g, 95%). TLC (ethyl acetate) R_{f}= 0.18.

### Part D: 2-Methylamino-N-{2-oxo-1-[(1,2,3,4-tetrahydro-naphthalen-1-ylcarbamoyl)-methyl]-azepan-3-yl}-propionamide Trifluoroacetic acid salt

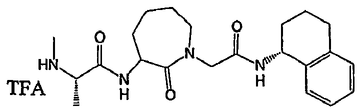

Methyl-(1-{2-oxo-1-[(1,2,3,4-tetrahydro-naphthalen-1-ylcarbamoyl)-methyl]-azepan-3-ylcarbamoyl}-ethyl)-carbamic acid tert-butyl ester was dissolved in ethyl acetate (.686mL). To this was added 4M HCl in dioxane (0.23mL, 0.914mmol, 4.4eq.). The mixture was stirred at room temperature for 18 hours, then the reaction mixture was concentrated under reduced pressure. The crude material was purified using reverse phase HPLC, using a C18 Vydac column and eluting with a gradient of 5% to 40% acetonitrile in water containing 0.1 % TFA over 30 minutes, yielding the title compound (0.075 g, 71%). MS(ES) for C₂₂H₃₂N₄O₃ (MW=400.5): positive 401.35 (M+H), negative 399.26 (M-H). Analytical HPLC Rₜ=8.3min.

### Example 28

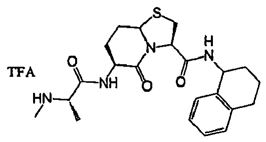

### 6-(2-Methylamino-propionylamino)-5-oxo-hexahydro-thiazolo[3,2-a]pyridine-3-carboxylic acid (1,2,3,4-tetrahydro-naphthalen-1-yl)-amide Trifluoroacetic acid salt

### Part A: 2-tert-Butoxycarbonylamino-5-hydroxy-pentanoic acid methyl ester

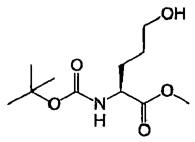

2-tert-Butoxycarbonylamino-pentanedioic acid 1-methyl ester (1.059g, 4.05mmol) was dissolved in THF (6.75mL, 0.6M) and cooled to -10°C. Ethyl chloroformate (581uL, 6.08mmol, 1.5eq.) was added followed by triethylamine (902uL, 6.49mmol, 1.6eq.). This mixture was allowed to stir 10 minutes then filtered into NaBH₄ (690mg, 18.24mmol, 4.5eq.) in 10mL water. The precipitate was rinsed with THF and added to the reaction mixture. After 20 minutes the reaction mixture was neutralized to ~pH7 w/ HCl. The reaction mixture was partitioned with ethyl acetate (100mL) and the ethyl acetate layer was washed with 10mL of 1M HC 1(2X), brine, saturated sodium bicarbonate solution (2X), and brine. The organic layers were dried with sodium sulfate and concentrated under reduced pressure. The crude product was purified by flash column chromatography eluting with 2/1 ethyl acetate/hexanes, yielding the title compound. TLC (2/1 ethyl acetate/hexanes) R_{f}=0.3.

### Part B: 2-tert-Butoxycarbonylamino-5-oxo-pentanoic acid methyl ester

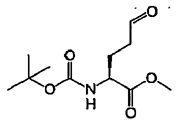

2-tert-Butoxycarbonylamino-5-hydroxy-pentanoic acid methyl ester (626mg, 2.53mmol) was dissolved in DMSO (12.5mL) and toluene (12.5mL), to this solution 1-(3-dimethylaminopropyl)-3-ethylcarbodiimide hydrochloride (EDCI, 4.86g, 25.31mmol, 10eq.) was added and the resulting suspension stirred. Dichloroacetic acid (1.04mL, 12.66mmol, 5eq.) was added and after 20 min of stirring all was in solution. TLC in 2/1 hexanes/ethyl acetate showed consumption of starting material. The reaction was diluted with 100mL ethyl acetate and washed (10mL) with 1M HCl (2X), Brine, saturated sodium bicarbonate solution (2X), brine. The organic layer was dried with sodium sulfate concentrated under reduced pressure to yield the crude title compound which was used directly for the next step.

### Part C: 6-tert-Butoxycarbonylamino-5-oxo-hexahydro-thiazolo[3,2-a]pyridine-3-carboxylic acid

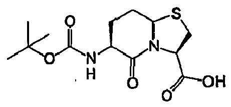

2-tert-Butoxycarbonylamino-5-oxo-pentanoic acid methyl ester (2.5mmol) was dissolved in pyridine (69mL, 0.037M). 4A molecular sieves (~25) were added followed by Cysteine hydrochloride (518mg, 3.29mmol, 1.3eq.). The mixture was stirred and heated (~125°C) to reflux for 18 hours. The resulting brown mixture was filtered and the sieves rinsed with methyl alcohol and the combined filtrates concentrated under reduced pressure. This was then re-suspended in 100mL ethyl acetate and washed (10mL) with 1M HCl(2X), and brine, dried (sodium sulfate) and concentrated under reduced pressure to yield the crude title compound which was used directly for next coupling.

### Part D: [5-Oxo-3-(1,2,3,4-tetrahydro-naphthalen-1-ylcarbamoyl)-hexahydro-thiazolo[3,2-a]pyridin-6-yl]-carbamic acid tert-butyl ester

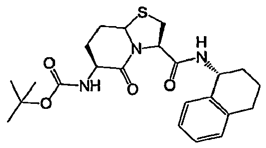

6-tert-Butoxycarbonylamino-5-oxo-hexahydro-thiazolo[3,2-a]pyridine-3-carboxylic acid was stirred as a slurry with 1,2,3,4-Tetrahydro-naphthalen-1-ylamine (559mg, 3.80mmol, 1.5eq.) in ethyl acetate (10.12mL, 0.25M). To this was added N-methylmorpholine (NMM, 2.78mL, 25.3mmol, 10eq.) followed by propane phosponic acid anhydride (T3P, 50% w/w soln in ethyl acetate) (3.21g, 5.06mmol, 2eq.) until a clear homogeneous mixture was achieved (20min). The mixture was then diluted to 200 mL with ethyl acetate. The reaction was washed with 20mL 1M HCl followed by 10mL washes with 1M HCl and brine. The ethyl acetate was dried (Na₂SO₄) and solvent was removed to yield an oil that was purified by flash column chromatography on silica gel eluting with 40% ethyl acetate/ methylene chloride, yielding the title compound. TLC (40% ethyl acetate/ methylene chloride) R_{f} = 0.2.

### Part E: 6-Amino-5-oxo-hexahydro-thiazolo[3,2-a]pyridine-3-carboxylic acid (1,2,3,4-tetrahydro-naphthalen-1-yl)-amide HCl Salt

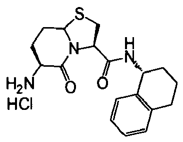

[5-Oxo-3-(1,2,3,4-tetrahydro-naphthalen-1-ylcarbamoyl)-hexahydro-thiazolo[3,2-a]pyridin-6-yl]-carbamic acid tert-butyl ester) (0.36g, 0.808mmol) was dissolved in ethyl acetate (6mL). To this was added 4M HCl in dioxane (2mL, 8mmol, 9.9eq.). The mixture was stirred at room temperature for 18 hours. Toluene was added and the solvents removed under reduced pressure yielding the crude title compound as a light brown solid.

### Part F: Methyl-{1-[5-oxo-3-(1,2,3,4-tetrahydro-naphthalen-1-ylcarbamoyl)-hexahydro-thiazolo[3,2-a]pyridin-6-ylcarbamoyl]-ethyl}-carbamic acid tert-butyl ester

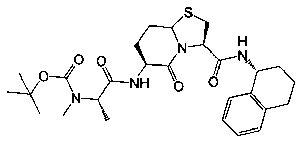

2-(tert-Butoxycarbonyl-methyl-amino)-propionic acid (25mg, 0.123mmol) was combined with 6-Amino-5-oxo-hexahydro-thiazolo[3,2-a]pyridine-3-carboxylic acid (1,2,3,4-tetrahydronaphthalen-1-yl)-amide HCl Salt (52mg, 0.135mmol, 1.1eq.) and slurried with ethyl acetate (0.5mL, 0.25M). To this was added N-methylmorpholine (NMM, 0.135mL, 1.23mmol, 10eq.) followed by propane phosponic acid anhydride (T3P, 50% w/w soln in ethyl acetate) (0.235g, 0.369mmol, 3eq.) until a clear homogeneous mixture was achieved (15min). The mixture was then diluted to 50 mL with ethyl acetate. The reaction was washed with 10mL 1M HCl followed by 5mL washes with 1M HCl and brine. The organic layer was dried (Na₂SO₄) and concentrated under reduced pressure to give the crude title compound. This material was used directly in the next deprotection step.

### Part G: 6-(2-Methylamino-propionylamino)-5-oxo-hexahydro-thiazolo[3,2a]pyridine-3-carboxylic acid (1,2,3,4-tetrahydro-naphthalen-1-yl)-amide Trifluoroacetic acid salt

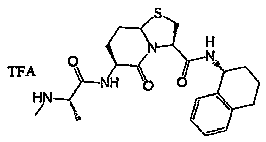

Methyl- {1-[5-oxo-3-(1,2,3,4-tetrahydro-naphthalen-1-ylcarbamoyl)-hexahydro-thiazolo[3,2-a]pyridin-6-ylcarbamoyl]-ethyl}-carbamic acid tert-butyl ester (0.123mmol) was dissolved in ethyl acetate (6mL). To this was added 4M HCl in dioxane (2mL, 8mmol, 65eq.). The mixture was stirred at room temperature for 18 hours. Toluene was added and the solvents removed under reduced pressure. The crude material was purified using reverse phase HPLC, using a C18 Vydac column and eluting with a gradient of 5% to 50% acetonitrile in water containing 0.1% TFA over 30 minutes, yielding (0.025g). MS(ES) for C₂₂H₃₀N₄O₃S(MW=430.56): positive 431.29 (M+H), negative 429.18 (M-H). Analytical HPLC Rₜ=8.19 min.

### Example 29

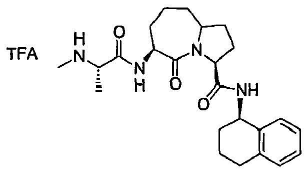

### 6-(2-Methylamino-propionylamino)-5-oxo-octahydro-pyrrolo[1,2-a]azepine-3-carboxylic acid (1,2,3,4-tetrahydro-naphthalen-1-yl)-amide

**Part A: [2-tert-Butoxycarbonylamino-5-oxo-hept-6-enoic acid benzyl ester**

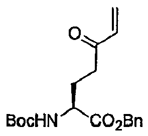

A solution of Boc-pyroglutamic acid benzyl ester (3.5 g, 10.97 mmol) in anhydrous THF (64 mL)at -40 °C was treated with vinylmagnesium bromide (1M in THF). The resulting mixture was stirred at -40°C for 2 hours, quenched with a mixture of acetic acid and methanol (1:1, 5 mL), then diluted with ethyl ether. The organic layer was washed with brine, dried (Na₂SO₄, and concentrated to a residue that was purified by flash column chromatography on silica gel using 25% ethyl acetate/hexane to give a colorless oil (1.33 g, 35%). MS (ES) for C₁₉H₂₅NO₅ (MW=347.41): positive 348 (M+H).

### Part B: 2-tert-Butoxycarbonylamino-5-hydroxy-hept-6-enoic acid benzyl ester

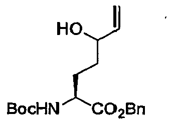

A solution of [2-tert-Butoxycarbonylamino-5-oxo-hept-6-enoic acid benzyl ester (710 mg, 2.05 mmol) in methanol (6 mL) was treated with CeCl₃.7 H₂O (764 mg, 2.056 mmol) and stirred at room temperature for 10 min. Solid sodium borohydride (78 mg, 2.05 mmol) was added at -15°C, and the resulting mixture was stirred for 5 minutes quenched with water, and diluted with ethyl ether (20 mL). The organic layer was separated, and the aqueous layer extracted with ethyl ether (2x 50 mL). The combined organic layers were washed with brine, dried (Na₂SO₄) and concentrated. The crude residue was purified by flash column chromatography on silica gel eluting with 20-40% ethyl acetate/hexane to give the title compound (537mg, 75%). MS (ES for C₁₉H₂₇NO₅ (MW=349.42): positive 350(M+H).

### Part C: 5-Vinyl-pyrrolidine-1,2-dicarboxylic acid 2-benzyl ester 1-tert-butyl ester

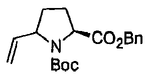

To a solution of -tert-Butoxycarbonylamino-5-hydroxy-hept-6-enoic acid benzyl ester (700mg, 2.00mmol) in methylene chloride (26 mL) at 0 °C was added methanesulfonyl chloride (0.92 mL, 11.91 mmol) and triethylamine (1.96 mL, 14.06mmol). The stirred mixture was stirred overnight allowing to warm to room temperature. The solvent was evaporated and the crude mixture was purified by flash column chromatography on silica gel eluting with 0-20% ethyl acetate/hexane to give the title compound as a distereomeric mixture (431 mg, 65%). MS (ES) for C₁₉H₂₅NO₄ (MW=331.41): positive 332(M+H).

### Part D: 1-(2-tert-Butoxycarbonylamino-pent-4-enoyl)-5-vinyl-pyrrolidine-2-carboxylic acid benzyl ester

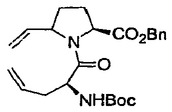

To a solution of 5-Vinyl-pyrrolidine-1,2-dicarboxylic acid 2-benzyl ester 1-tert-butyl ester (282 mg, 1.31 mol) in dimethylformamide (DMF, 92 mL) was added 1-hydroxybenzotriazole (HOBt, 194 mg, 1.43 mmol), 1-(3-dimethylaminopropyl)-3-ethylcarbodiimide hydrochloride (EDCI, 276 mg, 1.44 mol), N-Boc-allylglycine (192.31 mg, 0.72 mmol), and diisopropylethylamine (DIEA, 0.26 mL, 1.49 mmol). The reaction mixture was stirred at room temperature for 18 hours, then diluted with ethyl acetate (92 x 15 mL). The organic layers were washed with aqueous 5% KHSO₄ and brine, dried (Na₂SO₄), and concentrated. The resulting crude mixture was purified by flash column chromatography on silica gel eluting with 0-35% ethyl acetate /hexane to afford the title compound (160 mg, 52 %). MS (ES) for C₂₄H₃₂N₂O₅ (MW=428.52): positive 429(M+H).

### Part E: 6-tert-Butoxycarbonylamino-5-oxo-2,3,5,6,9,9a-hexahydro-1H-pyrrolo[1,2-a]azepine-3-carboxylic acid benzyl ester

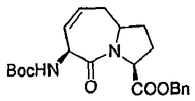

A solution of 1-(2-tert-Butoxycarbonylamino-pent-4-enoyl)-5-vinyl-pyrrolidine-2-carboxylic acid benzyl ester (210 mg, 0.49 mmol)) in methylene chloride was treated with *bis*(tricyclohexylphosphine-benzylidine ruthenium (IV) dichloride ( 80 mg) and the mixture was stirred at 40 °C for 18 hours. After evaporation of the methylene chloride in vacuo, the crude product was purified by flash column chromatography on silica gel using 35% ethyl acetate /hexane to give the title compound (130 mg, 66%). MS (ES) forC₂₂H₂₈N₂O₅ (MW=400.47): positive 401(M+H).

### Part F: [5-Oxo-3-(1,2,3,4-tetrahydro-naphthalen-1-ylcarbamoyl)-octahydropyrrolo[1,2-a]azepin-6-yl]-carbamic acid tert-butyl ester

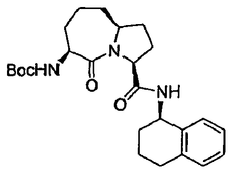

To a solution of 6-tert-Butoxycarbonylamino-5-oxo-2,3,5,6,9,9a-hexahydro-1H-pyrrolo[1,2-a]azepine-3-carboxylic acid benzyl ester (64 mg, 0.16 mmol) in ethyl acetate (10 mL) at room temperature was added 10% Pd/C (30 mg). The reaction mixture was stirred under hydrogen (1 atm) for 1.5 hours. After removal of the catalyst by filtration and evaporation of the ethyl acetate, the residue was dissolved in DMF (3mL) followed by addition of HOBt (35 mg, 0.16 mmol), EDCI (50 mg, 0.26 mmol), *R*-1,2,3,4-tetrahydro-1-naphthylamine, and DIEA. The resulting mixture was stirred at room temperature for 18 hours, then diluted with ethyl acetate. The organic layer was washed with 5% KHSO₄, saturated sodium bicarbonate solution, brine, dried (Na₂SO₄), then concentrated. The crude product was purified by flash column chromatography on silica gel eluting with 10-30% ethyl acetate/hexane to afford the title compound (63.58 mg, 90%). MS (ES) for C₂₅H₃₅N₃O₄ (MW=441.56): positive 442 (M+H).

### Part G: Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester

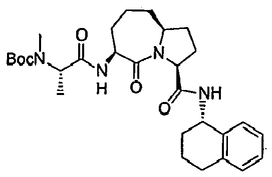

To a solution of [5-Oxo-3-(1,2,3,4-tetrahydro-naphthalen-1-ylcarbamoyl)-octahydro-pyrrolo[1,2-a]azepin-6-yl]-carbamic acid tert-butyl ester (61.8 mg, 0.14 mmol) in ethyl acetate (5 mL) was added 4M HCl in 1,4-dioxane (0.11 ml, 3 eq.). The resulting mixture was stirred at room temperature for 18 hours then concentrated under reduced pressure. The crude product that was added to a solution of Boc-N-Me-Ala-OH (53 mg, 0.20 mmol), HOBt (35 mg, 0.26 mmol), EDCI (50 mg, 0.26 mmol), DIEA (56µl, 0.32 mmol) in DMF (1 mL), and the resulting mixture was stirred at room temperature for 18 hours. It was then diluted with ethyl acetate, washed with 5% KHSO₄, saturated sodium bicarbonate solution, brine, (Na₂SO₄), and concentrated. The crude product was purified by flash column chromatography on silica gel eluting with 5% methanol/ methylene chloride to afford the title compound ( 44.24 mg, 60%). MS (ES) forC₂₉H₄₂N₄O₅ (MW=526.67): positive: 527(M+H).

### Part H: 6-(2-Methylamino-propionylamino)-5-oxo-octahydro-pyrrolo[1,2-a]azepine-3-carboxylic acid (1,2,3,4-tetrahydro-naphthalen-1-yl)-amide

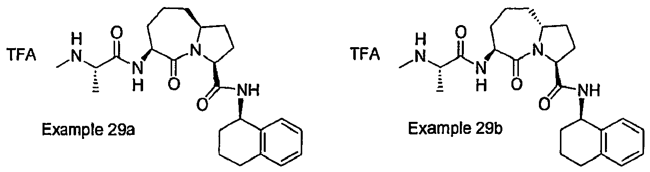

To a solution of Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester (73 mg, 0.14 mmol) in ethyl acetate (5mL) was added 4 M HCl in 1,4-dioxane (0.11mL). The reaction mixture was stirred at room temperature for 18 hours, concentrated, and was purified by reverse phase HPLC, using a C18 Vydec column and eluting with a gradient of 5% to 40% acetonitrile in water containing 0.1% TFA over 30 minutes, yielding two isomers. The first fraction **example 29a** : 15 mg, Analytical HPLC RT = 8.535 min. MS (ES) for C₂₄H₃₄N₄O₃ (MW=426.55): positive 427(M+H). The second fraction **example 29b**: 2 mg, MS (ES) for C₂₄H₃₄N₄O₃ (MW=426.55): positive 427(M+H).

### Example 30

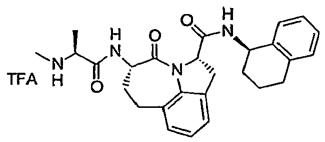

### 5-(2-Methylamino-propionylamino)-4-oxo-1,2,4,5,6,7-hexahydro-azepino[3,2,1-hi]indole-2-carboxylicacid (1,2,3,4-tetrahydro-naphthalen-1-yl)-amide

### Part A: [4-Ozo-2-(1,2,3,4-tetrahydro-naphthalen-1-ylcarbamoyl)-1,2,4,5,6,7-hexahydro-azepmo[3,2,1-hi]mdol-5-yl]-carbamic acid 9H-fluoren-9-ylmethyl ester

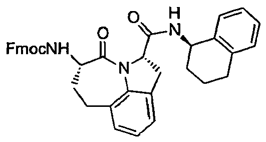

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.460 g, 0.98 mmol) was dissolved in dimethylformamide (DMF) (2 mL) and then added to aminodehydronaphthalene (0.172 g, 1.17mmol, 1.2eq) with an additional 2 mL DMF. The reaction mixture was stirred under nitrogen and cooled to 0°C using an ice bath. To this was added N-methylmorpholine (NMM) (1.08 mL, 9.8 mmol, 10 eq.) followed by propane phosponic acid anhydride (T3P, 50% w/w soln in DMF) (0.44 mL, 3.6 mmol, 3.7 eq). After stirring for 1.5 hr, the reaction mixture was diluted with ethyl acetate, poured into a dilute brine solution, then extracted 3 times with ethyl acetate. The organic layers were washed successively with 5% KHSO₄, saturated NaHCO₃, then saturated NaCl solutions, dried over anhydrous Na₂SO₄ and evaporated to a dryness (clear oil). The crude material was purified by flash column chromatography on silica gel eluting with ethyl acetate/hexane (25-50%) to give the title compound (0.432 g, 76%) as a white solid. TLC (50% ethyl acetate/hexane) R*_{f}*= 0.4.

### Part B: N 5-Amino-4-oxo-1,2,4,5,6,7-hexahydro-azepino[3,2,1-hi]indole-2-carboxylic acid (1,2,3,4-tetrahydro-naphthalen-1-yl)-amide

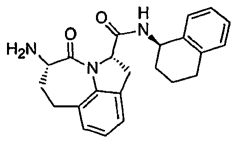

A solution of [4-Oxo-2-(1,2,3,4-tetrahydro-naphthalen-1-ylcarbamoyl)-1,2,4,5,6,7-hexahydro-azepino[3,2,1-hi]indol-5-yl]-carbamic acid 9H-fluoren-9-ylmethyl ester (0.136 g, 0.23 mmol) dissolved in dimethylformamide (DMF, 2 mL) at room temperature was treated with diethylamine (0.1mL, a 5% solution of diethylamine in DMF) and stirred for 1 hour at which time the reaction was complete by TLC. The reaction mixture was then evaporated under reduced pressure, azeotroping two times with methanol to give the crude title compound as light yellow oil, which was carried on to the next step without purification. TLC (90/10/1 CH₂Cl₂/methanol/acetic acid) R_{f}= 0.15; MS(ES) for C₂₃H₂₅N₃O₂ (MW=375.5) positive 376 (M+H), negative 374 (M-H).

### Part C: Methyl-{1-[4-ozo-2-(1,2,3,4-tetrahydro-naphthalen-1-ylcarbamoyl)-1,2,4,5,6,7-hexahydro-azepino[3,2,1-hi]indol-5-ylcarbamoyl]-ethyl}-carbamic acid tert-butyl ester

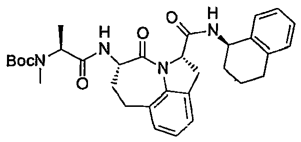

To a solution of crude *N* 5-Amino-4-oxo-1,2,4,5,6,7-hexahydro-azepino[3,2,1-hi]indole-2-carboxylic acid (1,2,3,4-tetrahydro-naphthalen-1-yl)-amide (0.086 g, 0.23 mmol, theoretical yield from previous reaction) in methylene chloride (2.3 mL) was added Boc-N-methyl-alanine carboxylic acid (0.056 g, 0.27 mmol, 1.2eq.). Then HATU was added (0.103 g, 0.27 mmol, 1.2eq), and finally diisopropylethylamine (0.06 mL, 0.46 mmol, 2eq.) After stirring 18 hrs the reaction was complete by TLC. The reaction mixture was diluted with ethyl acetate and washed successively with 5% KHSO₄, saturated NaHCO₃, then saturated NaCl solutions, dried over anhydrous Na₂SO₄ and evaporated to a dryness (clear oil). The crude material was purified by flash column chromatography on silica gel eluting with 30% ethyl acetate/hexane to give the title compound (0.1 g, 73% over 2 steps) as a clear oil. TLC (50% ethyl acetate/hexane) R_{f}= 0.35. MS(ES) for C₃₂H₄₀N₄O₅ (MW=560.68) positive 561 (M+H), negative 559 (M-H).

### Part D: 5-(2-Methylamino-propionylamino)-4-oxo-1,2,4,5,6,7-hexahydro-azepino[3,2,1-hi]indole-2-carboxylicacid (1,2,3,4-tetrahydro-naphthalen-1-yl)-amide

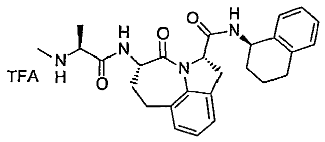

To a solution of methyl-{1-[4-oxo-2-(1,2,3,4-tetrahydro-naphthalen-1-ylcarbamoyl)-1,2,4,5,6,7-hexahydro-azepino[3,2,1-hi]indol-5-ylcarbamoyl]-ethyl}-carbamic acid tert-butyl ester (0.094 g, 0.17mmol) in ethyl acetate (1.2 mL) at 0°C (ice bath) was added 4M HCl in dioxane (0.4 mL, 1.5 mmol, 8.8 eq.). The reaction was stirred 16 hrs, slowly coming to room temperature as ice melted. Reaction was complete by TLC, and the solution was evaporated to dryness to give the crude title compound as an oil. The crude material was purified by reverse phase HPLC, using a C18 Vydec column and eluting with a gradient of 5% to 40% acetonitrile in water containing 0.1% TFA over 30 minutes, yielding (0.065 g, 76%). (90/10/1 CH₂Cl₂/methanol/acetic acid) R_{f}= 0.12; MS(ES) for C₂₇H₃₂N₄O₃ (MW=460.8): positive 461 (M+H).

### Example 31

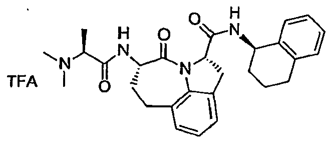

### 5-(2-Dimethylamino-propionylamino)-4-oxo-1,2,4,5,6,7-hexahydro-azepino[3,2,1-hi]indole-2-carboxyli c acid (1,2,3,4-tetrahydro-naphthalen-1-yl)-amide

To a solution of crude *N* 5-Amino-4-oxo-1,2,4,5,6,7-hexahydro-azepino[3,2,1-hi]indole-2-carboxylic acid (1,2,3,4-tetrahydro-naphthalen-1-yl)-amide (0.086 g, 0.23 mmol, theoretical yield from Part B of example 30) in DMF (4 mL) was added N,N-dimethyl-alanine carboxylic acid (0.035 g, 0.3 mmol, 1.2eq.) and the mixture cooled to 0°C. Then HOBt (0.037 g, 0.28 mmol, 1.2eq) and EDCI (0.071 g, 0.37 mmol, 1.6eq) were added, then finally diisopropylethylamine (0.2 mL, 1.15 mmol, 5eq.). After stirring 18 hrs (coming to room temperature overnight) the reaction was complete by TLC. The reaction mixture was diluted with ethyl acetate and washed successively with saturated NaHCO₃/saturated NaCl solution mixture, followed by a solution of 1N sodium hydroxide and saturated NaCl solution, then dried over anhydrous Na₂SO₄ and evaporated to a dryness (clear oil). The crude material was then taken up in of ethanol (10 mL) and cooled to 0°C. To this solution was added a 6M solution of HCl in ethyl acetate, and the mixture stirred for 16 hours, coming to room temperature overnight. The crude material was purified using reverse phase HPLC, with a C18 Vydec column eluting with a gradient of 5% to 40% acetonitrile in water containing 0.1% TFA over 30 minutes, yielding (0.065 g, 76%). TLC (90/10/1 CH₂Cl₂/methanol/acetic acid) R_{f}= 0.12; MS(ES) for C₂₈H₃₄N₄O₃ (MW=474.6): positive 475.36 (M+H) negative 473.22 (M-H).

### Example 32

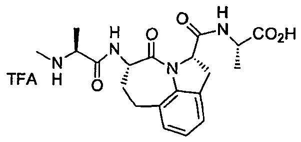

### 2-{[5-(2-Methylamino-propionylamino)-4-oxo-1,2,4,5,6,7-hexahydro-azepino[3,2,1-hi]indole-2-carbonyl]-amino}-propionic acid

### Part A: 2-{[5-(9H-Fluoren-9-ylmethoxycarbonylamino)-4-oxo-1,2,4,5,6,7-hexahydro-azepino[3,2,1-hi]indole-2-carbonyl]-amino}-propionic acid tert-butyl ester

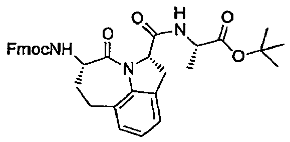

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.258 g, 0.55 mmol) was dissolved in dimethylformamide (DMF) (2 mL) and then added to alanine t-butyl ester HCl salt (0.12 g, 0.66 mmol, 1.2eq). The reaction mixture was then stirred under nitrogen and cooled to 0°C using an ice bath. To this was added N-methylmorpholine (NMM) (0.3 mL, 2.75 mmol, 5 eq.) followed by propane phosponic acid anhydride (T3P, 50% w/w soln in DMF) (1.1g, 1.65 mmol, 3 eq). After stirring for 1.5 hr, the reaction mixture was diluted with ethyl acetate, poured into a dilute brine solution, and extracted 3 times with ethyl acetate. The organic layers were washed successively with 5% KHSO₄, saturated NaHCO₃, then saturated NaCl solutions, dried over anhydrous Na₂SO₄ and evaporated to dryness (clear oil). The crude material was purified by flash column chromatography on silica gel eluting with ethyl acetate/hexane (25-50%) to give the title compound (0.291 g, 89%) as a white solid. TLC (50% ethyl acetate/hexane) R_{f}= 0.6.

### Part B: 2-[(5-Amino-4-oxo-1,2,4,5,6,7-hexahydro-azepino[3,2,1-hi]indole-2-carbonyl)-amino]-propionic acid tert-butyl ester

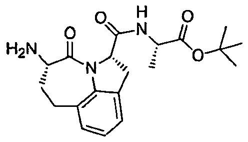

A solution of 2-{[5-(9H-Fluoren-9-ylmethoxycarbonylamino)-4-oxo-1,2,4,5,6,7-hexahydro-azepino[3,2,1-hi]indole-2-carbonyl]-amino}-propionic acid tert-butyl ester (0.255 g, 0.43 mmol) dissolved in dimethylformamide (DMF, 2 mL) at room temperature was treated with diethylamine (0.1mL, a 5% solution of diethylamine in DMF) and stirred for 30 minutes at which time the reaction was complete by TLC. The reaction mixture was then evaporated under reduced pressure, azeotroping two times with methanol to give the crude title compound as light yellow oil. TLC (1/1 ethyl acetate/hexanes) R_{f}= 0.1; MS(ES) for C₂₀H₂₇N₃O₄ (MW=373.45) positive 374 (M+H), negative 372.33 (M-H).

### Part C: 2-({5-[2-(tert-Butoxycarbonyl-methyl-amino)-propionylamino]-4-oxo-1,2,4,5,6,7-hexahydro-azepino[3,2,1-hi]indole-2-carbonyl}-amino)-propionic acid tert-butyl ester

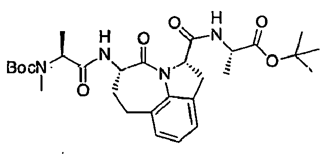

To a solution of crude 2-[(5-Amino-4-oxo-1,2,4,5,6,7-hexahydro-azepino[3,2,1-hi]indole-2-carbonyl)-amino]-propionic acid tert-butyl ester (0.187 g, 0.43 mmol, theoretical yield from previous reaction) in DMF (2 mL) was added Boc-N-methyl-alanine carboxylic acid (0.105 g, 0.52 mmol, 1.2eq.). To this was added N-methylmorpholine (NMM) (0.23 mL, 2.15 mmol, 5 eq.) followed by propane phosponic acid anhydride (T3P, 50% w/w soln in DMF) (0.82 g, 1.3 mmol, 3 eq). After stirring for 45 minutes the reaction was complete by TLC. The reaction mixture was diluted with ethyl acetate, poured into a dilute brine solution, then extracted 3 times with ethyl acetate. The organic layers were washed successively with 5% KHSO₄, saturated NaHCO₃, then saturated NaCl solutions, dried over anhydrous Na₂SO₄ and evaporated to dryness (clear oil). The crude material was purified by flash column chromatography on silica gel eluting with ethyl acetate/hexane (30-50%) to give the title compound (0.193 g, 80%) as an off-white sticky solid. TLC (1/1 ethyl acetate/hexane) R_{f}= 0.2. MS(ES) for C₂₉H₄₂N₄O₇ (MW=558.6) negative 557 (M-H).

### Part D: 2-{[5-(2-Methylamino-propionylamino)-4-oxo-1,2,4,5,6,7-hexahydro-azepino[3,2,1-hi]indole-2-carbonyl]-amino}-propionic acid tert-butyl ester

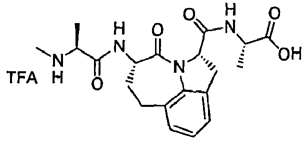

The starting material, 2-({5-[2-(tert-Butoxycarbonyl-methyl-amino)-propionylamino]-4-oxo-1,2,4,5,6,7-hexahydro-azepino[3, 2,1-hi]indole-2-carbonyl}-amino)-propionic acid tert-butyl ester (0.148 g, 0.27mmol) was taken up in a 50% v/v solution of TFA in methylene chloride (4 mL) at 0°C (ice bath). After stirring for 1.5 hours the reaction was complete by TLC. The reaction mixture was then evaporated to dryness to give the crude title compound as an oil. The crude material was purified using reverse phase HPLC, using a C18 Vydec column and eluting with a gradient of 5% to 50% acetonitrile in water containing 0.1% TFA over 30 minutes, yielding (0.07 g, 65%). Anayltical HPLC Rₜ-5.74 min. TLC (90/10/1 CH₂Cl₂/methanol/acetic acid) R_{f}= 0.12; MS(ES) for C₂₀H₂₆N₄O₅ (MW=402.4): positive 403. (M+H) negative 401 (M-H).

### Example 33

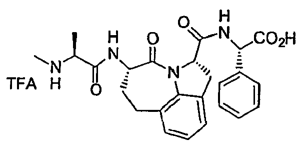

### {[5-(2-Methylamino-propionylamino)-4-oxo-1,2,4,5,6,7-hexahydro-azepino{3,2,1-hi]indole-2-carbonyl]-amino}-phenyl-acetic acid

### Part A: {[5-(9H-Fluoren-9-ylmethoxycarbonylamino)-4-oxo-1,2,4,5,6,7-hexahydro-azepino[3,2,1-h]indole-2-carbonyl]-amino]-phenyl-acetic acid tert-butyl ester

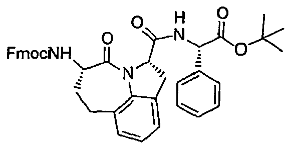

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.258 g, 0.55 mmol) was dissolved in dimethylformamide (DMF) (2 mL) and then added to phenylglycine t-butyl ester HCl salt (0.161 g, 0.66 mmol, 1.2eq). The reaction mixture was stirred under nitrogen and cooled to 0°C using an ice bath. To this was added N-methylmorpholine (NMM) (0.3 mL, 2.75 mmol, 5 eq.) followed by propane phosponic acid anhydride (T3P, 50% w/w soln in DMF) (1.1g, 1.65 mmol, 3 eq). After stirring for 1.5 hr, the reaction was complete by TLC. The reaction mixture was diluted with ethyl acetate, poured into a dilute brine solution, then extracted 3 times with ethyl acetate. The organic layers were washed successively with 5% KHSO₄, saturated NaHCO₃, then saturated NaCl solutions, dried over anhydrous Na₂SO₄ and evaporated to dryness (clear oil). The crude material was purified by flash column chromatography on silica gel eluting with ethyl acetate/hexane (25-50%) to give the title compound (0.391 g, 88%) as a white foam. TLC (50% ethyl acetate/hexane) R_{f}=0.8.

### Part B: [(5-Amino-4-oxo-1,2,4,5,6,7-hexahydro-azepino[3,2,1-hi]indole-2-carbonyl)-amino]-phenyl-acetic acid tert-butyl ester

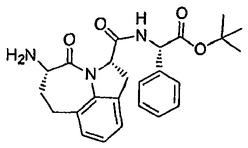

A solution of {[5-(9H-Fluoren-9-ylmethoxycarbonylamino)-4-oxo-1,2,4,5,6,7-hexahydro-azepino[3,2,1-hi]indole-2-carbonyl]-amino}-phenyl-acetic acid tert-butyl ester (0.278 g, 0.423 mmol) dissolved in dimethylformamide (DMF, 2 mL) at room temperature was treated with diethylamine (0.1mL, a 5% solution of diethylamine in DMF) and stirred for 30 minutes at which time the reaction was complete by TLC. The reaction mixture was then evaporated under reduced pressure, azeotroping two times with methanol to give the crude title compound as light yellow oil. TLC (90/10/1 methylene chloride, methanol, acetic acid) R_{f}= 0.35; MS(ES) for C₂₅H₂₉N₃O₄ (MW=435.52) positive 436.31 (M+H), negative 434.20 (M-H).

### Part C: ({5-[2-(tert-Butoxycarbonyl-methyl-amino)-propionylamino]-4-oxo-1,2,4,5,6,7-hexahydro-azepino[3,2,1-hi]indole-2-carbonyl}-amino)-phenyl-acetic acid isopropyl ester

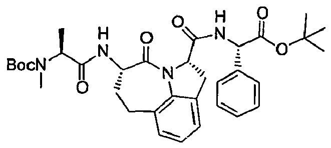

To a solution of crude [(5-Amino-4-oxo-1,2,4,5,6,7-hexahydro-azepino[3,2,1-hi]indole-2-carbonyl)-amino]-phenyl-acetic acid tert-butyl ester (0.184 g, 0.423 mmol, theoretical yield from previous reaction) in DMF (2 mL) was added Boc-N-methyl-alanine carboxylic acid (0.103 g, 0.51 mmol, 1.2eq.). To this was added N-methylmorpholine (NMM) (0.23 mL, 2.12 mmol, 5 eq.) followed by propane phosponic acid anhydride (T3P, 50% w/w soln in DMF) (0.8 g, 1.3 mmol, 3 eq). After stirring for 1 hour, the reaction was complete by TLC. The reaction mixture was diluted with ethyl acetate, poured into a dilute brine solution, and extracted 3 times with ethyl acetate. The organic layers were washed successively with 5% KHSO₄, saturated NaHCO₃, then saturated NaCl solutions, dried over anhydrous Na₂SO₄ and evaporated to a dryness (clear oil). The crude material was purified by flash column chromatography on silica gel eluting with ethyl acetate/hexane (30-50%) to give the title compound (0.201 g, 76%) as an off-white sticky solid. TLC (1/1 ethyl acetate/hexane) R_{f}= 0.2. MS(ES) for C₃₄N₄₄N₄O₇ (MW=620.7) positive 621.3 (M+H), negative 619.14 (M-H).

### Part D: {[5-(2-Methylamino-propionylamino)-4-oxo-1,2,4,5,6,7-hexahydro-azepino[3,2,1-hi]indole-2-carbonyl] -amino}-phenyl-acetic acid

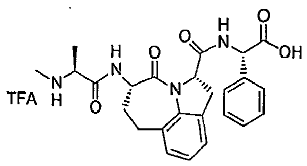

The starting material, ({5-[2-(tert-Butoxycarbonyl-methyl-amino)-propionylamino]-4-oxo-1,2,4,5,6,7-hexahydro-azepino[3,2, 1-hi]indole-2-carbonyl}-amino)-phenyl-acetic acid isopropyl ester (0.157 g, 0.25mmol) was taken up in a 50% v/v solution of TFA in methylene chloride (4 mL) at 0°C (ice bath). After stirring for 1.5 hours the reaction was complete by TLC. The reaction mixture was then evaporated to dryness to give the crude title compound as an oil. The crude material was purified using reverse phase HPLC, using a C18 Vydec column and eluting with a gradient of 5% to 50% acetonitrile in water containing 0.1 % TFA over 30 minutes, yielding (0.036 g, 31 %). (90/10/1 CH₂Cl₂/methanol/acetic acid) R_{f}= 0.15; MS(ES) for C₂₅H₂₈N₄O₅ (MW=464.5): positive 465.3 (M+H) negative 463.2 (M-H).

### Example 34

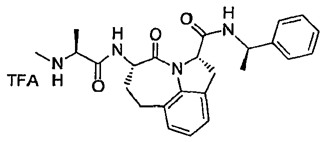

### 5-(2-Methylamino-propionylamino)-4-oxo-1,2,4,5,6,7-hexahydro-azepino[3,2,1-hi]indole-2-carboxylic acid (1-phenyl-ethyl)-amide

### Part A: [4-Oxo-2-(1-phenyl-ethylcarbamoyl)-1,2,4,5,6,7-hexahydro-azepino[3,2,1-hi]indol-5-yl]-carbamic acid 9H-fluoren-9-ylmethyl ester.

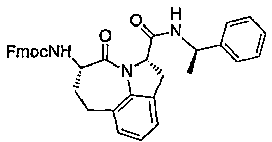

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.330 g, 0.7mmol) was dissolved in dimethylformamide (DMF) (2.8 mL) and then added to R(+)-alpha methylbenzylamine (0.102 g, 0.85 mmol, 1.2eq). The reaction mixture was then stirred under nitrogen and cooled to 0°C using an ice bath. To this was added N-methylmorpholine (NMM) (0.77 mL, 7.04 mmol, 10 eq.) followed by propane phosponic acid anhydride (T3P, 50% w/w soln in DMF) (1.34 g, 2.11 mmol, 3 eq). After stirring for 1.5 hr, the reaction was still not complete by TLC, so an additional amount of NMM (0.88 mL, 8 mmol, 11 eq.) followed by T3P (2.1 g, 3.3 mmol, 4.7 eq) and the reaction was allowed to come to room temperature overnight. The next day the reaction was complete by TLC, then diluted with ethyl acetate, poured into a dilute brine solution, then extracted 3 times with ethyl acetate. The organic layers were washed successively with 5% KHSO₄, then saturated NaCl solution, dried over anhydrous Na₂SO₄ and evaporated to dryness (clear oil). The crude material was purified by flash column chromatography on silica gel eluting with ethyl acetate/hexane (50%) to give the title compound (0.201 g, 50%) as a white solid. TLC (50% ethyl acetate/hexane) R_{f}= 0.35.

### Part B: 5-Amino-4-oxo-1,2,4,5,6,7-hexahydro-azepino[3,2,1-hi]indole-2-carboxylic acid (1-phenyl-ethyl)-amide

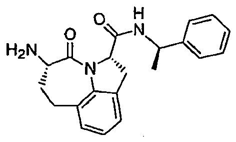

A solution of [4-Oxo-2-(1-phenyl-ethylcarbamoyl)-1,2,4,5,6,7-hexahydro-azepino[3,2,1-hi]indol-5-yl]-carbamic acid 9H-fluoren-9-ylmethyl ester (0.102 g, 0.18 mmol) dissolved in methylene chloride (2 mL) at room temperature was treated with diethylamine (1 mL, a 12.5% solution of diethylamine in CH₂Cl₂) and stirred for 2 hours at which time the reaction was complete by TLC. The reaction mixture was then evaporated under reduced pressure to give the crude title compound as light yellow oil. TLC (1/1 ethyl acetate/hexanes) R_{f}= 0.1.

### Part C: Methyl-{1-[4-oxo-2-(1-phenyl-ethylcarbamoyl)-1,2,4,5,6,7-hexahydro-azepino[3,2,1-hi]indol-5-ylcarbamoyl]-ethyl}-carbamic acid tert-butyl ester

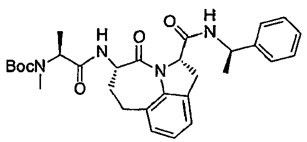

To a solution of crude 5-Amino-4-oxo-1,2,4,5,6,7-hexahydro-azepino[3,2,1-hi]indole-2-carboxylic acid (1-phenyl-ethyl)-amide (0.063 g, 0.18 mmol, theoretical yield from previous reaction) in DMF (0.7 mL) was added Boc-N-methyl-alanine carboxylic acid (0.037 g, 0.18 mmol, 1eq.). To this was added N-methylmorpholine (NMM) (0.18 g, 1.8 mmol, 10 eq.) followed by propane phosponic acid anhydride (T3P, 50% w/w soln in DMF) (0.344 g, 0.54 mmol, 3 eq). After stirring for 30 minutes the reaction was complete by TLC. The reaction mixture was diluted with ethyl acetate and washed successively with 1M HCl, saturated NaCl solution, saturated NaHCO₃, then saturated NaCl solution again, dried over anhydrous Na₂SO₄ and evaporated to a dryness (clear oil). The crude material was carried on without purification (0.128 g) as an oil. TLC (2/1 =ethyl acetate/hexane) R_{f} = 0.3 & 0.8.

### Part D: 5-(2-Methylamino-propionylamino)-4-oxo-1,2,4,5,6,7-hexahydro-azepino[3,2,1-hi]indole-2-carboxylic acid (1-phenyl-ethyl)-amide

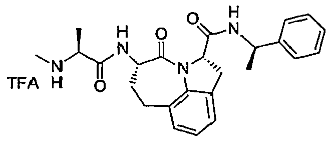

The crude starting material, methyl-{1-[4-oxo-2-(1-phenyl-ethylcarbamoyl)-1,2,4,5,6,7-hexahydro-azepino[3,2,1-hi]indol-5-ylcarbamoyl]-ethyl}-carbamic acid tert-butyl ester (0.063 g, 0.18 mmol, theoretical yield from previous reaction) was taken up in ethyl acetate (6 mL) at 0°C (ice bath), then treated with a 4M solution of HCl in dioxane (2 mL, 8 mmols, 44eq.). After stirring 16 hours the reaction was complete by TLC. Methanol was added to the reaction mixture, and the resulting crude hydrochloride salt precipitate filtered and isolated. The crude material was purified by reverse phase HPLC, using a C18 Vydec column and eluting with a gradient of 5% to 50% acetonitrile in water containing 0.1 % TFA over 30 minutes, yielding (0.034 g, 44% over 3 steps). Analytical HPLC- Rₜ-8.35 min.; MS(ES) for C₂₅H₃₀N₄O₃ (MW=434.53): positive 435.33 (M+H) negative 433.21 (M-H).

### Example 35

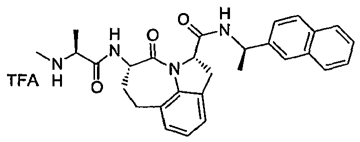

### 5-(2-Methylamino-propionylamino)-4-oxo-1,2,4,5,6,7-hexahydro-azepino[3,2,1-hi]indole-2-carboxylic acid (1-naphthalen-2-yl-ethyl)-amide type in exp detail

### Part A: [2-(1-Naphthalen-2-yl-ethylcarbamoyl)-4-oxo-1,2,4,5,6,7-hexahydro-azepino[3,2,1-hi]indol-5-yl]-carbamic acid 9H-fluoren-9-ylmethyl ester

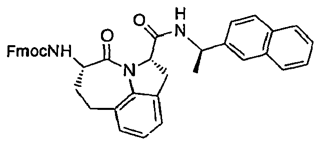

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.341 g, 0.73 mmol) was dissolved in dimethylformamide (DMF) (3 mL) and then added to R(+)-1-(2-naphthyl)ethylamine (0.15 g, 0.87 mmol, 1.2eq). The reaction mixture was then stirred under nitrogen and cooled to 0°C using an ice bath. To this was added N-methylmorpholine (NMM) (0.735 g, 7.28 mmol, 10 eq.) followed by propane phosponic acid anhydride (T3P, 50% w/w soln in DMF) (3.4 g, 5.34 mmol, 7.3 eq). After stirring for 3 hr, the reaction mixture was diluted with ethyl acetate (5 mL) and methanol (30 mL) to yield the title compound as a white precipitate that was filtered and collected (0.192 g, TLC (50% ethyl acetate/hexane) R_{f}= 0.35). The crude filtrate was concentrated under reduced pressure and purified by flash column chromatography on silica gel eluting with ethyl acetate/hexane (3/2) to give more title compound (0.098 g, combined precipitate and off the column,0.29 g, 64%) as a white solid.

### Part B: 5-Amino-4-oxo-1,2,4,5,6,7-hexahydro-azepino[3,2,1-hi]indole-2-carboxylic acid (1-naphthalen-2-yl-ethyl)-amide

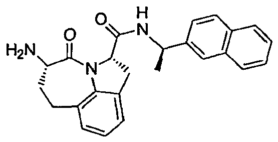

A solution of [2-(1-Naphthalen-2-yl-ethylcarbamoyl)-4-oxo-1,2,4,5,6,7-hexahydro-azepino[3,2,1-hi]indol-5-yl]-carbamic acid 9H-fluoren-9-ylmethyl ester (0.122 g, 0.2 mmol) dissolved in methylene chloride (2 mL) at room temperature was treated with diethylamine (1 mL, a 33% solution of diethylamine in CH₂Cl₂) and stirred for 3 hours at which time the reaction was complete by TLC. The reaction mixture was then evaporated under reduced pressure to give the crude title compound as light yellow oil. TLC (1/1 ethyl acetate hexanes) R_{f}= 0.1.

### Part C: Methyl-{1-[2-(1-naphthalen-2-yl-ethylcarbamoyl)-4-oxo-1,2,4,5,6,7-hexahydro-azepino[3,2,1-hi]indol-5-ylcarbamoyl]-ethyl}-carbamic acid tert-butyl ester

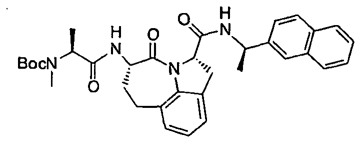

To a solution of crude 5-amino-4-oxo-1,2,4,5,6,7-hexahydro-azepino[3,2,1-hi]indole-2-carboxylic acid (1-naphthalen-2-yl-ethyl)-amide (0.078 g, 0.20 mmol, theoretical yield from previous reaction) in DMF (0.8 mL) was added Boc-N-methyl-alanine carboxylic acid (0.041 g, 0.20 mmol, 1eq.). To this was added N-methylmorpholine (NMM) (0.202 g, 2 mmol, 10 eq.) followed by propane phosponic acid anhydride (T3P, 50% w/w soln in DMF) (0.382 g, 0.6 mmol, 3 eq). After stirring for 3 hours the reaction was complete by TLC. The reaction mixture was diluted with ethyl acetate and washed successively with 1M HCl then saturated NaCl solution, dried over anhydrous Na₂SO₄, and evaporated to a dryness (clear oil). The crude material was carried on directly in the next reaction without purification.

### Part D: 5-(2-Methylamino-propionylamino)-4-oxo-1,2,4,5,6,7-hexahydro-azepino[3,2,1-hi]indole-2-carbozylic acid (1-naphthalen-2-yl-ethyl)-amide

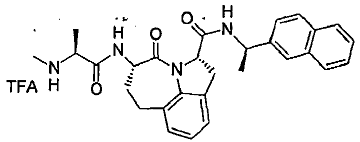

The starting material, methyl-{1-[2-(1-naphthalen-2-yl-ethylcarbamoyl)-4-oxo-1,2,4,5,6,7-hexahydro-azepino[3,2,1-hi]indol-5-ylcarbamoyl]-ethyl} -carbamic acid tert-butyl ester (0.078 g, 0.20 mmol, theoretical yield from previous reaction) was taken up in a 50% v/v solution of TFA in methylene chloride (10 mL) at 0°C (ice bath). After stirring for 1.5 hours the reaction was complete by TLC. Heptane was added and the crude reaction mixture was evaporated to dryness to give the crude title compound as an oil. The crude material was purified using reverse phase HPLC, using a C18 Vydec column and eluting with a gradient of 5% to 50% acetonitrile in water containing 0.1% TFA over 30 minutes, yielding (0.006 g, 7%). Analytical HPLC- Rₜ-8.35 min.; MS(ES) for C₂₉H₃₂N₄O₃ (MW=484.59): positive 785.36 (M+H) negative 483.24 (M-H).

### Example 36

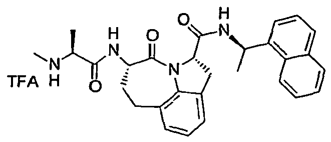

### 5-(2-Methylamino-propionylamino)-4-oxo-1,2,4,5,6,7-hexahydro-azepino[3,2,1-hi]indole-2-carboxylic acid (1-naphthalen-1-yl-ethyl)-amide

### Part A: [[2-(1-Naphthalen-1-yl-ethylcarbamoyl)-4-oxo-1,2,4,5,6,7-hexahydro-azepino[3,2,1-hi]indol-5-yl]-carbamic acid 9H-fluoren-9-ylmethyl ester

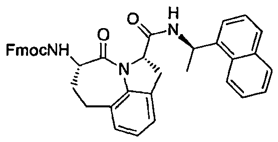

The 5-(9H-Fluoren-9-ylmethoxycarbonylamino)-4-oxo-1,2,4,5,6,7-hexahydro-azepino[3,2,1-hi]indole-2-carboxylic acid (FMOC-Haic acid) (0.325 g, 0.69 mmol) was dissolved in dimethylformamide (DMF) (2.77 mL) and then added to R(+)-1-aminoethylnaphthylamine (0.143 g, 0.83 mmol, 1.2eq). The reaction mixture was then stirred under nitrogen and cooled to 0°C using an ice bath. To this was added N-methylinorpholine (NMM) (0.7 g, 6.94 mmol, 10 eq.) followed by propane phosponic acid anhydride (T3P, 50% w/w soln in DMF) (1.32 g, 2.1 mmol, 3 eq). The reaction progression was followed by TLC over the course of 4 hours, requiring the addition of more NMM (0.92 g, 9 mmol) and T3P (1.5 g, 2.4 mmol) until the FMOC-Haic acid starting material was consumed. The reaction mixture was then diluted with ethyl acetate and washed successively with 1M HCl, brine, dried over anhydrous Na₂SO₄ and evaporated to dryness (clear oil). The crude product was treated with ethyl acetate (3 mL) and methanol (5 mL) and methylene chloride (5 mL) to yield the title compound as a white precipitate that was filtered and collected (0.214 g, TLC (50% ethyl acetate/hexane) R_{f}= 0.3). The crude filtrate was concentrated under reduced pressure and purified by flash column chromatography on silica gel eluting with ethyl acetate/hexane (1/1) to give more title compound (0.126 g; combined precipitate and off the column 0.34 g, 64%) as a white solid.

### Part B: 5-Amino-4-oxo-1,2,4,5,6,7-hexahydro-azepino[3,2,1-hi]indole-2-carboxylic acid (1-naphthalen-1-yl-ethyl)-amide

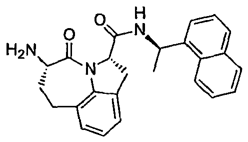

A solution of [2-(1-naphthalen-1-yl-ethylcarbamoyl)-4-oxo-1,2,4,5,6,7-hexahydro-azepino[3,2,1-hi]indol-5-yl]-carbamic acid 9H-fluoren-9-ylmethyl ester (0.115 g, 0.18 mmol) dissolved in methylene chloride (2 mL) at room temperature was treated with diethylamine (1 mL, a 33% solution of diethylamine in methylene chloride) and stirred for 3.5 hours at which time the reaction was complete by TLC. The reaction mixture was then evaporated under reduced pressure to give the crude title compound as light yellow oil. TLC (1/1 ethyl acetate hexanes) R_{f}= 0.1

### Part C: Methyl-{1-[2-(1-naphthalen-1-yl-ethylcarbamoyl)-4-oxo-1,2,4,5,6,7-hezahydro-azepino[3,2,1-hi]indol-5-ylcarbamoyl]-ethyl}-carbamic acid tert-butyl ester

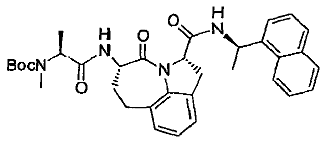

To a solution of crude 5-amino-4-oxo-1,2,4,5,6,7-hexahydro-azepino[3,2,1-hi]indole-2-carboxylic acid (1-naphthalen-1-yl-ethyl)-amide (0.072 g, 0.18 mmol, theoretical yield from previous reaction) in DMF (0.72 mL) was added Boc-N-methyl-alanine carboxylic acid (0.037 g, 0.18 mmol, 1eq.). To this was added N-methylmorpholine (NMM) (0.182 g, 1.8 mmol, 10 eq.) followed by propane phosponic acid anhydride (T3P, 50% w/w soln in DMF) (0.344 g, 0.54 mmol, 3 eq). After stirring for 1 hour the reaction was complete by TLC. The reaction mixture was diluted with ethyl acetate and washed successively with 1M HCl, brine, dried over anhydrous Na₂SO₄ and evaporated to a dryness (clear oil). The crude material was used in the next reaction without purification.

### Part D: 5-(2-Methylammo-propionylamino)-4-oxo-1,2,4,5,6,7-hexahydro-azepino[3,2,1-hi]indole-2-carboxylic acid (1-naphthalen-1-yl-ethyl)-amide

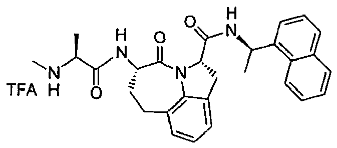

The starting material, methyl-{1-[2-(1-naphthalen-1-yl-ethylcarbamoyl)-4-oxo-1,2,4,5,6,7-hexahydro-azepino[3,2,1-hi]indol-5-ylcarbamoyl]-ethyl} -carbamic acid tert-butyl ester (0.105 g, 0.18 mmol, theoretical yield from previous reaction) was taken up in a 50% v/v solution of TFA in methylene chloride (6 mL) at 0°C (ice bath). After stirring for 1.5 hours the reaction was complete by TLC.. Heptane was added and the crude reaction mixture was evaporated to dryness to give the crude title compound that was purified by reverse phase HPLC, using a C18 Vydec column and eluting with a gradient of 5% to 50% acetonitrile in water containing 0.1% TFA over 30 minutes, yielding (0.026 g, 30% over 3 steps). Analytical HPLC- Rₜ- 10.28 min.; MS(ES) for C₂₉H₃₂N₄O₃ (MW=484.6): positive 485.31 (M+H) negative 483.2 (M-H).

### Example 37

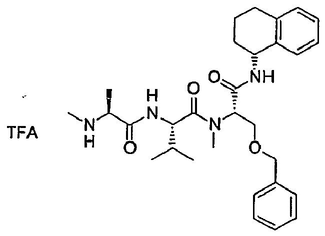

### N-[2-Benzyloxy-1-(1,2,3,4-tetrahydro-naphthalen-1-ylcarbamoyl)-ethyl]-3,N-dimethyl-2-(2-methylamino-propionylamino)-butyramide; trifluoroacetic acid salt

### Part A: [2-Benzyloxy-1-(1,2,3,4-tetrahydro-naphthalen-1-ylcarbamoyl)-ethyl]-methyl-carbamic acid 9H-fluoren-9-ylmethyl ester

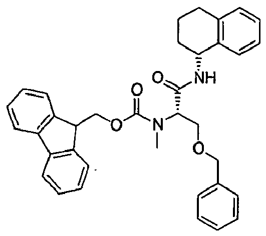

A solution of 3-benzyloxy-2-[(9*H*-fluoren-9-ylmethoxycarbonyl)-methyl-amino]-propionic acid (976.2 mg, 2.26 mmol) and (R)-1,2,3,4-tetrahydro-naphthalen-1-ylamine (398.0 mg, 2.70 mmol) in tetrahydrofuran (THF) (60 mL) was added 1-ethyl-3-(3-dimethylaminopropyl)carbodiimide hydrochloride (EDCI) (840 mg, 4.38 mmol) and 1-hydroxybenzotriazole monohydrate (HOBt) (420 mg, 3.33 mmol), followed by *N*-methyl-2-pyrrolidinone (0.75 mL) and 4-methyl morpholine (0.75 mL). This mixture was stirred at room temperature for 5 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (1.27 g, >95%) as a colorless oil: TLC (50% ethyl acetate/hexane) R*_{f}*= 0.48; MS (ES) for C₃₆H₃₆N₂O₄ (MW=560.68): positive 561 (M+H).

### Part B: 3-Benzyloxy-2-methylamino-N-(1,2,3,4-tetrahydro-naphthalen-1-yl-propionamide

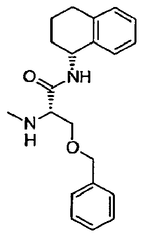

A solution of [2-Benzyloxy-1-(1,2,3,4-tetrahydro-naphthalen-1-ylcarbamoyl)-ethyl]-methyl-carbamic acid 9*H*-fluoren-9-ylmethyl ester (891.9 mg, 1.59 mmol) in dimethylformamide (DMF) (4 mL) was treated with diethylamine (DEA) (1 mL) at room temperature for 2 hours. The DEA was evaported, and the resulting solution was used without further purification in the subsequent reaction. TLC (ethyl acetate) R*_{f}*= 0.4; MS (ES) for C₂₁H₂₆N₂O₂ (MW=338.44): positive 339 (M+H), negative 337 (M-H).

### Part C: [1-(1-{[2-Benzyloxy-1-(1,2,3,4-tetrahydro-naphthalen-1-ylcarbamoyl)-ethyl]-methyl-carbamoyl}-2-methyl-propylcarbamoyl)-ethyl]-methyl-carbamic acid tert-butyl ester

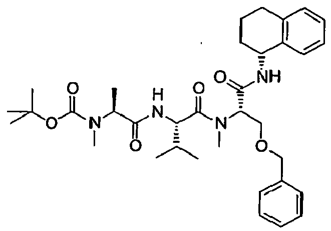

A solution of 2-[2-(*tert*-butoxycarbonyl-methyl-amino)-propionylamino]-3-methyl-butyric acid (530 mg, 1.75 mmol) and 3-benzyloxy-2-methylamino-*N*-(1-,2,3,4-tetrahydro-naphthalen-1-yl)-propionamide (1.59 mmol in DMF from above reaction) in tetrahydrofuran (THF) (40 mL) was added 1-ethyl-3-(3-dimethylaminopropyl)carbodiimide hydrochloride (EDCI) (700 mg, 3.65 mmol) and 1-hydroxybenzotriazole monohydrate (HOBt) (400 mg, 2.96 mmol), followed by *N*-methyl-2-pyrrolidinone (1 mL) and 4-methyl morpholine (1 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (674 mg, 39%) as a colorless oil. TLC (50% ethyl acetate/hexane) R*_{f}*= 0.26; MS (ES) for C₃₅H₅₀N₄O₆ (MW=622.79): positive 623 (M+H), negative 621 (M-H).

### Part D: N-[2-Benzyloxy-1-(1,2,3,4-tetrahydro-naphthalen-1-ylcarbamoyl)-ethyl]-3,N-dimethyl-2-(2-methylamino-propionylamino)-butyramide; trifluoroacetic acid salt

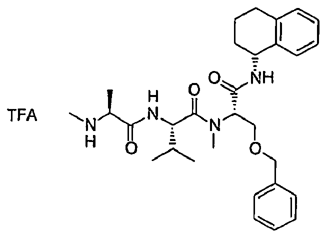

The [1-(1-{[2-benzyloxy-1-(1,2,3,4-tetrahydro-naphthalen-1-ylcarbamoyl)-ethyl]-methyl-carbamoyl}-2-methyl-propylcarbamoyl)-ethyl]-methyl-carbamic acid *tert-butyl* ester (575.5 mg, 0.924 mmol) was dissolved in dichloromethane (DCM) (10 mL). Trifluoroacetic acid (TFA) (8 mL) was added. After stirring at room temperature for 2 hours, the solvent was evaporated. The crude material was purified using reverse phase HPLC, using a C18 Vydec column and eluting with a gradient of 5% to 60% acetonitrile in water containing 0.1 % TFA over 30 minutes, yielding the title compound as a white solid (196 mg). Analytical HPLC Rₜ-12.24 min (58.5%) and 12.02 min (37.4%), rotomers); MS(ES) for C₃₀H₄₂N₄O₄ (MW=522.68): positive 523 (M+H), negative 521 (M-H).

### Example 38

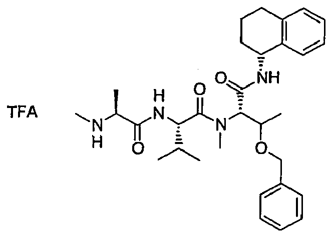

### N-[2-Benzyloxy-1-(1,2,3,4-tetrahydro-naphthalen-1-ylcarbamoyl)-propyl]-3,N-dimethyl-2-(2-methylamino-propionylamino)-butyramide; trifluoroacetic acid salt

### Part A: [2-Benzyloxy-1-(1,2,3,4-tetrahydro-naphthalen-1-ylcarbamoyl)-propyl]-methyl-carbamic acid tert-butyl ester

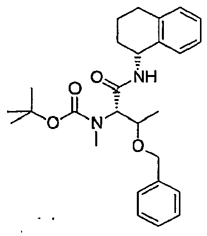

A solution of 3-benzyloxy-2-(*tert*-butoxycarbonyl-methyl-amino)-butyric acid (930 mg, 2.20 mmol) and (R)-1,2,3,4-tetrahydro-naphthalen-1-ylamine (510 mg, 3.43 mmol) in tetrahydrofuran (THF) (40 mL) was added 1-ethyl-3-(3-dimethylaminopropyl)carbodiimide hydrochloride (EDCI) (1.00 g, 5.21 mmol) and 1-hydroxybenzotriazole monohydrate (HOBt) (500 mg, 3.70 mmol), followed by *N*-methyl-2-pyrrolidinone (0.75 mL) and 4-methyl morpholine (0.75 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (996 mg, >95%) as a colorless oil. TLC (50% ethyl acetate/hexane) R*_{f}*= 0.26; MS (ES) for C₂₇H₃₆N₂O₄ (MW=452.59): positive 453 (M+H).

### Part B: 3-Benzyloxy-2-methylamino-N-(1,2,3,4-tetrahydro-naphthalen-1-yl)-butyramide; trifluoroacetic acid salt

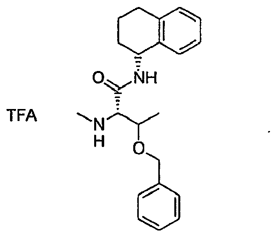

The [2-Benzyloxy-1-(1,2,3,4-tetrahydro-naphthalen-1-ylcarbamoyl)-propyl]-methyl-carbamic acid *tert-butyl* ester (605 mg, 1.34 mmol) was dissolved in dichloromethane (DCM) (10 mL). Trifluoroacetic acid (TFA) (6 mL) was added. After stirring at room temperature for 1 hour, the solvent was evaporated to give the title compound as an oil (623 mg, >95%). MS (ES) for C₂₂H₂₈N₂O₂ (MW=352.47): positive 353 (M+H).

### Part C: [1-(1-{[2-Benzyloxy-1-(1,2,3,4-tetrahydro-naphthalen-1-ylcarbamoyl)-propyl]-methyl-carbamoyl}-2-methyl-propylcarbamoyl)-ethyl]-methyl-carbamic acid tert-butyl ester

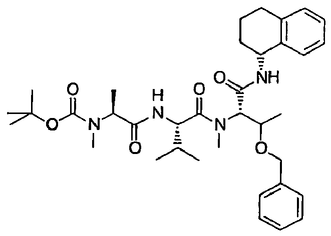

A solution of 2-[2-(*tert*-butoxycarbonyl-methyl-amino)propionylamino]-3-methyl-butyric acid (507 mg, 1.68 mmol) and 3-benzyloxy-2-metllylamino-*N*-(1,2,3,4-tetrahydro-naphthalen-1-yl)-butyramide; trifluoroacetic acid salt (623 mg, 1.34 mmol) in tetrahydrofuran (THF) (40 mL) was added 1-ethyl-3-(3-dimethylaminopropyl)carbodiimide hydrochloride (EDCI) (740 mg, 3.86-mmol) and 1-hydroxybenzotriazole monohydrate (HOBt) (358 mg, 2.64 mmol), followed by *N*-methyl-2-pyrrolidinone (1.5 mL) and 4-methyl morpholine (2 mL). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with ethyl acetate/hexane (0-100%) to give the title compound (334.5 mg, 39%) as a colorless oil. TLC (50% ethyl acetate/hexane) R*_{f}*= 0.18; MS (ES) for C₃₆H₅₂N₄O₆ (MW=636.82): positive 637 (M+H), negative 635 (M-H).

### Part D: N-[2-Benzyloxy-1-(1,2,3,4-tetrahydro-naphthalen-1-ylcarbamoyl)-propyl]-3,N-dimethyl-2-(2-methylamino-propionylamino)-butyramide; trifluoroacetic acid salt

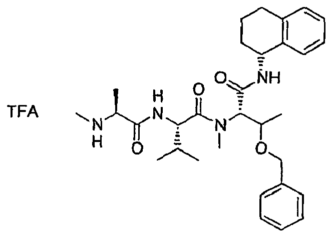

The [1-(1-{[2-benzyloxy-1-(1,2,3,4-tetrahydro-naphthalen-1-ylcarbamoyl)-propyl]-methyl-carbamoyl}-2-methyl-propylcarbamoyl)-ethyl]-methyl-carbamic acid *tert-butyl* ester (277 mg, 0.435 mmol) was dissolved in dichloromethane (DCM) (10 mL). Trifluoroacetic acid (TFA) (5 mL) was added. After stirring at room temperature for 3 hours, the solvent was evaporated. The residual material was purified via HPLC (reverse phase; H₂O with 0.1% TFA, acetonitrile), the appropriate fractions were collected and solvents removed to give the title compound as a white solid (21.5 mg). Analytical HPLC Rₜ- 12.68 min); MS (ES) for C₃₁H₄₄N₄O₄ (MW=536.71): positive 537 (M+H), negative 535 (M-H).

### Example 39

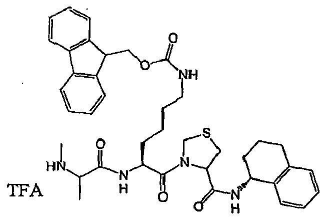

### {5-(2-Methylamino-propionylamino)-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester Trifluoroacetic acid salt

### Part A: 4-(1,2,3,4-Tetrahydro-naphthalen-1-ylcarbamoyl)-thiazolidine-3-carboxylic acid tert-butyl ester

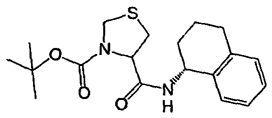

Thiazolidine-3,4-dicarboxylic acid 3-tert-butyl ester (6.63g, 28.42mmol) was stirred as a slurry with 1,2,3,4-Tetrahydro-naphthalen-1-ylamine (5.021g, 34.10mmol, 1.2eq.) in ethyl acetate (113.7mL, 0.25M). To this was added N-methylmorpholine (NMM, 31.2mL, 284.2mmol, 10eq.) followed by propane phosponic acid anhydride (T3P, 50% w/w soln in ethyl acetate) (54.25g, 85.26mmol, 3eq.) until a clear homogeneous mixture was achieved (20min). The mixture was then diluted to 1L with ethyl acetate. The reaction was washed with 200mL 1M HCl followed by 100mL washes with 1M HCl and brine. The ethyl acetate was dried (Na₂SO₄ and solvent was removed to yield the title compound.

### Part B: Thiazolidine-4-carboxylic acid (1,2,3,4-tetrahydro-naphthalen-1-yl)-amide HCl Salt

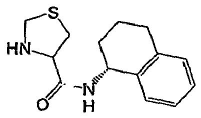

4-(1,2,3,4-Tetrahydro-naphthalen-1-ylcarbamoyl)-thiazolidine-3-carboxylic acid tert-butyl ester was dissolved in ethyl acetate (75mL). To this was added 4M HCl in dioxane (25mL, 100.1mmol, 4.4eq.). The mixture was stirred at room temperature for 18 hours. The reaction was diluted in half by the addition of diethyl ether the resulting precipitate was filtered, rinsed with ether and dried under vacuum to yield the title compound.

### Part C: {5-tert-Butoxycarbonylamino-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester

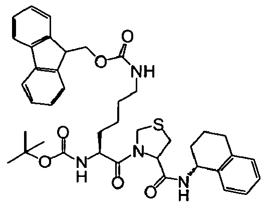

2-tert-Butoxycarbonylamino-6-(9H-fluoren-9-ylmethoxycarbonylamino)-hexanoic acid (470mg, 1.0mmol) and Thiazolidine-4-carboxylic acid (1,2,3,4-tetrahydro-naphthalen-1-yl)-amide HCl Salt (600mg, 2.01mmol, 1.3eq.) was stirred as a slurry with in ethyl acetate (4.01mL, 0.25M). To this was added N-methylmorpholine (NMM, 1.10mL, 10.0mmol, 10eq.) followed by propane phosponic acid anhydride (T3P, 50% w/w soln in ethyl acetate) (1.915g, 3.0mmol, 3eq.) until a clear homogeneous mixture was achieved (20min). The mixture was pre-absorbed on 5g silica and chromatographed on silica eluting with 33% hexanes in ethyl acetate to yield the title compound.. TLC (33% hexanes in ethyl acetate) R*_{f}*= 0.3.

### Part D: {5-Amino-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester HCl salt

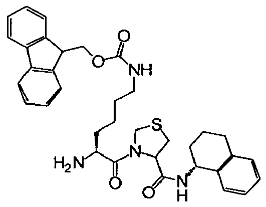

{5-tert-Butoxycarbonylamino-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester was dissolved in ethyl acetate (6mL). To this was added 4M HCl in dioxane (2mL, 8mmol, 8.7eq.). The mixture was stirred at room temperature for 18 hours. The reaction was diluted in half by the addition of diethyl ether the resulting precipitate was filtered, rinsed with ether and dried under vacuum to yield the title compound.

### Part E: {5-[2-(tert-Butoxycarbonyl-methyl-amino)-propionylamino]-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester

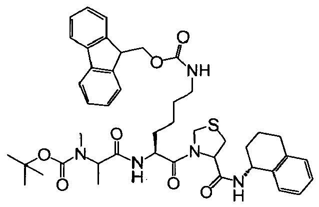

2-(tert-Butoxycarbonyl-methyl-amino)-propionic acid (142mg, 0.7mmol) was combined with {5-Amino-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester HCl salt (.499mg, 0.769mmol, 1.1eq.) and slurried with ethyl acetate (2.8mL, 0.25M). To this was added N-methylmorpholine (NMM, 0.77mL, 6.99mmol, 10eq.) followed by propane phosponic acid anhydride (T3P, 50% w/w soln in ethyl acetate) (1.33g, 2.09mmol, 3eq.) until a clear homogeneous mixture was achieved (15min). The mixture was then diluted to 100 mL with ethyl acetate. The reaction was washed with 20mL 1M HCl followed by 10mL washes with 1M HCl and brine. The organic layer was dried (Na₂SO₄) and concentrated under reduced pressure. This material was purified by flash column chromatography on silica eluting with neat ethyl acetate to yield the title compound. TLC (ethyl acetate) R*_{f}* = 0.4.

### Part F: {5-(2-Methylamino-propionylamino)-6-oxo-6-[4-(1,2,3,4tetrahydronaphthalen-1-ylcarbamoyl)-thiazolidin-3-yl]-hexyl}-carbamicacid 9H-fluoren-9-ylmethyl ester Trifluoroacetic acid salt

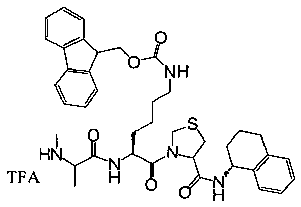

{5-[2-(tert-Butoxycarbonyl-methyl-amino)-propionylamino]-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester (50mg, 0.06mmol) was dissolved in methylene chloride (1mL, 0.06M). TFA (1mL, 0.06M) was added and the mixture stirred 3 hours. Heptanes was added and solvent removed under reduced pressure. The crude material was purified using reverse phase HPLC, using a C18 Vydac column and eluting with a gradient of 5% to 50% acetonitrile in water containing 0.1% TFA over 30 minutes. MS(ES) for C₃₉H₄₇N₅O₅S (MW=697.89): positive 698.29 (M+H), Analytical HPLC Rₜ=13.98 main.

### Example 40

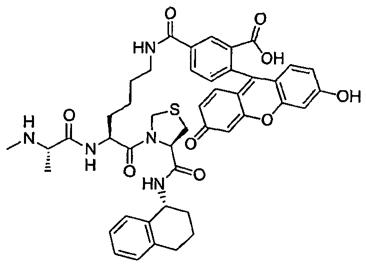

### 6-(6-Hydroxy-3-oxo-3H-xanthen-9-yl)-N-{5-(2-methylamino-propionylamino)-6-oxo-6-[4-(1,2,3,4-tetrahydro-naphthalen-1-ylcarbamoyl}-thiazolidin-3-yl]-hexyl}-isophthalamic acid

### Part A: (1-{5-Amino-l-[4-(1,2,3,4-tetrahydro-naphthalen-1-ylcarbamoyl)-thiazolidine-3-carbonyl]-pentylcarbamoyl}-ethyl)-methyl-carbamic acid tert-butyl ester

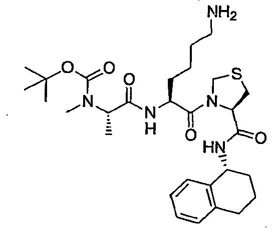

To a solution of {5-[2-(tert-Butoxycarbonyl-methyl-amino)-propionylamino]-6-oxo-6-[4-(1,2,3,4-tetrahydro-naphthalen-1-ylcarbamoyl)-thiazolidin-3-yl]-hexyl}-carbamic acid 9H-fluoren-9-ylmethyl ester (0.250 g, 0.31 mmol) in DMF (3 mL) was added diethylamine (35.2 µL, 0.34 mmol). The solution was stirred for 3 hour, followed by concentration of the reaction mixture under reduced pressure, and azeotroping with methylene chloride to yield the title compound (0.178 g, >99 %) which was used in the next step without further purification.

### Part B: N-{5-[2-(tert-Butoxycarbonyl-methyl-amino)-propionylamino]-6-oxo-6-[4-(1,2,3,4-tetrahydro-naphthalen-1-ylcarbamoyl)-thiazolidin-3-yl]-hexyl}-6-(6-hydroxy-3-oxo-3H-xanthen-9-yl)-isophthalamic acid

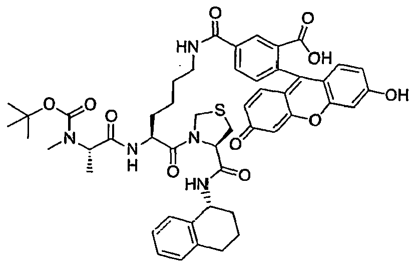

To a solution of (1-{5-Amino-1-[4-(1,2,3,4-tetrahydro-naphthalen-1-ylcarbamoyl)-thiazolidine-3-carbonyl]-pentylcarbamoyl}-ethyl)-methyl-carbamic acid tert-butyl ester (0.178 g, 0.31 mmol) in methylene chloride (3 mL) was added 4-(6-Hydroxy-3-oxo-3H-xanthen-9-yl)-isophthalic acid 3-(2,5-dioxo-pyrrolidin-1-yl) ester (0.147 g, 0.31 mmol). The resulting mixture was stirred for 48 hours and filtered to remove solids. The filtrate was concentrated under reduced pressure, taken up in ethyl acetate (20 mL), washed with 5% KHSO₄ (3x10 mL) and brine (10 mL); dried over Na₂SO₄, and concentrated under reduced pressure. The crude material was purified via flash column chromatography (ethyl acetate) to yield the title compound (47 mg, 16.2 %).

### Part C: 6-(6-Hydroxy-3-oxo-3H-xanthen-9-yl)-N-{5-(2-methylamino-proplonylamino)-6-oxo-6-[4-(1,2,3,4-tetrahydro-naphthalen-1-ylcarbamoyl)-thiazolidin-3-yl]-hexyl}-isophthalamic acid

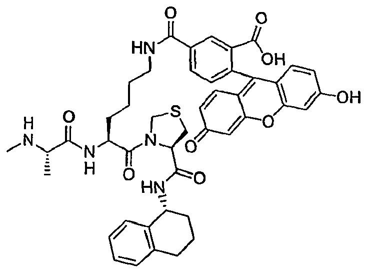

To a solution of N-{5-[2-(tert-Butoxycarbonyl-methyl-amino)-propionylamino]-6-oxo-6-[4-(1,2,3,4-tetrahydro-naphthalen-1-ylcarbamoyl)-thiazolidin-3-yl]-hexyl}-6-(6-hydroxy-3-oxo-3H-xanthen-9-yl)-isophthalamic acid (47 mg, 0.05 mmol) in methylene chloride (5 mL) was added TFA (1.5 mL). The resulting solution was stirred for 5 hours, then the reaction mixture was concentrated under reduced pressure. The crude material was purified using reverse phase HPLC, using a C 18 Vydac column and eluting with a gradient of 5% to 50% acetonitrile in water over 30 minutes to yield the title compound (10 mg, 24%). Analytical HPLC Rₜ-10.125 min. MS(ES) for C₄₅H₄₇N₅O₉S (MW=833.31): positive 834.22 (M+H), negative 831.61 (M-H).

### Example 41

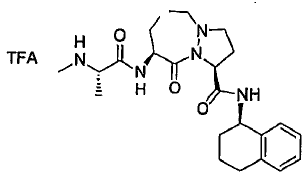

### 8-(2-Methylamino-propionylamino)-9-oxo-hexahydro-pyrazolo[1,2-a][1,2)diazepine-1-carboxylic acid (1,2,3,4-tetrahydro-naphthalen-1-yl)-amide

### Part A: 1-(10,10-Dimethyl-3,3-dioxo-3λ⁶-thia-4-aza-tricyclo[5.2.1.0^{1,5}]dec-4-yl)-propenone

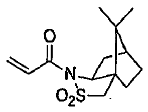

Sodium hydride dispersion in mineral oil (1.22 g, 27.85 mmol, 1.5eq) was suspended in toluene (20 mL) at 0°C. To this solution was added (*R*)-camphor sultam chiral auxiliary (4 g, 18.56 mmol)in toluene (5 mL) and the reaction mixture was stirred at 0 °C for 30minutes, allowing to warm to room temperature. Acryloyl chloride (0.44 mL, 5.57 mmol, 1.2eq.) was taken up in toluene (2 mL) and added slowly to the reaction mixture. The resulting mixture was stirred at room temperature for 1.5 hours, quenched with ice water (14 mL), then extracted with ethyl acetate, dried (Na₂SO₄), and concentrated to a residue that was purified by flash column chromatography on silica gel using 30% ethyl acetate/hexane to give a solid that recrystallizes from methanol to yield the title compound (0.4 g, 40%). TLC (30% ethyl acetatelhexane) R_{f} = 0.55.

### Part B: (3,4-Dihydro-2H-pyrazol-3-yl)-(10,10-dimethyl-3,3-dioxo-3λ⁶-thia-4-aza-tricyclo[5.2.1.0^{1,5}] dec-4-yl)-methanone

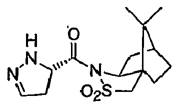

A solution of **1-(10,10-Dimethyl-3,3-dioxo-3λ⁶-6 -thia-4-aza-tricyclo[5.2.1.0^{1,5}]dec-4-yl)-propenone** (370 mg, 1.37 mmol) in a 1:1 mixture of toluene/hexanes (27 mL) was treated with trimethylsilyldiazomethane (2M in hexanes, 1 mL, 2.01 mmol, 1.5eq.). The resulting mixture stirred at room temperature until complete by TLC (2 days), then concentrated under reduced pressure. The crude intermediate was dissolved in methylene chloride (0.1M solution 14 mL) and was then treated with trifluoroacetic acid (0.12 mL, 1.51 mmol, 1.1 eq.). The resulting mixture was stirred for at room temperature for 30 minutes, then quenched with aqueous saturated sodium bicarbonate solution. The aqueous layer extracted with methylene chloride and the combined organic layers were dried (Na₂SO₄) and concentrated under reduced pressure. The crude residue was purified by flash column chromatography on silica gel eluting with (2:1 hexane/ethyl acetate) to give the title compound (220mg, 52%). MS (ES for C₁₄H₂₁N₃O₃S (MW=311.40): positive 312(M+H).

### Part C : 3-(10,10-Dimethyl-3,3-dioxo-3λ⁶-thia-4-aza-tricyclo[5.2.1.0^{1,5}]decane-4-carbonyl)-pyrazolidine-1-carboxylic acid benzyl ester

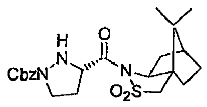

To a solution of **(3,4-Dihydro-2*H* pyrazol-3-yl)-(10,10-dimethyl-3,3-dioxo-3λ⁶-thia-4-aza-tricyclo[5.2.1.0^{1,5}]dec-4-yl)-methanone** (1.06 g, 3.4 mol) in acetic acid (11 mL) was added sodium cyanoborohydride (0.535 g, 8.5 mmol, 2.5eq.) The reaction mixture was stirred at room temperature for 1 hour, then diluted with ethyl acetate and quenched by addition of saturated potassium carbonate aqueous solution. The organic layers were washed with brine, dried (Na₂SO₄), and concentrated. The resulting crude residue was taken up in methylene chloride (12 mL) and treated with benzyl chloroformate (0.54 mL, 3.74 mmol, 1.1eq.) and triethylamine (0.5 mL 3.57 mmol, 1.05eq.) and the resulting solution was stirred for 1h. The reaction mixture was quenched with water and extracted with methylene chloride (3x). The organic layers were washed with brine, dried (Na₂SO₄), and concentrated. The crude product was purified by flash column chromatography on silica gel eluting with 25-35% ethyl acetate /hexane to afford the title compound as a colorless oil (500 mg, 33 %). MS (ES) for C₂₂H₂₉N₃O₅S (MW=447.55): positive 448(M+H).

### Part D: Pyrazolidine-1,3-dicarboxylic acid 1-benzyl ester 3-methyl ester

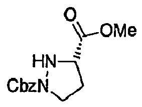

A solution of **3-(10,10-Dimethyl-3,3-dioxo-3λ⁶-thia-4-aza-tricycio [5.2.1.0^{1,5}]decane-4-carbonyl)-pyrazolidine-1-carboxylic acid benzyl ester** (500 mg, 1.12 mmol)) in 1:1 methylene chloride/methanol (15 mL) and the solution was cooled to 0°C. To this was added magnesium methoxide, ( Mg(OCH₃)₂, 6-10% in methanol) (2.73 mL, 2.01 mmol) and the mixture was stirred at 0°C for 1 hour. The reaction mixture was diluted with methylene chloride (25 mL) and quenched with saturated ammonium chloride solution (25 mL), then extracted with methylene chloride (5x20 mL), dried (Na₂SO₄), and concentrated under reduced pressure. The crude product was purified by flash column chromatography on silica gel using 50% ethyl acetate /hexane to give the title compound (130 mg, 66%). TLC (50% ethyl acetate/hexane) R_{f}= 0.2. MS (ES) for C₁₃H₁₆N₂O₄ (MW=264.28): positive 265(M+H).

### Part E: 2-(1,3-Dioxo-1,3-dihydro-isoindol-2-yl)-pent-4-enoic acid

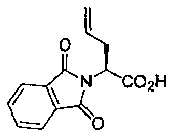

To a solution of Boc-allyl glycine (3 g, 13.95 mmol) and sodium carbonate (1.48 g, 13.95 mmol) in distilled water (21 mL) at 0°C was added N-ethoxycarbonylphthalimide (3.07 g, 13.95 mmol). The reaction mixture was stirred vigorously for 10 minutes, then filtered and the filtrate was acidified with 6N HCl to pH-3. This was then extracted with ethyl acetate several times, and the combined organic layers were dried (Na₂SO₄), and concentrated under reduced pressure. The crude product was purified by flash column chromatography on silica gel eluting with ethyl acetate to afford the title compound (1.2 g, 35%). MS (ES) for C₁₃H₁₁NO₄ (MW=245.23): positive 246 (M+H).

### Part F: 2-[2-(1,3-Dioxo-1,3-dihydro-isoindol-2-yl)-pent-4-enoyl]-pyrazolidine-1,3-dicarboxylic acid 1-benzyl ester 3-methyl ester

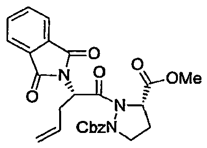

To a solution of 2-(1,3-Dioxo-1,3-dihydro-isoindol-2-yl)-pent-4-enoic acid (430 mg, 1.66 mmol 2eq.) in anhydrous ethyl ether (4 mL) at 0°C was added phosphorous pentachloride (PCl₅, 380.25 mg, 1.83 mmol, 2.2eq.). The resulting mixture was stirred, warming to room temperature over 1.5 hours then concentrated under reduced pressur (azeotroping with toluene). The crude acid chloride was taken up in toluene (3 mL), and added to a mixture of Pyrazolidine-1,3-dicarboxylic acid 1-benzyl ester 3-methyl ester (220 mg, 0.83 mmol, 1eq.) and in toluene (3 mL) and 10% aqueous solution of sodium bicarbonate (5 mL). The resulting mixture was stirred at room temperature for 2 hours at which time the reaction was complete by TLC. It was then diluted with ethyl acetate, washed with saturated sodium bicarbonate solution, and extracted several times with ethyl acetate. The combined organic layers were washed with brine, dried (Na₂SO₄), and concentrated. The crude product was purified by flash column chromatography on silica gel eluting with 50% ethyl acetate/hexane to afford the title compound TLC (50% ethyl acetate/hexane) R_{f}= 0.4. (400 mg, 98%). MS (ES) for C₂₆H₂₅N₃O₇ (MW=491.49): positive: 492(M+H).

### Part G: 2-[2-(1,3-Dioxo-1,3-dihydro-isoindol-2-yl)-5-hydroxy-pentanoyl]-pyrazolidine-1,3-dicarbozylic acid 1-benzyl ester 3-methyl ester

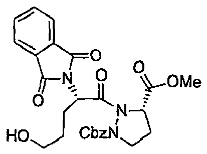

Borane-methyl sulfide complex (0.199 mL, 1.99 mmol, 2.5eq.) was dissolved in tetrahydrofuran (2.8 mL) and was cooled to 0°C and treated with 2-methyl-2-butene (0.43 mL, 3.95mmol, 5 eq.) and the resulting solution was stirred for 1 hour at 0°C. 2-[2-(1,3-Dioxo-1,3-dihydro-isoindol-2-yl)-pent-4-enoyl]-pyrazolidine-1,3-dicarboxylic acid 1-benzyl ester 3-methyl ester (390 mg, 0.79 mmol) in tetrahydrofuran (3 mL) was added to the reaction mixture and the resulting mixture was stirred at room temperature for 4 hours. The reaction mixture was diluted by addition of tetrahydrofuran (10 mL) and treated with saturated sodium acetate solution (4 mL) followed by slow addition of the aqeous hydrogen peroxide (30 wt.%, 4 mL). The resulting mixture was stirred for 8 additional hours and diluted with ethyl ether. The aqueous phase was extracted with ethyl ether (3x10 mL)), and the combined organic layers were washed with brine, dried (Na₂SO₄) and concentrated under reduced pressure. The crude product was purified by flash column chromatography on silica gel eluting with 50% ethyl acetate/hexane to afford the title compound (230 mg, 57%). TLC (50% ethyl acetate/hexane) R_{f}= 0.15. MS (ES) for C₂₆H₂₇N₃O₈ (MW=509.51): positive: 510(M+H).

### Part H: 2-[2-(1,3-Dioxo-1,3-dihydro-isoindol-2-yl)-5-oxo-pentanoyl]-pyrazolidine-1,3-dicarboxylic acid 1-benzyl ester 3-methyl ester

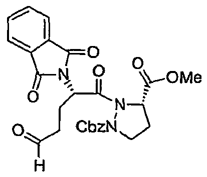

To a solution of **2-[2-(1,3-Dioxo-1,3-dihydro-isoindol-2-yl)-5-hydroxy-pentanoyl]-pyrazolidine-1,3-dicarboxylic acid 1-benzyl ester 3-methyl ester** (230 mg, 0.45 mmol) in methylene chloride (6 mL) was added Dess-Martin reagent (248 mg, 0.59 mmol, 1.3 eq.). The reaction mixture was stirred at room temperature for 3 hours, then quenched by addition of saturated sodium bicarbonate solution and saturated sodium thiosulfate solutional. The methylene chloride layer was dried (Na₂SO₄), and concentrated under reduced pressure. The crude product was purified by flash column chromatography on silica gel eluting with 50% ethyl acetate/hexane to afford the title compound (143 mg, 63%). TLC (50% ethyl acetate/hexane) R_{f}= 0.25.

### Part I: 8 8-(1,3-Dioxo-1,3-dihydro-isoindol-2-yl)-9-oxo-hexahydro-pyrazolo[1,2-a][1,2]diazepine-1-carboxylic acid methyl ester

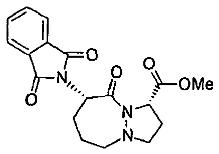

To a solution of Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester (143 mg, 0.28 mmol) in ethyl acetate (25mL) was added Pd/C ( 10%, 143 mg). The reaction mixture was stirred under hydrogen atmosphere overnight, filtrated and concentrated to afford a crude product which was used for the next step without further purification. MS (ES) for C₁₈H₁₉N₃O₅ (MW=357.36): positive: 358(M+H).

### Part J: 8-Amino-9-oxo-hexahydro-pyrazolo[1,2-a][1,2]diazepine-1-carboxylic acid methyl ester

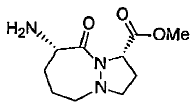

To a solution of Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl]-carbamic acid tert-butyl ester (100 mg, 0.28 mmol, theoretical yield from previous step) in MeOH (3mL) was added hydrate hydrazine (34.26w, 0.7 mmol). The reaction mixture was stirred at room temperature overnight, concentrated, and was purified by flash column chromatography on silica gel eluting with 5-15 % MeOH/DCM to afford the title compound (45 mg, 68%). TLC (15% MeOH/DCM) R_{f}= 0.37. MS (ES) for C₁₀H₁₇N₃O₃ (MW=227.26): positive 228(M+H).

### Part K: 8-[2-(tert-Butoxycarbonyl-methyl-amino)-propionylamino]-9-oxo-hexahydro-pyrazolo[1,2-a][1,2]diazepine-1-carboxylic acid methyl ester

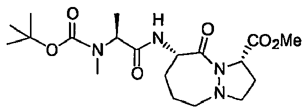

To a solution of Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester (20.45mg, 0.09) in DMF (2mL) was added HOBt (24.3 mg, 0.18 mmol), EDAC (34.5 mg, 0.18 mmol), Boc-Me-AlaOH (36.6 mg, 0.18 mmol), (*i*Pr)₂EtN. The reaction mixture was stirred at room temperature overnight, diluted with EtOAc (10 mL), washed with sat. NaHCO₃ and brine. The organic layer was dried(Na₂SO₄) and concentrated to residue which was purified by flash column chromatography on silica gel eluting with 40 % EtOAc/Hexanes to afford the title compound (30 mg, 80%). TLC (50% EtOAc/Hexanes) R_{f}= 0.12. MS (ES) for C₁₉H₃₂N₄O₆ (MW=412.48): positive 413 (M+H).

### Part L: 8-[2-(tert-Bntoxycarbonyl-methyl-amino)-propionylamino]-9-oxo-hexahydro-pyrazolo[1,2-a][1,2]diazepine-1-carboxylic acid

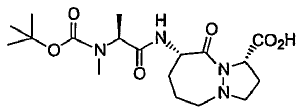

To a solution of methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester (73mg, 0.19 mmol) in THF (1mL) was added 1 M LiOH in H₂O (0.19mL). The reaction mixture was stirred at room temperature for 1 hour, diluted with H₂O, lyophilized, and the resulted crude product was used directly for the next step.

### Part M: Methyl-{1-[5-oxo-3-(1,2,3,4-tetrahydro-naphthalen-1-ylcarbamoyl)-hezahydro-pyrazolo[1,2-a][1,2]diazepin-6-ylcarbamoyl]-ethyl}-carbamic acid tert-butyl ester

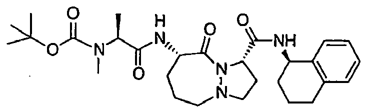

The above crude product was dissolved in DMF ( 1 mL). To this solution was added HOBt (34 mg, 0.22 mmol), EDAC (53.24 mg, 0.278 mmol), 1,2,3,4-tetrahydronaphthylamine ( 32.68 mg, 0.22 mmol), and NMM ( 31µL, 0.28 mmol). The reaction mixture was stirred at room temperature overnighrt, diluted with EtOAc, washed with sat.NaHCO₃, dried (NaSO₄) and concentrated to residuewhich was purified by flash chromatography on silica gel eluting with 10% MeOH/DCM to yield the title product ( 26 mg, 27%) and MS (ES) for C₂₈H₄₁N₅O₅ (MW=527.66): positive 428(M+H).

### Part N: 8-(2-Methylamino-propionylamino)-9-oxo-hexahydro-pyrazolo[1,2-a][1,2]diazepine-1-carboxylic acid (1,2,3,4-tetrahydro-naphthalen-1-yl)-amide

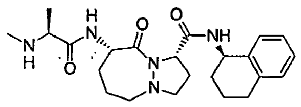

To a solution of Methyl-{1-[5-oxo-3-(1,2,3,4-tetrahydro-naphthalen-1-ylcarbamoyl)-octahydro-pyrrolo[1,2-a]azepin-6-ylcarbamoyl]-ethyl}-carbamic acid tert-butyl ester (26 mg, 0.05 mmol) in DCM (3mL) was added TFA( 3mL). The reaction mixture was stirred at room temperature for 1.7 hours, concentrated, and purified with HPLC to afford the desired product (18 mg, 66%). Analytical HPLC: RT 7.5 min. MS (ES) for C₂₃H₃₃N₅O₃ (MW=427.54): positive 428(M+H).

### Example 42

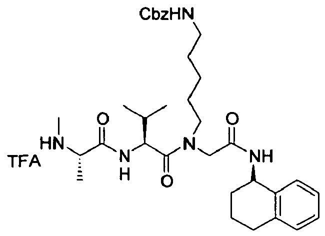

### (5-{3-Methyl-2-(2-methylamino-propionylamino)-butyryl]-[(1,2,3,4-tetrahydro-naphthalen-1-ylcarbamoyl)-methyl]-amino)-carbamic acid benzyl ester trifluoro acetic acid salt

### Part A: (5-{[1,2,3,4-tetrahydro-naphthalen-1-ylcarbamoyl)-methyl]-amino}-pentyl)-carbamic acid benzyl ester

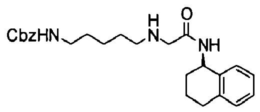

A solution of (5-Oxo-pentyl)-carbamic acid benzyl ester (500mg, 4.2 mmol)and 2-amino-N-(1,2,3,4-tetrahydro-naphthalen-1-yl)-acetamide (1.00g, 4.16 mmol) were dissolved in DCM (20 mL) followed by addition of NaCNBH₃(264mg, 4.2 mmol). The mixture was stirred at rt overnight and quenched with sat.NaHCO₃, extracted withDCM(100 mL). The organic phase was dried, concentrated,. The residue was purified by chromatography with MeOH/DCM(5-10%) to give the title compound (1g, 56%).

### Part B: (5-{(2-tert-Butoxycarbonylamino-3-methyl-butyryl)-[(1,2,3,4-tetrahydro-naphthalen-1-1ylcarbamoyl)-methyl]-aminol-pentyl)-carbamic acid benzyl ester

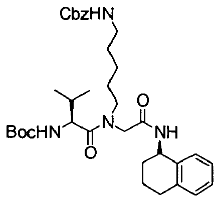

**(5-{[1,2,3,4-tetrahydro-naphthalen-1-ylcarbamoyl)-methyl]-amino}-pentyl)-carbamic acid benzyl ester(974mg, 2.3 mmol) and L-2-*ter*ₜ-Butoxycarbonylamino-3-methyl-butyric acid**(911 mg, 4.2 mmol) were dissolved in DCM/NMP (20 ml, 1:1) followed by addition of HATU(1.92g, 5.04 mmol) and Et₃N(1.4 mL, 10.08 mmol). The reaction mixture was stirred at rt overnight and then treated with water (20 mL), then partitioned between ethyl acetate/water. The organic phase was washed witch 1N HCl (2 x 25 mL), saturated sodium bicarbonate solution (50 mL) and brine solution (50 mL), dried over Na₂SO₄ and evaporated to dryness. The residue was purified by medium pressure column chromatography on silica gel eluting with MeOH/DCM (7%) to give the title compound (1.26g, 65%). MS (ES) for C₃₅H₅₀N₄O₆ (MW=622.79): positive 623 (M+H).

### Part C: (5-{3-Methyl-2-(2-methylamino-propionylamino)-butyryl]-[(1,2,3,4-tetrahydro-naphthalen-1-ylcarbamoyl)-methyl]-amino}-carbamic acid benzyl ester trifluoro acetic acid salt.

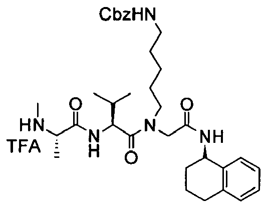

**(5-{(2-tert-Butoxycarbonylamino-3-methyl-butyryl)-[(1,2,3,4-tetrahydro-naphthalen-1-1ylcarbamoyl)-methyl]-amino}-pentyl)-carbamic acid benzyl ester** (1.7g, 2.73 mmol) was dissolved in HCl in EtOAc(4N, 15 mL). The mixture was stirred at rt for 1 h and then evaporated to dryness. The residue was dissolved in DCM/NMP( 50 mL, 1:1) followed by addition of L-2-(ter-Butoxycarbonyl-methyl-amino-propionic acid (555mg, 2.73 mmoL), HATU( 1.25g, 3.28 mmol), and Et₃N (0.91 mL, 6.55 mmol). This mixture was stirred at room temperature for 18 hours. The reaction mixture was treated with water (20 mL), then partitioned between ethyl acetate/water. The organic phase was washed with 1N HCl (2 x 25 mL), saturated sodium bicarbonate solution (50 mL) and brine solution (50 mL), dried over Na₂SO₄ and evaporated to dryness. The residue was dissolved in TFA/DCM( 30%, 15 mL) and stirred at rt for 1h. The reaction mixture was concentrated to dryness and purified by HPLC to give the title compound (60, 3%) MS (ES) for C₃₆H₅₀F₃N₄O₆ (MW=636.82): positive 637 (M+H).

### Example 43

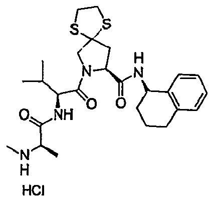

### 7-[3-Methyl)-2-(2-methylamino-propionylamino)-butyryl]-1,4-dithia-7-aza-spiro[4,4]nonane-8-carboxylic acid(1,2,3,4-tetrahydro-naphthalen-1-yl)amide

### Part A: 4-hydroxy-2-91,2,3,4-tetrahydro-naphthalen-1-ylcarbamoyl)-pyrrolidine-1-carboxylic acid benzyl ester

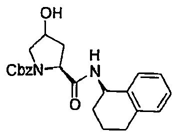

4-Hydroxy-pyrrolidine-1,2-dicarboxylic acid 1-benzyl ester

A solution of 4-hydroxy pyrrolidine-1,2-dicarboxylic acid 1-benzyl ester(2.70g, 7.81 mmol) and 1,2,3,4-tetrahydro-naphthalen-1-yl -amine ( 1.28g, 8.59 mmol) in DMF was added 1-ethyl-3-(3-dimethylaminopropyl)carbodiimide hydrochloride (EDCI) (1.95g ,10.17 mmol)) and 1-hydroxybenzotriazole monohydrate (HOBt) (1.37g, 10.15 mmol,), followed by triethylamine (2.00 mL, 11.72 mmol). This mixture was stirred at room temperature for 18 hours. The reaction mixture was diluted with EtOAc (150 mL) with water (100 mL) and washed with 1N HCl (100 mL), saturated sodium bicarbonate solution (100 mL) and brine solution (100 mL), dried over Na₂SO₄ and evaporated to dryness. The residue was trituated with ether to give the title compound (2.7g,88%) . MS (ES) for C₂₃H₂₆N₂O₄ (MW=394.46): positive 395 (M+H).

### Part B: {1-[4-Hydroxy-2-(1,2,3,4)tetrahydro-naphthalen-1-ylcarbamoyl)-pyrrolidine-1-carbonyl]-2-methylpropyl}-carbamic acid tert-butyl ester

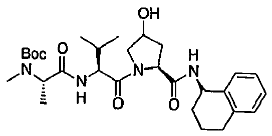

4-Hydroxy-2-(1,2,3,4-tetrahydro-naphthalen-1-yl-carbamoyl) pyrrolidine-1-carboxylic benzylester(1.31g, 3.31 mmol) was dissolved in ethylacetate (100 mL) followed by addition of Pd/C(10%). The mixture was stirred overnight and then filtree through a pad of celite. The crude product and 2-[2-(tert-Butoxycarcarbonyl-methyl-amino)-propionylamino]-3-methyl-butyzic acid(1.00g, 3.31 mmol) were dissolved in DMF followed by addition of 1-ethyl-3-(3-dimethylaminopropyl)carbodiimide hydrochloride (EDCI) (0.82g, 4.3 mmol)) and 1-hydroxybenzotriazole monohydrate (HOBt) (0.58g, 3.32 mmol), 10.15 mmol,), and diisopropylethyl amine (2.00 mL, 4.95 mmol). This mixture was stirred at room temperature for 18 hours. The reaction mixture was diluted with EtOAc (150 mL) with water (100 mL) and washed with 1N HCl (100 mL), saturated sodium bicarbonate solution (100 mL) and brine solution (100 mL), dried over Na₂SO₄ and evaporated to dryness. The residue was trituated with ether to give the title compound (1.4g, 80%). MS (ES) for C₂₉H₄₄N₄O₆ (MW=544.68): positive 545 (M+H).

### Part C: Methyl-(1-{2-methyl-1-[4-oxo-2-(1,2,3,4-tetrahydro-naphthalen-1-ylcarbamoyl)-pyrrolidine-1-carbonyl]-propylcarbamoyl]-ethyl)-carbamic acid tert-butyl ester

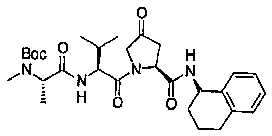

A solution of **{1-[4-Hydroxy-2-(1,2,3,4)tetrahydro-naphthalen-1-ylcarbamoyl)-pyrrolidine-1-carbonyl]-2-methylpropyl}-carbamic acid tert-butyl ester( 1.15g, 2.1 mmol) in DCM** was added Dess-Martin reagent. The mixture was stirred at rt for 2h ,quenched with saturated aq. NaHCO3, and extracted with EtOAc. The organic phase was dried over Na₂SO₄ and evaporated to dryness. The residue was purified by medium pressure column chromatography on silica gel eluting with MeOH/DCM (7%) to give the title compound (1g, 88%) MS (ES) for C₂₉H₄₂N₄O₆ (MW=542.67): positive 543 (M+H).

### Part D: 7-[3-methyl-2-(2-methylamino)-butyryl]-1,4-dithia-7-aza-spiro[4,4]nonane-8-carboxylic acid(1,2,3,4-tetrahydro-naphthalen-1-yl)-amide

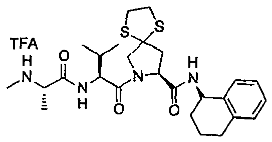

**Methyl-(1-{2-methyl-1-[4-oxo-2-(1,2,3,4-tetrahydro-naphthalen-1-ylcarbamoyl)-pyrrolidine-1-carbonyl]-propylcarbamoyl]-ethyl)-carbamic acid *tert-***butyl ester (1g, 1.85 mmol) was dissolved in DCM followed by addition of ethane-1,2-dithiol and trifluoro borane. After stirring at room temperature overnight,the reaction was diluted with EtOAc and the solvent was evaporated. The residual material was purified via HPLC (reverse phase; H₂O with 0.1 % TFA, acetonitrile), the appropriate fractions were collected and solvents removed to give the title compound as a white solid (21.5 mg). MS (ES) for C₂₆H₃₈N₄O₃S₂ (MW=518.74): positive 519 (M+H).

### Example 44

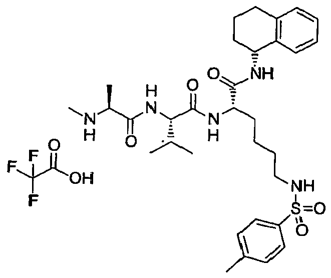

### 2-[3-Methyl-2-(2-methylamino-propionylamino)-butyrylamino]-6-(toluene-4-sulfonylamino)-hexanoic acid

### (1,2,3,4-tetrahydro-naphthalen-1-yl)-amide; compound with trifluoro-acetic acid

### Part A: [1-(1,2,3,4-Tetrahydro-naphthalen-1-ylcarbamoyl)-5-(toluene-4-sulfonylamino)-pentyl]-carbamic acid tert-butyl ester

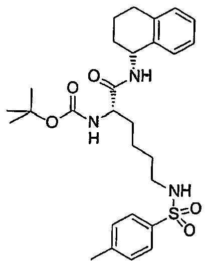

A solution 2-tert-Butoxycarbonylamino-6-(toluene-4-sulfonylamino)-hexanoic acid (1.462 g, 3.65 mmol) and (R)-1,2,3,4-tetrahydro-naphthalen-1-ylamine (645mg, 4.38 mmol) in Acetonitrile(MeCN) (14.6 mL) was added 1-ethyl-3-(3-dimethylaminopropyl)carbodiimide hydrochloride (EDCI) 1.049 g, 5.48 mmol) and 1-hydroxybenzotriazole monohydrate (HOBt) (.670 mg, 4.38 mmol), followed by diisopropylethylamine(0.3.12 mL, 18.25mmol). This mixture was stirred at room temperature for 18 hours. The reaction mixture was pumped down and re-suspended in 200mL ethyl acetate (EtOAC). The organic phase was washed with 1N HCl (2 x 20 mL), brine solution (20 mL), saturated sodium bicarbonate solution (2 x 20mL) and brine solution (20 mL), dried over Na₂SO₄ and evaporated to dryness. The residue was purified by medium pressure column chromatography on silica gel eluting with ethyl acetate/hexane (1/1) to give the title compound (1.605 g,83%) as a pink foam. TLC (50% ethyl acetate/hexane) R*_{f}*= 0.33; MS (ES) for C₂₇H₃₆N₂O₄ (MW=529.69): positive 530 (M+H).

### Part B: 2-Amino-6-(toluene-4-sulfonylamino)-hexanoic acid (1,2,3,4-tetrahydro-naphthalen-1-yl)-amide; hydrochloride

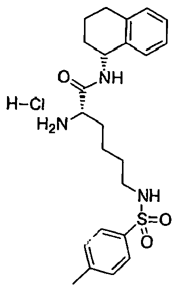

The [1-(1,2,3,4-Tetrahydro-naphthalen-1-ylcarbamoyl)-5-(toluene-4-sulfonylamino)-pentyl]-carbamic acid tert-butyl ester (270 mg, .497 mmol) was dissolved in dichloromethane (DCM) (3 mL). HCl saturated methanol (3 mL) was added. After stirring at room temperature for 10min, the solvent was evaporated to give the title compound as a white solid (225 mg, >95%). MS (ES) for C₂₃H₃₁N₃O₃S (MW=429.58): positive 430 (M+H).

### Part C: Methyl-(1-{2-methyl-l- [1-(1,2,3,4-tetrahydro-naphthalen-1-ylcarbamoyl)-5-(toluene-4-sulfonylamino)-pentylcarbamoyl]-propylcarbamoyl}-ethyl)-carbamic acid tert-butyl ester

A solution of 2-[2-(tert-Butoxycarbonyl-methyl-amino)-propionylamino]-3-methyl-butyric acid (.120 mg, .40 mmol) and 2-Amino-6-(toluene-4-sulfonylamino)-hexanoic acid (1,2,3,4-tetrahydro-naphthalen-1-yl)-amide; hydrochloride (222 mg, .48 mmol) in Acetonitrile (MeCN) (2 mL) was added 1-ethyl-3-(3-dimethylaminopropyl)carbodiimide hydrochloride (EDCI) (114 mg, .60 mmol) and 1-hydroxybenzotriazole monohydrate (HOBt) (73 mg, .48 mmol), followed by diisopropylethylamine(0.34 mL, 1.98mmol). This mixture was stirred at room temperature for 18 hours. The reaction mixture was pumped down and re-suspended in 200mL ethyl acetate (EtOAC). The organic phase was washed with 1N HCl (2 x 20 mL), brine solution (20 mL), saturated sodium bicarbonate solution (2 x 20mL) and brine solution (20 mL), dried over Na₂SO₄ and evaporated to dryness. The residue was purified by medium pressure column chromatography on silica gel eluting with ethyl acetate to give the title compound (218 mg, 84%) as a white solid. RP-HPLC Rₜ = 17.1min; MS (ES) for C₃₇H₅₅N₅O₇S (MW=713.93): positive 715 (M+H), negative 713 (M-H).

### Part D: 2-[3-Methyl-2-(2-methylamino-propionylamino)-butyrylamino]-6-(toluene-4-sulfonylamino)-hexanoic acid (1,2,3,4-tetrahydro-naphthalen-1-yl)-amide; compound with trifluoro-acetic acid

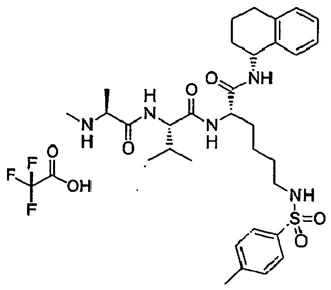

The Methyl-(1-{2-methyl-1-[1-(1,2,3,4-tetrahydro-naphthalen-1-ylcarbamoyl)-5-(toluene-4-sulfonylamino)-pentylcarbamoyl]-propylcarbamoyl}-ethyl)-carbamic acid tert-butyl ester (218 mg, 0.311 mmol) was dissolved in dichloromethane (DCM) (5 mL). Trifluoroacetic acid (TFA) (5 mL) was added. After stirring at room temperature for 1 hours, the solvent was evaporated. The residual material was purified via HPLC (reverse phase; H₂O with 0.1% TFA, acetonitrile), the appropriate fractions were collected and solvents removed to give the title compound as a white solid (182 mg, 82%). Analytical HPLC Rₜ- 11.92 min); MS (ES) for C₃₂H₄₇N₅O₅S (MW=613.33): positive 614 (M+H), negative 612 (M-H).

### Example 45

### Caspase-9 Rescue Assay

Relief of BIR3-mediated Caspase-9 inhibition by BIR3 inhibitors was assayed by measuring Caspase-9 reactivation using the fluorogenic substrate Ac-LEHD-7-amido-4-methylcoumarin. A truncated form of human Caspase-9 that lacks the N-terminal Caspase recruitment domain (ΔPro-Caspase-9 or p35/p12) was used in the assay. This form of the enzyme has the full catalytic activity and the same substrate specificity as the full-length protein in the Apaf-1/Cytochrome C/dATP/Caspase-9 reconstituted system. Rat or Chicken BIR3 was used in this assay. In a typical assay, the 150-µL reaction mixture contains 37.5 nM Caspase-9, 45 nM BIR3, 50 µM Ac-LEHD-amc and various concentration of tested compounds in the assay buffer (20 mM Hepes, 0.1 mM EDTA, 0.1% Chaps, 10% sucrose, and 15 mM dithiothreitol) at pH 6.5. This mixture was incubated for 40 min to allow equilibration at 25°C, and the assay progression was subsequently followed by monitoring the formation of fluorescent 7-amido-4-. methylcoumarin over time at 460 nm using an excitation wavelength of 360 nm. Under these experimental conditions, Caspase-9 activity is typically 70-80% inhibited in the control mixtures that contain no rescuer compound or BIR3 inhibitor. EC₅₀ values of tested BIR3 inhibitors or Caspase-9 rescuers were calculated from the percentage of Caspase-9 reactivation in the mixtures containing serially diluted concentrations of tested compound. Typically, 10-point curves were generated for such EC₅₀ determination with compound concentration ranging from 50 nm to 50 µm.

Average EC₅₀ of the compounds provided herein for relief of BIR3-mediated Caspase-9 inhibition are provided in Table 1.

### Example 46

### Fluorescence Polarization Assay for Measuring Ki of SMAC Mimetics to BIR3 from Rat XIAP

The binding affinity of compounds provided herein for rat BIR3 was measured by competitive displacement of a fluorescent probe using fluorescence polarization anisotropy. The BIR3 domain of rat XIAP (aa 241-355 of Genbank ID AF183429), expressed in E. coli as fusion protein with an N-terminal His-tag and thrombin cleavage site, purified to homogeneity, and reconstituted in 50 mM Tris pH 8.0 (HCl), 120 mM NaCl was used as receptor. The hexapeptide H-Ala-Val-Pro-Phe-Ala-Lys-OH covalently modified on the epsilon-amino group of the C-terminal lysine with 5-carboxyfluorescein was used as probe (probe 1). The binding constant (Kd) of probe 1 for rat BIR3 was measured as 96 nM. Serial dilutions of BIR3 antagonist at final concentrations typically between 100 µM and 1 nM were mixed in a half-area black 96-well microtiter plate with 80 nM receptor and 5 nM probe in a final volume of 100 µl of binding buffer (50 mM Tris pH 8.0 (HCl), 120 mM NaCl, 0.1 % bovine gamma-globulin) and the anisotropy of the solution was measured with an Analyst AD 96-well plate reader (LJL, Molecular Dynamics, Sunnyvale, CA) using fluorescein optics. Control conditions were set up on each plate and include free probe 1 alone (5 nM), fully bound probe (5 µM BIR3 + 5 nM probe 1) and binding buffer blank. The EC₅₀ of binding was computed from a 4-parameter fit of the measured anisotropies using the SoftMax Pro 4.6 software package (Molecular Dynamics, Sunnyvale, CA). Inhibition constants were computed using an implementation of the algorithm by Z. Wang, FEBS Letters 360:111-114 (1995) in Microsoft Excel 2000 (Redmond, Washington).

Alternatively, probe 1 was substituted with probe 2: 6-(6-Hydroxy-3-oxo-3H-xanthen-9-yl)-N- {5-(2-methylamino-propionylamino)-6-oxo-6-[4-(1,2,3,4-tetrahydro-naphthalen-1-ylcarbamoyl)-thiazolidin-3-yl]-hexyl]-isophthalamic acid, for rat BIR3 and the concentrations of assay components changed. Serial dilutions of BIR3 antagonist at final concentrations typically between 1 µM and 0.1 nM were mixed with 15 nM receptor and 1 nM probe in a final volume of 100 µl of binding buffer and the anisotropy measured as described above. Controls were adjusted accordingly.

Average EC₅₀ of the compounds provided herein for binding to BIR3 domain of rat XIAP are provided in Table 1.

Since modifications will be apparent to those of skill in this art, it is intended that the subject matter claimed herein be limited only by the scope of the appended claims.

**The invention also relates to the following items:**
1. A compound of formula: 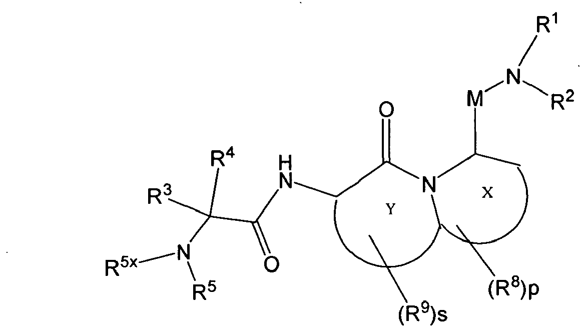 or pharmaceutically acceptable derivatives thereof, where:
   M is CO, SO or SO₂;
   X and Y are each independently selected from 4-7 membered heterocyclic and heteroaryl rings containing one or two heteroatoms;
   s and p are each independently 0-3;
   R¹ and R² are each independently selected as follows:
      (i) R¹ is hydrogen, alkyl, alkenyl, alkynyl, aryl, heteroaryl, cycloalkyl or heterocyclyl; and R² is selected from hydrogen, alkenyl, alkynyl, aryl, heteroaryl, cycloalkyl, heterocyclyl. OR¹¹, NR¹⁵R¹⁶ and 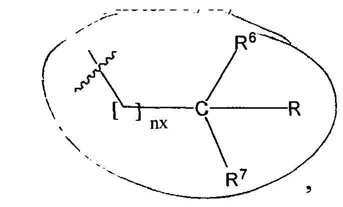 where nx is 0-6; R is hydrogen, alkyl, alkenyl, alkynyl, aryl, heteroaryl, heteroarylium, cycloalkyl, heterocyclyl, C(A)R¹⁰, OR¹¹ or NR¹⁵R¹⁶; and R⁶ and R⁷ are each independently selected from hydrogen, alkyl, alkenyl, alkynyl, aryl, heteroaryl, cycloalkyl and heterocyclyl; or
      (ii) R¹ and R² together with the nitrogen atom on which they are substituted form a heterocyclic or heteroaryl ring;
   R³, R⁴ and R⁵ are each independently selected from (i) or (ii) as follows:
      (i) R³ and R⁴ are each independently hydrogen, lower alkyl, lower alkenyl, alkynyl, lower cycloalkyl, lower heterocyclyl; and R⁵ is selected from hydrogen, alkyl, alkenyl, alkynyl, aryl, heteroaryl, cycloalkyl, heterocyclyl and NR¹⁵R¹⁶;
      (ii) R⁵ and R³ or R⁵ and R⁴ together with the atoms on which they are substituted form heterocyclic or heteroaryl ring and the other of R³ or R⁴ is selected as (i);
   R^{5x} is hydrogen, lower alkyl, lower alkenyl or lower alkynyl;
   R⁸ and R⁹ are each independently selected from (i), (ii) or (iii) as follows:
      (i) R⁸ and R⁹ are each independently selected from lower alkyl, lower alkenyl, lower alkynyl, aryl, heteroaryl, cycloalkyl, heterocyclyl, C(A)R¹⁰, halo, pseudohalo, OR¹¹, oxo, thio, S(D)nR¹², NR¹⁵R¹⁶ N⁺R¹⁵R¹⁶R¹⁶, =NR²⁵ and =CR¹³R¹⁴;
      (ii) R⁸ and R⁹ together with the atoms on which they are substituted form a cycloalkyl, aryl, heterocyclic or heteroaryl ring; or
      (iii) two R⁸ substituents together with the carbon atoms on which they are substituted form a cycloalkyl, aryl, heteroaryl or heterocyclyl ring;
   A is O, S or NR²⁵;
   R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴_{;}
   D is 0 or NR²⁵;
   n is 0, 1 or 2;
   when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
   when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
   R¹³ and R¹⁴ are each independently selected from hydrogen, alkyl, alkenyl, alkynyl, aryl, heteroaryl, heteroarylium, cycloalkyl, heterocyclyl, C(A)R²⁹, OR²⁵ or NR³²R³³_{;}
   R¹⁵, R¹⁶ and R¹⁷ are each independently selected from (a) and (b) as follows:
      (a) hydrogen, alkyl, alkenyl, alkynyl, aryl, heteroaryl, heteroarylium, cycloalkyl, heterocyclyl, C(A)R²⁹, OR²⁵ or NR³²R³³; or
      (b) any two of R¹⁵, R¹⁶ and R¹⁷ together form alkylene, alkenylene, alkynylene, heteroalkylene, and the other is selected as in (a);
   R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
   R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R21 together form alkylene, alkenylene, alkynylene, heteroalkylene;
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
      (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
      (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR⁴⁰ or NR³²R³³_{;}
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R, R^{5x} and R¹-R³³ are each independently unsubstituted or substituted with one or more substituents, each independently selected from Q¹;
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfmyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
   each Q¹ is independently unsubstituted or substituted with one or more substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroalyl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfmyloxy, alkylsulfonyloxy, arylsulfmyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfmyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e.*, -O-(CH₂)y-O-), thioalkylenoxy (*i.e*., -S-(CH₂)_{y}-O-)or alkylenedithioxy (*i.e.,* -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹ R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹,
   with the proviso that when a) Y is a 7 membered heterocyclic ring with 1 heteroatom in the ring, b) X is a 5 membered heterocyclic ring with 1 heteroatom in the ring, c) R⁸ and R⁹ together with the atoms on which they are substituted form a phenyl ring, and d) nx is 0, then R, R⁶, and R⁷ are not heterocyclyl or heteroaryl.
2. The compound of item 1, wherein R, R^{5x} and R¹-R³³ are each independently unsubstituted or substituted with one, two or three Q¹ substituents.
3. The compound of items 1 or 2, wherein the compound has formula: 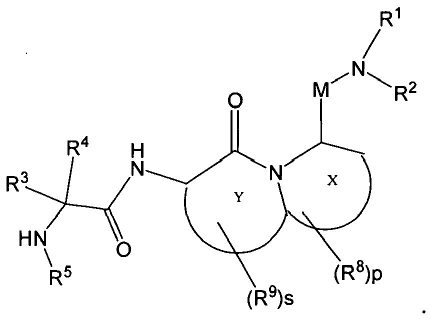
4. The compound of any of items 1-3, wherein the compound has formula: 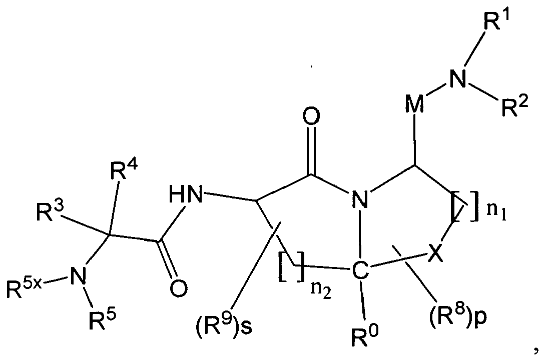 where
   X is NH, NR⁸, S, O, CH₂, CHR⁸ or C(R⁸)₂
   CR⁰ is CH or CR⁸;
   n₁ and n₂ are each independently 0-3 and s and p are each independently selected as follows:
   when n₁ is 0, p is 0 or 1;
   when n₁ is 1, p is 0, 1 or 2;
   when n₁ is 2, p is 0, 1, 2 or 3;
   when n₁ is 3, p is 0, 1, 2, 3 or 4;
   when n₂ is 0, s is 0 or 1;
   when n₂ is 1, s is 0, 1 or 2;
   when n₂ is 2, s is 0, 1, 2 or 3; and
   when n₂ is 3, s is 0, 1, 2, 3 or 4.
5. The compound of any of items 1-3, wherein
   M is CO;
   n₁ is 3 and n₂ is 1;
   R¹ is hydrogen or lower alkyl;
   R² is substituted or unsubstituted aryl, NR¹⁵R¹⁶ or 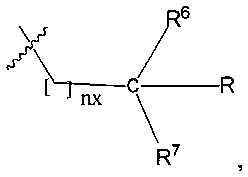 where nx is 0 or 1; R⁶ is selected from hydrogen and substituted or unsubstituted alkyl; R⁷ is selected from hydrogen, substituted or unsubstituted alkyl, and substituted or unsubstituted cycloalkyl;
   R is hydrogen or substituted or unsubstituted alkyl;
   R³, R⁴ and R⁵ are each independently selected from (i) or (ii) as follows:
   (i) R³ and R⁴ are each independently hydrogen or substituted or unsubstituted alkyl; and R⁵ is selected from hydrogen, substituted or unsubstituted alkyl, substituted or unsubstituted cycloalkyl and NR¹⁵R¹⁶;
   (ii) R⁵ and R³ or R⁵ and R⁴ together with the atoms on which they are substituted form substituted or unsubstituted heterocyclic or substituted or unsubstituted heteroaryl ring and the other of R³ or R⁴ is selected as (i);
   R^{5x} is hydrogen or lower alkyl;
   R⁸ and R⁹ are each independently selected from (i), (ii) or (iii) as follows:
   i) R⁸ and R⁹ are each independently selected from substituted or unsubstituted lower alkyl and oxo;
   ii) R⁸ and R⁹ together with the atoms on which they are substituted form substituted or unsubstituted aryl ring, wherein substituents when present, are selected from Q¹; or
   (iii) two R⁸ substituents together with the carbon atoms on which they are substituted form an aryl ring and R⁹ is selected as (i).
6. The compound of any of items 1-5, wherein R¹ is hydrogen.
7. The compound of any of items 1-6, wherein R² is selected from hydrogen, substituted or unsubstituted aryl, substituted or unsubstituted cycloalkyl and NR¹⁵R¹⁶.
8. The compound of any of items 1-6, wherein R² is selected from carboxyaralkyl, aryl, cycloalkyl and arylalkylamine.
9. The compound of any of items 1-6, wherein R² is selected from benzylmethyl, carboxybenzyl, naphthyl, tetrahydronaphthyl and methylphenylamine.
10. The compound of any of items 1-4, wherein R² is selected from 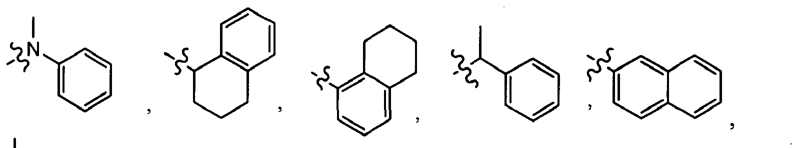 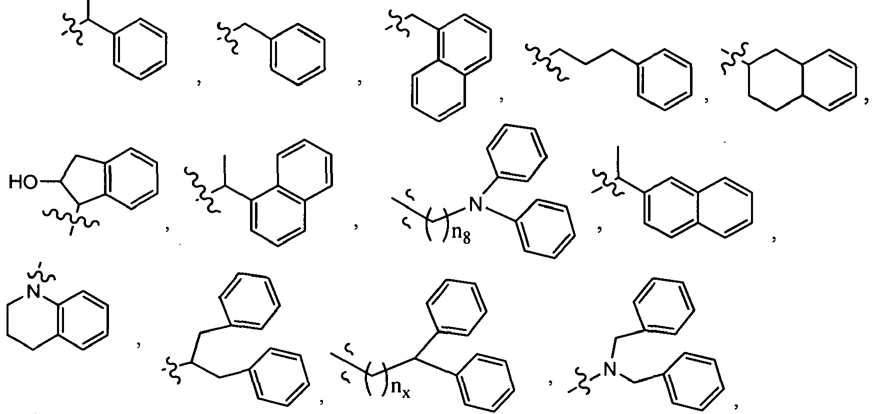 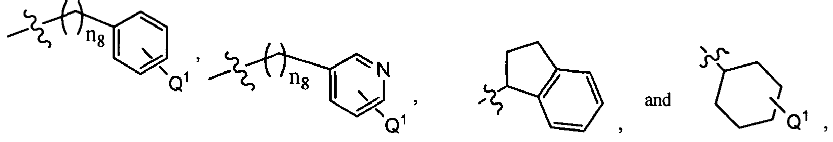 wherein n₈ is 0 to 3.
11. The compound of any of items 1-4, and 10 wherein R² is selected from 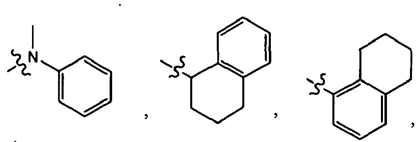 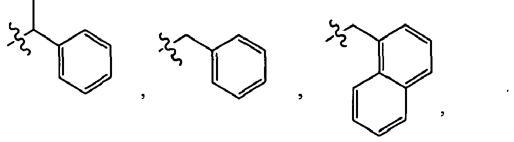 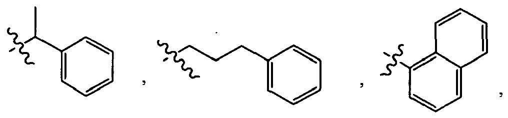 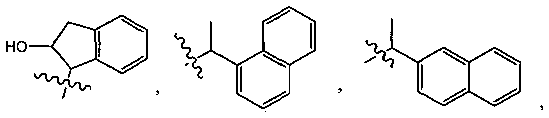 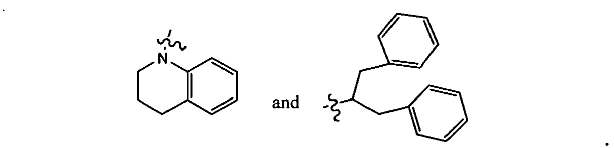
12. The compound of any of items 1-11, wherein the X ring is a 5 membered heterocyclic ring with two heteroatoms in the ring.
13. The compound of any of items 1-12, wherein the X ring is a thiazole ring.
14. The compound of any of items 1-12, wherein the X ring is pyrrolidine ring.
15. The compound of any of items 1-12, wherein the Y ring is pyrrolidine ring.
16. The compound of any of items 1-12, wherein the Y ring is 7-membered ring.
17. The compound of any of items 1-12, wherein the X and Y rings together are selected from: 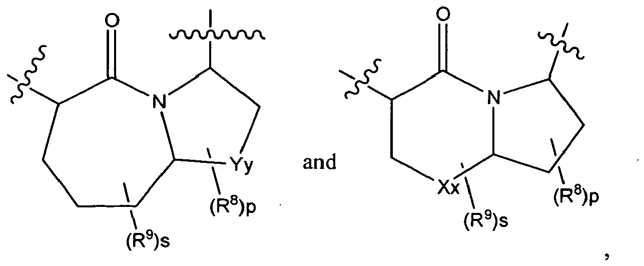 where Y_{y} is S, O or NR⁸ and Xₓ is S, O or NR⁹_{.}
18. The compound of any of items 1-12, wherein the X and Y rings together are selected from: 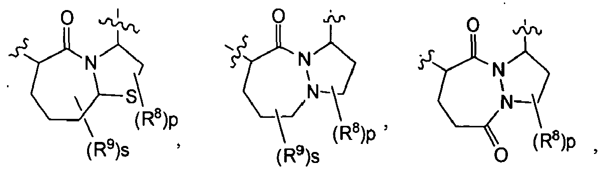 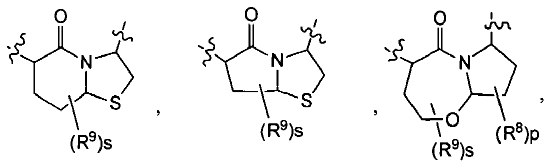 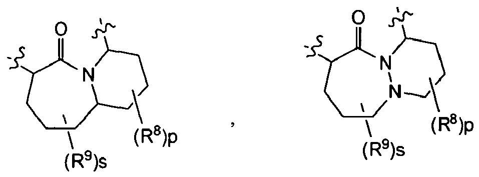 and 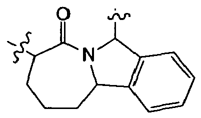
19. The compound of any of items 1-12, wherein the X and Y rings form: 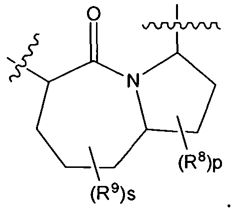
20. The compound of any of items 1-19, wherein R⁸ and R⁹ are selected from: (i) R⁸ and R⁹ together with the atoms on which they are substituted form an aryl ring or (ii) two R⁸ substituents together with the carbon atoms on which they are substituted form an aryl ring.
21. The compound of any of items 1-20, wherein R⁸ and R⁹ together with the atoms on which they are substituted form a phenyl ring.
22. The compound of any of items 1-21, wherein R³ and R⁴ are each independently hydrogen or lower alkyl.
23. The compound of any of items 1-22, wherein R³ is hydrogen.
24. The compound of any of items 1-22, wherein R³ is lower alkyl.
25. The compound of any of items 1-22, wherein R³ is methyl.
26. The compound of any of items 1-22, wherein R⁴ is hydrogen or lower alkyl.
27. The compound of any of items 1-22, wherein R⁴ is methyl.
28. The compound of any of items 1-27, wherein R⁵ is hydrogen, alkyl, cycloalkylalkyl, or aralkyl.
29. The compound of any of items 1-25, wherein R⁵ is methyl, cyclohexylmethyl, cyclopropylmethyl, isopropyl, benzyl or phenylpropyl.
30. The compound of any of items 1-21, wherein R⁵ and R³ or R⁵ and R⁴ together with the atoms on which they are substituted form a heterocyclic or heteroaryl ring and the other of R³ or R⁴ is selected from hydrogen and lower alkyl.
31. The compound of any of items 1-21 and 30, wherein R⁵ and R³ form a heterocyclic ring as follows: 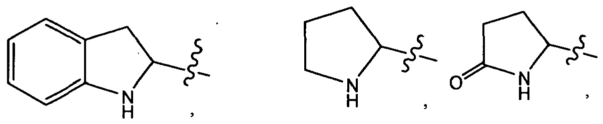 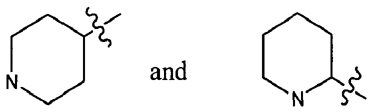
32. The compound of any of items 1-21 and 30, wherein R⁵ and R³ form a heterocyclic ring as follows: 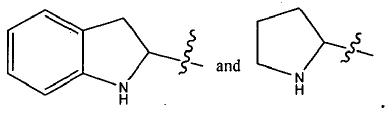
33. The compound of any of items 1-32, wherein R^{5x} is hydrogen or lower alkyl.
34. The compound of any of items 1-33, wherein R^{5x} is hydrogen or methyl.
35. The compound of items 1 or 2, wherein the compound has formula 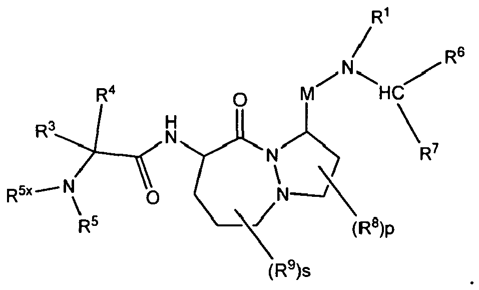
36. The compound of any of items 1-10, wherein the compound has formula: 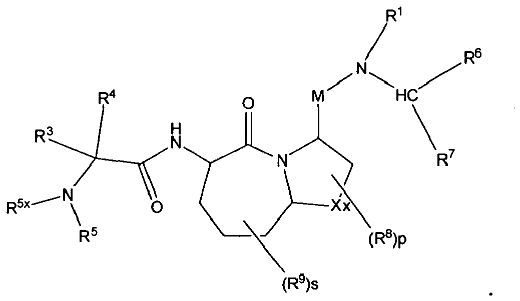
37. The compound of any of items 1-10, wherein the compound has formula: 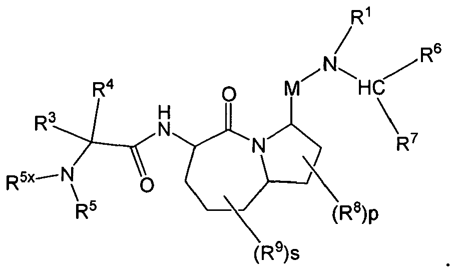
38. The compound of any of items 1-10, wherein the compound has formula: 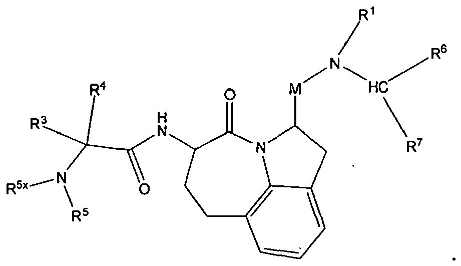
39. The compound of any of items 1-10, wherein the compound has formula 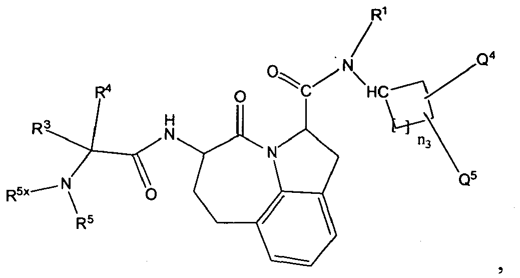 where n₃ is 1-3, Q⁴ and Q⁵ are each independently alkyl, cycloalkyl, aryl or Q⁴ and Q⁵ together with the carbon atoms on which they are substituted form a cycloalkyl or aryl ring.
40. The compound of any of items 1-10,wherein the compound has formula: 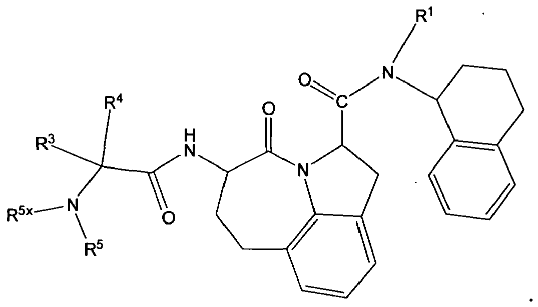
41. The compound of any of items 1-10, wherein the compound has formula: 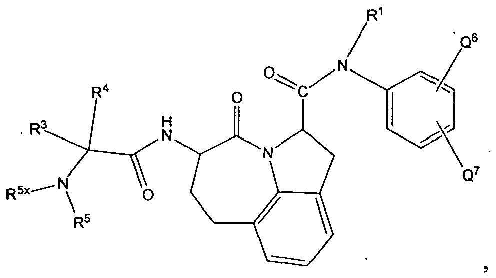 where Q⁶ and Q⁷ are each independently alkyl, cycloalkyl, aryl or Q⁶ and Q⁷ together with the carbon atoms on which they are substituted form a cycloalkyl or aryl ring.
42. The compound of any of items 1-10, wherein the compound has formula: 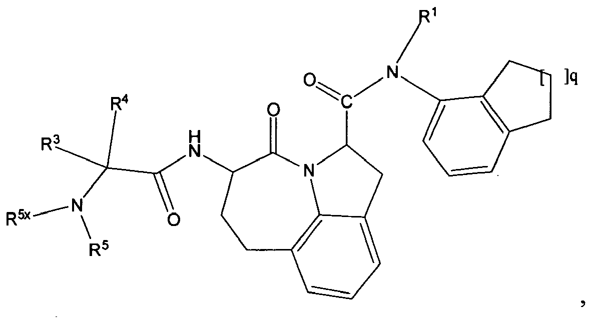 where q is 1-3.
43. The compound of any of items 1-10, wherein the compound has formula: 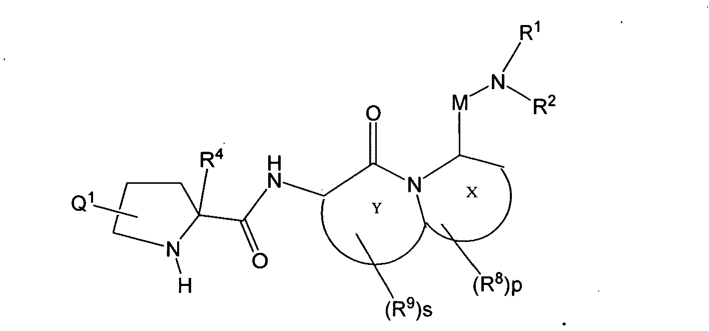
44. A compound of formula: 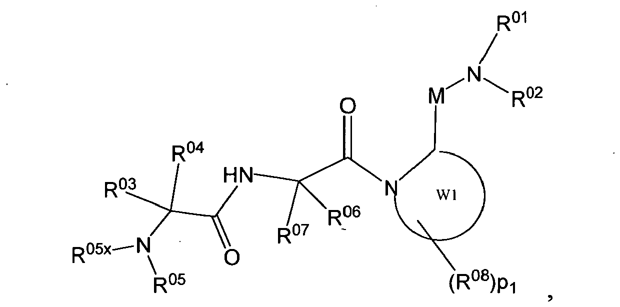 or pharmaceutically acceptable derivative thereof, where:
   M is CO, SO or SO₂ W1 is a 5-7 membered heterocyclic or heteroaryl ring containing 1 or 2 heteroatoms;
   R⁰¹ and R⁰² are each independently selected from (i) or (ii) as follows:
      (i) ) R⁰¹ is hydrogen, alkyl, alkenyl, alkynyl, aryl, heteroaryl, cycloalkyl or heterocyclyl; and R⁰² is selected from hydrogen, alkyl, alkenyl, alkynyl, aryl, heteroaryl, cycloalkyl, heterocyclyl, OR¹¹, NR¹⁵R¹⁶, or
      (ii) R⁰¹ and R⁰² together with the nitrogen atom on which they are substituted form a heterocyclic or heteroaryl ring;
   R⁰³, R⁰⁴ and R⁰⁵ are each independently selected from (i) or (ii) as follows:
      (i) R⁰³ and R⁰⁴ are each independently hydrogen, lower alkyl, lower alkenyl, alkynyl, lower cycloalkyl, lower heterocyclyl; and R⁰⁵ is selected from hydrogen, alkyl, alkenyl, alkynyl, aryl, heteroaryl, cycloalkyl, heterocyclyl and NR¹⁵R¹⁶;
      (ii) R⁰⁵ and R⁰³ or R⁰⁵ and R⁰⁴ together with the atoms on which they are substituted form a heterocyclic or heteroaryl ring and the other of R⁰³ or R⁰⁴ is selected as (i);
   R^{05x} is hydrogen, lower alkyl, lower alkenyl or lower alkynyl;
   R⁰⁶ and R⁰⁷ are each independently hydrogen, alkyl, alkenyl, alkynyl, aryl, heteroaryl, cycloalkyl and heterocyclyl;
   p₁ is 0-4;
   R⁰⁸ is selected from
      i) alkyl, alkenyl, alkynyl, aryl, heteroaryl, heteroarylium, cycloalkyl, heterocyclyl, C(A)R¹⁰, halo, pseudohalo, OR¹¹, oxo, thio, S(D)nR¹² NR¹⁵R¹⁶, N⁺R¹⁵R¹⁶R¹⁷, =NR²⁵ and =CR¹³R¹⁴; or
      ii) two R⁰⁸ substituents together with the atoms on which they are substituted form an aryl, cycloalkyl, heteroaryl or heterocyclyl ring;
   A is O, S or NR²⁵;
   R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
   D is O or NR²⁵;
   n is 0, 1 or 2;
   when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
   when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
   R¹³ and R¹⁴ are each independently selected from hydrogen, alkyl, alkenyl, alkynyl, aryl, heteroaryl, heteroarylium, cycloalkyl, heterocyclyl, C(A)R²⁹, OR²⁵ or NR³²R³³;
   R¹⁵, R¹⁶ and R¹⁷ are each independently selected from (a) and (b) as follows:
      (a) hydrogen, alkyl, alkenyl, alkynyl, aryl, heteroaryl, heteroarylium, cycloalkyl, heterocyclyl, C(A)R²⁹, OR²⁵ or NR³²R³³; or
      (b) any two of R¹⁵, R¹⁶ and R¹⁷ together form alkylene, alkenylene, alkynylene, heteroalkylene, and the other is selected as in (a);
   R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
   R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R21 together form alkylene, alkenylene, alkynylene, heteroalkylene;
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
      (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroalylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
      (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR⁴⁰ or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R⁰¹-R⁰⁸, R^{05x} and R¹⁰-R³³ are each independently unsubstituted or substituted with one or more substituents, each independently selected from Q¹;
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e.,* -S-(CH₂)_{y}-S-) where y is 1 or 2; or two Q¹ groups, which substitute the same atom, together form alkylene; and
   each Q¹ is independently unsubstituted or substituted with one or more substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹;
   with the proviso that when ring W1 is a heterocyclic ring with one heteroatom in the ring, p₁ is 0, R⁰¹ is hydrogen, R^{05x} is hydrogen or methyl, and R⁵⁰ is alkyl, optionally substituted with halo, hydroxy, alkoxy, mercapto, alkylthio, thiocyano, pesudohalo, then R⁰² is not hydrogen, alkyl, cycloalkyl, naphthyl or COR¹⁰, where R¹⁰ is alkyl or phenyl.
45. The compound of item 44, wherein R¹⁰-R⁰⁸, R^{5x} and R¹⁰-R³³ are each independently unsubstituted or substituted with one, two or three Q¹ substituents.
46. The compound of items 44 or 45, wherein each Q¹ substituent is independently substituted with one, two or three Q² substituents.
47. The compound of any of items 44-46, wherein the compound has formula: 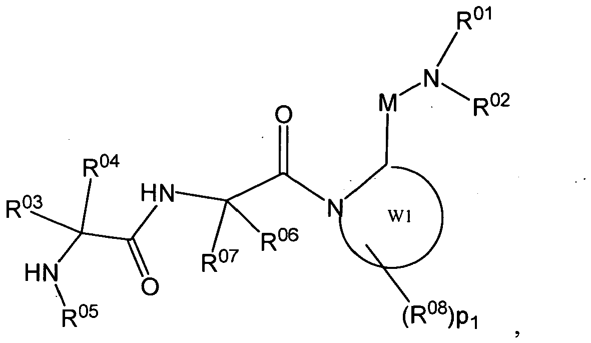 where W1 is a 5-7 membered heterocyclic ring containing 1 or 2 heteroatoms;
   M is CO;
   R⁰¹ is hydrogen, alkyl, alkenyl or alkynyl; and R⁰² is selected from hydrogen, alkyl, aryl, heteroaryl, cycloalkyl, heterocyclyl, NR¹⁵R¹⁶;
   R⁰³, R⁰⁴ and R⁰⁵ are each independently selected from (i) or (ii) as follows:
   (i) R⁰³ and R⁰⁴ are each independently hydrogen and alkyl and R⁰⁵ is selected form hydrogen, alkyl, aryl or NR¹⁵R¹⁶;
   (ii) R⁰⁵ and R⁰³ or R⁰⁵ and R⁰⁴ together with the atoms on which they are substituted form a heterocyclic ring and the other of R⁰³ or R⁰⁴ is selected as (i);
   R⁰⁶ and R⁰⁷ are each independently hydrogen, alkyl and cycloalkyl;
   p₁ is 0-2;
   R⁰⁸ is selected from
   i) alkyl or oxo, or
   ii) two R⁰⁸ substituents together with the atoms on which they are substituted form an aryl, heteroaryl or heterocyclyl ring;
   R⁰¹-R⁰⁸ are each independently unsubstituted or substituted with one to five substituents, each independently selected from Q¹.
48. The compound of any of items 44-47, wherein R⁰¹ is hydrogen or substituted or unsubstituted lower alkyl.
49. The compound of any of items 44-48, wherein R⁰¹ is hydrogen.
50. The compound of any of items 44-49, wherein R⁰² is selected from hydrogen, substituted or unsubstituted alkyl, substituted or unsubstituted aryl, substituted or unsubstituted cycloalkyl and NR¹⁵R¹⁶.
51. The compound of any of items 44-50, wherein R⁰² is selected from carboxyaralkyl, aralkyl, bicyclic aryl, bicyclic cycloalkyl and arylalkylamine.
52. The compound of any of items 44-51, wherein R⁰² is selected from benzylmethyl, 2-benzyl-3-phenylpropyl, carboxybenzyl, naphthyl, tetrahydronaphthyl and methylphenylamine.
53. The compound of any of items 44-50, wherein R⁰² is selected from: 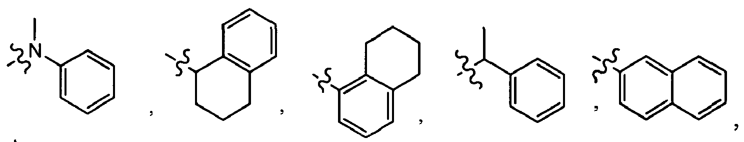 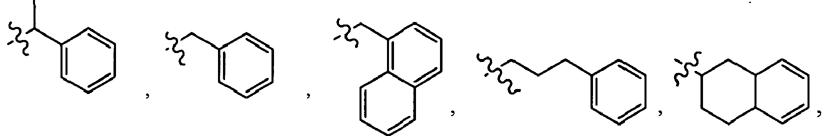 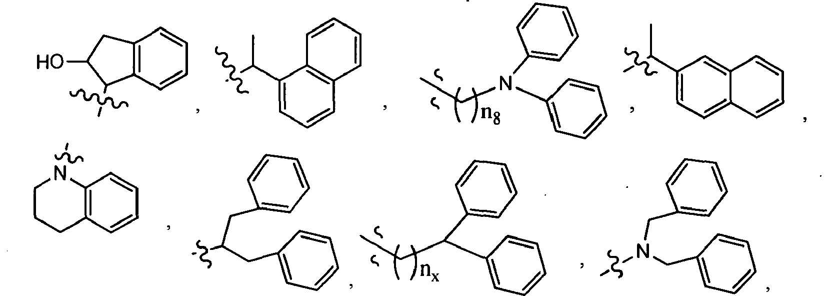 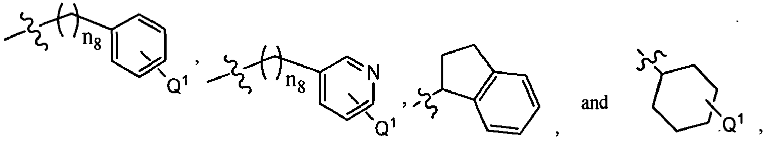 n₈ is 0 to 3.
54. The compound of any of items 44-50, wherein R⁰² is selected from 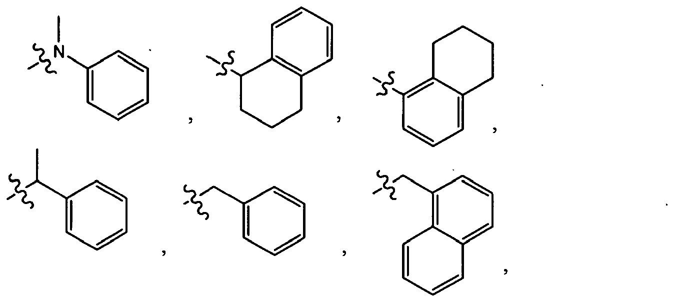 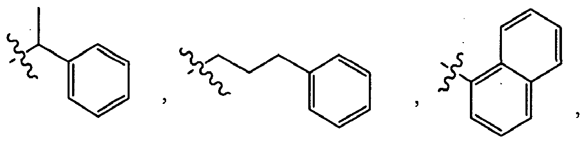 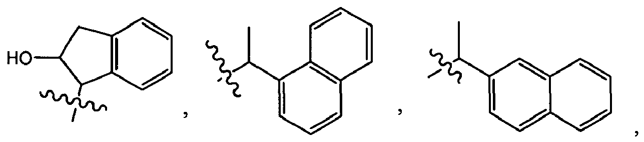 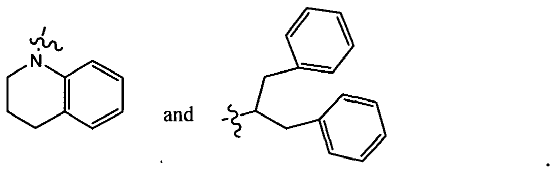
55. The compound of any of items 44-54, wherein the W1 ring is a 5 membered heterocyclic ring with two heteroatoms in the ring.
56. The compound of any of items 44-55, wherein the W1 ring is a thiazole ring.
57. The compound of any of items 44-55, wherein the W1 ring is pyrrolidine ring.
58. The compound of any of items 44-55, wherein the W1 ring is selected from: 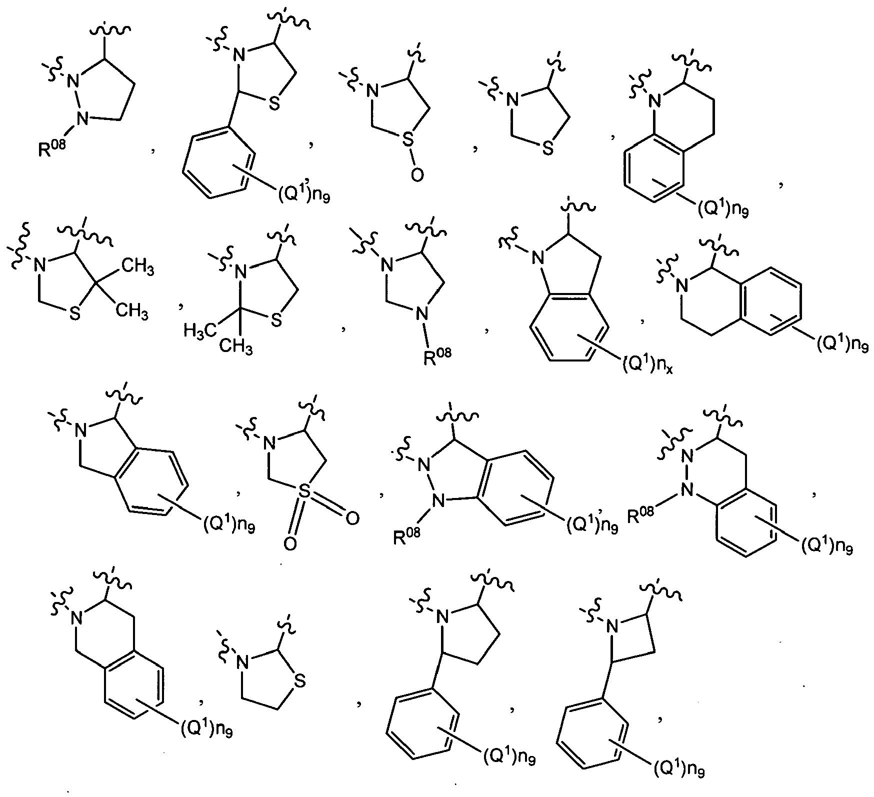 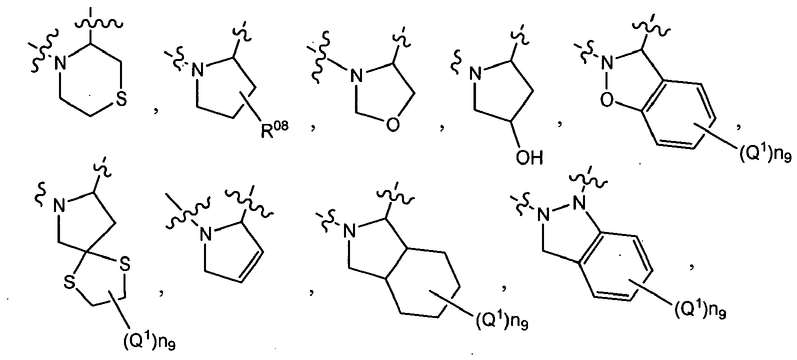 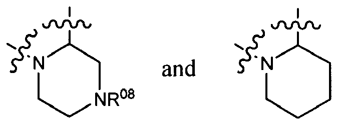 where n₉ is 0-5.
59. The compound of any of items 44-55, wherein the W1 ring is selected from: 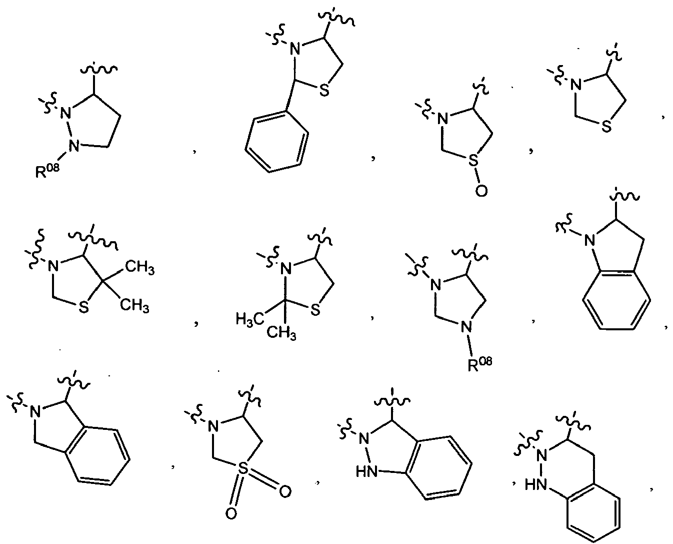 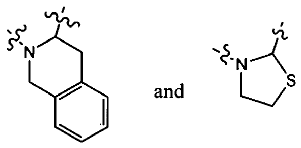
60. The compound of any of items 44-59, wherein R⁰³ and R⁰⁴ are each independently hydrogen or lower alkyl.
61. The compound of any of items 44-60, wherein R⁰³ is hydrogen.
62. The compound of any of items 44-60, wherein R⁰³ is lower alkyl.
63. The compound of any of items 44-60, wherein R⁰³ is methyl.
64. The compound of any of items 44-60, wherein R⁰⁴ is hydrogen.
65. The compound of any of items 44-60, wherein R⁰⁴ is lower alkyl.
66. The compound of any of items 44-60, wherein R⁰⁴ is methyl.
67. The compound of any of items 44-66, wherein R⁰⁵ is hydrogen, alkyl, cycloalkylalkyl, aralkyl or NR¹⁵R¹⁶, where R¹⁵ and R¹⁶ are each independently hydrogen or lower alkyl.
68. The compound of any of items 44-67, wherein R⁰⁵ is methyl, cyclohexylmethyl, cyclopropylmethyl, isopropyl, benzyl, phenylpropyl or NH₂.
69. The compound of any of items 44-57, wherein R⁰⁵ and R⁰³ or R⁰⁵ and R⁰⁴ together with the atoms on which they are substituted form a heterocyclic or heteroaryl ring and other of R⁰³ or R⁰⁴ is selected from hydrogen and lower alkyl.
70. The compound of any of items 44-57 and 69, wherein R⁰⁵ and R⁰³ form a heterocyclic ring as follows: 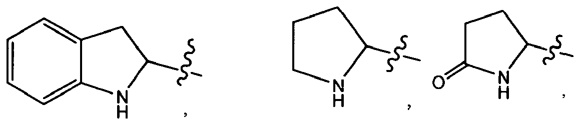 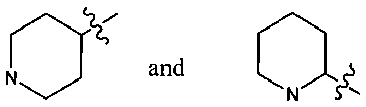
71. The compound of any of items 44-57 and 69-70, wherein R⁰⁵ and R⁰³ form a heterocyclic ring as follows: 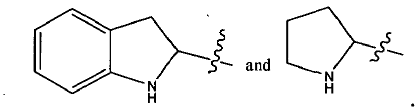
72. The compound of any of items 44-71, wherein R^{5x} is hydrogen or lower alkyl.
73. The compound of any of items 44-71, wherein R^{5x} is hydrogen or methyl.
74. The compound of any of items 44-54, wherein the compound has formula: 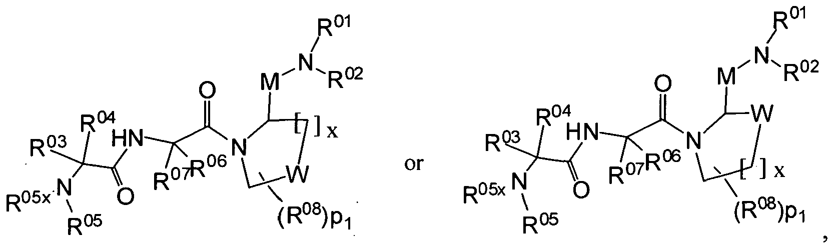 where W is S, N, NH, NR⁰⁸, CH or CHR⁰⁸; x is 1-3.
75. The compound of any of items 44-47, wherein the compound has formula: 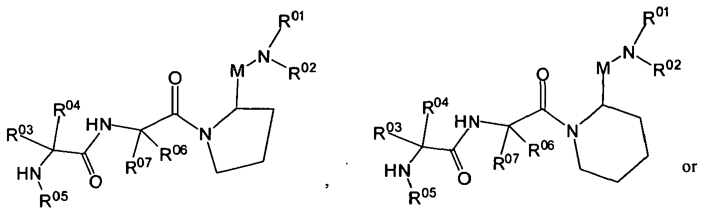 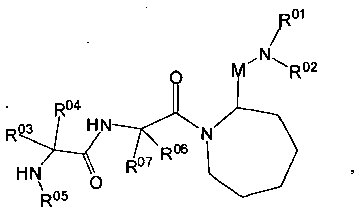
76. The compound of any of items 44-47, wherein the compound has formula: 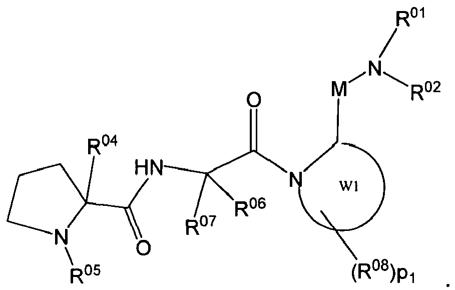
77. The compound of any of items 44-47, wherein the compound has formula: 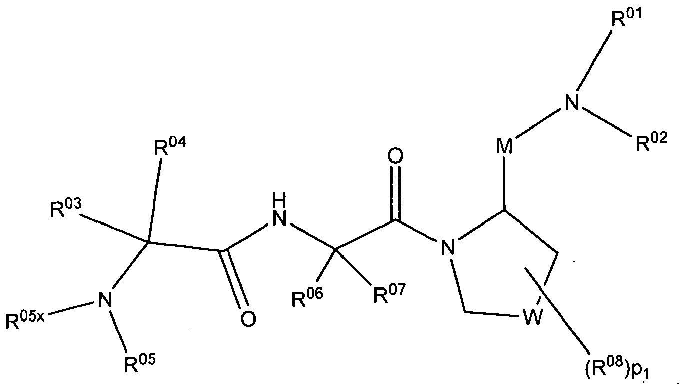
78. The compound of any of items 44-47, wherein the compound has formula: 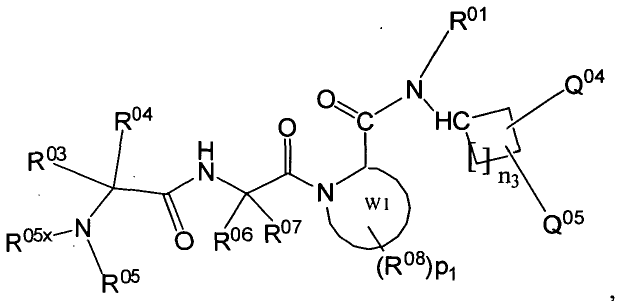 where n₃ is 1-3, Q⁰⁴ and Q⁰⁵ are each independently alkyl, cycloalkyl, aryl or Q⁰⁴ and Q⁰⁵ together with the carbon atoms on which they are substituted form a cycloalkyl or aryl ring.
79. The compound of any of items 44-47, wherein the compound has formula: 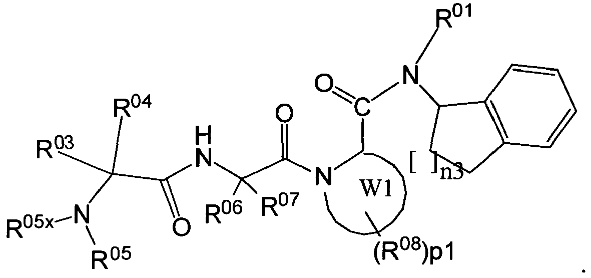
80. The compound of any of items 44-47, wherein the compound has formula: 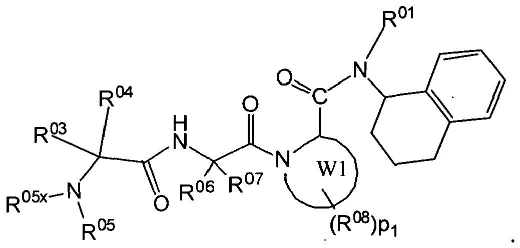
81. The compound of any of items 44-47, wherein the compound has formula: 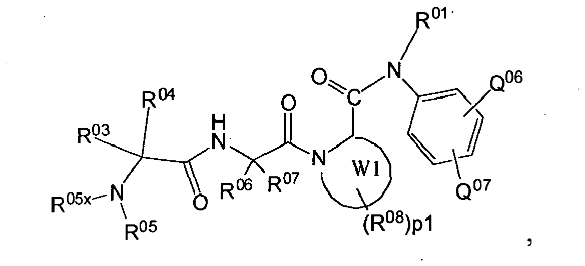 where Q⁰⁶ and Q⁰⁷ are each independently alkyl, cycloalkyl, aryl or Q⁰⁶ and Q⁰⁷ together with the carbon atoms on which they are substituted form a cycloalkyl or aryl ring.
82. The compound of any of items 44-47, wherein the compound has formula: 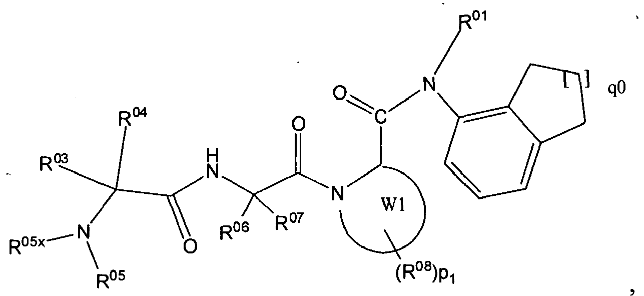 where q⁰ is 1-3.
83. The compound of any of items 44-47, wherein the compound has formula: 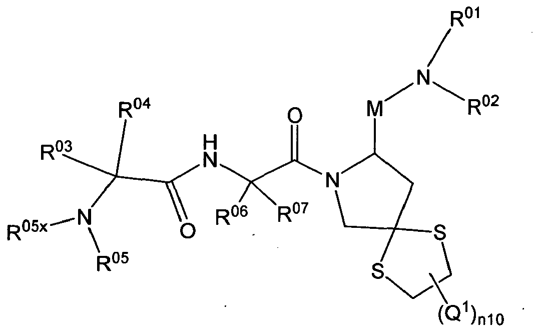 or 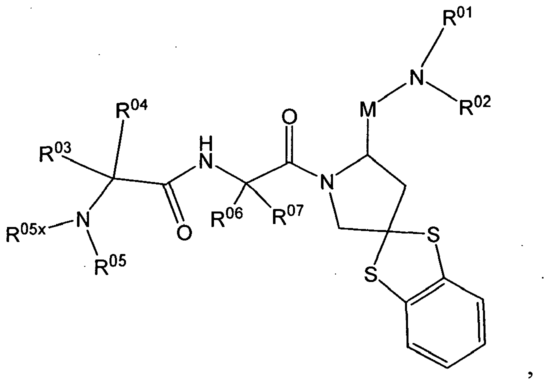 where n10 is 0 to 2.
84. The compound of any of claims 44-47, wherein the compound has formula: 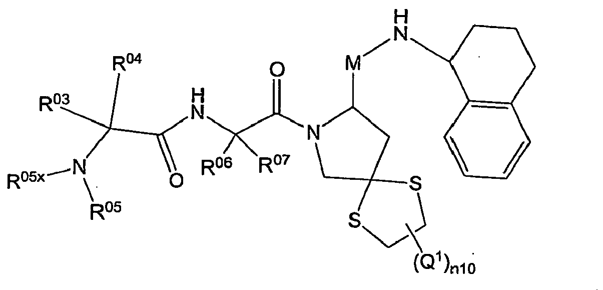 or 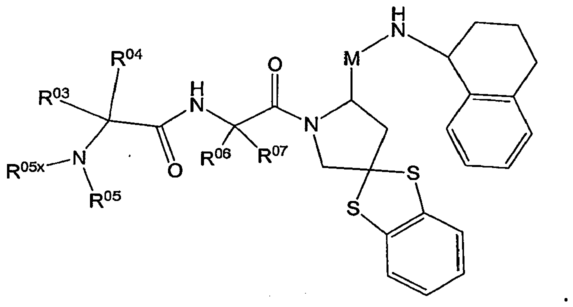
85. The compound of any of claims 44-47, wherein the compound has formula: 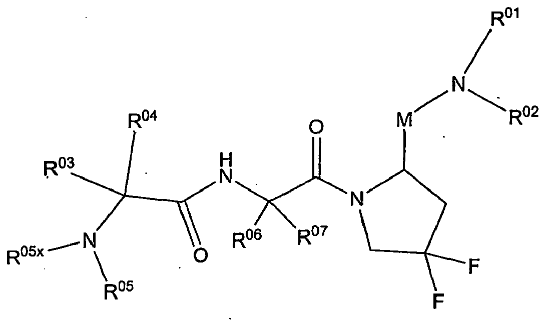 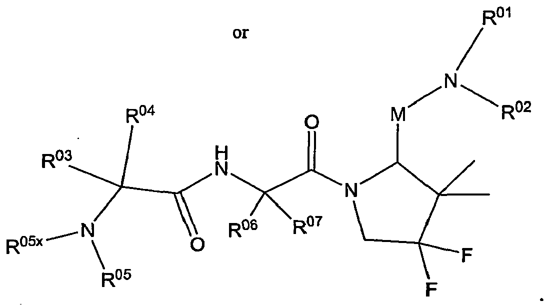
86. The compound of any of claims 44-47, wherein the compound has formula: 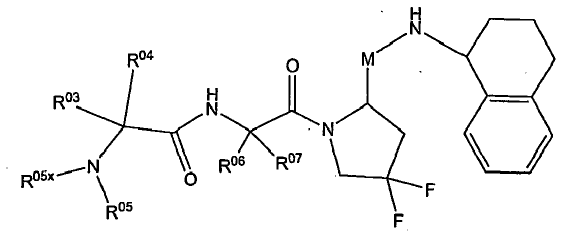 or 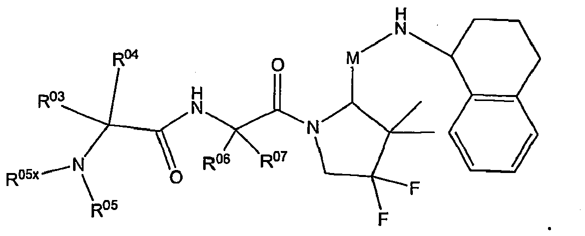
87. A compound of formula: 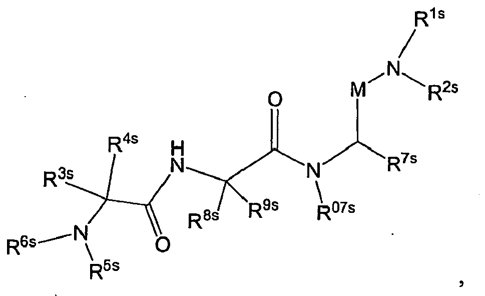 or a pharmaceutically acceptable derivative thereof,
   where:
   M is CO, SO or SO₂;
   R^{1s} and R^{2s} are selected as follows:
      (i) R^{1s} is hydrogen, alkyl, alkenyl, alkynyl, aryl, heteroaryl, cycloalkyl or heterocyclyl; and R^{2s} is selected from hydrogen, alkenyl, alkynyl, aryl, heteroaryl, cycloalkyl, heterocyclyl, OR¹¹, NR¹⁵R¹⁶ or
      (ii) R^{1s} and R^{2s} together with the nitrogen atom on which they are substituted form a heterocyclic or heteroaryl ring;
   R^{3s}, R^{4s} and R^{5s} are each independently selected from (i) or (ii) as follows:
      (i) R^{3s} is hydrogen or alkyl; R^{4s} is alkyl; and R^{5s} is selected from hydrogen, alkyl, alkenyl, alkynyl, aryl, heteroaryl, heteroarylium, cycloalkyl, heterocyclyl and NR¹⁵R¹⁶;
      (ii) R^{5s} and R^{3s} or R^{5s} and R^{4s} together with the atoms on which they are substituted form heterocyclic or heteroaryl ring and the other of R^{3s} or R^{4s} is selected as (ii);
   R^{6s} is hydrogen, lower alkyl, lower alkenyl or lower alkynyl;
   R^{7s} is hydrogen, alkyl, alkenyl, alkynyl, aryl, heteroaryl, cycloalkyl or heterocyclyl;
   R^{07s}, R^{8s} and R^{9s} are each selected as follows:
      (i) R^{8s} and R^{9s} are each independently hydrogen, alkyl, alkenyl, alkynyl, aryl, heteroaryl, cycloalkyl, heterocyclyl and R^{07s} is alkyl, aryl or cycloalkyl,; or
      (ii)) R^{8s} and R^{07s} or R^{9s} and R^{07s} together with the atoms on which they are substituted form a 5-7 membered heterocyclic or heteroaryl ring containing 1 or 2 heteroatoms in the ring;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
   A is O, S or NR²⁵;
   R¹⁵, R¹⁶ and R¹⁷ are each independently selected from (a) and (b) as follows:
      (a) hydrogen, alkyl, alkenyl, alkynyl, aryl, heteroaryl, heteroarylium, cycloalkyl, heterocyclyl, C(A)R²⁹, OR²⁵ or NR³²R³³; or
      (b) any two of R¹⁵, R¹⁶ and R¹⁷ together form alkylene, alkenylene, alkynylene, heteroalkylene, and the other is selected as in (a);
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁹ is hydrogen, alkyl, alkenyl, alkynyl, aryl, heteroaryl, heteroarylium, cycloalkyl, heterocyclyl, OR⁴⁰ or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R^{1s}-R^{9s}, R^{07s} and R¹⁰-R³³ are each independently unsubstituted or substituted with one or more substituents, each independently selected from Q¹;
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
   each Q¹ is independently unsubstituted or substituted with one or more substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(-O)(R⁵⁰)₂, OP(=O)(R⁵⁰)₂, -NR⁶⁰C(-O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e.,* -S-(CH₂)y-O-)or alkylenedithioxy (*i.e.,* -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹.
88. The compound of item 87, wherein R^{1s}-R^{9s}, R^{07s} and R¹⁰-R³³ are each independently unsubstituted or substituted with one, two or three Q¹ substituents.
89. The compound of items 87 or 88, wherein each Q¹ substituent is independently substituted with one, two or three Q² substituents.
90. The compound of any of items 87-89, wherein 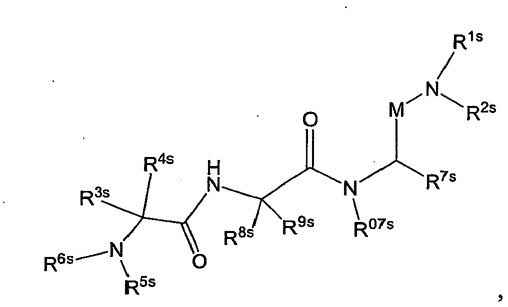 or a pharmaceutically acceptable derivative thereof,
   where:
   M is CO, SO or SO₂;
   R^{1s} is hydrogen;
   R^{2s} is selected from aryl, heteroaryl, cycloalkyl, cycloalkylalkyl and heterocyclyl;
   R^{3s}, R^{4s} and R^{5s} are each independently selected from (i) or (ii) as follows:
      (i) R^{3s} is hydrogen or alkyl; R^{4s} is alkyl; and R^{5s} is selected from hydrogen, alkyl, alkenyl, alkynyl, aryl, heteroaryl, heteroarylium, cycloalkyl, heterocyclyl and NR¹⁵R¹⁶;
      (ii) R^{5s} and R^{3s} or R^{5s} and R^{4s} together with the atoms on which they are substituted form heterocyclic or heteroaryl ring and the other of R^{3s} or R^{4s} is selected as (ii);
   R^{6s} is hydrogen, lower alkyl, lower alkenyl or lower alkynyl;
   R^{7s} is hydrogen, alkyl, alkenyl, alkynyl, aryl, heteroaryl, cycloalkyl or heterocyclyl;
   R^{07s}, R^{8s} and R^{9s} are each selected as follows:
      (i) R^{8s} and R^{9s} are each independently hydrogen, alkyl, alkenyl, alkynyl, aryl, heteroaryl, cycloalkyl, heterocyclyl and R^{07s} is alkyl, aryl or cycloalkyl; or
      (ii) ) R^{8s} and R^{07s} or R^{9s} and R^{07s} together with the atoms on which they are substituted form a 5-7 membered heterocyclic or heteroaryl ring containing 1 or 2 heteroatoms in the ring.
91. The compound of any of claims 87-90, wherein the compound has formula: 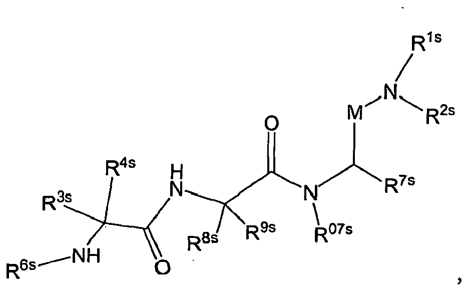 where M is CO;
   R^{1s} is hydrogen; and R^{2s} is selected from aryl, heteroaryl, cycloalkyl cycloalkylalkyl and heterocyclyl;
   R^{3s}, R^{4s} and R^{5s} are each independently selected from (i) or (ii) as follows:
   (i) R^{3s} is hydrogen or alkyl; R^{4s} is alkyl and R^{5s} is selected form hydrogen, alkyl and aryl;
   (ii) R^{5s} and R^{3s} or R^{5s} and R^{4s} together with the atoms on which they are substituted form a heterocyclic ring and the other of R^{3s} or R^{4s} is selected as (i);
   R^{6s} is hydrogen, or lower alkyl;
   R^{7s} is hydrogen or alkyl;
   R^{07s}, R^{8s} and R^{9s} are each selected as follows:
   (i) R^{8s} and R^{9s} are each independently hydrogen or alkyl; and R^{07s} is lower alkyl; or
   (ii)) R^{8s} and R^{07s} or R^{9s} and R^{07s} together with the atoms on which they are substituted form a 5-7 membered heterocyclic or heteroaryl ring containing 1 heteroatom in the ring;
   R^{1s}-R^{8s} and R^{07s} are each independently unsubstituted or substituted with one to five substituents, each independently selected from Q¹.
92. The compound of any of claims 87-91, wherein R^{1s} is hydrogen and R^{2s} is selected from substituted or unsubstituted aryl, substituted or unsubstituted heteroaryl, substituted or unsubstituted heterocyclyl, substituted or unsubstituted cycloalkyl and substituted or unsubstituted cycloalkylalkylalkyl.
93. The compound of any of claims 87-92, wherein R^{2s} is selected from substituted or unsubstituted aryl and substituted or unsubstituted cycloalkyl.
94. The compound of any of claims 87-93, wherein R^{2s} is cycloalkyl.
95. The compound of any of items 87-94, wherein R^{2s} is tetrahydronaphthyl.
96. The compound of any of items 87-92, wherein R^{2s} is selected from: 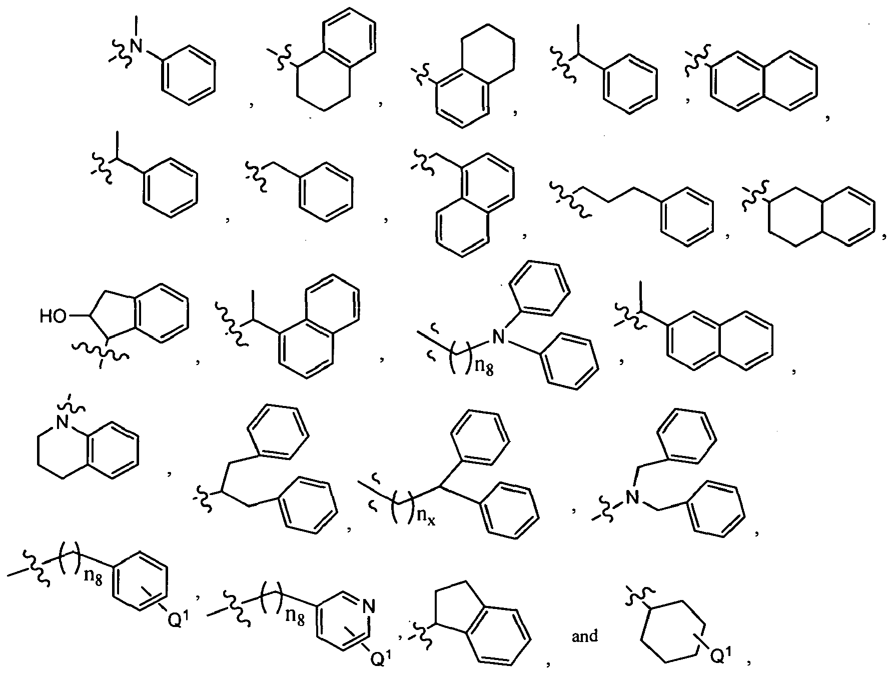 n₈ is 0 to 3.
97. The compound of any of items 87-92, wherein R^{2s} is selected from 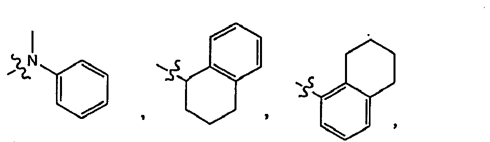 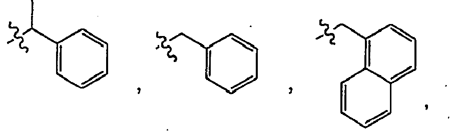 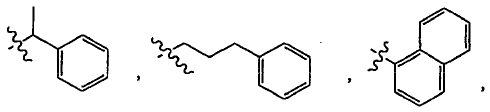 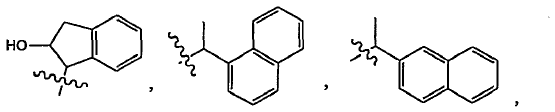 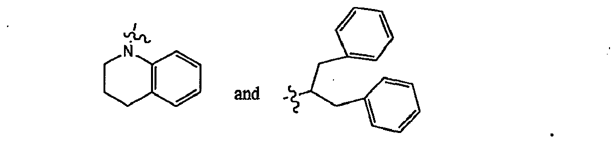
98. The compound of any of claims 87-97, wherein R^{3s} is hydrogen or lower alkyl.
99. The compound of any of claims 87-98, wherein R^{3s} is hydrogen.
100. The compound of any of claims 87-98, wherein R^{3s} is lower alkyl.
101. The compound of any of claims 87-98, wherein R^{3s} is methyl.
102. The compound of any of claims 87-98, wherein R^{4s} is lower alkyl.
103. The compound of any of claims 87-98, wherein R^{4s} is methyl.
104. The compound of any of claims 87-98, wherein R^{5s} is hydrogen, alkyl, cycloalkylalkyl or aralkyl.
105. The compound of any of claims 87-104, wherein R^{5s} is lower alkyl.
106. The compound of any of claims 87-105, wherein R^{5s} is methyl.
107. The compound of any of claims 87-106, wherein R^{5s} and R^{3s} or R^{5s} and R^{4s} together with the atoms on which they are substituted form a heterocyclic or heteroaryl ring and other of R^{3s} or R^{4s} is selected from hydrogen and lower alkyl.
108. The compound of any of items 87-98 and 107, wherein R^{5s} and R^{3s} form a heterocyclic ring as follows: 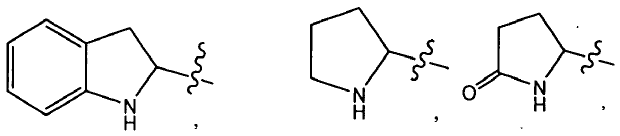 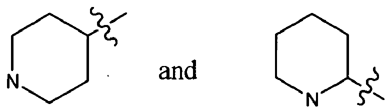
109. The compound of any of items 87-98 and 107, wherein R^{5s} and R^{3s} form a heterocyclic ring as follows: 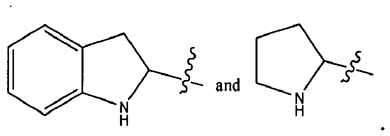
110. The compound of any of items 87-109, wherein R^{6s} is hydrogen or lower alkyl.
111. The compound of any of items 87-110, wherein R^{6s} is hydrogen or methyl.
112. The compound of any of items 87-111, wherein the compound has formula: 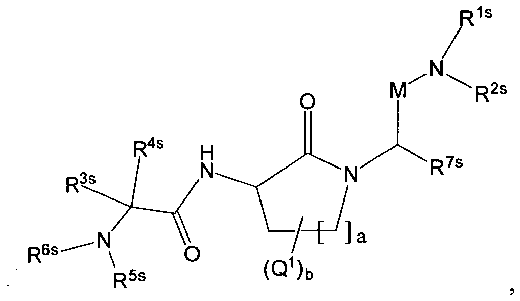 where a is 1-3 and b is 0-4 and is selected as follows:
   when a is 1, b is 0, 1 or 2,
   when a is 2, b is 0, 1, 2 or 3,
   when a is 3, b is 0, 1, 2, 3 or 4.
113. The compound of any of items 87-89, wherein the compound has formula: 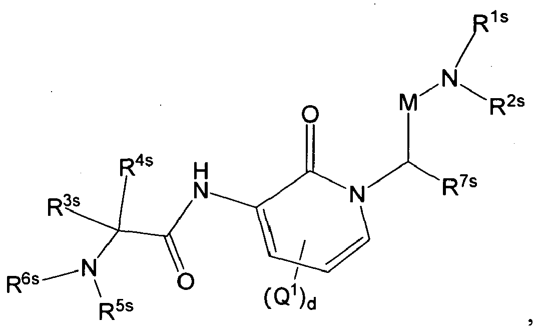 where d is 0-3.
114. The compound of any of items 87-89, wherein the compound has formula: 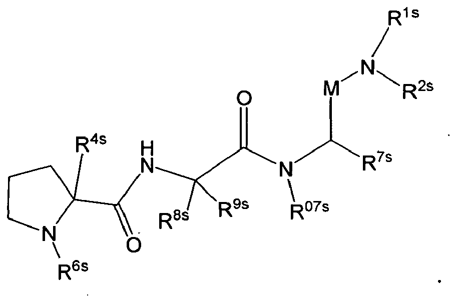
115. The compound of any of items 87-89, wherein the compound has formula: 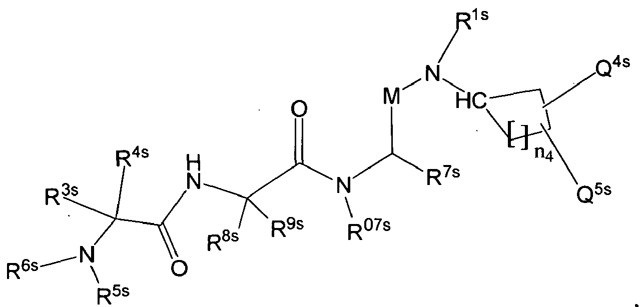 where n₄ is 1-3, Q^{4s} and Q^{5s} are each independently alkyl, cycloalkyl, aryl or Q^{4s} and Q^{5s} together with the carbon atoms on which they are substituted form a cycloalkyl or aryl ring.
116. The compound of any of items 87-89, wherein the compound has formula: 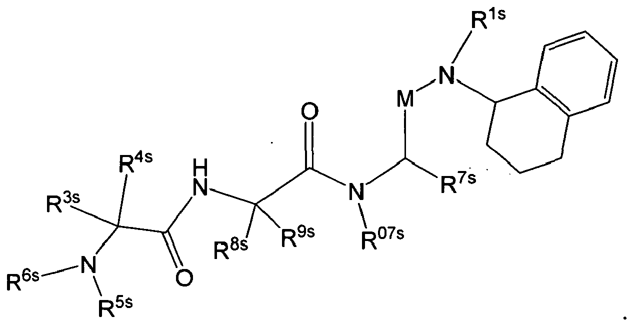
117. The compound of any of items 87-89, wherein the compound has formula: 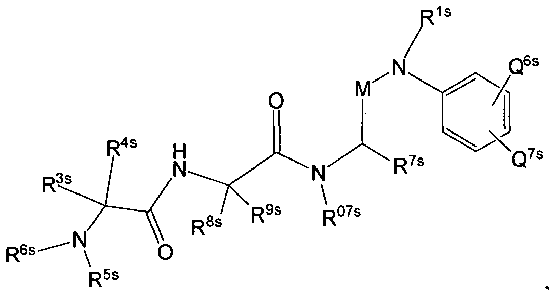 where Q^{6s} and Q^{7s} are each independently alkyl, cycloalkyl, aryl or Q^{6s} and Q^{7s} together with the carbon atoms on which they are substituted form a cycloalkyl or aryl ring.
118. The compound of any of items 87-89 and 117, wherein the compound has formula: 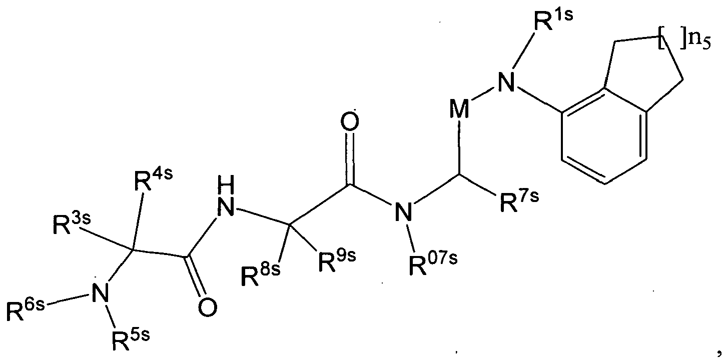 where n₅ is 1-3.
119. The compound of any of claims 87-118, wherein R^{7s} is selected from: 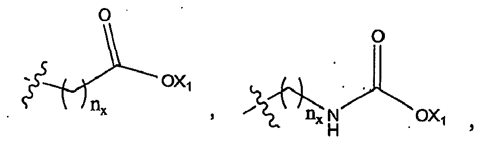 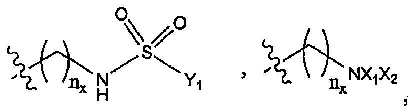 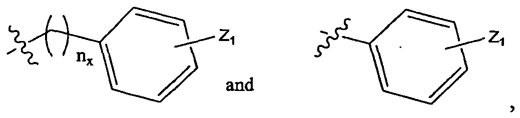 where nₓ is 1-6;
   X₁ and X₂ are selected as follows:
   (i) X₁ is selected from hydrogen, alkyl, aryl, aralkyl and X₂ is selected from hydrogen, alkyl, aryl, aralkyl, NX₄X₅, and CAX₃, or
   (ii) X₁ and X₂ together with the nitrogen atom on which they are substituted form a heteroaryl or heterocyclic ring;
   X₃ is hydrogen, alkyl, aryl or NH₂;
   A is O, S or NX₄;
   X₄ is hydrogen, alkyl, or aryl;
   Y₁ is alkyl, aryl, aralkyl;
   Z₁ hydrogen, halogen, alkyl, alkenyl, alkynyl, haloalkyl, aryl, aralkyl, nitro and COX₃; and
   X₁, X₂ and Y₁ can be substituted with 1, 2 or 3 substituents selected from Z₁.
120. The compound of any of claims 87-118, wherein R^{7s} is hydrogen, benzyloxymethyl, benzyloxyethyl, benzyloxybenzyl, benzyl, 4-benzyloxycarbonylaminobutyl, 5- benzyloxycarbonylaminopentyl or methyl.
121. The compound of any of claims 87-118, wherein R^{7s} is hydrogen, benzyloxymethyl, benzyloxyethyl, benzyloxycarbonylaminobutyl or methyl.
122. The compound of any of claims 87-118, wherein R^{7s} is alkyl or aralkyl.
123. The compound of any of claims 87-118, wherein R^{7s} is methyl or 2-phenylethyl.
124. The compound of any of claims 87-118, wherein R^{7s} is hydrogen, alkyl, aralkoxycarbonylaminoalkyl or aralkoxyalkyl.
125. The compound of any of claims 87-119, wherein R^{7s} is hydrogen, benzyloxymethyl, benzyloxyethyl, benzyloxycarbonylaminobutyl or methyl.
126. The compound of any of claims 87-119, wherein R^{07s} is methyl.
127. The compound of any of claims 87-119, wherein R^{8s} is hydrogen or lower alkyl.
128. The compound of any of claims 87-119, wherein R^{8s} is hydrogen or isopropyl.
129. The compound of any of claims 87-128, wherein R^{9s} is hydrogen or lower alkyl.
130. The compound of any of claims 87-129, wherein R^{9s} is hydrogen.
131. The compound of any of claims 44-86 wherein R⁰⁶ and R⁰⁷ are each independently hydrogen or alkyl.
132. The compound of any of claims 44-86 and 131 wherein R⁰⁶ is hydrogen or alkyl.
133. The compound of any of claims 44-86 and 132, wherein R⁰⁶ is hydrogen, tertiary butyl or isopropyl.
134. The compound of any of claims 44-86 and 1131, wherein R⁰⁷ is hydrogen. 135. A compound of formula: 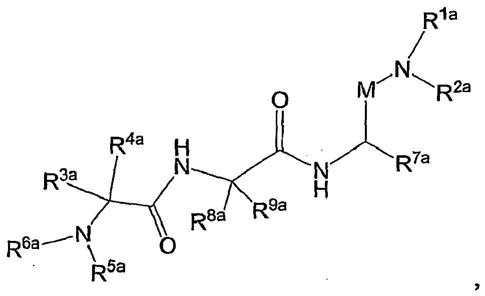 or pharmaceutically acceptable derivative thereof, where:
   M is CO, SO or SO₂;
   R^{1a} is hydrogen, alkyl, alkenyl, alkynyl, aryl, heteroaryl, cycloalkyl or heterocyclyl; and R^{2a} is selected from hydrogen, alkyl, alkenyl, alkynyl, aryl, heteroaryl, cycloalkyl, heterocyclyl, OR¹¹, NR¹⁵R¹⁶ or
   R^{2a} is selected from substituted or unsubstituted aryl, substituted or unsubstituted aralkyl and substituted or unsubstituted cycloalkyl,
   R^{3a}, R^{4a} and R^{5a} are each independently selected from (i) or (ii) as follows:
      (i) R^{3a} and R^{4a} are each independently hydrogen, lower alkyl, lower alkenyl, alkynyl, lower cycloalkyl, lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶;
      (ii) R^{5a} and R^{3a} or R^{5a} and R^{4a} together with the atoms on which they are substituted form heterocyclic or heteroaryl ring and the other of R^{3s} or R^{4s} is selected as (ii);
   R^{6a} is hydrogen, lower alkyl, lower alkenyl or lower alkynyl;
   R^{7a} is hydrogen, alkyl, alkenyl, alkynyl, aryl, C(A)R²⁹, heteroaryl, cycloalkyl or heterocyclyl;
   R^{8a} and R^{9a} are each independently hydrogen, alkyl, alkenyl, alkynyl, aryl, heteroaryl, cycloalkyl, heterocyclyl;
   A is O, S or NR²⁵;
   R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
   R¹⁵ and R¹⁶ are each independently selected hydrogen, alkyl, alkenyl, alkynyl, aryl, heteroaryl, heteroarylium, cycloalkyl, heterocyclyl, C(A)R²⁹, OR²⁵ or NR³²R³³; or
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
      (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
      (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR⁴⁰ or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R^{1a}-R^{9a} and R¹⁰-R³³ are each independently unsubstituted or substituted with one, two or three substituents, each independently selected from Q¹;
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-)or alkylenedithioxy (*i.e.,* -S-(CH₂)_{y}-S-) where y is 1 or 2; or two Q¹ groups, which substitute the same atom, together form alkylene; and
   each Q¹ is independently unsubstituted or substituted with one, two or three substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, 5 diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹.
136. The compound of claim 135, wherein the compound has formula: 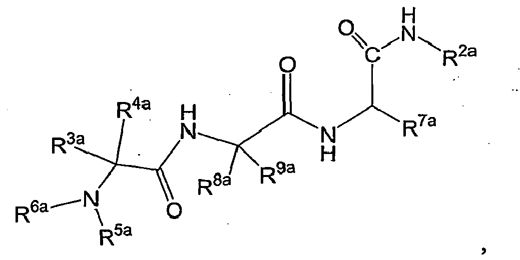 or pharmaceutically acceptable derivative thereof, where:
   R^{2a} is selected from substituted or unsubstituted arylalkyl and substituted or unsubstituted cycloalkyl;
   R^{3a}, R^{4a} and R^{5a} are each independently selected from (i) or (ii) as follows:
      (i) R^{3a} and R^{4a} are each independently hydrogen, lower alkyl, lower alkenyl, alkynyl, lower cycloalkyl, lower heterocyclyl; and R^{5a} is selected from hydrogen, alkyl, alkenyl, alkynyl, aryl, heteroaryl, heteroarylium, cycloalkyl, heterocyclyl and NR¹⁵R¹⁶;
      (ii) R^{5a} and R^{3a} or R^{5a} and R^{4a} together with the atoms on which they are substituted form heterocyclic or heteroaryl ring and the other of R^{3a} or R^{4a} is selected as (ii);
   R^{6a} is hydrogen, lower alkyl, lower alkenyl or lower alkynyl;
   R^{7a} is hydrogen, alkyl, alkenyl, alkynyl, C(A)R29, aryl, heteroaryl, cycloalkyl or heterocyclyl;
   R^{8a} and R^{9a} are each independently hydrogen, alkyl, alkenyl, alkynyl, aryl, heteroaryl, cycloalkyl, heterocyclyl; and
   R²⁹ is hydrogen, alkyl, alkenyl, alkynyl, aryl, heteroaryl, heteroarylium, cycloallcyl or heterocyclyl,
   R^{1a}-R^{9a} are each independently unsubstituted or substituted with one to five substituents, each independently selected from Q¹.
137. The compound of item 135 or 136, wherein R^{1a} is hydrogen; and R^{2s} is selected from aryl, heteroaryl, cycloalkyl, cycloalkylalkyl and heterocyclyl.
138. The compound of any items 135-137, wherein R^{1a} is hydrogen.
139. The compound of any items 135-139, wherein R^{2a} is selected from substituted or unsubstituted aryl, substituted or unsubstituted aralkyl and substituted or unsubstituted cycloallcyl.
140. The compound of any items 135-139, wherein R^{2a} is selected from substituted or unsubstituted aryl and substituted or unsubstituted cycloalkyl.
141. The compound of any items 135-140, wherein R^{2a} is selected from bicyclic aryl and bicyclic cycloalkyl.
142. The compound of any items 135-141, wherein R^{2a} is tetrahydronaphthyl.
143. The compound of any items 135-139, wherein R^{2a} is selected from: 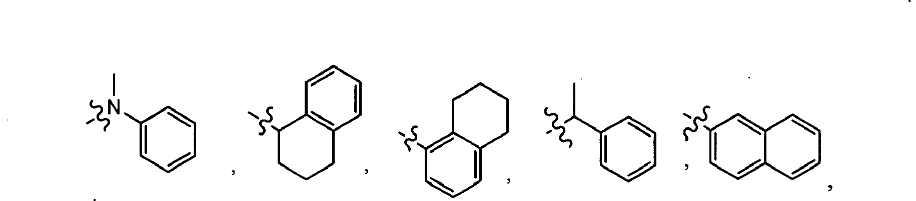 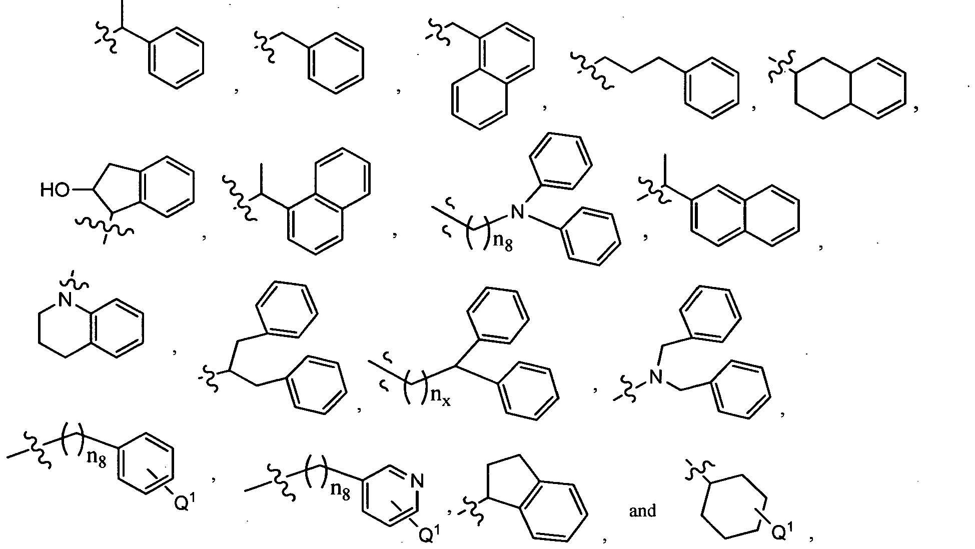 n₈ is 0 to 3.
144. The compound of any items 135-139, wherein R^{2a} is selected from substituted or unsubstituted arylalkyl and substituted or unsubstituted cycloalkyl.
145. The compound of any items 135-139, wherein R^{2a} is selected from: 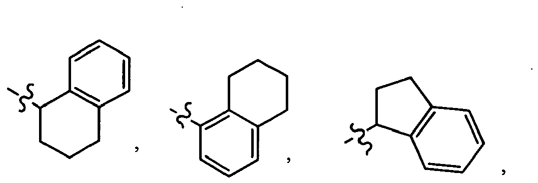 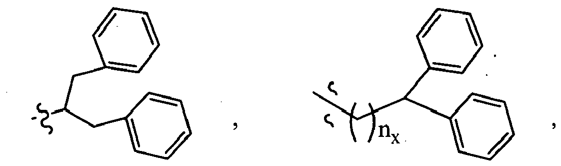 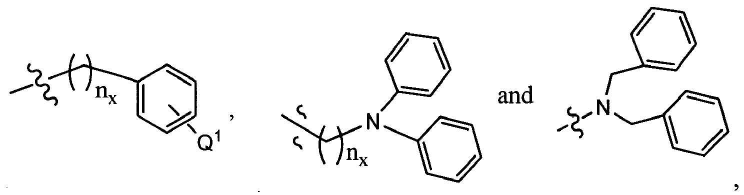 where nx is 1-6.
146. The compound of any items 135-139, wherein R^{3a} is lower alkyl or hydrogen.
147. The compound of any items 135-139, wherein R^{3a} is hydrogen.
148. The compound of any items 135-139, wherein R^{3a} is methyl.
149. The compound of any items 135-139, wherein R^{4a} is lower alkyl.
150. The compound of any items 135-139, wherein R^{4a} is methyl.
151. The compound of any items 135-150, wherein R^{5a} is hydrogen, alkyl, cycloalkylalkyl or aralkyl.
152. The compound of any items 135-151, wherein R^{5a} is methyl.
153. The compound of any items 135-145, wherein R^{5a} and R^{3a} or R^{5a} and R^{4a} together with the atoms on which they are substituted form a heterocyclic or heteroaryl ring and other of R^{3a} or R^{4a} is selected from hydrogen and lower alkyl.
154. The compound of any items 135-145, wherein R^{5a} and R^{3a} form an optinally substituted heterocyclic ring selected from: 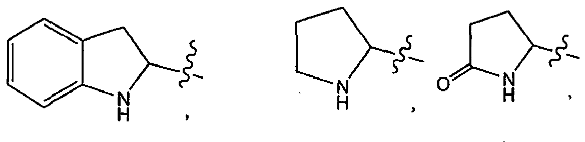 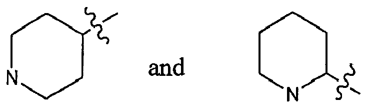
155. The compound of any claims 135-145, wherein R^{5a} and R^{3a} form a heterocyclic ring selected from: 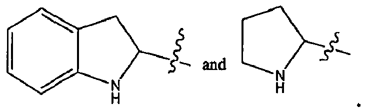
156. The compound of any claims 135-139, wherein R^{6a} is hydrogen or lower alkyl.
157. The compound of any claims 135-139, wherein R^{6a} is hydrogen or methyl. 158. The compound of any claims 135-145, wherein R^{7a} 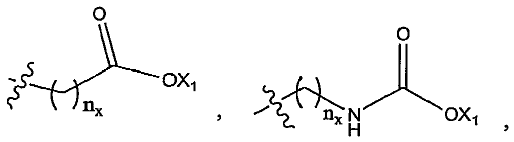 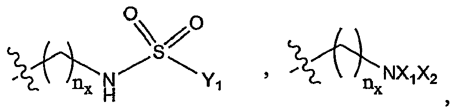 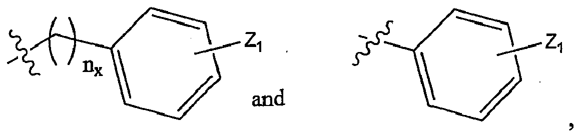 where nx is 1-6, X₁ is selected from hydrogen, alkyl, aryl, aralkyl;
   X₂ is selected from hydrogen, alkyl, aryl, aralkyl, COX₃, where X₃ is alkyl or aryl;
   Y₁ is alkyl, aryl, aralkyl;
   Z₁ hydrogen, halogen, alkyl, alkenyl, alkynyl, haloalkyl, aryl, aralkyl, nitro and COX₃; and
   X₁, X₂ and Y₁ can be substituted with 1, 2 or 3 substituents selected from Z₁,
159. The compound of any of claims 135-145, wherein R^{7a} is hydrogen, benzyloxymethyl, benzylthiomethyl, 2-benzyloxyethyl, benzyloxybenzyl, benzyl, 4-aminobutyryl, 3-aminopropyl, 4- benzyloxycarbonylaminobutyryl, 3-benzyloxycarbonylaminopropyl, 4-(benzyloxycarbonylamino)benzyl, 4-(2-chlorobenzyloxycarbonylamino)butyl, 4-(p-tolylsulfonylamino)butyryl, 3-(p-tolylsulfonylamino)propyl, butyryl, 5- benzyloxycarbonylaminopentyl, cyclohexyl, methyl, 4-methylsulfonylaminobutyryl, 4-(4-butyl)-sulfonylaminobutyryl, 4-benzylsulfonylaminobutyryl, 4-trifluoromethylsulfonylaminobutyryl, 4-(4-methoxyphenylsulfonylamino)butyryl, 4-(4-nitrophenylsulfonylamino)butyryl, benzyloxycarbonylmethyl, 2-(benzyloxycarbonyl)ethyl, 2-aminoethyl, propyl, 4-(N, N-dimethylamino)butyryl, 4-(N, N-dibutyrylamino)butyryl, 4-acetylaminobutyryl, 2-hydroxyethyl and phenyl.
160. The compound of any of items 135-139, wherein R^{7a} is hydrogen, alkyl, aralkylthioalkyl, aralkoxycarbonylaminoalkyl or aralkoxyalkyl.
161. The compound of item 158, wherein Y₁ is methyl, butyl, benzyl, tolyl, trifluoromethyl, methoxyphenyl, and nitrophenyl.
162. The compound of item 158, wherein X₁ is benzyl or chlorobenzyl.
163. The compound of item 158, wherein X₂ is selected from hydrogen, methyl, butyl, and acetyl.
164. The compound of item 158, wherein X₃ is alkyl, aryl or aralkyl.
165. The compound of item 158, wherein X₃ is methyl.
166. The compound of any of items 135-145, wherein R^{8a} is hydrogen or alkyl.
167. The compound of any of items 135-145, wherein R^{8a} is hydrogen, isopropyl or tert-butyl.
168. The compound of any of items 135-145, wherein R^{9a} is hydrogen or alkyl.
169. The compound of any of items 135-145, wherein R^{9a} is hydrogen.
170. The compound of any of items 135-145, wherein the compound has formula: 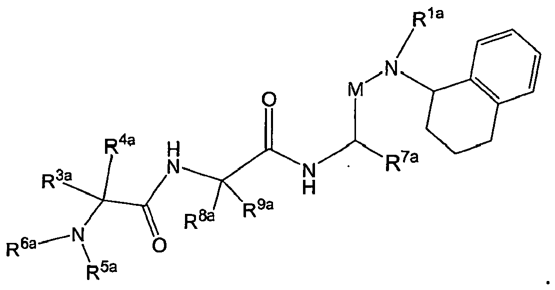
171. The compound of any of claims 135-145, wherein the compound has formula: 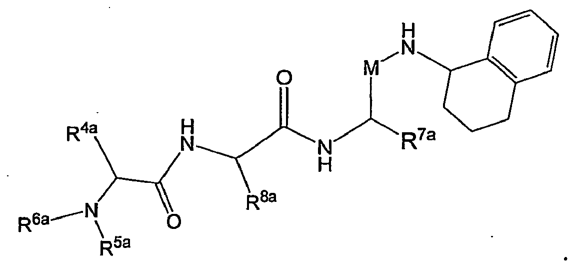
172. The compound of any of claims 135-145, wherein the compound has formula: 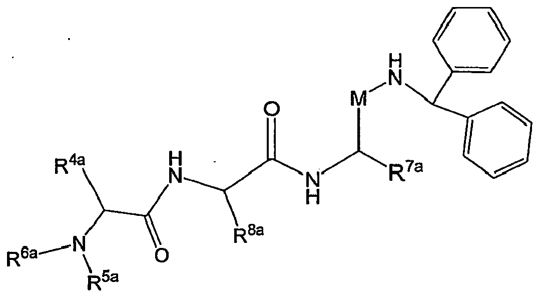
173. The compound of any of claims 135-145, wherein the compound has formula: 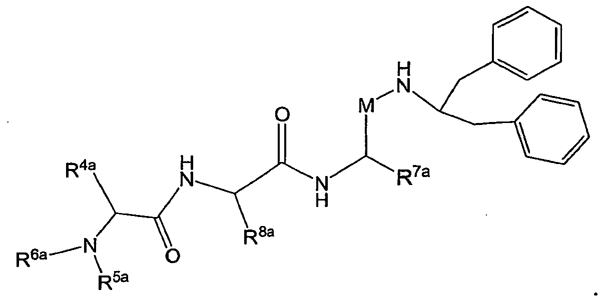
174. A compound of formula: 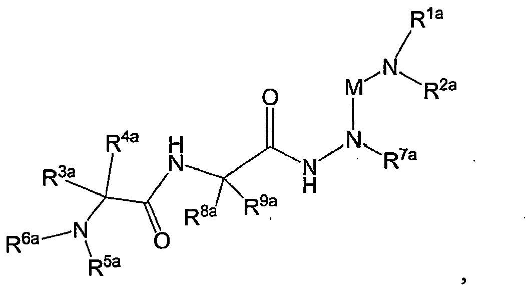 or pharmaceutically acceptable derivative thereof, wherein:
   M is CO, SO or SO₂;
   R^{1a} and R^{2a} are selected as follows:
      (i) R^{1a} is hydrogen, alkyl, alkenyl, alkynyl, aryl, heteroaryl, cycloalkyl or heterocyclyl; and R^{2a} is selected from hydrogen, alkyl, allcenyl, allcynyl, aryl, heteroaryl, cycloalkyl, heterocyclyl, OR¹¹, NR¹⁵R¹⁶ or
      (ii) R^{1a} and R^{2a} together with the nitrogen atom on which they are substituted form a heterocyclic or heteroaryl ring; provided that both R^{1a} and R^{2a} are not hydrogen,
   R^{3a}, R^{4a} and R^{5a} are each independently selected from (i) or (ii) as follows:
      (i) R^{3a} and R^{4a} are each independently hydrogen, lower alkyl, lower alkenyl, alkynyl, lower cycloalkyl, lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶;
      (ii) R^{5a} and R^{3a} or R^{5a} and R^{4a} together with the atoms on which they are substituted form heterocyclic or heteroaryl ring and the other of R^{3s} or R^{4s} is selected as (ii);
   R^{6a} is hydrogen, lower alkyl, lower alkenyl or lower alkynyl;
   R^{7a} is hydrogen, alkyl, alkenyl, alkynyl, aryl, C(A)R²⁹, heteroaryl, cycloalkyl or heterocyclyl;
   R^{8a} and R^{9a} are each independently hydrogen, alkyl, alkenyl, alkynyl, aryl, heteroaryl, cycloalkyl, heterocyclyl;
   A is O, S or NR²⁵;
   R¹⁰ is hydrogen, alkyl, allcenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
   R¹⁵ and R¹⁶ are each independently selected hydrogen, allcyl, alkenyl, alkynyl, aryl, heteroaryl, heteroarylium, cycloalkyl, heterocyclyl, C(A)R²⁹, OR²⁵ or NR³²R³³; or
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
      (i) R²⁷ and R²⁸ are each independently hydrogen, alkyl, allcenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, allcyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
      (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR⁴⁰ or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or R³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R^{1a}-R^{9a} and R¹⁰-R³³ are each independently unsubstituted or substituted with one, two or three substituents, each independently selected from Q¹;
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, arallcynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfmyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
   each Q¹ is independently unsubstituted or substituted with one, two or three substituents, each independently selected from Q²
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(-O)(R⁵⁰)₂, OP(=O)(R⁵⁰)₂, -NR⁶⁰C(-O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfmyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (i.e., -O-(CH₂)_{y}-O-), thioalkylenoxy (i.e., -S-(CH₂)_{y}-O-)or alkylenedithioxy (i.e., -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹.
175. The compound of item 174, wherein the compound has formula: 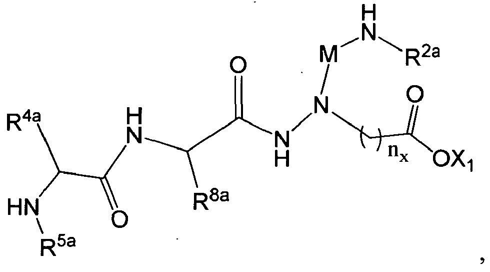 wherein X₁ is selected from hydrogen, alkyl, aryl, and aralky;
   X₁ is optionally substituted with 1-3 substituents selected from Z;
   Z₁ is hydrogen, halogen, alkyl, alkenyl, alkynyl, haloalkyl, aryl, aralkyl, nitro, alkylcarbonyl or arylcarbonyl; and nₓ is 1-5.
176. The compound of item 174, wherein the compound has formula: 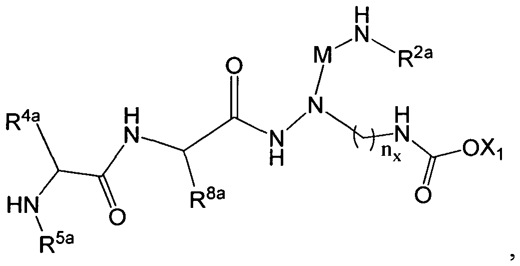 wherein X₁ is selected from hydrogen, alkyl, aryl, and aralky;
   X₁ is optionally substituted with 1-3 substituents selected from Z;
   Z₁ is hydrogen, halogen, alkyl, alkenyl, alkynyl, haloalkyl, aryl, aralkyl, nitro alkylcarbonyl or arylcarbonyl; and nₓ is 1-5.
177. The compound of claim 176, wherein the compound is: 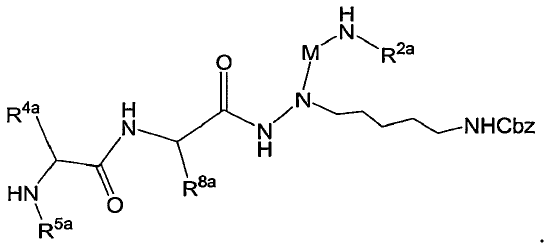
178. A compound of formula: 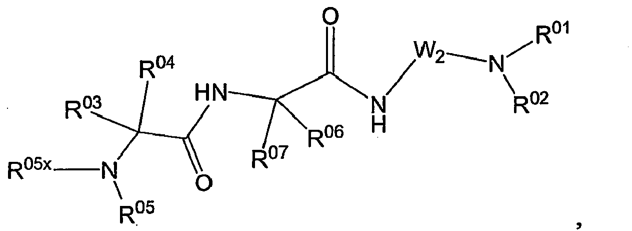 or pharmaceutically acceptable derivatives thereof, wherein W₂ is an optionally substituted 5-7 membered heteroaryl ring containing 1-3 heteroatoms selected from O, S and N, wherein the substituents, when present, are selected from R⁰⁸;
   R⁰¹ and R⁰² are each independently selected from (i) or (ii) as follows:
   (i) ) R⁰¹ is hydrogen, alkyl, alkenyl, alkynyl, aryl, heteroaryl, cycloalkyl or heterocyclyl; and R⁰² is selected from hydrogen, alkyl, alkenyl, alkynyl, aryl, heteroaryl, cyloalkyl, heterocyclyl, OR¹¹, NR¹⁵R¹⁶, or
   (ii) R⁰¹ and R⁰² together with the nitrogen atom on which they are substituted form a heterocyclic or heteroaryl ring;
R⁰³, R⁰⁴ and R⁰⁵ are each independently selected from (i) or (ii) as follows:
   (i) R⁰³ and R⁰⁴ are each independently hydrogen, lower alkyl, lower alkenyl, alkynyl, lower cycloalkyl, lower heterocyclyl; and R⁰⁵ is selected from hydrogen, alkyl, alkenyl, alkynyl, aryl, heteroaryl, cycloalkyl, heterocyclyl and NR¹⁵R¹⁶;
   (ii) R⁰⁵ and R⁰³ or R⁰⁵ and R⁰⁴ together with the atoms on which they are substituted form a heterocyclic or heteroaryl ring and the other of R⁰³ or R⁰⁴ is selected as (i);
      R^{05x} is hydrogen, lower alkyl, lower alkenyl or lower alkynyl;
      R⁰⁶ and R⁰⁷ are each independently hydrogen, alkyl, alkenyl, alkynyl, aryl, heteroaryl, cycloalkyl and heterocyclyl;
      p₁ is 0-4;
   R⁰⁸ is selected from
   i) alkyl, alkenyl, alkynyl, aryl, heteroaryl, heteroarylium, cycloalkyl, heterocyclyl, C(A)R¹⁰, halo, pseudohalo, OR¹¹, oxo, thio, S(D)ₙR¹², NR¹⁵R¹⁶, N+R¹⁵R¹⁶R¹⁷, =NR²⁵ and =CR¹³R¹⁴; or
   ii) two R⁰⁸ substituents together with the atoms on which they are substituted form an aryl, cycloalkyl, heteroaryl or heterocyclyl ring;
   A is O, S or NR²⁵_{;}
   R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴_{;}
   D is O or NR²⁵
   n is 0, 1 or 2;
   when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
   when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
   R¹³ and R¹⁴ are each independently selected from hydrogen, alkyl, alkenyl, alkynyl, aryl, heteroaryl, heteroarylium, cycloallcyl, heterocyclyl, C(A)R²⁹, OR²⁵ or NR²R³³;
   R¹⁵, R¹⁶ and R¹⁷ are each independently selected from (a) and (b) as follows:
      (a) hydrogen, alkyl, alkenyl, alkynyl, aryl, heteroaryl, heteroarylium, cycloalkyl, heterocyclyl, C(A)R²⁹, OR²⁵ or NR³²R³³_{;} or
      (b) any two of R¹⁵, R¹⁶ and R¹⁷ together form alkylene, alkenylene, alkynylene, heteroalkylene, and the other is selected as in (a);
   R¹⁸ and R¹⁹ are each independently selected from alkyl, alkenyl, alkynyl, aryl, heteroaryl, heteroarylium, cycloalkyl, heterocyclyl or OR²⁵, or R¹⁸ and R¹⁹ together form alkylene, alkenylene, alkynylene, heteroalkylene;
   R²⁰ and R²¹ are each independently selected from alkyl, alkenyl, alkynyl, aryl, heteroaryl, heteroarylium, cycloalkyl, heterocyclyl, OR²⁵ and NR³²R³³, or R20 and R²¹ together form alkylene, alkenylene, alkynylene, heteroalkylene;
   R²², R²³ and R²⁴ are each independently selected as in (i) or (ii) as follows:
      (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
      (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
      (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
      (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR^{4o} or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R⁰¹-R⁰⁸, R^{05x} and R¹⁰-R³³ are each independently unsubstituted or substituted with one or more substituents, each independently selected from Q¹
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaₗninoalkyl, alkylarylaminoalkyl, alkylamino, dialkylamino, haloalkylamino, arylamino, diarylamino, alkylarylmuino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroalylthio, azido, -N⁺R⁵¹R⁵²R⁵³, P(R⁵⁰)₂, P(=O)(R⁵⁰)₂, OP(=O)(R⁵⁰)₂, -NR^{6o}C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q¹ groups, which substitute the same atom, together form alkylene; and
   each Q¹ is independently unsubstituted or substituted with one or more substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfmyloxy, alkylsulfonyloxy, arylsulfmyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-0-)or alkylenedithioxy (*i.e*., -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹.
179. The compound of claim 178, wherein W2 is a 5 membered ring.
180. The compound of claim 178 or 179, wherein the compound has formula: 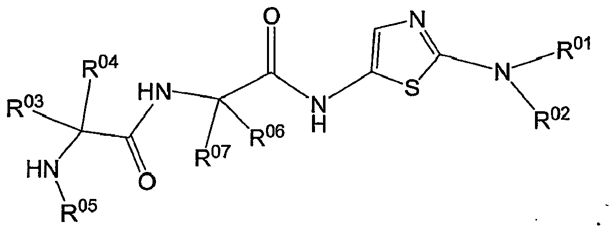
181. The compound of any of claims 178-180, wherein the compound has formula: 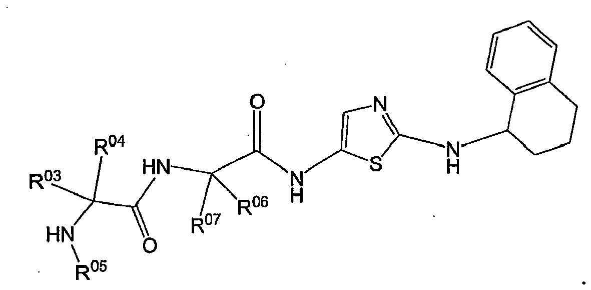
182. The compound of any of claims 178-181, wherein the compound is: 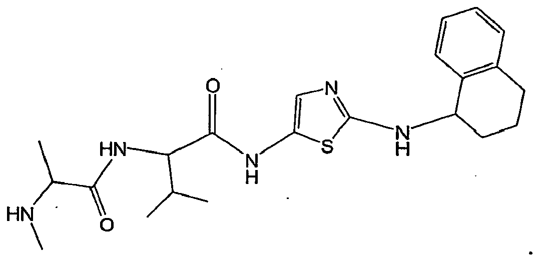
183. A compound of formula: 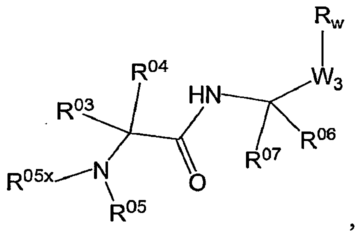 or pharmaceutically acceptable derivatives thereof, where
   W₃ is an optionally substituted 5-7 membered heteroaryl ring containing 1-3 heteroatoms selected from O, S and N, wherein the substituents, when present, are selected from R⁰⁸;
   R_{w} is hydrogen, halo, pseudohalo, alkyl, alkenyl, alkynyl, aryl, heteroaryl, cycloalkyl, heterocyclyl, C(A)R¹⁰, OR¹¹, oxo, thio, S(D)nR¹² or NR¹⁵R¹⁶_{;}
   R⁰³, R⁰⁴ and R⁰⁵ are each independently selected from (i) or (ii) as follows:
   (i) R⁰³ and R⁰⁴ are each independently hydrogen, lower alkyl, lower alkenyl, alkynyl, lower cycloalkyl, lower heterocyclyl; and R⁰⁵ is selected from hydrogen, alkyl, alkenyl, alkynyl, aryl, heteroaryl, cycloalkyl, heterocyclyl and NR¹⁵R¹⁶;
   (ii) R⁰⁵ and R⁰³ or R⁰⁵ and R⁰⁴ together with the atoms on which they are substituted form a heterocyclic or heteroaryl ring and the other of R⁰³ or R⁰⁴ is selected as (i);
   R^{05x} is hydrogen, lower alkyl, lower alkenyl or lower alkynyl;
   R⁰⁶ and R⁰⁷ are each independently hydrogen, alkyl, alkenyl, alkynyl, aryl, heteroaryl, cycloalkyl and heterocyclyl;
   A is O, S or NR²⁵;
   R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ and SiR²²R²³R²⁴;
   R¹¹ is hydrogen, alkyl, alkenyl, alkynyl, aryl, heteroaryl, heteroarylium, cycloalkyl, heterocyclyl, C(A)R²⁹, NR³⁰R³¹ and SiR²²R²³R²⁴;
   D is O or NR²⁵;
   n is 0, 1 or 2;
   when n is 1 or 2, R¹² is selected from hydrogen, alkyl, alkenyl, alkynyl, aryl, heteroaryl, heteroarylium, cycloalkyl, heterocyclyl, halo, pseudohalo, OR²⁵, SR²⁵ and NR³²R³³;
   when n is 0, R¹² is selected from hydrogen, alkyl, alkenyl, alkynyl, aryl, heteroaryl, heteroarylium, cycloalkyl, heterocyclyl, SR²⁵ and C(A)R²⁹;
   R¹⁵ and R¹⁶ are each independently selected from (a) and (b) as follows:
   (a) hydrogen, alkyl, alkenyl, alkynyl, aryl, heteroaryl, heteroarylium, cycloalkyl, heterocyclyl, C(A)R²⁹, OR²⁵ or NR³²R³³; or
   (b) R¹⁵ and R¹⁶ together form alkylene, alkenylene, alkynylene, heteroalkylene, and the other is selected as in (a);
   R²², R²³ and R²⁴ are each independently selected as in (i) or (ii) as follows:
   (i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
   (ii) any two of R²², R²³ and R²⁴ together form alkylene, alkenylene, alkynylene, heteroalkylene; and the other is selected as in (i);
   R²⁵ is hydrogen, alkyl, alkenyl, alkynyl, aryl, heteroaryl, heteroarylium, cycloalkyl or heterocyclyl;
   R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
   R²⁷ and R²⁸ are each independently selected as in (i) or (ii) as follows:
   (i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene, heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl, heterocyclyl; or
   (ii) R²⁷ and R²⁸ together form alkylene, alkenylene, alkynylene, heteroalkylene;
   R²⁹ is hydrogen, alkyl, alkenyl, alkynyl, aryl, heteroaryl, heteroarylium, cycloalkyl, heterocyclyl, OR⁴⁰ or NR³²R³³;
   R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene, heteroalkylene;
   R³² and R³³ are each independently hydrogen, alkyl, alkenyl, alkynyl, aryl, heteroaryl, heteroarylium, cycloalkyl, heterocyclyl, or R³² and R³³ together form alkylene, alkenylene, alkynylene, heteroalkylene;
   R⁰³, R⁰⁴, R⁰⁵, R⁰⁶, R⁰⁷, R^{05x} and R¹⁰-R³³ are each independently unsubstituted or substituted with one or more substituents, each independently selected from Q¹
   Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -NR⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-)or alkylenedithioxy (*i.e.,* -S-(CH₂)_{y}-S-) where y is 1 or 2; or two Q¹ groups, which substitute the same atom, together form alkylene; and
   each Q¹ is independently unsubstituted or substituted with one or more substituents, each independently selected from Q²;
   each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfmyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-)or alkylenedithioxy (*i.e.,* -S-(CH₂)_{y}-S-) where y is 1 or 2; or two Q² groups, which substitute the same atom, together form alkylene;
   R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
   R⁵¹, R⁵² and R⁵³ are each independently hydrogen, alkyl, aryl, aralkyl, heteroaryl, heteroaralkyl, heterocyclyl or heterocyclylalkyl;
   R⁶⁰ is hydrogen, alkyl, aryl, aralkyl, heteroaryl, heteroaralkyl, heterocyclyl or heterocyclylalkyl; and
   R⁶³ is alkoxy, aralkoxy, alkyl, heteroaryl, heterocyclyl, aryl or -NR⁷⁰R⁷¹.
184. The compound of claim 183, wherein R_{w} is hydrogen, C(A)R¹⁰ or NR¹⁵R¹⁶.
185. The compound of claim 183 or 184, wherein W3 is a 5 membered ring.
186. The compound of any of claims 183-185, wherein the compound has formula: 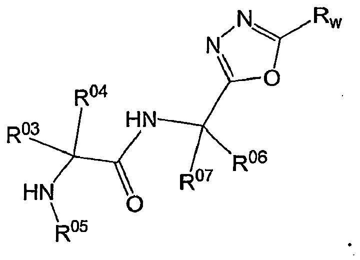
187. The compound of any of claims 183-186, wherein the compound has formula: 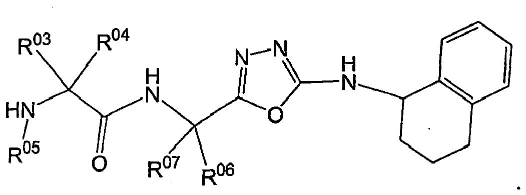 188. The compound of any of claims 183-187, wherein the compound is: 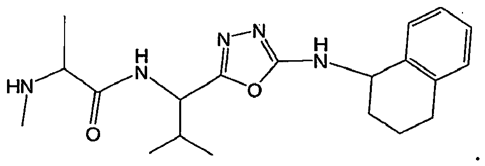
189. A compound selected from:
   2-Methylamino-N-{2-oxo-1-[(1,2,3,4-tetrahydro-naphthalen-1-ylcarbamoyl)-methyl]-azepan-3-yl}-propionamide; compound with trifluoro-acetic acid
   5-(2-Methylamino-propionylamino)-4-oxo-1,2,4,5,6,7-hexahydro-azepino[3,2,1-hi]indole-2-carboxylic 'acid (1,2,3,4-tetrahydro-naphthalen-1-yl)-amide
   1-[2-(2-Isobutylamino-propionylamino)-3,3-dimethyl-butyryl]-pyrrolidine-2-carboxylic acid (1,2,3,4-tetrahydro-naphthalen-1-yl)-amide
   3,3-Dimethyl-2-(2-methylamino-propionylamino)-N-phenethyl-N-[(1,2,3,4-tetrahydro-naphthalen-1-ylcarbamoyl)-methyl]-butyramide TFA salt
   1-{2-[2-(Cyclopropylmethyl-amino)-propionylamino]-3,3-dimethyl-butyryl}-pyrrolidine-2-carboxylic acid (1,2,3,4-tetrahydro-naphthalen-1-yl)-amide
   3-[3-Methyl-2-(2-methylamino-propionylamino)-butyryl]-thiazolidine-4-carboxylic acid (1,2,3,4-tetrahydro-naphthalen-1-yl)-amide
   1-[3,3-Dimethyl-2-(2-propylamino-propionylamino)-butyryl]-pyrrolidine-2-carboxylic acid (1,2,3,4-tetrahydro-naphthalen-1-yl)-amide
   N-{2,2-Dimethyl-1-[2-(N'-methyl-N'-phenyl-hydrazinocarbonyl)-pyrrolidine-1-carbonyl]-propyl}-2-methylamino-propionamide
   6-(2-Methylamino-propionylamino)-5-oxo-octahydro-pyrrolo[1,2-a]azepine-3-carboxylic acid (1,2,3,4-tetrahydro-naphthalen-1-yl)-amide
   3-[3-Methyl-2-(2-methylamino-propionylamino)-butyryl]-oxazolidine-4-carboxylic acid (1,2,3,4-tetrahydro-naphthalen-1-yl)-amide
   1-[3-Methyl-2-(2-methylamino-propionylamino)-butyryl]-octahydro-indole-2-carboxylic acid (1,2,3,4-tetrahydro-naphthalen-1-yl)-amide
   3-[3-Methyl-2-(2-methylamino-propionylamino)-butyryl]-thiazolidine-2-carboxylic acid (1,2,3,4-tetrahydro-naphthalen-1-yl)-amide
   {5-(2-Methylamino-propionylamino)-6-oxo-6-[4-(1,2,3,4-tetrahydro-naphthalen-1-ylcarbamoyl)-thiazolidin-3-yl]-hexyl}-carbmic acid 9H-fluoren-9-ylmethyl ester
   3-[3-Methyl-2-(2-methylamino-propionylamino)-butyryl]-1,1-dioxo-116-thiazolidine-4-carboxylic acid (1,2,3,4-tetrahydro-naphthalen-1-yl)-amide
   2-[3-Methyl-2-(2-methylamino-propionylamino)-butyryl]-2,3-dihydro-1H-isoindole-1-carboxylic acid (1,2,3,4-tetrahydro-naphthalen-1-yl)-amide
   [5-{Methyl-[3-methyl-2-(2-methylamino-propionylamino)-butyryl]-amino}-5-(1,2,3,4-tetrahydro-naphthalen-1-ylcarbamoyl)-pentyl]-carbamic acid benzyl ester
   8-(2-Methylamino-propionylamino)-9-oxo-hexahydro-pyrazolo[1,2-a][1,2]diazepine-1-carboxylic acid (1,2,3,4-tetrahydro-naphthalen-1-yl)-amide
   2-[3-Methyl-2-(2-methylamino-propionylamino)-butyryl]-1,2,3,4-tetrahydroisoquinoline-3-carboxylic acid (1,2,3,4-tetrahydro-naphthalen-1-yl)-amide
   N-[2-Benzyloxy-1-(1,2,3,4-tetrahydro-naphthalen-1-ylcarbamoyl)-ethyl]-3-methyl-2-(2-methylamino-propionylamino)-butyramide
   1-[3,3-Dimethyl-2-(2-methylamino-propionylamino)-butyryl]-pyrrolidine-2-carboxylic acid benzhydryl-amide
   3-[3-Methyl-2-(2-methylamino-propionylamino)-butyryl]-thiazolidine-4-carboxylic acid benzhydryl-amide
   3-[3-Methyl-2-(2-methylamino-propionylamino)-butyryl]-thiazolidine-4-carboxylic acid (1-benzyl-2-phenyl-ethyl)-amide
   N-{1-[4-(N',N'-Diphenyl-hydrazinocarbonyl)-thiazolidine-3-carbonyl]-2-methyl-propyl}-2-methylamino-propionamide
   N-{1-[4-(N',N'-Dibenzyl-hydrazinocarbonyl)-thiazolidine-3-carbonyl]-2-methyl-propyl}-2-methylamino-propionamide
   5,5-Dimethyl-3-[3-methyl-2-(2-methylamino-propionylamino)-butyryl]-thiazolidine-4-carboxylic acid (1,2,3,4-tetrahydro-naphthalen-1-yl)-amide
   2-[3-Methyl-2-(2-methylamino-propionylamino)-butyryl]-1,2,3,4-tetrahydroisoquinoline-3-carboxylic acid benzhydryl-amide
   2-[3-Methyl-2-(2-methylamino-propionylamino)-butyryl]-1,2,3,4-tetrahydroisoquinoline-3-carboxylic acid (1-benzyl-2-phenyl-ethyl)-amide
   2-[3-Methyl-2-(2-methylamino-propionylamino)-butyryl]-1,2,3,4-tetrahydroisoquinoline-3-carboxylic acid (1-benzyl-2-phenyl-ethyl)-amide
   [5-[3-Methyl-2-(2-methylamino-propionylamino)-butyrylamino]-5-(1,2,3,4-tetrahydro-naphthalen-1-ylcarbamoyl)-pentyl]-carbamic acid benzyl ester
   1-[3,3-Dimethyl-2-(2-methylamino-propionylamino)-butyryl]-pyrrolidine-2-carboxylic acid (3,3-diphenyl-propyl)-amide
   N-{1-[2-(N',N'-Dibenzyl-hydrazinocarbonyl)-pyrrolidine-1-carbonyl]-2,2-dimethylpropyl}-2-methylamino-propionamide
   N-{1-[3-(N',N'-Diphenyl-hydrazinocarbonyl)-3,4-dihydro-1H-isoquinoline-2-carbonyl]-2-methyl-propyl}-2-methylamino-propionamide
   7-[3-Methyl-2-(2-methylamino-propionylamino)-butyryl]-1,4-dithia-7-aza-spiro[4.4]nonane-8-carboxylic acid (1,2,3,4-tetrahydro-naphthalen-1-yl)-amide
   [4-[3-Methyl-2-(2-methylamino-propionylamino)-butyrylamino]-4-(1,2,3,4-tetrahydro-naphthalen-1-ylcarbamoyl)-butyl]-carbamic acid benzyl ester
   6-Amino-2-[3-methyl-2-(2-methylamino-propionylamino)-butyrylamino]-hexanoic acid (1,2,3,4-tetrahydro-naphthalen-1-yl)-amide
   [3-[3-Methyl-2-(2-methylamino-propionylamino)-butyrylamino]-3-(1,2,3,4-tetrahydro-naphthalen-1-ylcarbamoyl)-propyl]-carbamic acid benzyl ester
   8-(2-Methylamino-propionylamino)-5,9-dioxo-hexahydro-pyrazolo[1,2-a][1,2] diazepine-1-carboxylic acid (1,2,3,4-tetrahydro-naphthalen-1-yl)-amide
   [5-[3-Methyl-2-(2-methylamino-propionylamino)-butyrylamino]-5-(1,2,3,4-tetrahydro-naphthalen-1-ylcarbamoyl)-pentyl]-carbamic acid 2-chloro-benzyl ester
   2-[3-Methyl-2-(2-methylamino-propionylamino)-butyrylamino]-6-(toluene-4-sulfonylamino)-hexanoic acid (1,2,3,4-tetrahydro-naphthalen-1-yl)-amide
   2-[3-Methyl-2-(2-methylamino-propionylamino)-butyrylamino]-hexanoic acid (1,2,3,4-tetrahydro-naphthalen-1-yl)-amide
   5-Amino-2-[3-methyl-2-(2-methylamino-propionylamino)-butyrylamino]-pentanoic acid (1,2,3,4-tetrahydro-naphthalen-1-yl)-amide
   (5-{[3-Methyl-2-(2-methylamino-propionylamino)-butyryl]-[(1,2,3,4-tetrahydro-naphthalen-1-ylcarbamoyl)-methyl]-amino}-pentyl)-carbamic acid benzyl ester
   N-[3-Benzyloxy-1-(1,2,3,4-tetrahydro-naphthalen-1-ylcarbamoyl)-propyl]-3-methyl-2-(2-methylamino-propionylamino)-butyramide
   3-Methyl-2-(2-methylamino-propionylamino)-N-[1-(1,2,3,4-tetrahydro-naphthalen-1-ylcarbamoyl)-ethyl]-butyramide
   N-[Cyclohexyl-(1,2,3,4-tetrahydro-naphthalen-1-ylcarbamoyl)-methyl]-3-methyl-2-(2-methylamino-propionylamino)-butyramide
   N-{3,7-Dioxo-1-pentyl-2-[(1,2,3,4-tetrahydro-naphthalen-1-ylcarbamoyl)-methyl]-[1,2]diazepan-4-yl} -2-methylamino-propionamide
   6-Methanesulfonylamino-2-[3-methyl-2-(2-methylamino-propionylamino)-butyrylamino]-hexanoic acid (1,2,3,4-tetrahydro-naphthalen-1-yl)-amide
   2-[3-Methyl-2-(2-methylamino-propionylamino)-butyrylamino]-6-phenylmethanesulfonylamino-hexanoic acid (1,2,3,4-tetrahydro-naphthalen-1-yl)-amide
   2-[3-Methyl-2-(2-methylamino-propionylamino)-butyrylamino]-6-trifluoromethanesulfonylamino-hexanoic acid (1,2,3,4-tetrahydro-naphthalen-1-yl)-amide
   6-(4-Methoxy-benzenesulfonylamino)-2-[3-methyl-2-(2-methylamino-propionylamino)-butyrylamino]-hexanoic acid (1,2,3,4-tetrahydro-naphthalen-1-yl)-amide
   2-[3-Methyl-2-(2-methylamino-propionylamino)-butyrylamino]-6-(4-nitro-benzenesulfonylamino)-hexanoic acid (1,2,3,4-tetrahydro-naphthalen-1-yl)-amide
   6-(Butane-1-sulfonylamino)-2-[3-methyl-2-(2-methylamino-propionylamino)-butyrylamino]-hexanoic acid (1,2,3,4-tetrahydro-naphthalen-1-yl)-amide
   4-[3-Methyl-2-(2-methylamino-propionylamino)-butyryl]-thiomorpholine-3-carboxylic acid (1,2,3,4-tetrahydro-naphthalen-1-yl)-amide
   (S)-2-((S)-N,3-dimethyl-2-((S)-2-(methylamino)propanamido)butanamido)-N-((R)-1,2,3,4-tetrahydronaphthalen-1-yl)-5-(3-Htosylguanidino)pentanamide; 2,2,2-trifluoroacetic acid salt
   3-[3-Methyl-2-(2-methylamino-propionylamino)-butyrylamino]-N-(1,2,3,4-tetrahydro-naphthalen-1-yl)-succinamic acid benzyl ester
   4-[3-Methyl-2-(2-methylamino-propionylamino)-butyrylamino]-4-(1,2,3,4-tetrahydro-naphthalen-1-ylcarbamoyl)-butyric acid benzyl ester
   N-[3-Amino-1-(1,2,3,4-tetrahydro-naphthalen-1-ylcarbamoyl)-propyl]-3-methyl-2-(2-methylamino-propionylamino)-butyramide
   N-[3-Hydroxy-1-(1,2,3,4-tetrahydro-naphthalen-1-ylcarbamoyl)-propyl]-3-methyl-2-(2-methylamino-propionylamino)-butyramide
   6-Dimethylamino-2-[3-methyl-2-(2-methylamino-propionylamino)-butyrylamino]-hexanoic acid (1,2,3,4-tetrahydro-naphthalen-1-yl)-amide
   3-[3-Methyl-2-(2-methylamino-propionylamino)-butyryl]-thiazolidine-4-carboxylic acid indan-1-ylamide
   6-Acetylasnino-2-[3-methyl-2-(2-methylamino-propionylamino)-butyrylamino]-hexanoic acid (1,2,3,4-tetrahydro-naphthalen-1-yl)-amide
   3-Methyl-2-(2-methylamino-propionylamino)-N-[phenyl-(1,2,3,4-tetrahydro-naphthalen-1-ylcarbamoyl)-methyl]-butyramide
   3-Methyl-2-(2-methylamino-propionylamino)-N-[(1,2,3,4-tetrahydro-naphthalen-1-ylcarbamoyl)-methyl]-butyramide
   6-Dibutylamino-2-[3-methyl-2-(2-methylamino-propionylamino)-butyrylamino]-hexanoic acid (1,2,3,4-tetrahydro-naphthalen-1-yl)-amide
   3-Methyl-2-(2-methylamino-propionylamino)-N-[2-phenyl-1-(1,2,3,4-tetrahydro-naphthalen-1-ylcarbamoyl)-ethyl]-butyramide
   3-[3-Methyl-2-(2-methylamino-propionylamino)-butyrylamino]-N-(1,2,3,4-tetrahydro-naphthalen-1-yl)-succinamic acid and
   4-[3-Methyl-2-(2-methylamino-propionylamino)-butyrylamino]-4-(1,2,3,4-tetrahydro-naphthalen-1-ylcarbamoyl)-butyric acid
190. A pharmaceutical composition comprising, in a pharmaceutically acceptable carrier, a compound of any of items 1-188.
191. An article of manufacture comprising packaging material and a compound of any of items 1-188.
192. A method for treatment, prevention, or amelioration of one or more symptoms of diseases or disorder that are modulated by caspase-9 activity comprising
   administering to a subject in need thereof an effective amount of a compound of any of items 1-188.
193. The method of item 192, wherein the disease is selected from hyperproliferative diseases, autoimmune diseases, psoriasis, hyperplasia and restenosis.
194. The method of item 193, wherein the hyperproliferative disease is cancer.
195. The method of, item 194, wherein the cancer is selected from neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, toung carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometriuni carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, nonsmall cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.
196. The method of item 195, wherein the autoimmune disease is selected from collagen diseases, Lupus erythematodes disseeminatus, Sharp syndrome, CREST syndrome, dermatomyositis, vasculitis, renal diseases, endocrine diseases, autoimmune polyendocrinopathy-candidiasis-ectodermal dystrophy, autoimmune parathyreoidism, pernicious anemia, gonad insufficiency, idiopathic Morbus Addison, hyperthyreosis, Hashimoto thyreoiditis, primary myxedemia, skin diseases, liver, neuronal diseases, blood and infectious diseases.
197. The method of item 196, wherein the autoimmune disease is selected from rheumatoid arthritis, calcinosis, Raynaud syndrome, esophageal dysmotility, teleangiectasia, Goodpasture syndrome, rapidly-progressing glomerulonephritis and membrane-proliferative glomerulonephritis type II, type-I diabetes, Pemphigus vulgaris, bullous pemphigoid , Herpes gestationis, Epidermolysis bullosa Erythema multiforme major, primary biliary cirrhosis, autoimmune cholangitis, autoimmune hepatitis type-1, autoimmune hepatitis type-2, primary sclerosing cholangitis, multiple sclerosis, myastenia gravis, myasthenic Lambert-Eaton syndrome, acquired neuromyotony, Guillain-Barré syndrome, Stiff-man syndrome, cerebellar degeneration, ataxia, opsoklonus, sensoric neuropathy, achalasia, autoimmune hemolytic anemia idiopathic thrombocytopenic purpura, AIDS , Malaria and Chagas disease.
198. A method of inducing apoptosis comprising administering to a subject in need thereof an effective amount of a compound of any of items 1-188.
199. A method of promoting apoptosis comprising administering to a subject in need thereof an effective amount of a compound of any of items 1-188.
200. A method of modulating BIR domain of an IAP comprising administering to a subject in need thereof an effective amount of a compound of any of items 1-188.
201. A method of modulating BIR domain of XIAP comprising administering to a subject in need thereof an effective amount of a compound of any of items 1-188.
202. A method of modulating BIR3 domain of XIAP comprising administering to a subject in need thereof an effective amount of a compound of any of items 1-188.
203. A method of antagonizing BIR domain of an IAP comprising administering to a subject in need thereof an effective amount of a compound of any of items 1-188.
204. A method of antagonizing BIR domain of XIAP comprising administering to a subject in need thereof an effective amount of a compound of any of items 1-188.
205. A method of antagonizing BIR3 domain of XIAP comprising administering to a subject in need thereof an effective amount of a compound of any of items 1-188.
206. A method of antagonizing XIAP comprising administering to a subject in need thereof an effective amount of a compound of any of items 1-188.
207. A method of reducing an inhibitory effect of XIAP comprising administering to a subject in need thereof an effective amount of a compound of any of items 1-188.
208. A method of reducing an inhibitory effect IAP comprising administering to a subject in need thereof an effective amount of a compound of any of items 1-176.
209. A method of rescuing caspase-9 comprising administering to a subject in need thereof an effective amount of a compound of any of items 1-176.
210. A method of upregulating an activity of caspase-9 comprising administering to a subj ect in need thereof an effective amount of a compound of any of items 1-188.
211. A method of promoting cell death comprising administering to a subject in need thereof an effective amount of a compound of any of items 1-188.
212. The compound of any of items 1-188 coupled to a tag.
213. The compound of item 112, wherein the compound is 6-(6-Hydroxy-3-oxo-3H-xanthen-9-yl)-N-{5-(2-methylamino-propionylamino)-6-oxo-6-[4-(1,2,3,4-tetrahydro-naphthalen-1-ylcarbamoyl)-thiazolidin-3-yl]-hexyl}-isophthalamic acid.
214. The composition of item 213 further comprising one or more active agents selected from the anthracycline family of drugs, the vinca drugs, the mitomycins, the bleomycins, the cytotoxic nucleosides, the pteridine family of drugs, diynenes, the maytansinoids, the epothilones, the taxanes and the podophyllotoxins.

## Claims

1. A compound of formula: 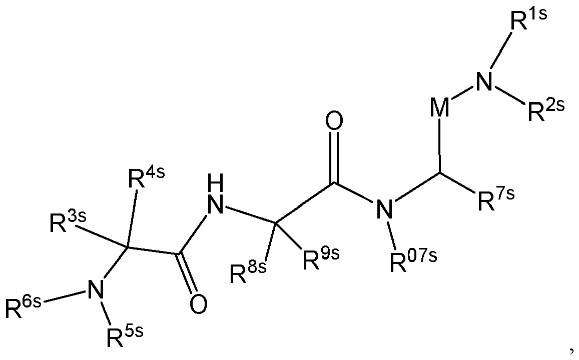 or a pharmaceutically acceptable derivative thereof,
where:
M is CO, SO or SO₂;
R^{1s} and R^{2s} are selected as follows:
(i) R^{1s} is hydrogen, alkyl, alkenyl, alkynyl, aryl, heteroaryl, cycloalkyl or heterocyclyl; and R^{2s} is selected from hydrogen, alkenyl, alkynyl, aryl, heteroaryl, cycloalkyl, heterocyclyl, OR¹¹ and NR¹⁵R¹⁶, or
(ii) R^{1s} and R^{2s} together with the nitrogen atom on which they are substituted form a heterocyclic or heteroaryl ring;
R^{3s}, R^{4s} and R^{5s} are each independently selected from (i) or (ii) as follows:
(i) R^{3s} is hydrogen or alkyl; R^{4s} is alkyl; and R^{5s} is selected from hydrogen, alkyl, alkenyl, alkynyl, aryl, heteroaryl, heteroarylium, cycloalkyl, heterocyclyl and NR¹⁵R¹⁶.
(ii) R^{5s} and R^{3s} or R^{5s} and R^{4s} together with the atoms on which they are substituted form heterocyclic or heteroaryl ring and the other of R^{3s} or R^{4s} is selected as (ii);
R^{6s} is hydrogen, lower alkyl, lower alkenyl or lower alkynyl;
k^{7s} is hydrogen, alkyl, alkenyl, alkynyl, aryl, heteroaryl, cycloalkyl or heterocyclyl;
R^{07s}, R^{8s} and R^{9s} are each selected as follows:
(i) R^{8s} and R^{9s} are each independently hydrogen, alkyl, alkenyl, alkynyl, aryl, heteroaryl, cycloalkyl, or heterocyclyl and R^{07s} is alkyl, aryl or cycloalkyl; or
(ii) R^{8s} and R^{07s} or R^{9s} and R^{07s} together with the atoms on which they are substituted form a 5-7 membered heterocyclic or heteroaryl ring containing 1 or 2 heteroatoms in the ring;
R¹¹ is hydrogen, alkyl, alkenyl, alkynyl, aryl, heteroaryl, heteroarylium, cycloalkyl, heterocyclyl, C(A)R²⁹, NR³⁰R³¹ or SiR²²R²³R²⁴;
A is O, S or NR²⁵;
R¹⁵, R¹⁶ and R¹⁷ are each independently selected from (a) and (b) as follows:
(a) hydrogen, alkyl, alkenyl, alkynyl, aryl, heteroaryl, heteroarylium, cycloalkyl, heterocyclyl, C(A)R²⁹, OR²⁵ or NR³²R³³; or
(b) any two of R¹⁵, R¹⁶ and R¹⁷ together form alkylene, alkenylene, alkynylene or heteroalkylene, and the other is selected as in (a);
R²², R²³ and R²⁴ are each independently selected as in (i) or (ii) as follows:
(i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
(ii) any two of R²², R²³ and R²⁴ together form alkylene, alkenylene, alkynylene or heteroalkylene; and the other is selected as in (i);
R²⁵ is hydrogen, alkyl, alkenyl, alkynyl, aryl, heteroaryl, heteroarylium, cycloalkyl or heterocyclyl;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene or heteroalkylene;
R³² and R³³ are each independently hydrogen, alkyl, alkenyl, alkynyl, aryl, heteroaryl, heteroarylium, cycloalkyl or heterocyclyl, or R³² and R³³ together form alkylene, alkenylene, alkynylene or heteroalkylene;
R^{1s}-R^{9s}, R^{07s} and R¹⁰-R³³ are each independently unsubstituted or substituted with one or more substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-) or alkylenedithioxy (*i.e.,* -S-(CH₂)_{y}-S-) where y is 1 or 2; or two Q¹ groups, which substitute the same atom, together form alkylene; and
each Q¹ is independently unsubstituted or substituted with one or more substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, ⁻N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e.,* -O-(CH₂)_{y}-O-), thioalkylenoxy (*i.e.,* -S-(CH₂)_{y}-O-) or alkylenedithioxy (*i.e.,* -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹.

2. The compound of claim 1,
where:
M is CO, SO or SO₂;
R^{1s} is hydrogen;
R^{2s} is selected from aryl, heteroaryl, cycloalkyl, cycloalkylalkyl and heterocyclyl;
R^{3s}, R^{4s} and R^{5s} are each independently selected from (i) or (ii) as follows:
(i) R^{3s} is hydrogen or alkyl; R^{4s} is alkyl; and R^{5s} is selected from hydrogen, alkyl, alkenyl, alkynyl, aryl, heteroaryl, heteroarylium, cycloalkyl, heterocyclyl and NR¹⁵R¹⁶.
(ii) R^{5s} and R^{3s} or R^{5s} and R^{4s} together with the atoms on which they are substituted form heterocyclic or heteroaryl ring and the other of R^{3s} or R^{4s} is selected as (ii);
R^{6s} is hydrogen, lower alkyl, lower alkenyl or lower alkynyl;
R^{7s} is hydrogen, alkyl, alkenyl, alkynyl, aryl, heteroaryl, cycloalkyl or heterocyclyl;
R^{07s}, R^{8s} and R^{9s} are each selected as follows:
(i) R^{8s} and R^{9s} are each independently hydrogen, alkyl, alkenyl, alkynyl, aryl, heteroaryl, cycloalkyl, heterocyclyl and R^{07s} is alkyl, aryl or cycloalkyl; or
(ii)) R^{8s} and R^{07s} or R^{9s} and R^{07s} together with the atoms on which they are substituted form a 5-7 membered heterocyclic or heteroaryl ring containing 1 or 2 heteroatoms in the ring.

3. The compound of claim 1 or 2, wherein R^{1s} is hydrogen and R^{2s} is selected from substituted or unsubstituted aryl, substituted or unsubstituted heteroaryl, substituted or unsubstituted heterocyclyl, substituted or unsubstituted cycloalkyl and substituted or unsubstituted cycloalkylalkylalkyl.

4. The compound of any of claims 1-3, wherein R^{2s} is selected from substituted or unsubstituted aryl and substituted or unsubstituted cycloalkyl.

5. The compound of any of claims 1-4, wherein R^{2s} is tetrahydronaphthyl.

6. The compound of any of claims 1-5, wherein R^{3s} is hydrogen or lower alkyl.

7. The compound of any of claims 1-6, wherein R^{5s} is hydrogen, alkyl, cycloalkylalkyl or aralkyl.

8. The compound of any of claims 1-7, wherein R^{7s} is selected from: 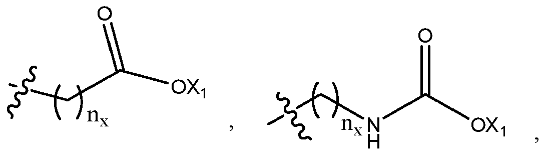 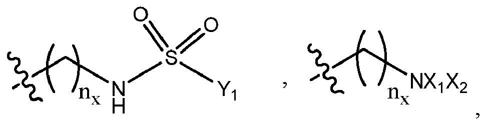 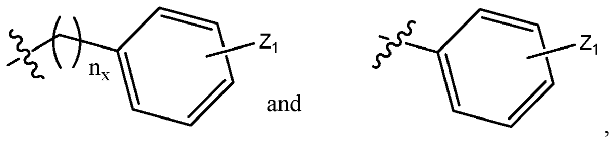 where nₓ is 1-6;
X₁ and X₂ are selected as follows:
(i) X₁ is selected from hydrogen, alkyl, aryl and aralkyl and X₂ is selected from hydrogen, alkyl, aryl, aralkyl, NX₄X₅ and CAX₃, or
(ii) X₁ and X₂ together with the nitrogen atom on which they are substituted form a heteroaryl or heterocyclic ring;
X₃ is hydrogen, alkyl, aryl or NH₂;
A is O, S or NX₄;
X₄ is hydrogen, alkyl or aryl;
Y₁ is alkyl, aryl or aralkyl;
Z₁ is hydrogen, halogen, alkyl, alkenyl, alkynyl, haloalkyl, aryl, aralkyl, nitro or COX₃; and
X₁, X₂ and Y₁ can be substituted with 1, 2 or 3 substituents selected from Z₁.

9. The compound of any of claims 1-8, wherein R^{7s} is hydrogen, benzyloxymethyl, benzyloxyethyl, benzyloxybenzyl, benzyl, 4-benzyloxycarbonylaminobutyl, 5-benzyloxycarbonylaminopentyl or methyl.

10. The compound of any of claims 1-8, wherein R^{7s} is alkyl or aralkyl.

11. The compound of any of claims 1-8, wherein R^{7s} is hydrogen, alkyl, aralkoxycarbonylaminoalkyl or aralkoxyalkyl.

12. A compound of formula: 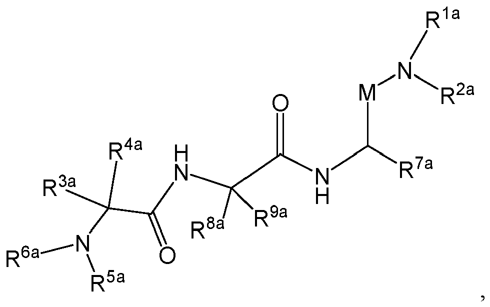 or a pharmaceutically acceptable derivative thereof, where:
M is CO, SO or SO₂;
R^{1a} is hydrogen, alkyl, alkenyl, alkynyl, aryl, heteroaryl, cycloalkyl or heterocyclyl; and R^{2a} is selected from hydrogen, alkyl, alkenyl, alkynyl, aryl, heteroaryl, cycloalkyl, heterocyclyl, OR¹¹ and NR¹⁵R¹⁶;
R^{2a} is selected from substituted or unsubstituted aryl, substituted or unsubstituted aralkyl and substituted or unsubstituted cycloalkyl;
R^{3a}, R^{4a} and R^{5a} are each independently selected from (i) or (ii) as follows:
(i) R^{3a} and R^{4a} are each independently hydrogen, lower alkyl, lower alkenyl, alkynyl, lower cycloalkyl or lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶.
(ii) R^{5a} and R^{3a} or R^{5a} and R^{4a} together with the atoms on which they are substituted form heterocyclic or heteroaryl ring and the other of R^{3s} or R^{4s} is selected as (ii);
R^{6a} is hydrogen, lower alkyl, lower alkenyl or lower alkynyl;
R^{7a} is hydrogen, alkyl, alkenyl, alkynyl, aryl, C(A)R²⁹, heteroaryl, cycloalkyl or heterocyclyl;
R^{8a} and R^{9a} are each independently hydrogen, alkyl, alkenyl, alkynyl, aryl, heteroaryl, cycloalkyl or heterocyclyl;
A is O, S or NR²⁵;
R¹⁰ is hydrogen, alkyl, alkenyl, alkynyl, aryl, heteroaryl, heteroarylium, cycloalkyl, heterocyclyl, C(A)R²⁶, halo, pseudohalo, OR²⁵, SR²⁵, NR²⁷R²⁸ or SiR²²R²¹R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ or SiR²²R²³R²⁴;
R¹⁵ and R¹⁶ are each independently hydrogen, alkyl, alkenyl, alkynyl, aryl, heteroaryl, heteroarylium, cycloalkyl, heterocyclyl, C(A)R²⁹, OR²⁵ or NR³²R³³; or
R²², R²³ and R²⁴ are each independently selected as in (i) or (ii) as follows:
(i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
(ii) any two of R²², R²³ and R²⁴ together form alkylene, alkenylene, alkynylene or heteroalkylene; and the other is selected as in (i);
R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene, heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
(i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene or heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl or heterocyclyl; or
(ii) R²⁷ and R²⁸ together form alkylene, alkenylene, alkynylene or heteroalkylene;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene or heteroalkylene;
R³² and R³³ are each independently hydrogen, alkyl, alkenyl, alkynyl, aryl, heteroaryl, heteroarylium, cycloalkyl or heterocyclyl, or R³² and R³³ together form alkylene, alkenylene, alkynylene or heteroalkylene;
R^{1a}-R^{9a} and R¹⁰-R³³ are each independently unsubstituted or substituted with one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-) or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-)or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹.

13. The compound of claim 12, wherein the compound has formula: 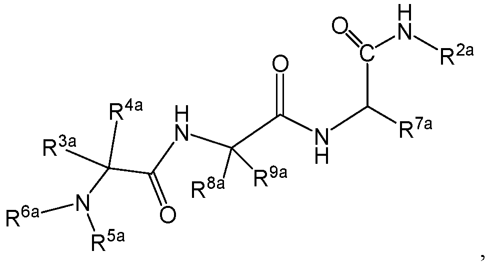 or pharmaceutically acceptable derivative thereof, where:
R^{2a} is selected from substituted or unsubstituted arylalkyl and substituted or unsubstituted cycloalkyl;
R^{3a}, R^{4a} and R^{5a} are each independently selected from (i) or (ii) as follows:
(i) R^{3a} and R^{4a} are each independently hydrogen, lower alkyl, lower alkenyl, alkynyl, lower cycloalkyl or lower heterocyclyl; and R^{5a} is selected from hydrogen, alkyl, alkenyl, alkynyl, aryl, heteroaryl, heteroarylium, cycloalkyl, heterocyclyl and NR¹⁵R¹⁶;
(ii) R^{5a} and R^{3a} or R^{5a} and R^{4a} together with the atoms on which they are substituted form heterocyclic or heteroaryl ring and the other of R^{3a} or R^{4a} is selected as (ii);
R^{6a} is hydrogen, lower alkyl, lower alkenyl or lower alkynyl;
R^{7a} is hydrogen, alkyl, alkenyl, alkynyl, C(A)R29, aryl, heteroaryl, cycloalkyl or heterocyclyl;
R^{8a} and R^{9a} are each independently hydrogen, alkyl, alkenyl, alkynyl, aryl, heteroaryl, cycloalkyl or heterocyclyl; and
R²⁹ is hydrogen, alkyl, alkenyl, alkynyl, aryl, heteroaryl, heteroarylium, cycloalkyl or heterocyclyl,
R^{1a}-R^{9a} are each independently unsubstituted or substituted with one to five substituents, each independently selected from Q¹.

14. The compound claim 12 or 13, wherein R^{7a} is selected from 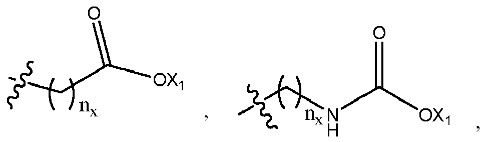 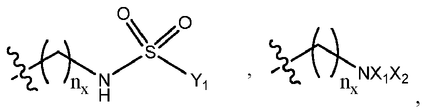 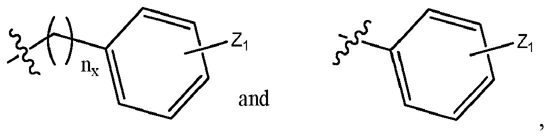 where nₓ is 1-6;
X₁ is selected from hydrogen, alkyl, aryl, aralkyl;
X₂ is selected from hydrogen, alkyl, aryl, aralkyl and COX₃, where X₃ is alkyl or aryl;
Y₁ is alkyl, aryl or aralkyl;
Z₁ is hydrogen, halogen, alkyl, alkenyl, alkynyl, haloalkyl, aryl, aralkyl, nitro or COX₃; and
X₁, X₂ and Y₁ can be substituted with 1, 2 or 3 substituents selected from Z₁

15. A compound of formula: 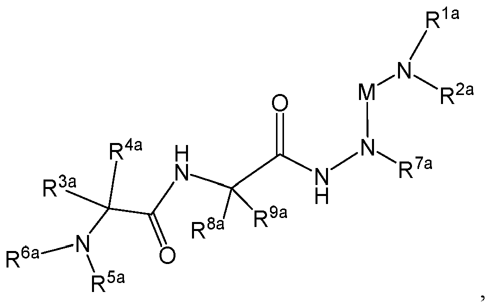 or a pharmaceutically acceptable derivative thereof, wherein: M is CO, SO or SO₂;
R^{1a} and R^{2a} are selected as follows:
(i) R^{1a} is hydrogen, alkyl, alkenyl, alkynyl, aryl, heteroaryl, cycloalkyl or heterocyclyl; and R^{2a} is selected from hydrogen, alkyl, alkenyl, alkynyl, aryl, heteroaryl, cycloalkyl, heterocyclyl, OR¹¹ and NR¹⁵R¹⁶, or
(ii) R^{1a} and R^{2a} together with the nitrogen atom on which they are substituted form a heterocyclic or heteroaryl ring; provided that both R^{1a} and R^{2a} are not hydrogen,
R^{3a}, R^{4a} and R^{5a} are each independently selected from (i) or (ii) as follows:
(i) R^{3a} and R^{4a} are each independently hydrogen, lower alkyl, lower alkenyl, alkynyl, lower cycloalkyl or lower heterocyclyl; and R^{5s} is selected from hydrogen, alkyl, alkenyl, alkynyl, aryl, heteroaryl, heteroarylium, cycloalkyl, heterocyclyl and NR¹⁵R¹⁶;
(ii) R^{5a} and R^{3a} or R^{5a} and R^{4a} together with the atoms on which they are substituted form heterocyclic or heteroaryl ring and the other of R^{3s}, or R^{4s} is selected as (ii);
R^{6a} is hydrogen, lower alkyl, lower alkenyl or lower alkynyl;
R^{7a} is hydrogen, alkyl, alkenyl, alkynyl, aryl, C(A)R²⁹, heteroaryl, cycloalkyl or heterocyclyl;
R^{8a} and R^{9a} are each independently hydrogen, alkyl, alkenyl, alkynyl, aryl, heteroaryl, cycloalkyl or heterocyclyl;
A is O, S or NR²5;
R¹⁰ is hydrogen, alkyl, alkenyl, alkynyl, aryl, heteroaryl, heteroarylium, cycloalkyl, heterocyclyl., C(A)R²⁶ , halo, pseudohalo, OR²⁵, SR²⁵, NR²⁷R²⁸ or SiR²²R²³R²⁴;
R¹¹ is hydrogen, alkyl, alkenyl, alkynyl, aryl, heteroaryl, heteroarylium, cycloalkyl, heterocyclyl, C(A)R²⁹, NR³⁰R³¹ or SiR²²R²³R²⁴;
R¹⁵ and R¹⁶ are each independently hydrogen, alkyl, alkenyl, alkynyl, aryl, heteroaryl, heteroarylium, cycloalkyl, heterocyclyl, C(A)R²⁹, OR²⁵ or NR³²R³³; or
R²², R²³ and R²⁴ are each independently selected as in (i) or (ii) as follows:
(i) R²², R²³ and R²⁴ are each independently hydrogen, alkyl, alkenyl, alkynyl, aryl, heteroaryl, heteroarylium, cycloalkyl, heterocyclyl, OR²⁵ or NR³²R³³; or
(ii) any two of R²², R²³ and R²⁴ together form alkylene, alkenylene, alkynylene or heteroalkylene; and the other is selected as in (i);
R²⁵ is hydrogen, alkyl, alkenyl, alkynyl, aryl, heteroaryl, heteroarylium, cycloalkyl or heterocyclyl;
R²⁶ is hydrogen, alkyl, alkenyl, alkynyl, aryl, heteroaryl, heteroarylium, cycloalkyl, heterocyclyl, OR²⁵ or NR³²R³³, where R³⁴ and R³⁵ are each independently hydrogen, alkyl, alkenyl, alkynyl, aryl, heteroaryl, heteroarylium, cycloalkyl, heterocyclyl, OR³⁶ or NR³²R³³, or R³⁴ and R³⁵ together form alkylene, alkenylene, alkynylene or heteroalkylene, where R³⁶ is hydrogen, alkyl, alkenyl, alkynyl, aryl, heteroaryl, heteroarylium, cycloalkyl or heterocyclyl;
R²⁷ and R²⁸ are each independently selected as in (i) or (ii) as follows:
(i) R²⁷ and R²⁸ are each independently hydrogen, alkyl, alkenyl, alkynyl, aryl, heteroaryl, heteroarylium, cycloalkyl, heterocyclyl, OR²⁵, NR³⁷R³⁸ or C(A)R³⁹, where R³⁷ and R³⁸ are each independently hydrogen, alkyl, alkenyl, alkynyl, aryl, heteroaryl, heteroarylium, cycloalkyl or heterocyclyl, or together form alkylene, alkenylene, alkynylene or heteroalkylene; and R³⁹ is hydrogen, alkyl, alkenyl, alkynyl, aryl, heteroaryl, heteroarylium, cycloalkyl, heterocyclyl, OR⁴⁰ or NR³²R³³, where R⁴⁰ is alkyl, alkenyl, alkynyl, aryl, heteroaryl, heteroarylium, cycloalkyl or heterocyclyl; or
(ii) R²⁷ and R²⁸ together form alkylene, alkenylene, alkynylene or heteroalkylene;
R²⁹ is hydrogen, alkyl, alkenyl, alkynyl, aryl, heteroaryl, heteroarylium, cycloalkyl, heterocyclyl, OR⁴⁰ or NR³²R³³;
R³⁰ and R³¹ are each independently hydrogen, alkyl, alkenyl, alkynyl, aryl, heteroaryl, heteroarylium, cycloalkyl, heterocyclyl or C(A)R⁴¹, where R⁴¹ is alkyl, alkenyl, alkynyl, aryl, heteroaryl, heteroarylium, cycloalkyl, heterocyclyl, OR⁴⁰ or NR³²R³³, or R³⁰ and R³¹ together form alkylene, alkenylene, alkynylene or heteroalkylene;
R³² and R³³ are each independently hydrogen, alkyl, alkenyl, alkynyl, aryl, heteroaryl, heteroarylium, cycloalkyl or heterocyclyl, or R³² and R³³ together form alkylene, alkenylene, alkynylene or heteroalkylene;
R^{1a}-R^{9a} and R¹⁰-R³³ are each independently unsubstituted or substituted with one, two or three substituents, each independently selected from Q¹;
Q¹ is halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺R⁵¹R⁵²R⁵³, P(R⁵⁰)₂, P(=O)(R⁵⁰)₂, OP(=O)(R⁵⁰)₂, -NR⁶⁰C(=O)R⁶³, dialkylphosphonyl, alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q¹ groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-) or alkylenedithioxy (*i.e*., -S-(CH₂)_{y}-S-) where y is 1 or 2; or two Q¹ groups, which substitute the same atom, together form alkylene; and
each Q¹ is independently unsubstituted or substituted with one, two or three substituents, each independently selected from Q²;
each Q² is independently halo, pseudohalo, hydroxy, oxo, thia, nitrile, nitro, formyl, mercapto, hydroxycarbonyl, hydroxycarbonylalkyl, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, heterocyclyl, heterocyclylalkyl, aryl, heteroaryl, aralkyl, aralkenyl, aralkynyl, heteroarylalkyl, trialkylsilyl, dialkylarylsilyl, alkyldiarylsilyl, triarylsilyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aralkoxycarbonyl, aralkoxycarbonylalkyl, arylcarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, heteroaryloxy, heteroaralkoxy, heterocyclyloxy, cycloalkoxy, perfluoroalkoxy, alkenyloxy, alkynyloxy, aralkoxy, alkylcarbonyloxy, arylcarbonyloxy, aralkylcarbonyloxy, alkoxycarbonyloxy, aryloxycarbonyloxy, aralkoxycarbonyloxy, aminocarbonyloxy, alkylaminocarbonyloxy, dialkylaminocarbonyloxy, alkylarylaminocarbonyloxy, diarylaminocarbonyloxy, guanidino, isothioureido, ureido, N-alkylureido, N-arylureido, N'-alkylureido, N',N'-dialkylureido, N'-alkyl-N'-arylureido, N',N'-diarylureido, N'-arylureido, N,N'-dialkylureido, N-alkyl-N'-arylureido, N-aryl-N'-alkylureido, N,N'-diarylureido, N,N',N'-trialkylureido, N,N'-dialkyl-N'-arylureido, N-alkyl-N',N'-diarylureido, N-aryl-N',N'-dialkylureido, N,N'-diaryl-N'-alkylureido, N,N',N'-triarylureido, amidino, alkylamidino, arylamidino, aminothiocarbonyl, alkylaminothiocarbonyl, arylaminothiocarbonyl, amino, aminoalkyl, alkylaminoalkyl, dialkylaminoalkyl, arylaminoalkyl, diarylaminoalkyl, alkylarylaminoalkyl, alkylamino, dialkylamino, haloalkylamino, arylamino, diarylamino, alkylarylamino, alkylcarbonylamino, alkoxycarbonylamino, aralkoxycarbonylamino, arylcarbonylamino, arylcarbonylaminoalkyl, aryloxycarbonylaminoalkyl, aryloxyarylcarbonylamino, aryloxycarbonylamino, alkylsulfonylamino, arylsulfonylamino, heteroarylsulfonylamino, heterocyclylsulfonylamino, heteroarylthio, azido, -N⁺P⁵¹,R⁵²R⁵³, P(R⁵⁰)₂, P(=O)(R⁵⁰)₂, OP(=O)(R⁵⁰)₂, -NR⁶⁰C(=O)R⁶³, dialkylphosphonyl,
alkylarylphosphonyl, diarylphosphonyl, hydroxyphosphonyl, alkylthio, arylthio, perfluoroalkylthio, hydroxycarbonylalkylthio, thiocyano, isothiocyano, alkylsulfinyloxy, alkylsulfonyloxy, arylsulfinyloxy, arylsulfonyloxy, hydroxysulfonyloxy, alkoxysulfonyloxy, aminosulfonyloxy, alkylaminosulfonyloxy, dialkylaminosulfonyloxy, arylaminosulfonyloxy, diarylaminosulfonyloxy, alkylarylaminosulfonyloxy, alkylsulfinyl, alkylsulfonyl, arylsulfinyl, arylsulfonyl, hydroxysulfonyl, alkoxysulfonyl, aminosulfonyl, alkylaminosulfonyl, dialkylaminosulfonyl, arylaminosulfonyl, diarylaminosulfonyl or alkylarylaminosulfonyl; or two Q² groups, which substitute atoms in a 1,2 or 1,3 arrangement, together form alkylenedioxy (*i.e*., -O-(CH₂)_{y}-O-), thioalkylenoxy (*i.e*., -S-(CH₂)_{y}-O-) or alkylenedithioxy (*i.e*., -S-(CH₂)_{y}-S-) where y is 1 or 2; or two Q² groups, which substitute the same atom, together form alkylene;
R⁵⁰ is hydroxy, alkoxy, aralkoxy, alkyl, heteroaryl, heterocyclyl, aryl or -NR⁷⁰R⁷¹, where R⁷⁰ and R⁷¹ are each independently hydrogen, alkyl, aralkyl, aryl, heteroaryl, heteroaralkyl or heterocyclyl, or R⁷⁰ and R⁷¹ together form alkylene, azaalkylene, oxaalkylene or thiaalkylene;
R⁵¹, R⁵² and R⁵³ are each independently hydrogen, alkyl, aryl, aralkyl, heteroaryl, heteroaralkyl, heterocyclyl or heterocyclylalkyl;
R⁶⁰ is hydrogen, alkyl, aryl, aralkyl, heteroaryl, heteroaralkyl, heterocyclyl or heterocyclylalkyl; and
R⁶³ is alkoxy, aralkoxy, alkyl, heteroaryl, heterocyclyl, aryl or -NR⁷⁰R⁷¹.

16. A compound selected from 3,3-Dimethyl-2-(2-methylamino-propionylamino)-N-phenethyl-N-[(1,2,3,4-tetrahydro-naphthalen-1-ylcarbamoyl)-methyl]-butyramide TFA salt, [5-{Methyl-[3-methyl-2-(2-methylamino-propionylamino)-butyryl]-amino}-5-(1,2,3,4-tetrahydro-naphthalen-1-ylcarbamoyl)-pentyl]-carbamic acid benzyl ester, N-[2-Benzyloxy-1-(1,2,3,4-tetrahydro-naphthalen-1-ylcarbamoyl)-ethyl]-3-methyl-2-(2-methylamino-propionylamino)-butyramide, [3-[3-Methyl-2-(2-methylamino-propionylamino)-butyrylamino]-3-(1,2,3,4-tetrahydro-naphthalen-1-ylcarbamoyl)-propyl]-carbamic acid benzyl ester, [5-[3-Methyl-2-(2-methylamino-propionylamino)-butyrylamino]-5-(1,2,3,4-tetrahydro-naphthalen-1-ylcarbamoyl)-pentyl]-carbamic acid 2-chloro-benzyl ester, 2-[3-Methyl-2-(2-methylamino-propionylamino)-butyrylamino]-6-(toluene-4-sulfonylamino)-hexanoic acid (1,2,3,4-tetrahydro-naphthalen-1-yl)-amide, 2-[3-Methyl-2-(2-methylamino-propionylamino)-butyrylamino]-hexanoic acid (1,2,3,4-tetrahydro-naphthalen-1-yl)-amide, 5-Amino-2-[3-methyl-2-(2-methylamino-propionylamino)-butyrylamino]-pentanoic acid (1,2,3,4-tetrahydro-naphthalen-1-yl)-amide, (5-{[3-Methyl-2-(2-methylamino-propionylamino)-butyryl]-[(1,2,3,4-tetrahydro-naphthalen-1-ylcarbamoyl)-methyl]-amino}-pentyl)-carbamic acid benzyl ester, N-[3-Benzyloxy-1-(1,2,3,4-tetrahydro-naphthalen-1-ylcarbamoyl)-propyl]-3-methyl-2-(2-methylamino-propionylamino)-butyramide, 3-Methyl-2-(2-methylamino-propionylamino)-N-[1-(1,2,3,4-tetrahydro-naphthalen-1-ylcarbamoyl)-ethyl]-butyramide, N-[Cyclohexyl-(1,2,3,4-tetrahydro-naphthalen-1-ylcarbamoyl)-methyl]-3-methyl-2-(2-methylamino-propionylamino)-butyramide, 6-Methanesulfonylamino-2-[3-methyl-2-(2-methylamino-propionylamino)-butyrylamino]-hexanoic acid (1,2,3,4-tetrahydro-naphthalen-1-yl)-amide, 2-[3-Methyl-2-(2-methylamino-propionylamino)-butyrylamino]-6-phenylmethanesulfonylamino-hexanoic acid (1,2,3,4-tetrahydro-naphthalen-1-yl)-amide, 2-[3-Methyl-2-(2-methylamino-propionylamino)-butyrylamino]-6-trifluoromethanesulfonylamino-hexanoic acid (1,2,3,4-tetrahydro-naphthalen-1-yl)-amide, 6-(4-Methoxy-benzenesulfonylamino)-2-[3-methyl-2-(2-methylamino-propionylamino)-butyrylamino]-hexanoic acid (1,2,3,4-tetrahydro-naphthalen-1-yl)-amide, 2-[3-Methyl-2-(2-methylamino-propionylamino)-butyrylamino]-6-(4-nitro-benzenesulfonylamino)-hexanoic acid (1,2,3,4-tetrahydro-naphthalen-1-yl)-amide, 6-(Butane-1-sulfonylamino)-2-[3-methyl-2-(2-methylamino-propionylamino)-butyrylamino]-hexanoic acid (1,2,3,4-tetrahydro-naphthalen-1-yl)-amide, 3-[3-Methyl-2-(2-methylamino-propionylamino)-butyrylamino]-N-(1,2,3,4-tetrahydro-naphthalen-1-yl)-succinamic acid benzyl ester, 4-[3-Methyl-2-(2-methylamino-propionylamino)-butyrylamino]-4-(1,2,3,4-tetrahydro-naphthalen-1-ylcarbamoyl)-butyric acid benzyl ester, N-[3-Amino-1-(1,2,3,4-tetrahydro-naphthalen-1-ylcarbamoyl)-propyl]-3-methyl-2-(2-methylamino-propionylamino)-butyramide, N-[3-Hydroxy-1-(1,2,3,4-tetrahydro-naphthalen-1-ylcarbamoyl)-propyl]-3-methyl-2-(2-methylamino-propionylamino)-butyramide, 6-Dimethylamino-2-[3-methyl-2-(2-methylamino-propionylamino)-butyrylamino]-hexanoic acid (1,2,3,4-tetrahydro-naphthalen-1-yl)-amide, 6-Acetylamino-2-[3-methyl-2-(2-methylamino-propionylamino)-butyrylamino]-hexanoic acid (1,2,3,4-tetrahydro-naphthalen-1-yl)-amide, 3-Methyl-2-(2-methylamino-propionylamino)-N-[phenyl-(1,2,3,4-tetrahydro-naphthalen-1-ylcarbamoyl)-methyl]-butyramide, 3-Methyl-2-(2-methylamino-propionylamino)-N-[(1,2,3,4-tetrahydro-naphthalen-1-ylcarbamoyl)-methyl]-butyramide, 6-Dibutylamino-2-[3-methyl-2-(2-methylamino-propionylamino)-butyrylamino]-hexanoic acid (1,2,3,4-tetrahydro-naphthalen-1-yl)-amide, 3-Methyl-2-(2-methylamino-propionylamino)-N-[2-phenyl-1-(1,2,3,4-tetrahydro-naphthalen-1-ylcarbamoyl)-ethyl]-butyramide, 3-[3-Methyl-2-(2-methylamino-propionylamino)-butyrylamino]-N-(1,2,3,4-tetrahydro-naphthalen-1-yl)-succinamic acid and 4-[3-Methyl-2-(2-methylamino-propionylamino)-butyrylamino]-4-(1,2,3,4-tetrahydro-naphthalen-1-ylcarbamoyl)-butyric acid.

17. A pharmaceutical composition comprising, in a pharmaceutically acceptable carrier, the compound of any of claims 1-16.

18. The compound of any of claims 1-16 for use in a method for treatment, prevention, or amelioration of one or more symptoms of diseases or disorder that are modulated by caspase-9 activity.
